# EUROPEAN PATENT APPLICATION

(11) **EP 4 480 965 A1**
(43) Date of publication of application: **25.12.2024**
(21) Application number: 23306002.9
(22) Date of filing: 22.06.2023
(51) Int. Cl.: C07K 16/18, A61P 25/28, C12N 15/11, A61K 39/395

(54) **TRANSFERRIN RECEPTOR-BINDING MOLECULES, CONJUGATES THEREOF AND THEIR USES TO PREVENT OR TREAT CNS DISEASES**

(71) Applicant: Vect-Horus, 13005 Marseille (FR); Centre National de la Recherche Scientifique, 75016 Paris (FR); Université d'Aix Marseille, 13007 Marseille (FR)
(72) Inventor: DAVID, Marion, 13010 MARSEILLE (FR); COHEN, Romy, 13010 MARSEILLE (FR); JACQUOT, Guillaume, 13100 AIX EN PROVENCE (FR); TEMSAMANI, Jamal, 30900 NIMES (FR); LECORCHE, Pascaline, 13004 MARSEILLE (FR); KHRESTCHATISKY, Michel, 13007 MARSEILLE (FR)
(74) Representative: Cabinet Becker et Associés

(57) **Abstract**

The invention relates to Variable Domain of Camelid Heavy Chain-only (VHH) molecules which bind TfR, the conjugate compounds comprising such VHH molecules, and the uses thereof e.g., to transport molecules of pharmaceutical interest into cells and in organs, in pathological conditions of the CNS, including cancer.

## Description

The invention relates to Transferrin receptor (TfR)-binding molecules and uses thereof. The invention particularly relates to Variable Domain of Camelid Heavy Chain-only (VHH) molecules, which bind TfR at the surface of cell membranes such as the blood-brain barrier (BBB). More particularly, the invention relates to conjugate compounds comprising such VHH molecules, and to uses thereof e.g., to transport molecules of pharmaceutical interest into cells of the central nervous system or TfR-expressing tissues or organs, and/or to treat various CNS diseases, including cancer.

### Background

According to *Global Industry Analysts,* the global market for drugs treating central nervous system (CNS, brain and spinal cord) pathologies was approximately 100 billion dollars in 2015, with nearly 9 billion dollars of this amount representing products arising from drug delivery technologies (Jain, 2008, Jain PharmaBiotech Report, Drug Delivery in CNS disorders)*.* Thus, neurology is today one of the three largest therapeutic areas, along with cardiovascular medicine and oncology. Although the number of people suffering from CNS disorders and pathologies throughout the world is larger than that of people with cardiovascular diseases or cancers, neurology remains an under-developed market. This is explained by the fact that 98% of potential drugs for treating CNS pathologies do not cross the BBB (Pardridge, 2003, Mol. Interv., 3, 90-105)*.*

Indeed, the brain is protected from potentially toxic substances by the presence of two principal physiological barrier systems: the BBB, and the blood-cerebrospinal fluid barrier (BCSFB). The BBB is regarded as the principal route for the uptake of plasma ligands. Its surface area is approximately 5000 times larger than that of the BCSFB. The overall length of the constitutive blood vessels of the BBB is approximately 600 km. Each cm³ of cerebral cortex contains the equivalent of 1 km of blood vessels. The total surface area of the BBB is estimated at 20 m*² (*De Boer et al., 2007, Clin. Pharmacokinet., 46(7), 553-576). Thus, the cerebral endothelium, which constitutes the BBB, represents a large surface of potential exchange between the blood and nervous tissue. However, this cerebral endothelium, because of its specific properties, is also a major obstacle to the use of drugs to treat CNS disorders.

Indeed, the BBB is composed of brain capillary endothelial cells (BCECs) that present unique properties, not found in the fenestrated endothelial cells that compose the vascular system of other organs. BCECs form tight junctions, they are surrounded by a basal lamina, astrocyte end-feet, pericytes and microglial and neuronal cells that all together compose a very selective barrier, that controls molecular exchanges between the blood and the brain, that maintains brain homeostasis and that very efficiently protects the brain from toxins and pathogens. The drawback is that the BBB is also impermeable to most molecules, including drugs and imaging agents. As a general rule, only a few small lipophilic molecules of approximately 450 to 600 Daltons can pass through the BBB (only 2% of all drug candidates), and most if not all higher molecular weight molecules, such as therapeutic peptides, proteins, antibodies, which show promising results in *in vitro* studies and in animal studies for treating CNS disorders, do not pass the BBB.

The BBB is thus regarded as a major obstacle to overcome in the development of novel therapies for treating CNS disorders (Neuwelt et al., 2008, Lancet Neurol., 7, 84-96). One of the research priorities to be associated with the discovery of molecules for treating, diagnosing or imaging CNS pathologies is the development of strategies that will allow/increase the passage of active substances across the BBB.

One approach to avoid the BBB is to administer drugs by direct injection into the CNS (e.g., intraventricular, intracerebral or intrathecal), or to disrupt the BBB. Such highly invasive approaches, however, have drawbacks (such as costs, short effect) and potential risks.

Pharmacological strategies have been contemplated, based on the addition of lipid or lipophilic groups to active substances (transcellular lipophilic diffusion, TLD) or on the use of liposomes (Zhou et al., 1992, J. Control. Release, 19, 459-486)*.* However, the addition of lipid or lipophilic groups or the use of liposomes often leads to large and non-specific complexes above the optimal limit of 450 Daltons, which are relatively non effective for crossing the BBB (Levin, 1980, J. Med. Chem., 23, 682-684*;* Schackert et al., 1989, Selective Cancer Ther., 5, 73-79)*.*

Among the strategies evaluated to deliver protein therapeutics into the brain, hijacking the cellular machinery involved in the transport of natural nutrients and endogenous ligands across the BBB appears as the safest and most effective (Fang et al., 2017; Jones and Shusta, 2007; Pardridge et al., 1992). The transport of macromolecules across the BBB can be facilitated by receptor-mediated transcytosis (RMT), a physiological process involving binding of a ligand to its receptor expressed by BCECs, internalization by endocytosis, intracellular trafficking and dissociation from the receptor in sorting endosomes, followed by its release at the abluminal side of the BBB (Tuma and Hubbard, 2003; Xiao and Gan, 2013). In this regard, WO2010/046588 and WO2011/131896 disclose various peptides with high affinity for LDL receptor, which are capable of transporting drugs or other molecules through the BBB.

Another receptor studied to transport drugs across the BBB is the transferrin receptor (TfR), which is involved in iron transport into the brain by its ligand transferrin (Tf) (Fishman et al., 1987). This receptor was shown to be highly expressed in brain endothelium (Jefferies et al., 1984; Pardridge et al., 1987), albeit it is also abundant in blood cells and lung (Chan and Gerhardt, 1992). Although the use of Tf as a transporter has been studied (Chang et al., 2009; Jain et al., 2011; Yan et al., 2013), the transport mechanism of this molecule is saturable and competes with endogenous Tf. Anti-TfR monoclonal antibodies have been studied as vectors for brain delivery, including the OX26 antibody that targets the rat TfR (Moos and Morgan, 2001; Pardridge et al., 1991; Ulbrich et al., 2009), or the 8D3 (Pardridge, 2015; Zhang and Pardridge, 2005; Zhou et al., 2010) and R17-217 antibodies (Lee et al., 2000; Pardridge, 2015; Ulbrich et al., 2009) that target the mouse TfR (see also WO2012075037, WO2013177062, WO201275037, WO2016077840, WO2016208695). However, drawbacks of these antibodies include their absence of cross-species reactivity, and especially their absence of binding to the human TfR, which precludes preclinical or clinical studies. Also, the ability of such antibodies to effectively transport drugs across BBB still remains of debate.

Accordingly, despite progress in the field, there is a need in the art for further effective methods and agents capable of improving drug access to the CNS.

### Summary of the invention

The present invention provides particular VHH molecules that advantageously target transferrin receptor (TfR) in CNS tissues, and uses thereof to effectively transport various therapeutic agents to the CNS. The invention also discloses VHH molecules that bind human, non-human primate and/or rodent TfR and which can deliver drugs to the CNS across BBB through transcytosis. The invention demonstrates that VHH molecules of the invention can effectively transmigrate through the CNS and deliver conjugated drugs or imaging agents *in vivo.* Such VHH thus represent valuable molecules for use in therapeutic or diagnostic approaches.

More particularly, the invention provides conjugate compounds comprising such VHH molecules, which are optimized for addressing CNS and mediating effective functional delivery of drugs to the CNS cells. The invention demonstrates that the conjugate compounds of the invention can effectively accumulate in the CNS and deliver conjugated therapeutics in the CNS tissue, and thus are suitable for efficient prevention and treatment of various CNS diseases.

An object of the invention relates to a conjugate compound comprising:
(i) one or more VHH molecule(s) of formula FR1-CDR1-FR2-CDR2-FR3-CDR3-FR4, and
(ii) at least one therapeutic compound or a vehicle comprising such a therapeutic compound,
   wherein said VHH molecule binds TfR at the surface of a cell of the central nervous system (CNS), and
   wherein said VHH molecule comprises:
      - a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710 and 711, and
      - a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611 and 712, and
      - a CDR3 comprising an amino acid sequence selected from SEQ ID NOs: 3, 7, 11, 117, 119, 121, 123, 177, 180, 188, 192, 196, 200, 203, 207, 452, 455, 609, 612 and 713-715,
   with the proviso that:
      . when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
      . when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
      . when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.

Preferably, the therapeutic compound is selected from a peptide, a polypeptide, a protein, an antibody and a nucleic acid, the nucleic acid being preferably selected from a mRNA, a ribozyme or an oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA and bridged nucleic acid (BNA). Preferably, the CNS cell is a brain or spinal cord cell.

A further object of the invention relates to a conjugate compound, wherein the VHH molecule comprises: SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7.

Another object of the invention relates to a conjugate compound, wherein the VHH molecule comprises an amino acid sequence selected from any one of SEQ ID NOs: 273, 276-284, 412, 415, 418, 421, 423, 425, 428, 430, 433, 436, 439, 441, 443, 445, 447, 449, 451, 454, 457, 677, 678 and 702-709.

Another object of the invention relates to a conjugate compound, wherein the VHH molecule comprises an amino acid sequence selected from any one of SEQ ID NOs: 242-271, 274, 275, 675, 676 and 701.

Another object of the invention relates to a conjugate compound, wherein the VHH molecule comprises an amino acid sequence selected from any one of SEQ ID NOs: 285-299.

Another object of the invention relates to a conjugate compound, wherein the VHH molecule comprises an amino acid sequence selected from any one of SEQ ID NOs: 613-615.

Another object of the invention relates to a conjugate compound, wherein the VHH molecule further comprises a tag and/or a linker.

Another object of the invention relates to a conjugate compound, wherein the VHH molecule is humanized, and preferably selected from SEQ ID NO: 130-149, 152-154, 252-271 and 273-275.

Another object of the invention relates to a conjugate compound, wherein said VHH molecule binds the human, non-human primate and/or rodent TfR, preferably TfR1.

Another object of the invention relates to a conjugate compound, wherein said VHH molecule can cross the human blood-brain barrier ("BBB").

Another object of the invention relates to the conjugate compounds, wherein said VHH molecule binds to TfR with an affinity (Kd) of 0.1 nM to 2500 nM.

The conjugate compound of the invention may also contain at least one additional compound, for example, a half-life extending moiety or stabilizing group or scaffold such as an antibody or a fragment thereof (such as a Fc fragment), a VHH molecule, PEG, a serum albumin protein, or a serum albumin moiety. Preferably, the additional compound is a Fc fragment.

A further object of the invention relates to conjugate compounds, wherein the Fc fragment is a Fc heterodimer comprising a modified Fc having a sequence of SEQ ID NO: 664 on the knob arm, and a modified Fc having a sequence of SEQ ID NO: 665 on the hole arm.

The invention further provides a pharmaceutical composition comprising a conjugate compound as defined herein and, a pharmaceutically acceptable excipient, support or carrier.

The invention further provides nucleic acids, vectors, and host cells encoding a VHH or chimeric agent as defined above.

The invention also provides methods for making a VHH or chimeric agent, comprising culturing a host cell as defined above under conditions allowing expression of the nucleic acid.

The invention further provides methods for making a conjugate compound, comprising conjugating one or more VHH as defined above to a molecule or agent or scaffold, covalently or non-covalently.

Another object of the invention relates to a conjugate compound as defined herein for use as a medicament.

Another object of the invention relates to the use of a conjugate compound of the invention, as defined herein, for increasing the biological activity and/or CNS delivery of a substance of interest.

Another object of the invention relates to a method for improving or enabling passage of a molecule across the BBB, using a conjugate compound as defined herein.

The conjugate of the invention, or a composition comprising such a conjugate may be administered systemically, intravenously, intramuscularly, subcutaneously, intracerebrally, intracerebroventricularly or intrathecally.

The invention can be used in any mammal, in particular in human subjects. It is suitable to prevent or treat any CNS disease, for example Alzheimer's disease, Parkinson's disease, stroke, Creutzfeldt-Jakob disease, bovine spongiform encephalopathy, multiple sclerosis, amyotrophic lateral sclerosis, epilepsy, migraine, encephalitis, CNS pain or glioblastoma.

### Legend to the Figures

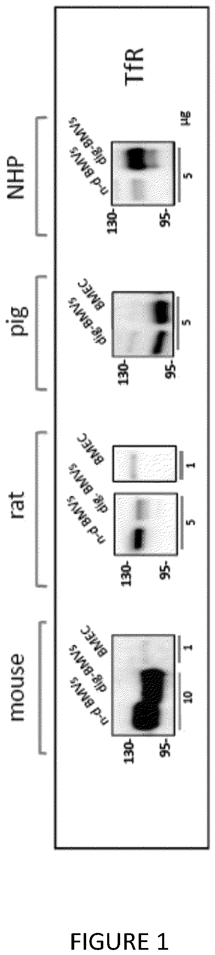

**Figure 1****: TfR expression at the BBB.** Western blots were performed on the membrane fraction of brain microvessels (BMVs) and brain microvessel endothelial cells (BMEC) from mouse, rat, pig and non-human primate (NHP; rhesus macaque). The amount of protein loaded is indicated under the picture. n-d: non-digested; dig-: digested.

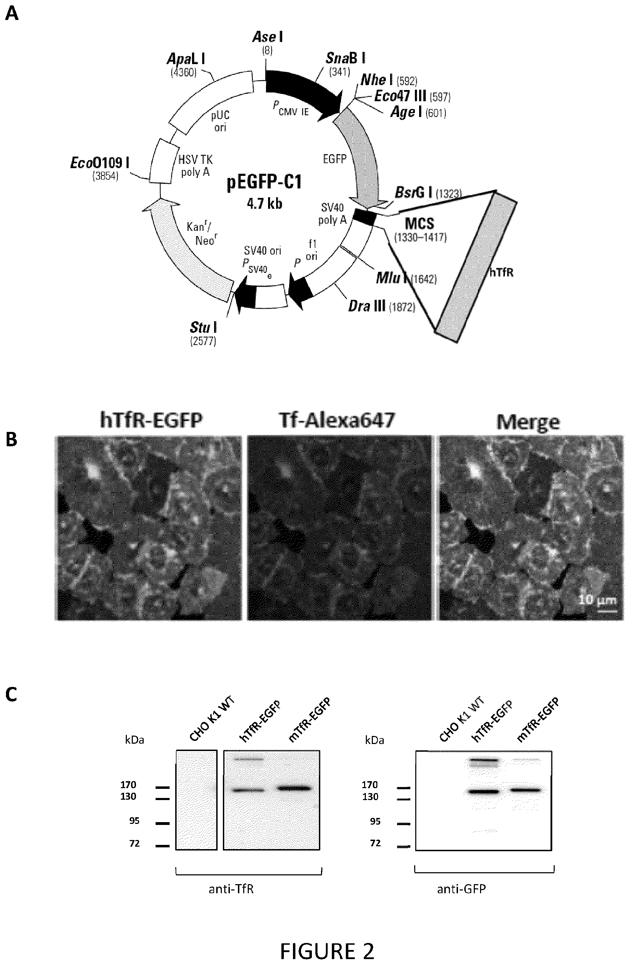

**Figure 2****: Validation of CHO cell lines expressing the human or mouse TfR.** (A) Map of the plasmid construct used to generate the CHO-hTfR-EGFP cell line. (B) Representative confocal photomicrographs of CHO-hTfR-EGFP cells (green) incubated 1 hr at 37 °C with Tf-Alexa647 (250 µg/ml, red). Cell nuclei were labeled with Hoechst#33342 at 0.5 µg/mL (blue). Co-labeling appears in yellow in the merged picture. (C) Western blots performed on cell membrane preparations of CHO cells expressing hTfR-EGFP and mTfR-EGFP compared to CHO WT, using a rabbit anti-TfR antibody (1/1000) or a mouse anti-GFP antibody (1/1000), followed by HRP-conjugated anti-rabbit or anti-mouse secondary antibodies (1/10000).

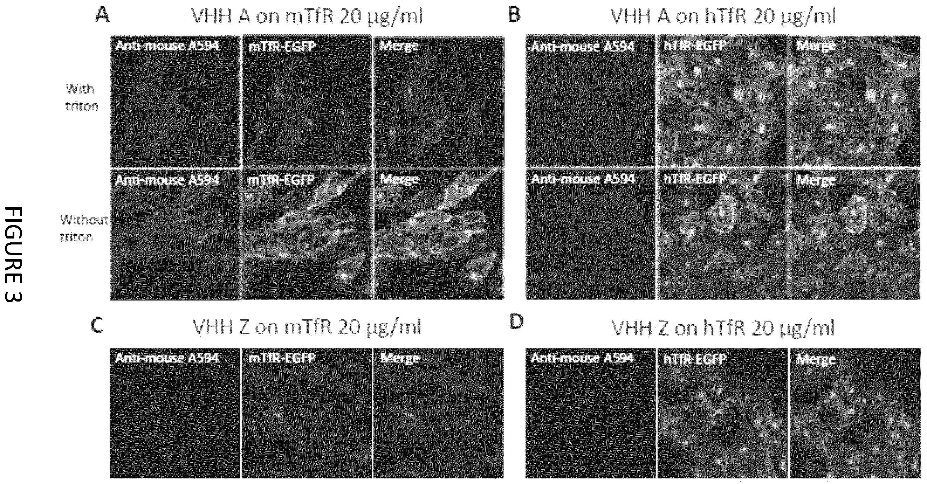

**Figure 3****: Cell surface binding and endocytosis of VHH A and VHH Z on CHO cells expressing hTfR and mTfR.** Representative confocal photomicrographs of CHO-hTfR-EGFP and CHO-mTfR-EGFP cells (green) incubated 1 hr at 37 °C with VHH A (A, B) and with the control VHH Z (C, D) at 20 µg/ml, detected post-PFA fixation and following or not triton X-100 permeabilization of cell membranes, with a mouse anti-cMyc (1/1000) and an Alexa594-conjugated anti-mouse secondary antibody (1/800, red). Cell nuclei were labeled with Hoechst#33342 at 0.5 µg/ml (blue). Co-labeling appears in yellow/orange in the merged pictures.

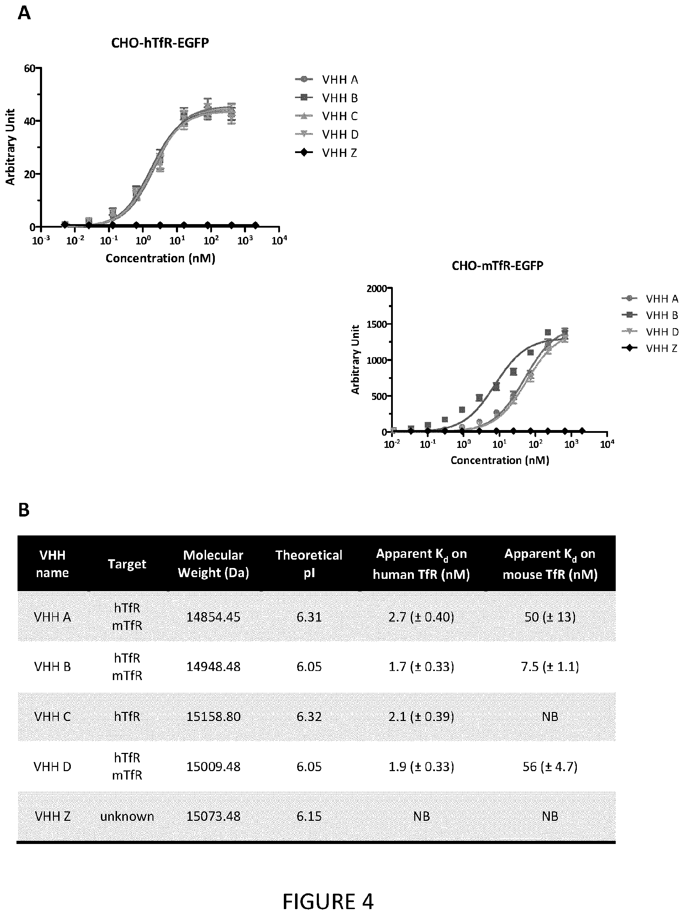

**Figure 4****: Apparent K_{d} determination of VHHs on hTfR- and mTfR- expressing CHO cell lines.** (A) CHO-hTfR-EGFP and CHO-mTfR-EGFP cells were incubated 1 hr at 4 °C with various concentrations of VHHs, detected with a mouse anti-6His (1/1000) and an Alexa647-conjugated anti-mouse secondary antibody (1/200 or 1/400). Measurements were performed using flow cytometry. The ratio of fluorescence intensity for each point was normalized with the corresponding EGFP signal (receptor expression) and gave rise to the arbitrary unit. Data are presented as mean ± SEM of 3 independent experiments. (B) Characteristics of selected VHHs: Molecular Weight (Da); Theoretical pI; Apparent K_{d} on human TfR (nM); Apparent K_{d} on mouse TfR (nM). Data are presented as mean ± SEM of 3 independent experiments. NB: no binding.

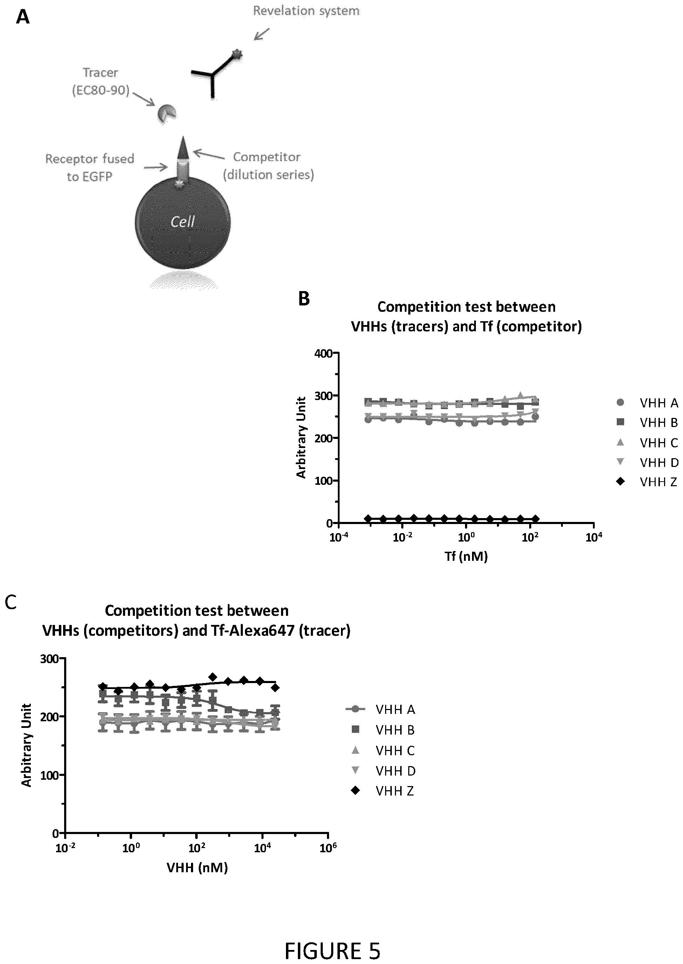

**Figure 5****: Competition assays between VHHs and Tf.** (A) Principle of competition test. In a first step, CHO-hTfR-EGFP cells were incubated 1 hr at 4 °C with the competitor in dilution series. Second, the tracer at EC90 was added and incubated for 1 hr at 4 °C. Tracer was then revealed with the appropriate revelation system. Measurements were performed using flow cytometry. The ratio of fluorescence intensity for each point was normalized with the corresponding EGFP signal (receptor expression) and gave rise to the arbitrary unit. (B) CHO-hTfR-EGFP cells were incubated with the competitor (Tf). Tracers (VHHs) at EC90 were then added and detected with a mouse anti-cMyc antibody (1/50) and an Alexa647-conjugated anti-mouse secondary antibody (1/200). (C) CHO-hTfR-EGFP cells were incubated with competitors (VHHs). Tracer (Tf-Alexa647) at EC90 was then added and detected directly. Data are presented as mean ± SEM of 3 independent experiments.

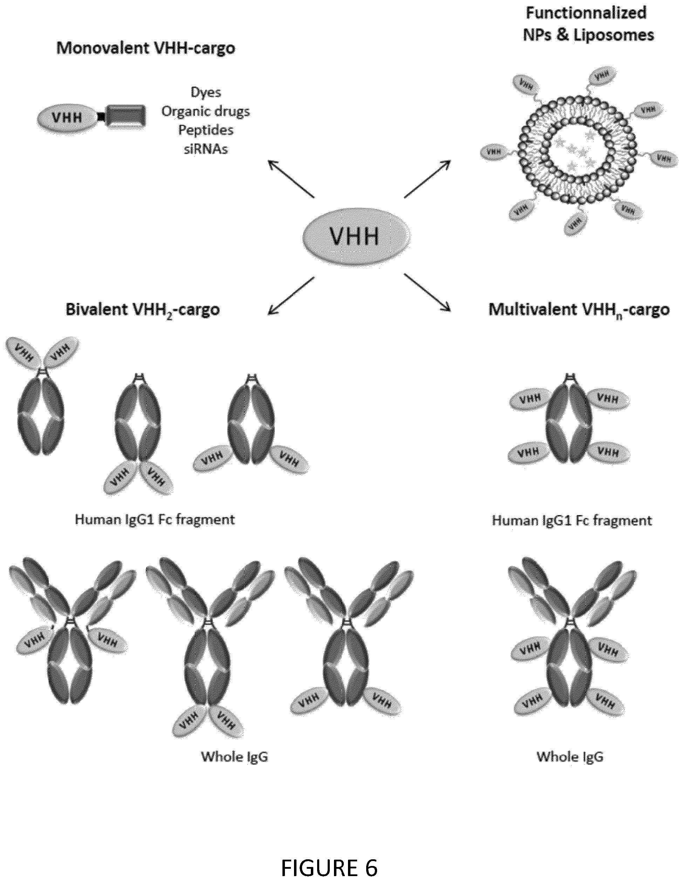

**Figure 6****: VHH conjugation strategies.** Using either chemical conjugation or recombinant fusion, VHHs can be used to vectorize all kinds of molecules, including non-exhaustively peptides, siRNAs, dyes, nanoparticles (NPs), liposomes, imaging agents and antibodies. Moreover, VHHs can be used to vectorize a molecule as a monovalent (VHH) or multivalent (VHHₙ) conjugate.

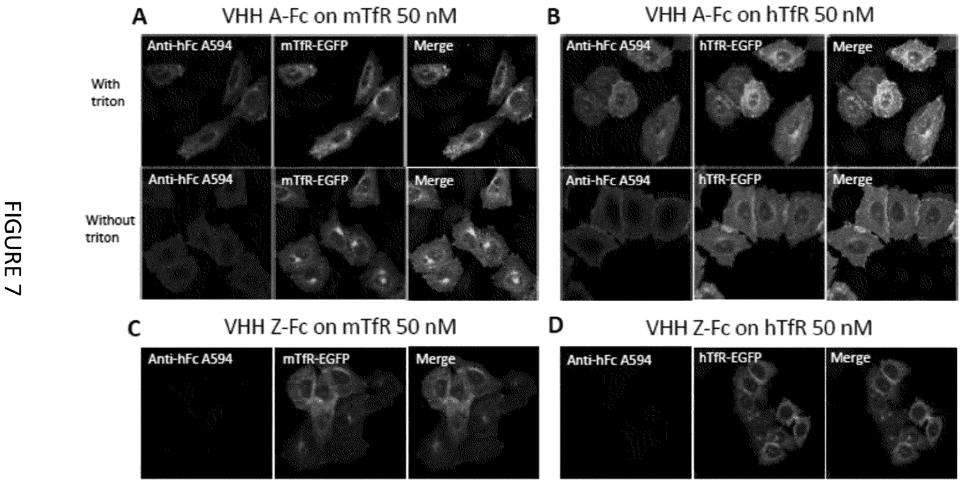

**Figure 7****: Cell surface binding and endocytosis of VHH A-Fc and VHH Z-Fc fusion proteins on hTfR- and mTfR-expressing CHO cells.** Representative confocal photomicrographs of CHO-hTfR-EGFP and CHO-mTfR-EGFP cells (green) incubated 1 hr at 37 °C with 50 nM of VHH A-Fc (A, B) and with the control VHH Z-Fc (C, D), detected post- PFA fixation and following or not triton X-100 permeabilization of cell membranes, with an Alexa594-conjugated anti-hFc antibody (1/1000, red). Cell nuclei were labeled with Hoechst#33342 at 0.5 µg/ml (blue). Co-labeling appears in yellow/orange in the merged pictures.

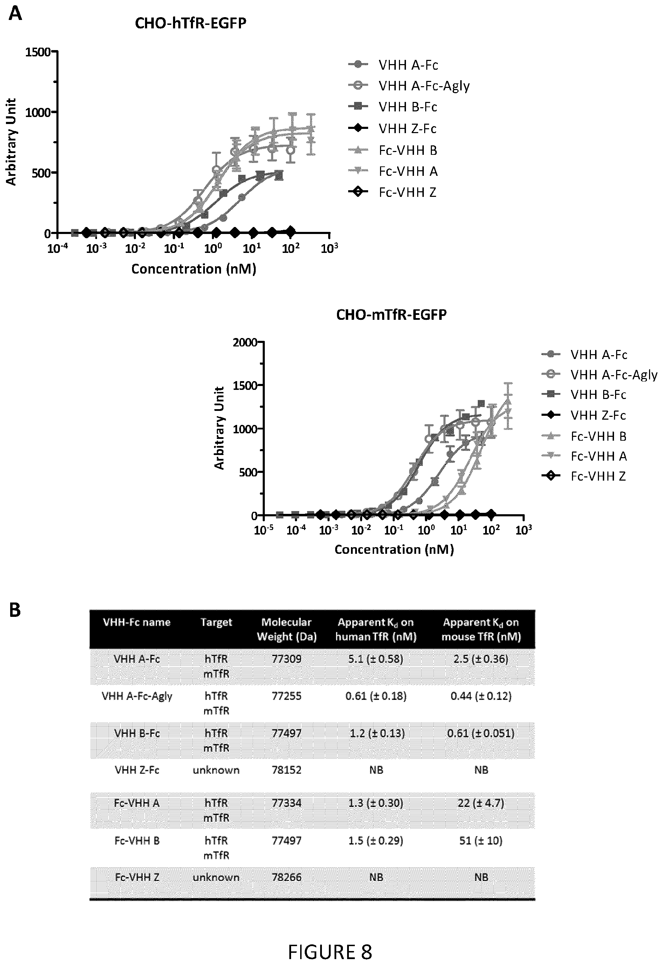

**Figure 8****: Apparent K_{d} determination of VHH-Fcs and Fc-VHHs on hTfR- and mTfR- expressing CHO cell lines.** (A) CHO-hTfR-EGFP and CHO-mTfR-EGFP cells were incubated 1 hr at 4 °C with various concentrations of VHH-Fcs or Fc-VHHs, detected with an Alexa647-conjugated anti-hFc antibody (1/400). Measurements were performed using flow cytometry. The ratio of fluorescence intensity for each point was normalized with the corresponding EGFP signal (receptor expression) and gave rise to the arbitrary unit. Data are presented as mean ± SEM of 3 independent experiments. (B) Characteristics of selected VHH-Fcs and Fc-VHHs: Molecular Weight (Da); Apparent K_{d} on human TfR (nM); Apparent K_{d} on mouse TfR (nM). Data are presented as mean ± SEM of 3 independent experiments. NB: no binding for the control VHH (VHH Z).

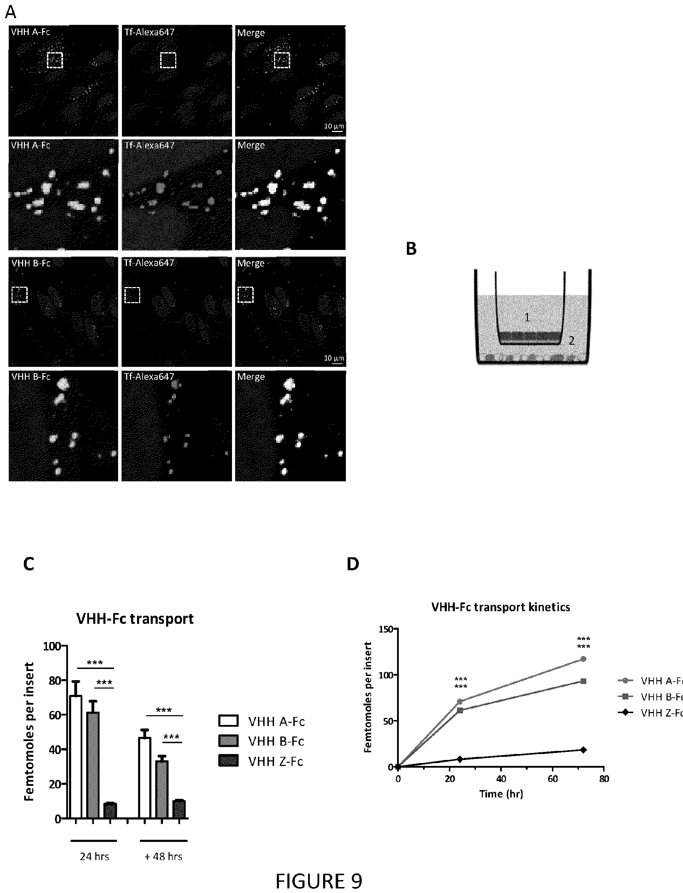

**Figure 9****: Uptake and transport of VHH A-Fc and VHH B-Fc fusion proteins in an *in vitro* BBB model.** (A) Representative photomicrographs of rat brain microvascular endothelial cell (rBMEC) monolayers probed for uptake of 500 nM VHH A-Fc and VHH B-Fc, co-incubated with Tf-Alexa647 at 200 nM (red), for 2 hrs on live cells, detected following PFA fixation and triton X-100 permeabilization of cell membranes with an Alexa488-conjugated anti-hFc antibody (1/50, green). Cell nuclei were labeled with Hoechst#33342 at 0.5 µg/ml (blue). Co-labeling appears in yellow in the merged pictures. (B) Schematic representation of the *in vitro* BBB model, a co-culture system with primary rBMECs plated on collagen type IV/fibronectin-coated filter in the upper compartment (1) and primary astrocytes in the lower compartment (2). (C, D) Transport of VHH A-Fc, VHH B-Fc and VHH Z-Fc fusion proteins across rBMEC monolayers from the luminal (upper) to the abluminal (lower) compartment. (C) VHH A-Fc, VHH B-Fc and VHH Z-Fc were incubated at 10 nM in the luminal compartment for 24 hrs and transport to the abluminal compartment was evaluated (named 24 hrs). Then the inserts containing the VHH-Fc solutions were transferred to another 96-well plate containing fresh transport buffer for another transport interval of 48 hrs (named +48 hrs) to the abluminal compartment. (D) Kinetic presentation of the experiment described in (C) (the 72 hrs transport is the sum of the 24 hrs and 48 hrs transport intervals). The content of Fc fragment in the abluminal compartment was quantified using an in-house anti-Fc ELISA assay. Absorbance units were transformed in femtomoles per insert (surface area of 0,143 cm² for inserts of a 96-well plate). Three independent experiments of at least 12 inserts were assayed for each conjugate. Data are presented as mean ± SEM (*** p ≤ 0.001).

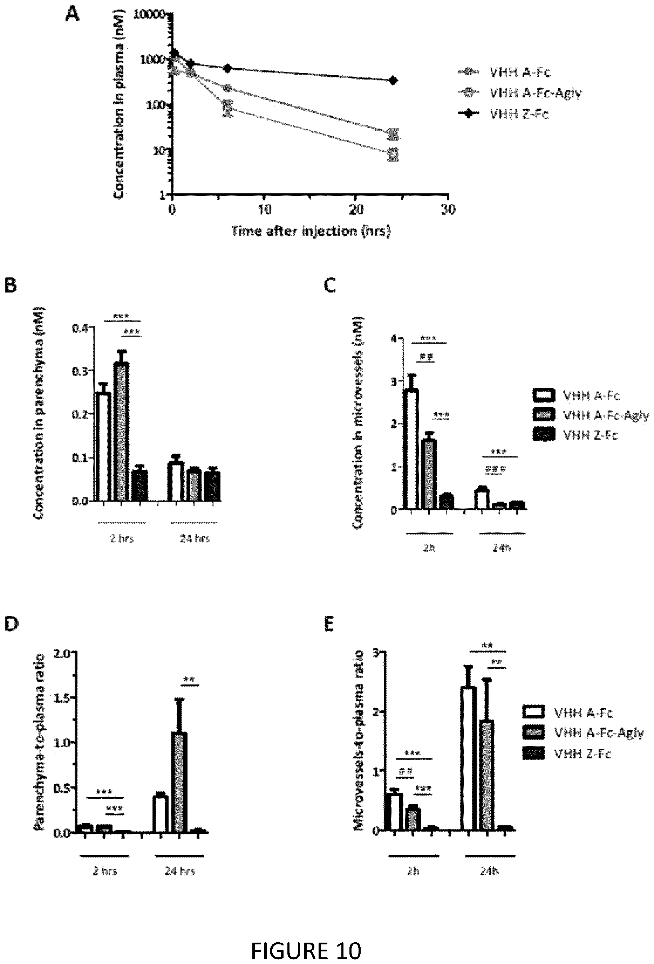

**Figure 10****: Distribution of VHH-Fc fusion proteins in WT C57Bl/6 mice at 2 and 24 hrs post-injection (p.i.).** VHH A-Fc, VHH A-Fc-Agly and VHH Z-Fc fusions were injected into the tail vein at 5 mg/kg and mice were perfused with saline at either 2 or 24 hrs p.i., after collection of plasmas. Intermediate plasma samples were also collected using retro-orbital sampling at 15 min and 6 hrs p.i. Brains were processed to isolate brain parenchyma from capillary. Amounts of VHH-Fcs in each tissue were assessed using an in-house anti-Fc ELISA assay. Data are presented as mean ± SEM of VHH-Fc concentrations in plasma (A), parenchyma (B) and microvessels (C), or by mean ± SEM of parenchyma-to-plasma ratio (D), and microvessel-to-plasma ratio (E). (4 < n < 12 per group per time point; * p ≤ 0.05, ** p ≤ 0.01, *** p ≤ 0.001).

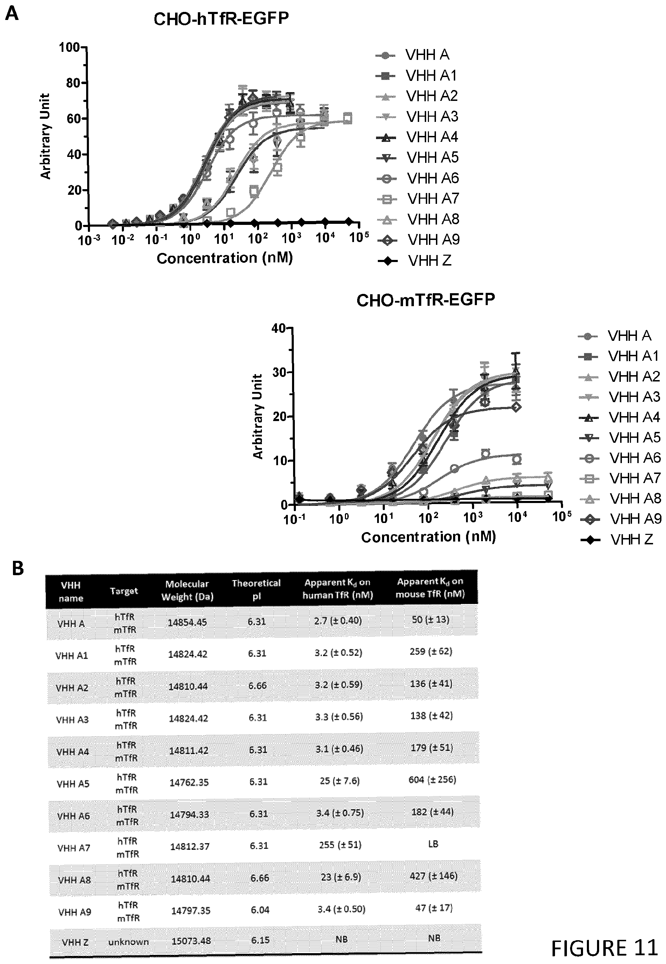

**Figure 11****: Apparent K_{d} determination of VHH A1 to A9 on CHO cell lines stably expressing hTfR and mTfR.** (A) CHO-hTfR-EGFP and CHO-mTfR-EGFP cells were incubated 1 hr at 4 °C with various concentrations of VHHs, detected with a mouse anti-6His (1/1000) and an Alexa647-conjugated anti-mouse secondary antibody (1/400). Measurements were performed using flow cytometry. The ratio of fluorescence intensity for each point was normalized with the corresponding EGFP signal (receptor expression) and gave rise to the arbitrary unit. Data are presented as mean ± SEM of 3 independent experiments. (B) Characteristics of VHHs: Molecular Weight (Da); Theoretical pI; Apparent K_{d} on human TfR (nM); Apparent K_{d} on mouse TfR (nM). Data are presented as mean ± SEM of 3 independent experiments. NB: no binding, LB: low binding.

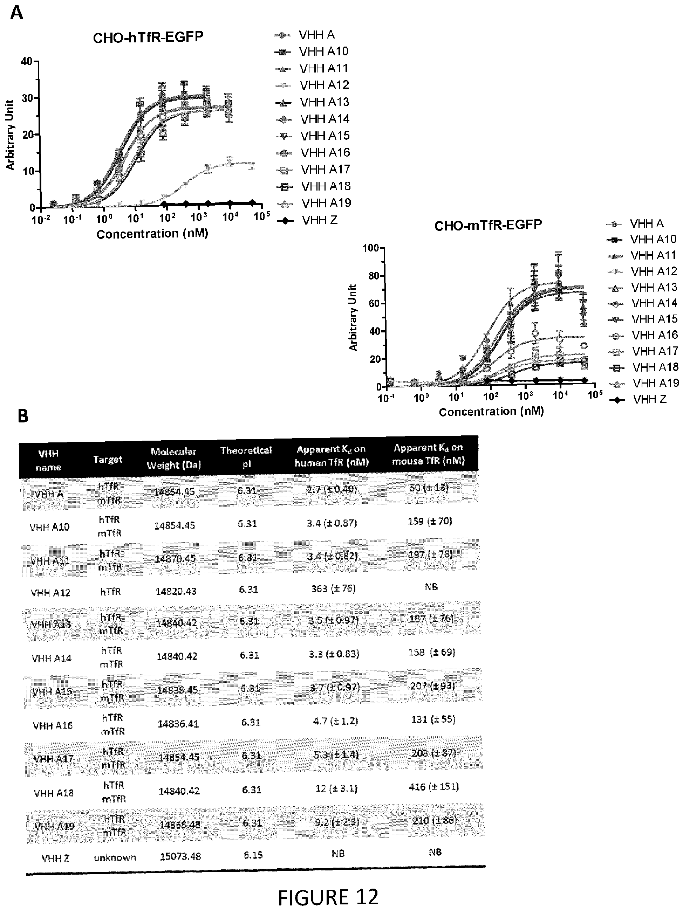

**Figure 12****: Apparent K_{d} determination of VHH A10 to A19 on CHO cell lines stably expressing hTfR and mTfR.** (A) CHO-hTfR-EGFP and CHO-mTfR-EGFP cells were incubated 1 hr at 4 °C with various concentrations of VHHs, detected with a mouse anti-6His (1/1000) and an Alexa647-conjugated anti-mouse secondary antibody (1/400). Measurements were performed using flow cytometry. The ratio of fluorescence intensity for each point was normalized with the corresponding EGFP signal (receptor expression) and gave rise to the arbitrary unit. Data are presented as mean ± SEM of 3 independent experiments. (B) Characteristics of VHHs: Molecular Weight (Da); Theoretical pI; Apparent K_{d} on human TfR (nM); Apparent K_{d} on mouse TfR (nM). Data are presented as mean ± SEM of 3 independent experiments. NB: no binding.

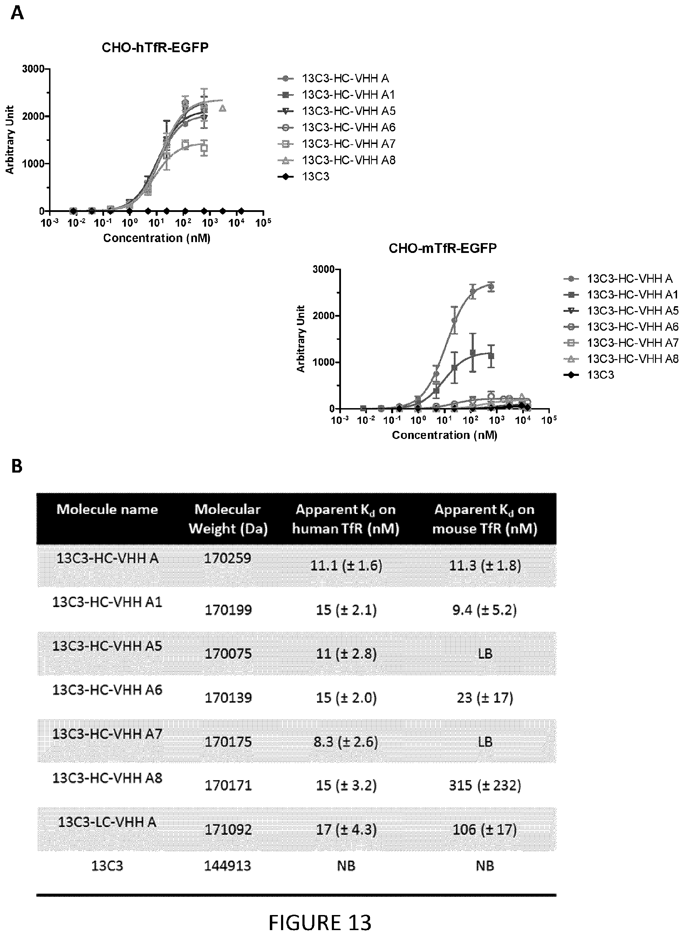

**Figure 13****: Apparent K_{d} determination of 13C3-HC-VHH fusions on hTfR- and mTfR- expressing CHO cell lines.** (A) CHO-hTfR-EGFP and CHO-mTfR-EGFP cells were incubated 1 hr at 4 °C with various concentrations of 13C3 fusions and detected with an Alexa647-conjugated anti-mouse antibody (1/400). Measurements were performed using flow cytometry. The ratio of fluorescence intensity for each point was normalized with the corresponding EGFP signal (receptor expression) and gave rise to the arbitrary unit. Data are presented as mean ± SEM of 3 independent experiments. (B) Characteristics of 13C3 fusions: Molecular Weight (Da); Apparent K_{d} on human TfR (nM); Apparent K_{d} on mouse TfR (nM). Data are presented as mean ± SEM of 3 independent experiments. NB: no binding, LB: low binding.

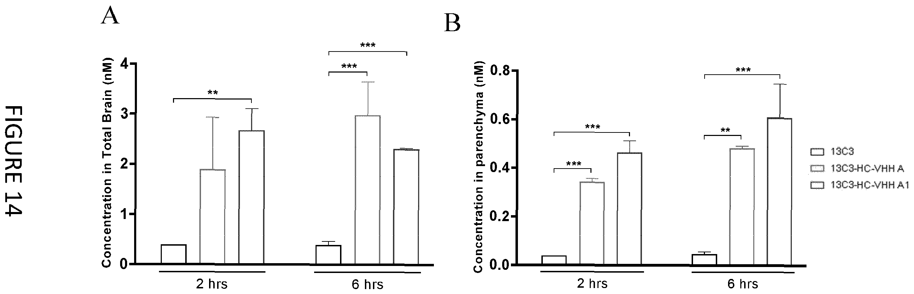

**Figure 14****: Distribution of 13C3 monoclonal antibody and 13C3-HC-VHH fusions in WT C57Bl/6 mice at 2 and 6 hrs post-injection (p.i).** 13C3, 13C3-HC-VHH A, and 13C3-HC-VHH A1, were injected into the tail vein at 35 nmoles/kg and mice were perfused with saline at either 2 or 6 hrs p.i. Brains were processed to isolate brain parenchyma from capillary. Amounts of 13C3 and 13C3-HC-VHH A/A1 in each tissue/compartment were assessed using a qualified Meso Scale Discovery (MSD) direct coating (Abeta) immunoassay (%CV<20 % and recovery ± 30 %). Data are presented as mean ± SEM of 13C3 and 13C3-HC-VHH A/A1 concentration in total brain (A) and parenchyma (B) (1 < n < 4 per group per time point; * p ≤ 0.05, ** p ≤ 0.01, *** p ≤ 0.001).

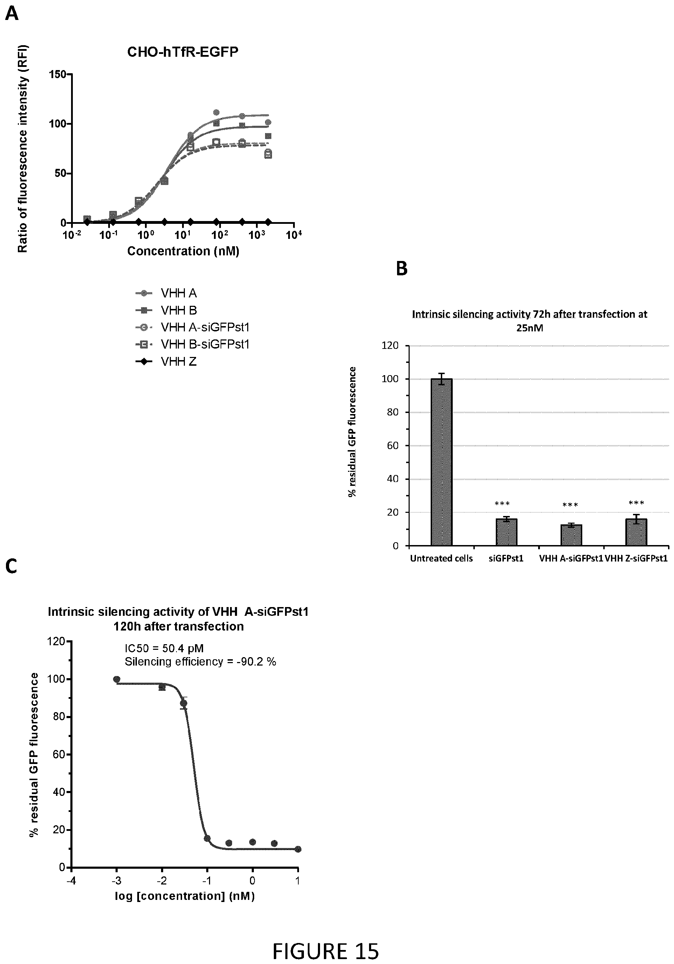

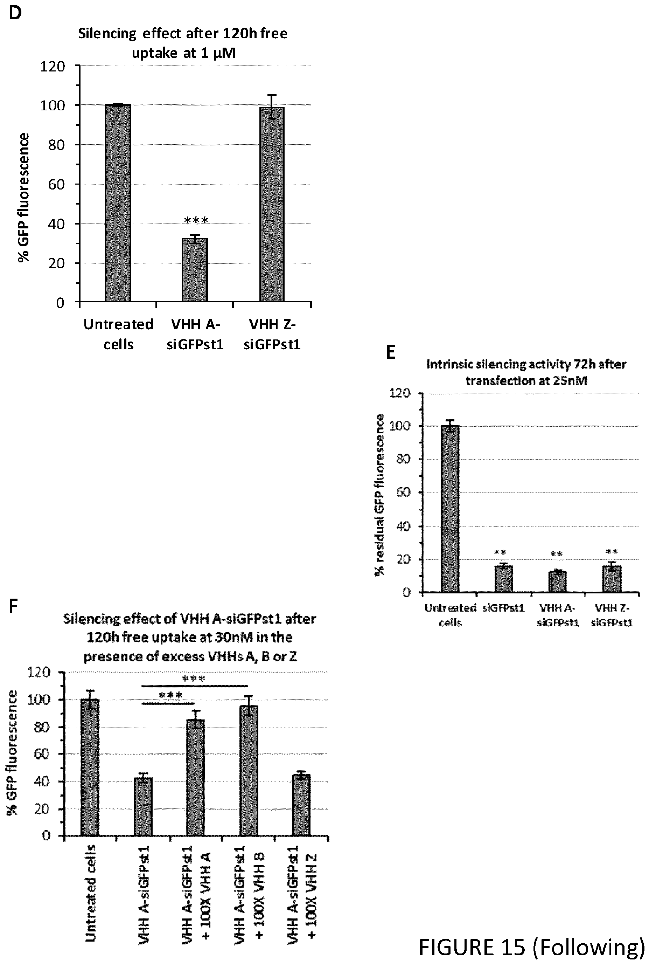

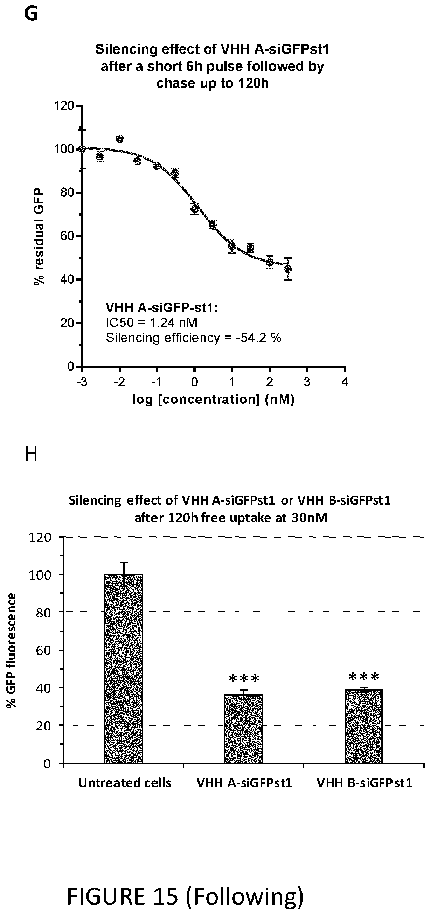

**Figure 15****: *In vitro* gene silencing activity of VHH-siGFPst1 bioconjugates.** (A) The VHH A-siGFPst1 and VHH B-siGFPst1 bioconjugates bind hTfR. CHO-hTfR-EGFP cells were incubated 1 hr at 4 °C with various concentrations of the indicated compounds. Detection of VHHs was performed using a primary mouse anti-6His (1/1000) and an Alexa647-conjugated anti-mouse secondary antibody (1/400). Measurements of cell-surface signal associated to VHH were performed using flow cytometry. The results are expressed as the ratio of Alexa647-associated fluorescence intensity of test compounds to that of background fluorescence. (B) The VHH A-siGFPst1 bioconjugate displays gene silencing efficiency. CHO-hTfR-EGFP cells were transfected with the indicated compound at 25 nM using Dharmafect 1 (Dharmacon) during 72h at 37°C. The total fluorescence associated to the EGFP protein was then quantified using flow cytometry and rationalized to that of untreated (control) cells (set at 100%) *** p<0.001. (C) The VHH A-siGFPst1 bioconjugate displays an intrinsic gene silencing activity in the picomolar range upon direct delivery into the cytosol. CHO-hTfR-EGFP cells were transfected with various concentrations of the VHH A-siGFPst1 bioconjugate using Dharmafect 1 (Dharmacon) during 120 hrs at 37°C. The total fluorescence associated with the EGFP protein was then quantified using flow cytometry and rationalized to that of untreated (control) cells (set at 100%). Data were fit using a nonlinear regression using GraphPad Prism^{®} software (solid line) to estimate the IC50 (concentration allowing 50% reduction of GFP protein levels) and the maximum effect (bottom plateau). (D) The VHH A-siGFPst1 bioconjugate triggers specific and efficient TfR-mediated gene silencing. CHO-hTfR-EGFP cells were incubated with the indicated compounds at 1 µM during 120 hrs at 37°C. Data were processed and analyzed as described in (B). *** p<0.001 vs. untreated cells. (E) hTfR-mediated binding and uptake of the VHH A-siGFPst1 bioconjugate allows cytosol delivery and subsequent gene silencing at nanomolar concentrations. CHO-hTfR-EGFP cells were incubated with various concentrations of the VHH A-siGFPst1 bioconjugate during 120 hrs at 37°C. The total fluorescence associated with the EGFP protein was then quantified using flow cytometry and rationalized to that of untreated (control) cells (set at 100%). Data were processed and analyzed as described in (C). (F) The gene silencing effect of the VHH A-siGFPst1 bioconjugate is inhibited by co-incubation with an excess of free TfR-binding VHHs A and B but not with the irrelevant VHH Z. CHO-hTfR-EGFP cells were incubated with VHH A-siGFPst1 at 30 nM alone or in the presence of a 100X excess of the free VHHs A, B or Z during 120 hrs at 37°C. Data were processed and analyzed as described in (C). (G) Cellular exposure to the VHH A-siGFPst1 bioconjugate during a short 6-hour pulse is sufficient to trigger efficient gene silencing. CHO-hTfR-EGFP cells were incubated with various concentrations of the VHH A-siGFPst1 bioconjugate during a short 6-hour pulse followed by chase up to 120 hrs in ligand-free medium. Data were processed and analyzed as described in (B). (H) The gene silencing effect of the VHH B-siGFPst1 bioconjugate was similar to that observed with VHH A-siGFPst1. CHO-hTfR-EGFP cells were incubated with VHH A-siGFPst1 or VHH B-siGFPst1 at 30 nM (saturating concentration based on the IC50 obtained with VHH A-siGFPst1) during 120 hrs at 37°C. Data were processed and analyzed as described in (C).

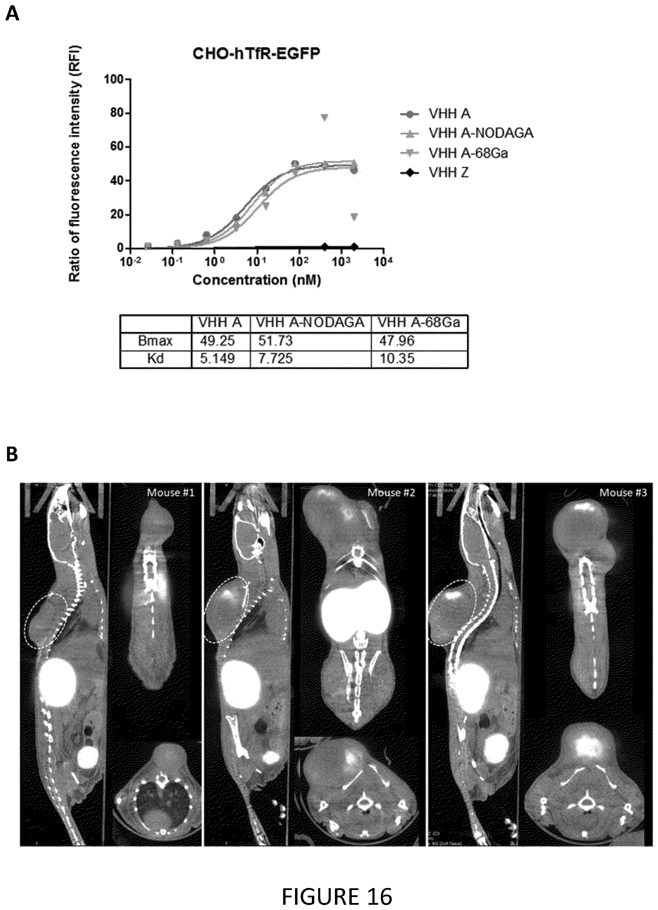

**Figure 16****: PET imaging of VHH A-68Ga bioconjugate in a subcutaneous mouse model of glioblastoma tumor.** (A) The VHH A-NODAGA and VHH A-68Ga bioconjugates bind hTfR as efficiently as the non-conjugated VHH A compound. CHO-hTfR-EGFP cells were incubated 1 hr at 4 °C with various concentrations of the indicated compounds. Detection of VHHs was performed using a primary mouse anti-6His (1/1000) and an Alexa647-conjugated anti-mouse secondary antibody (1/400). Measurements of cell-surface signal associated with VHH were performed using flow cytometry. The results are expressed as the ratio of Alexa647-associated fluorescence intensity of test compounds to that of background fluorescence. (B) PET imaging of mice administered with VHH A-68Ga at day 28 after implantation with U87-MG cells (2 hrs post injection). The glioblastoma tumor is indicated by a circle in the sagittal view.

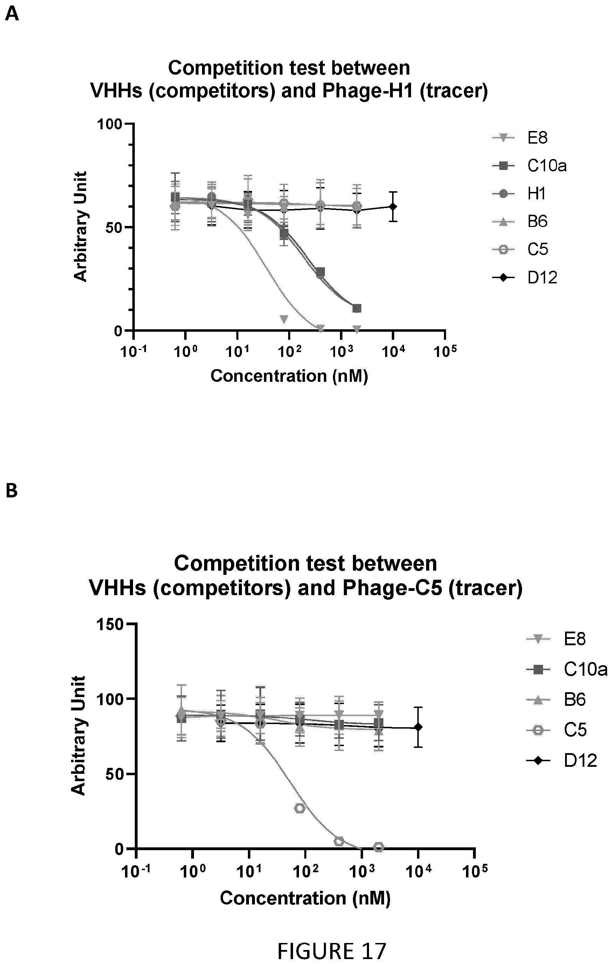

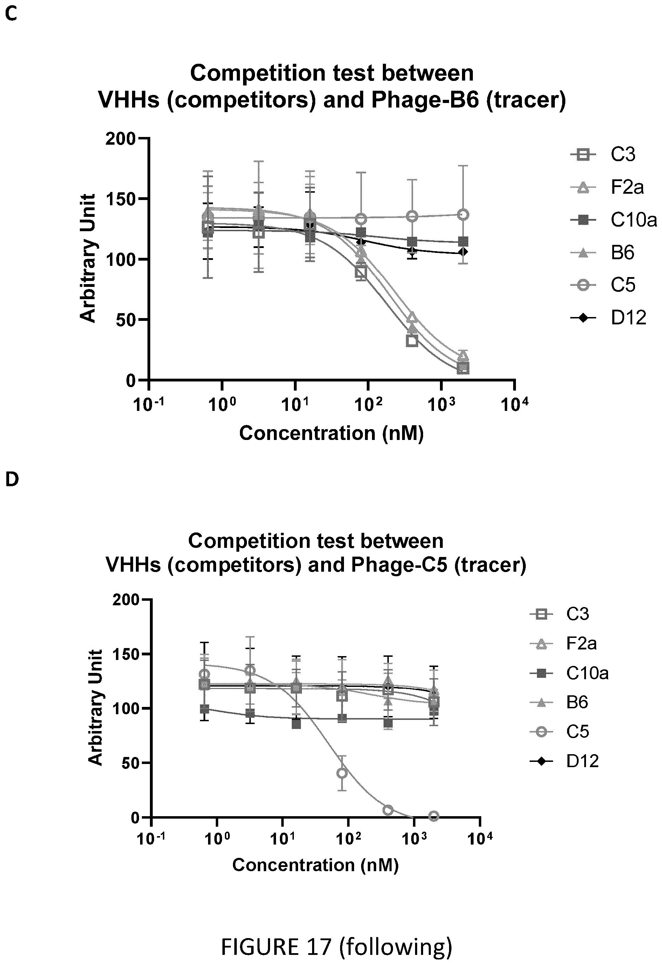

**Figure 17****: Competition assays between VHHs and phage-VHHs.** CHO-hTfR-EGFP cells were incubated 1 hr at 4 °C with VHHs (competitors) at increasing concentrations. Phage-VHHs (tracers) were then added at EC80 and incubated for 1 more hr . Phage-VHHs were detected with an anti-M13-A647 antibody (1/200). Fluorescence levels were assessed using flow cytometry. The ratio of fluorescence intensity for each point was normalized with the corresponding EGFP signal (receptor expression) and gave rise to the arbitrary unit. Data are presented as mean ± SEM of independent experiments. (A) Competition between VHHs identified after phage display selections on the rhTfR and phage-H1. (B) Competition between VHHs identified after phage display selections on the rhTfR and phage-C5. (C) Competition between VHHs identified after phage display selections on the apical domain of the TfR and phage-B6. (D) Competition between VHHs identified after phage display selections on the apical domain of the TfR and phage-C5.

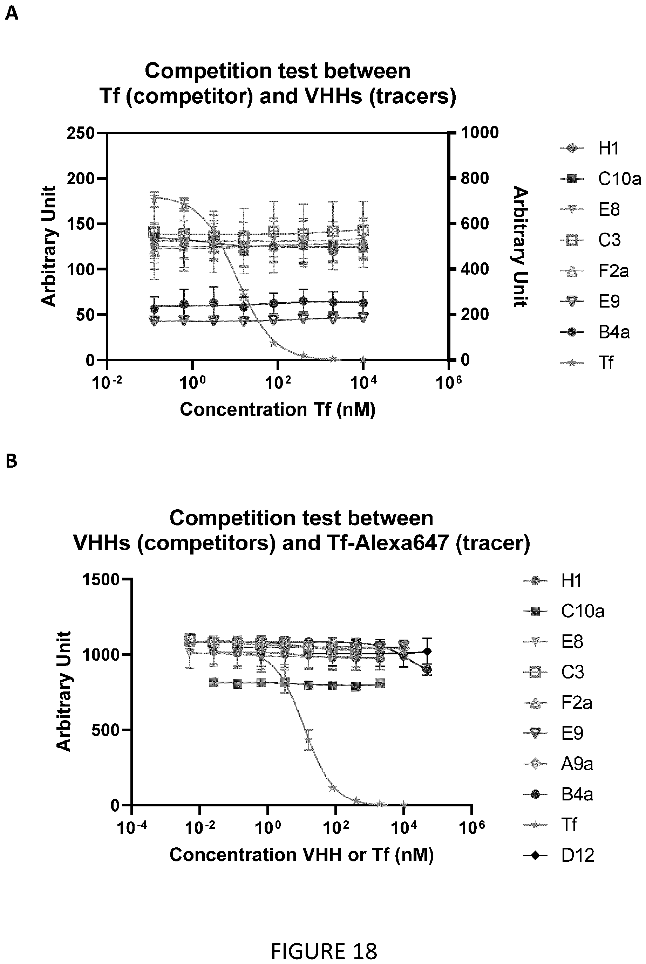

**Figure 18****: Competition assays between VHHs and Tf.** (A) CHO-hTfR-EGFP cells were incubated 1 hr at 4 °C with the Tf (competitor) at increasing concentrations. VHHs (tracers), or Tf-A647 as positive control, were then added at EC80 and incubated for 1 more hr. VHHs were detected with a mouse anti-6His (1/1000) and an Alexa647-conjugated anti-mouse secondary antibody (1/400). Fluorescence levels were assessed using flow cytometry. The ratio of fluorescence intensity for each point was normalized with the corresponding EGFP signal (receptor expression) and gave rise to the arbitrary unit. The Y left axis corresponds to the VHH binding, the Y right axis corresponds to Tf-A647 binding. Data are presented as mean ± SEM of independent experiments. (B) CHO-hTfR-EGFP cells were incubated 1 hr at 4 °C with VHHs (competitors) or Tf as positive control, at increasing concentrations. Tf-A647 (tracer) at EC80 was then added and incubated 1 more hr. Fluorescence levels were assessed using flow cytometry. The ratio of fluorescence intensity for each point was normalized with the corresponding EGFP signal (receptor expression) and gave rise to the arbitrary unit. Data are presented as mean ± SEM of independent experiments.

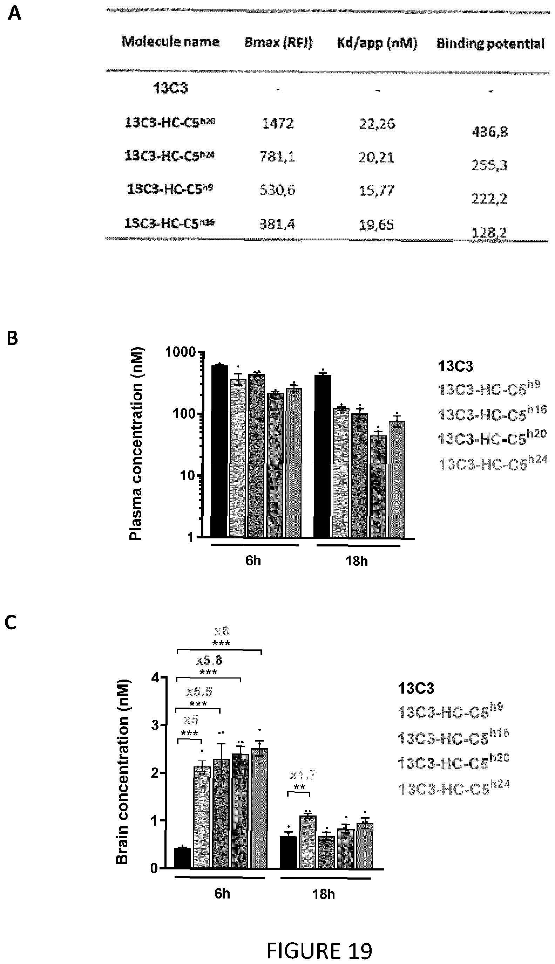

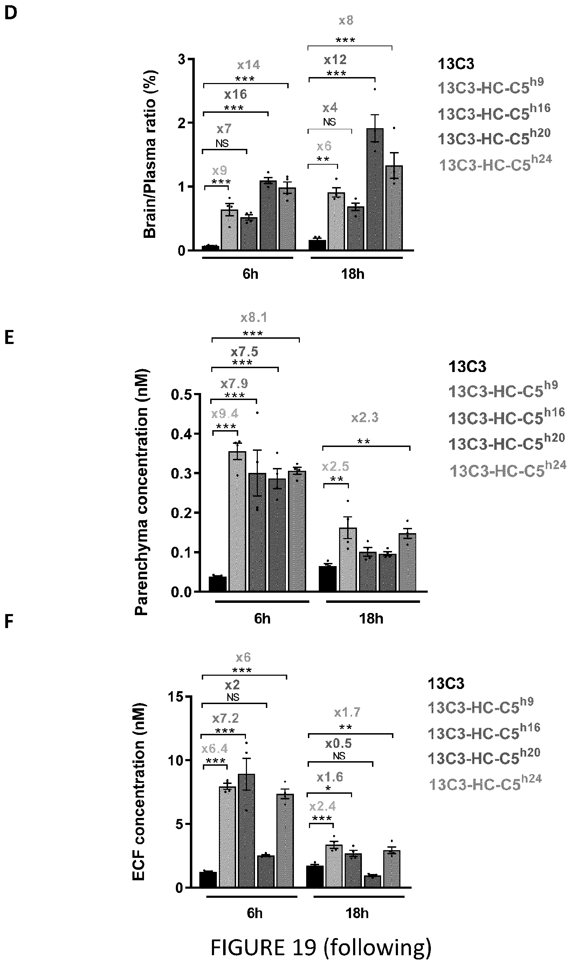

**Figure 19****: Binding potential to mTfR1 and *in vivo* distribution of 13C3 monoclonal antibody and 13C3-HC-VHH homodimeric fusions in WT C57Bl/6 mice at 6 and 18 hrs post-injection (p.i).** 13C3, 13C3-HC-C5h20, 13C3-HC-C5h9, 13C3-HC-C5h16, 13C3-HC-C5h24 were evaluated for their binding (Bmax, K_{d/app} and binding potential: ratio of normalized Bmax/K_{d/app} x 100) to mTfR in a cell-based assay (A). 13C3 and 13C3-HC-C5h20 / C5h9 / C5h16 / C5h24 were injected into the tail vein at 35 nmoles/kg and mice were perfused with saline post-injection. Brains were processed to isolate brain parenchyma and extra-cellular fluid (ECF) from capillary. Amounts of 13C3 and 13C3-HC-C5h20 / C5h9 / C5h16 / C5h24 in each tissues/compartment were assessed using a qualified Meso Scale Discovery (MSD) direct coating (Abeta) immunoassay (%CV<20 % and recovery ± 30 %). Data are presented as mean ± SEM of 13C3 and 13C3-HC-C5h20 / C5h9 / C5h16 / C5h24 concentration in Plasma (B), Total brain (C), parenchyma (E) and ECF (F) and are presented as brain/plasma concentration percentage (D) (n =4 per group per time point; * p ≤ 0.05, ** p ≤ 0.01, *** p ≤ 0.001).

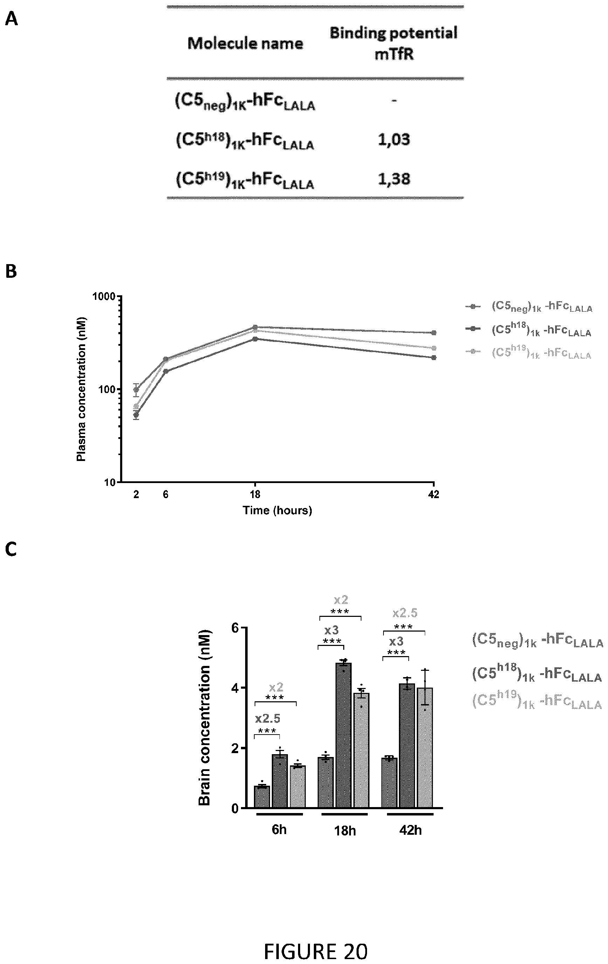

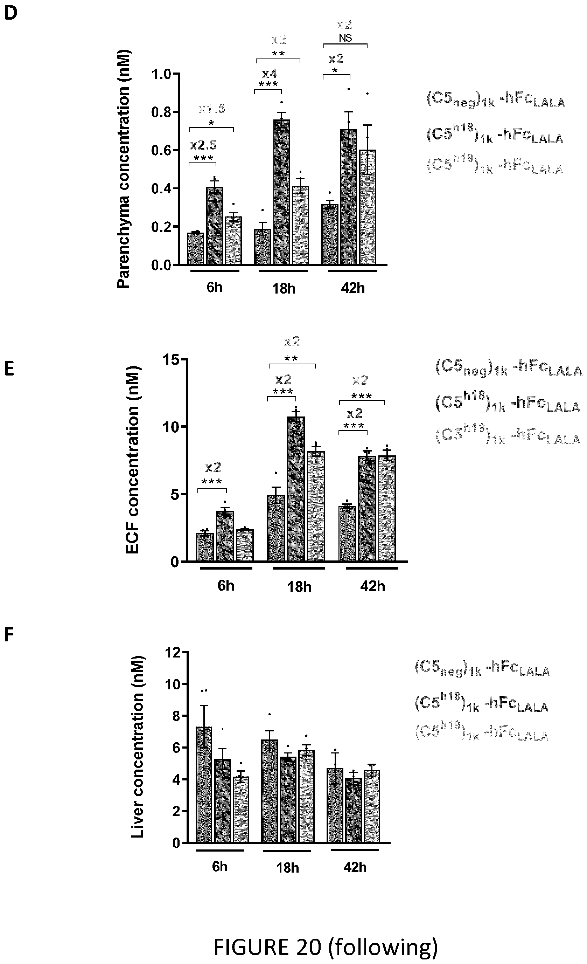

**Figure 20****: Binding potential to mTfR1 and *in vivo* distribution of (VHH)₁ₖ-hFc_{LALA} heterodimeric fusions in WT C57Bl/6 mice at 6,18 and 42 hrs post-injection (p.i).** (C5^{neg})₁ₖ-hFc_{LALA}, (C5^{h18})₁ₖ-hFc_{LALA} and (C5^{h19})₁ₖ-hFc_{LALA} were evaluated for their binding potential to mTfR: ratio of normalized Bmax/K_{d/app} x 100) to mTfR in a cell-based assay (A). (C5^{neg})₁ₖ-hFc_{LALA}, (C5^{h18})₁ₖ-hFc_{LALA} and (C5^{h19})₁ₖ-hFc_{LALA} were injected subcutaneously at 70 nmoles/kg and mice were perfused with saline post-injection. Brains were processed to isolate brain parenchyma and extra-cellular fluid (ECF) from capillary. Amounts of (C5^{neg})₁ₖ-hFc_{LALA}, (C5^{h18})₁ₖ-hFc_{LALA} and (C5^{h19})₁ₖ-hFc_{LALA} in each tissues/compartment were assessed using an anti-hFc ELISA assay (%CV<20 % and recovery ± 30 %). Data are presented as mean ± SEM of hFc_{LALA} and (C5^{h18/h19})₁ₖ-hFc_{LALA} concentration in Plasma (B), Total brain (C), parenchyma (D), ECF (E) and liver (F) (n =4 per group per time point; * p ≤ 0.05, ** p ≤ 0.01, *** p ≤ 0.001).

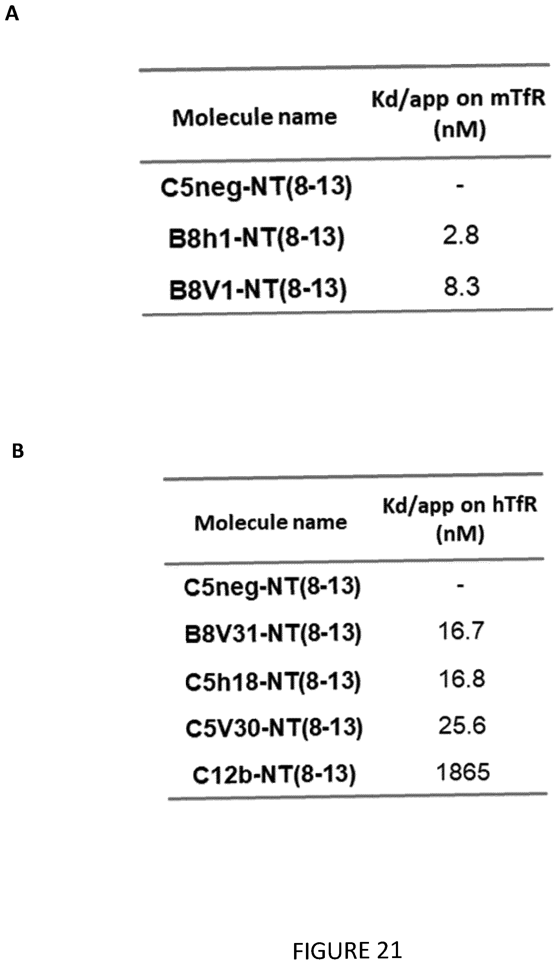

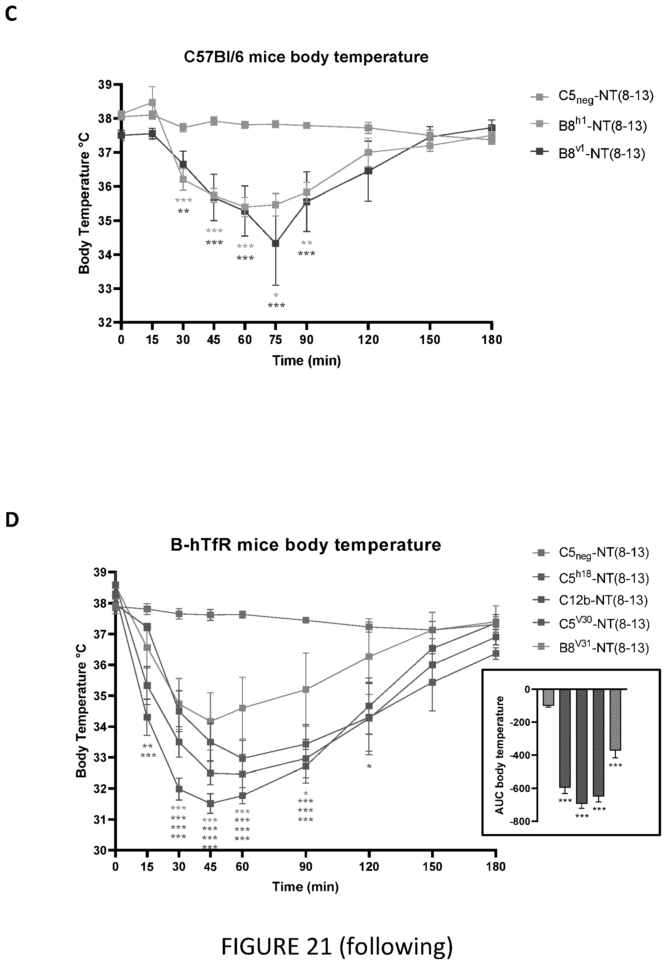

**Figure 21****: Hypothermia induction of VHH-NT(8-13) fusions after single IV injection at 500 nmoles/Kg in C57Bl/6 or B-hTfR mice**. C5_{neg}-NT(8-13), B8^{h1}-NT(8-13), B8^{V1}-NT(8-13), B8^{v31}-NT(8-13), C5^{h18}-NT(8-13), C5^{v30}-NT(8-13) and C12b-NT(8-13) were evaluated for their apparent affinity for mTfR (A) or hTfR (B) in a cell-based assay. C5_{neg}-NT(8-13), B8^{h1}-NT(8-13), B8^{V1}-NT(8-13), B8^{v31}-NT(8-13), C5^{h18}-NT(8-13), C5^{v30}-NT(8-13) and C12b-NT(8-13) were injected intravenously at 500 nmoles/kg in C57Bl/6 mice (C) or B-hTfR mice (D). After injection, mouse body temperature was assessed every 15 min until 90 min post-injection, then every 30 min until 180 min post-injection (n =4 to 3 per group per time point). Results are expressed as mean +/- SEM. * p ≤ 0.05, ** p ≤ 0.01, *** p ≤ 0.001 VHH-NT(8-13) fusions versus C5neg-NT(8-13). Area under the curve (AUC) were determined by comparison to the mice temperature baseline (≈ 38°C). Differences between groups were tested using one-way ANOVA with Tukey post hoc test (Graph Pad 9.4.1).

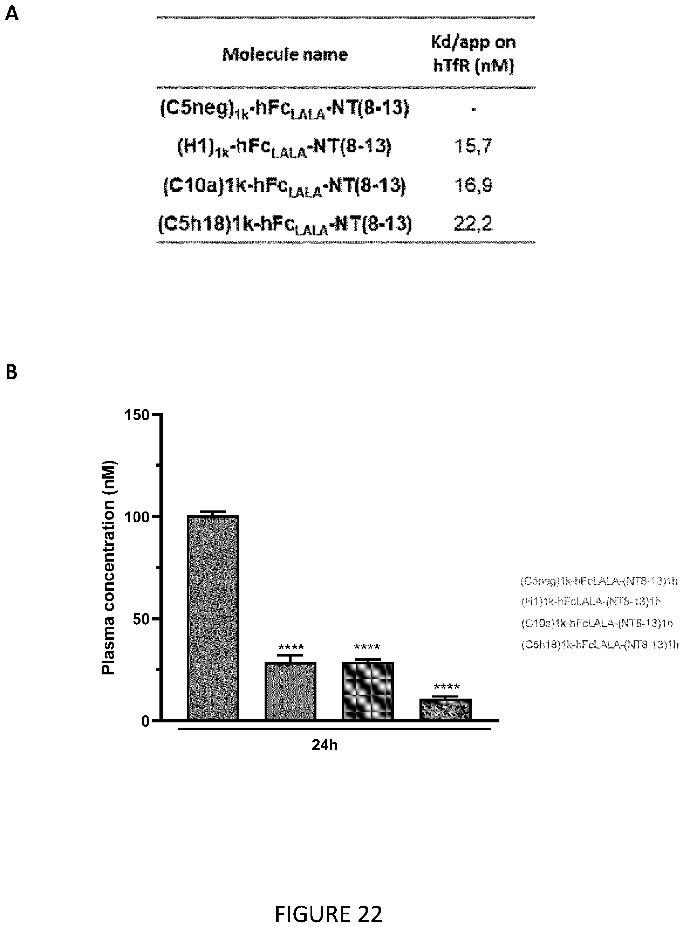

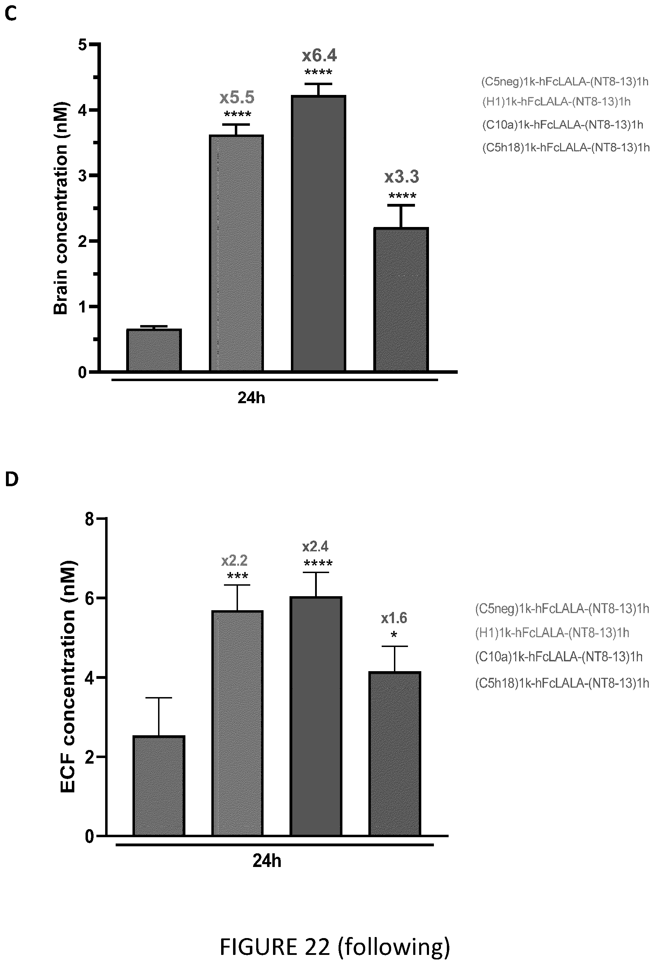

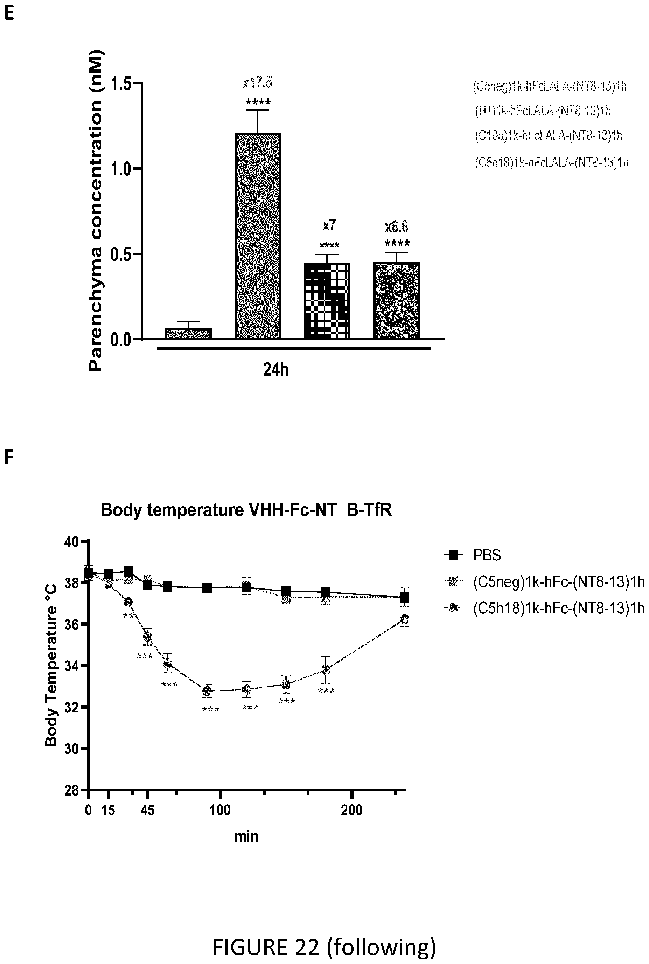

**Figure 22****: Binding affinity for hTfR, *in vivo* brain uptake and hypothermia induction of heterodimeric (VHH)₁ₖ-Fc-NT(8-13) fusions after single IV injection at 20 nmoles/Kg in B-hTfR mice.** (C5neg)₁ₖ-hFc_{LALA}-NT(8-13), (C5h18)₁ₖ-hFc_{LALA}-NT(8-13), (C10a)₁ₖ-hFc_{LALA}-NT(8-13) and (H1)₁ₖ-hFc_{LALA}-NT(8-13) were evaluated for their apparent affinity for hTfR in a cell-based assay (A). (C5neg)₁ₖ-hFc_{LALA}-NT(8-13), (C5h18)₁ₖ-hFc_{LALA}-NT(8-13), (C10a)₁ₖ-hFc_{LALA}-NT(8-13) and (H1)₁ₖ-hFc_{LALA}-NT(8-13) were injected in B-hTfR mice intravenously at 20 nmoles/kg. At 24 hrs post-injection, blood was recovered by cardiac puncture and mice were perfused with saline solution. Brains were processed to isolate brain parenchyma and extra-cellular fluid (ECF) from capillary. Amount of (VHH)₁ₖ-hFc_{LALA}-NT(8-13) in each tissues/compartment was assessed using an anti-hFc ELISA assay. Data are presented as mean ± SEM of (VHH)₁ₖ-hFc_{LALA}-NT(8-13) concentration in Plasma (B), Total brain (C), ECF (D) and parenchyma (E) (n =4 per group per time point; * p ≤ 0.05, ** p ≤ 0.01, *** p ≤ 0.001). To study the ability of (C5h18)₁ₖ-hFc_{LALA}-NT(8-13) to rapidly cross the BBB the fusion was injected together with the negative control fusion (C5neg)₁ₖ-hFc_{LALA}-NT(8-13) at 20 nmoles/Kg in B-hTfR mice, mouse body temperature was assessed every 15 min until 90 min post-injection, then every 30 min until 240 min post-injection (n =4 per group per time point). Results are expressed as mean +/- SEM. * p ≤ 0.05, ** p ≤ 0.01, *** p ≤ 0.001 (C5h18)₁ₖ-hFc_{LALA}-NT(8-13) fusion versus (C5neg)₁ₖ-hFc_{LALA}-NT(8-13) (F).

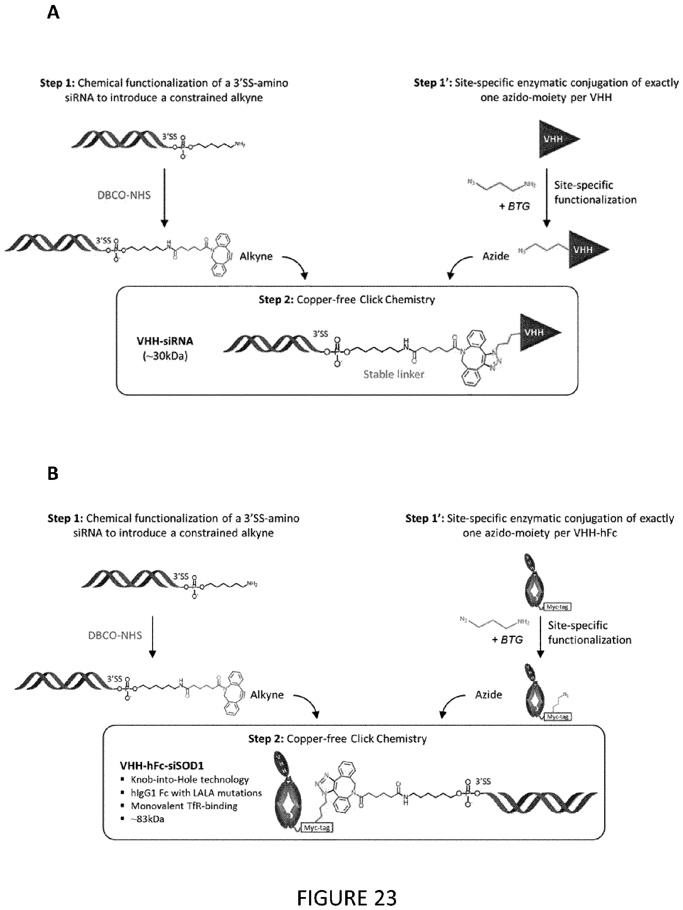

**Figure 23****: Conjugation Strategy.** Example of a conjugation strategy yielding VHH-Oligo (A) or heterodimeric VHH-hFc-Oligo (B) conjugates with a stable linker. Such a general conjugation strategy involves a convergent synthesis with the parallel modification of: (i) the VHH (A) or the heterodimeric VHH-hFc (B) to site-specifically introduce for example an azido-linker; and (ii) the oligonucleotide to introduce for example a constrained alkyne group complementary to the azido functional group. In a final step, both functionalized azido-VHH (A) or VHH-hFc-azido (B) and alkyne-Oligo precursors are linked to each other using preferably a copper-free click reaction, yielding VHH-Oligo or VHH-hFc-Oligo conjugates with a stable linker.

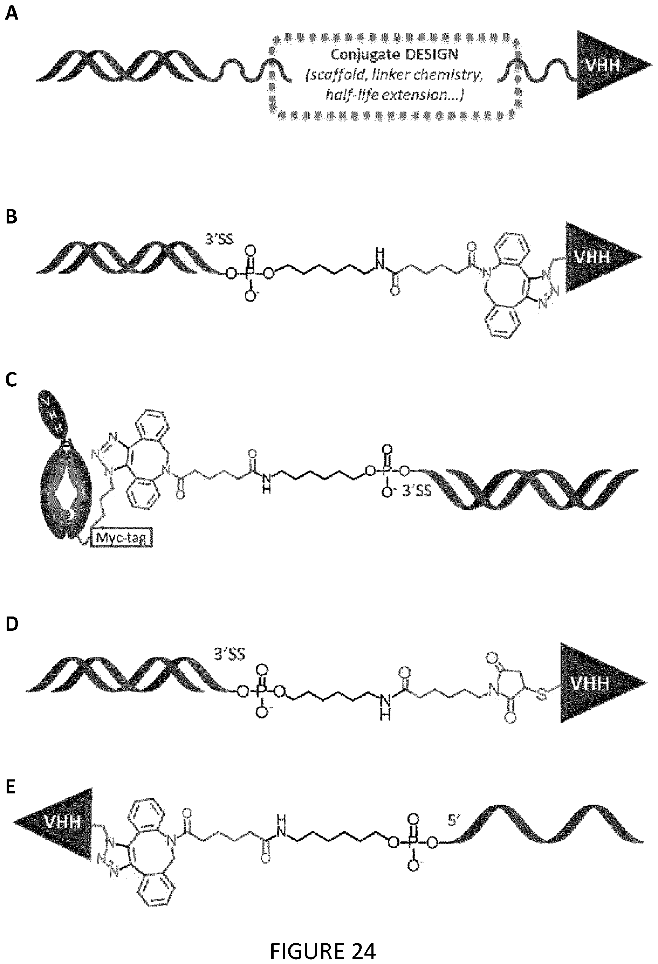

**Figure 24****: General VHH-oligo Structure.** (A) the general structure of VHH-Oligo conjugates of the present invention comprising i) the oligo moiety, which can be any oligonucleotide-based drug such as a single-stranded ASO or a duplex siRNA, ii) the VHH and iii) a linking moiety, which can consist of a half-life extending entity onto which the oligo and the VHH are linked at two distinct sites, and (B-E) a detailed structure of the linker used for some of the conjugates evaluated in the experimental part such as a VHH-siRNA conjugate (B), a VHH-hFc-siRNA conjugate (C), a VHH-thiol-Mal-siRNA (D) or a VHH-ASO conjugate (E).

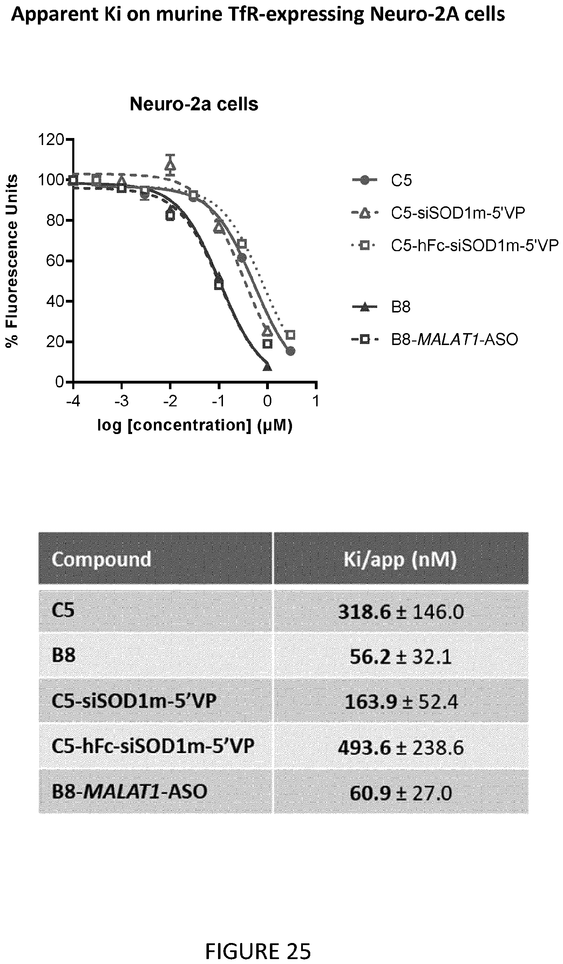

**Figure 25****: Apparent Ki on murine TfR expressing cells.** Concentration-dependent binding and apparent binding affinity values (Ki/app) of VHH-Oligo conjugates (C5-siSOD1m-5'VP, C5-hFc-siSOD1m-5'VP or B8-MALAT1-ASO) and free VHHs (C5 or B8), in competition with a fluorescent reference TfR-binding VHH, to murine TfR expressed by murine Neuro-2A.

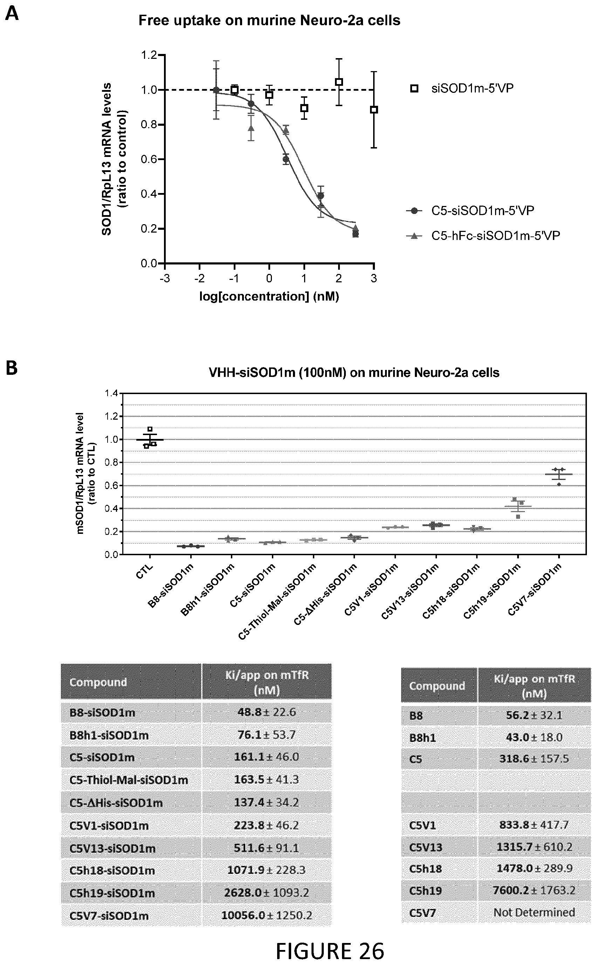

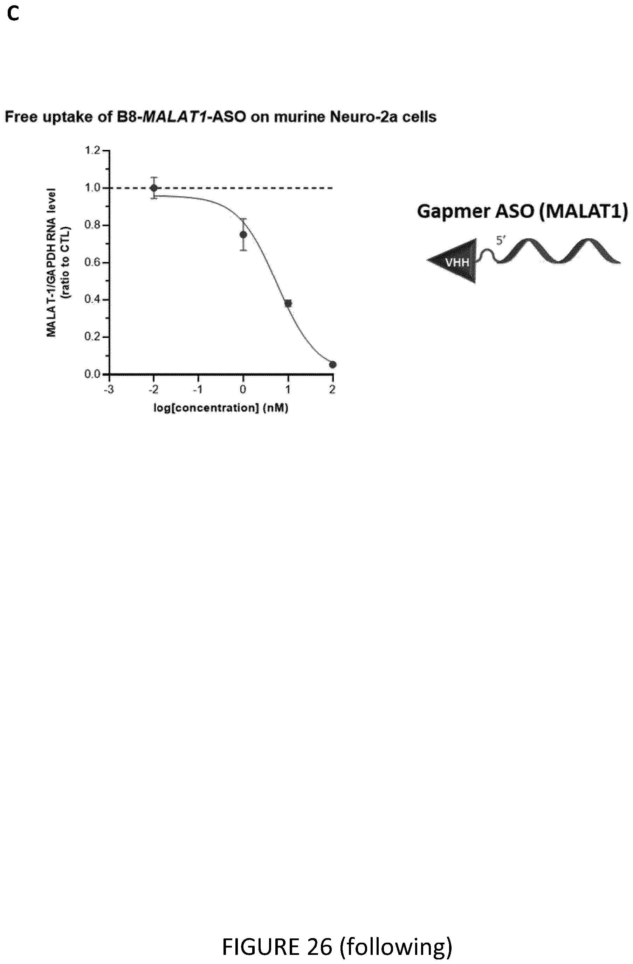

**Figure 26****: In vitro silencing activity.** mRNA or long coding RNA lncRNA downregulation potential of TfR-binding VHH-Oligo conjugates after free uptake on the murine Neuro-2A cell line. Figure 26A shows concentration-dependent downregulation of *SOD1* mRNA levels obtained with TfR-binding VHH-siRNA or VHH-hFc-siRNA conjugates (VHH-siSOD1m-5'VP and VHH-hFc-siSOD1m-5'VP wherein VHH is C5), compared to unconjugated siSOD1m-5'VP. Figure 26B shows downregulation of *SOD1* mRNA levels obtained with various examples of TfR-binding VHH-siRNA conjugates targeting the murine *SOD1* mRNA (i.e.,VHH-siSOD1m or VHH-thiol-Mal-siSOD1m or VHH-ΔHis-siSOD1m, wherein VHH is or comprises C5, B8, B8h1, C5V1, C5V13, C5h18, C5h19 or C5V7) and apparent binding affinity values (Ki/app) of tested conjugates for TfR evaluated on murine Neuro-2A cells as shown in Figure 25. Figure 26C shows downregulation of *MALAT-1* lncRNA levels obtained with a VHH-MALAT1-ASO conjugate targeting the human and mouse *MALAT-1* long non-coding RNA (VHH-MALAT1-ASO wherein VHH is B8).

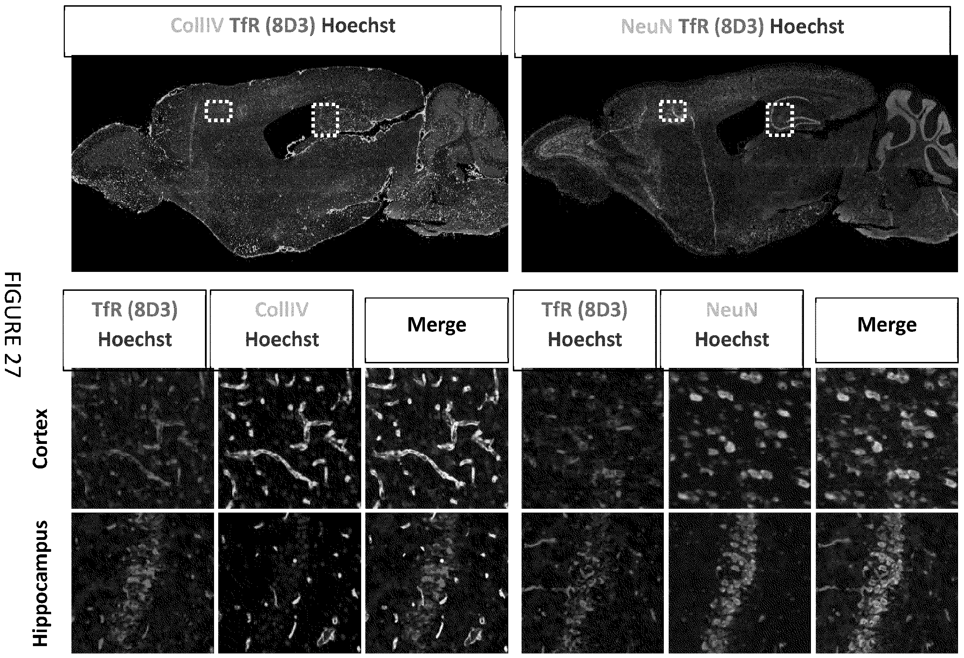

**Figure 27****: TfR distribution in brain microvessels and parenchyma cells.** TfR distribution using anti-mouse TfR immunohistochemistry in brain microvessels (co-stained using an anti-Collagen IV antibody), and parenchyma cells, including in neurons (co-stained using an anti-NeuN antibody).

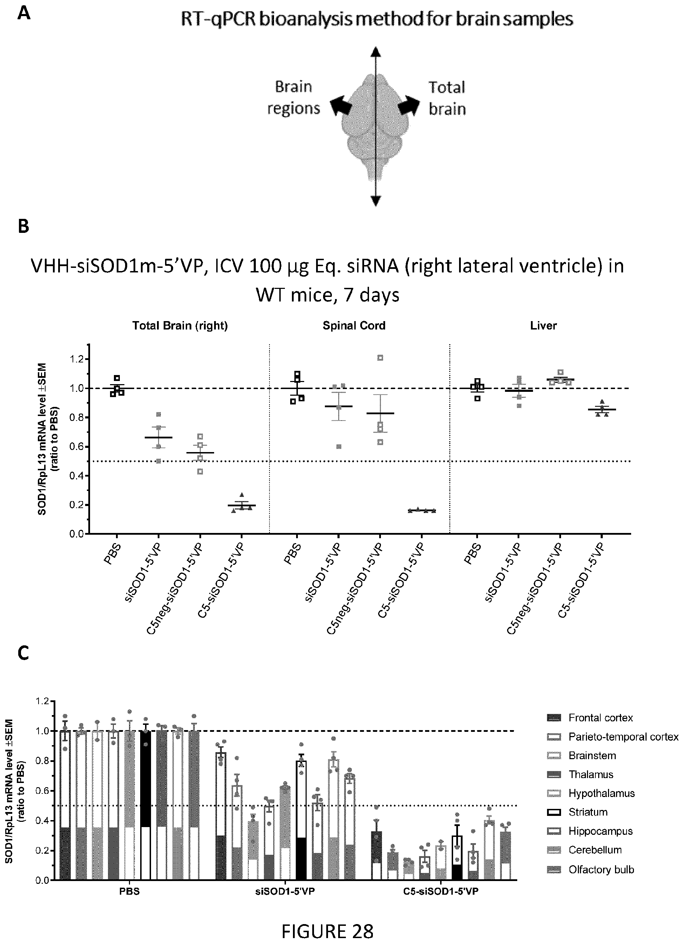

**Figure 28****: CNS knock-down effect of VHH-siRNA conjugates.** CNS- specific knock-down of murine *SOD1* mRNA after local ICV administration of VHH-siRNA conjugates (wherein VHH is C5) in wild-type C57B1/6 mice.

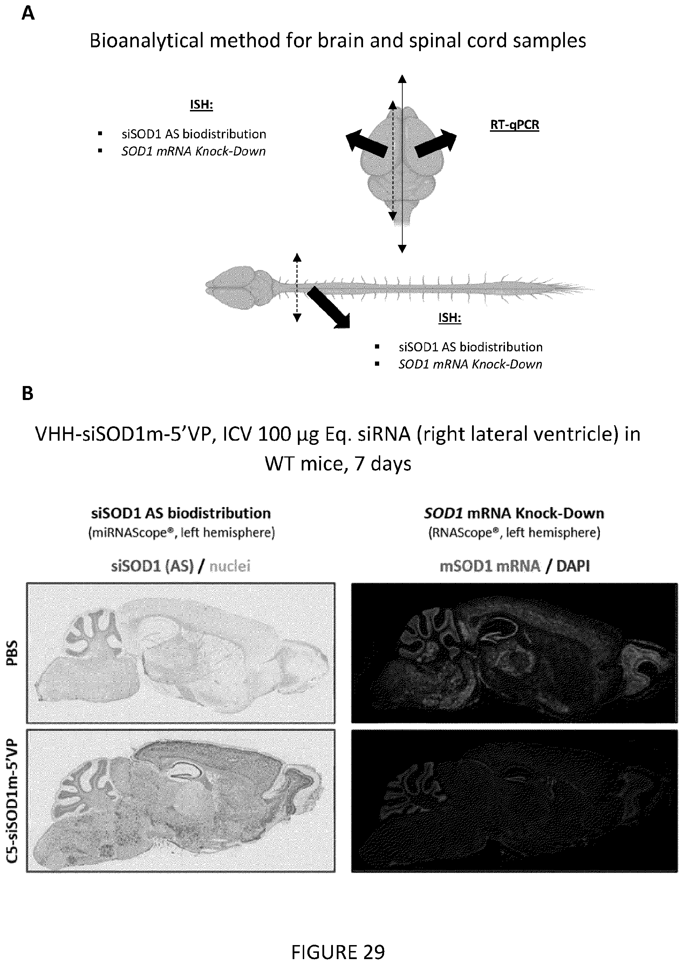

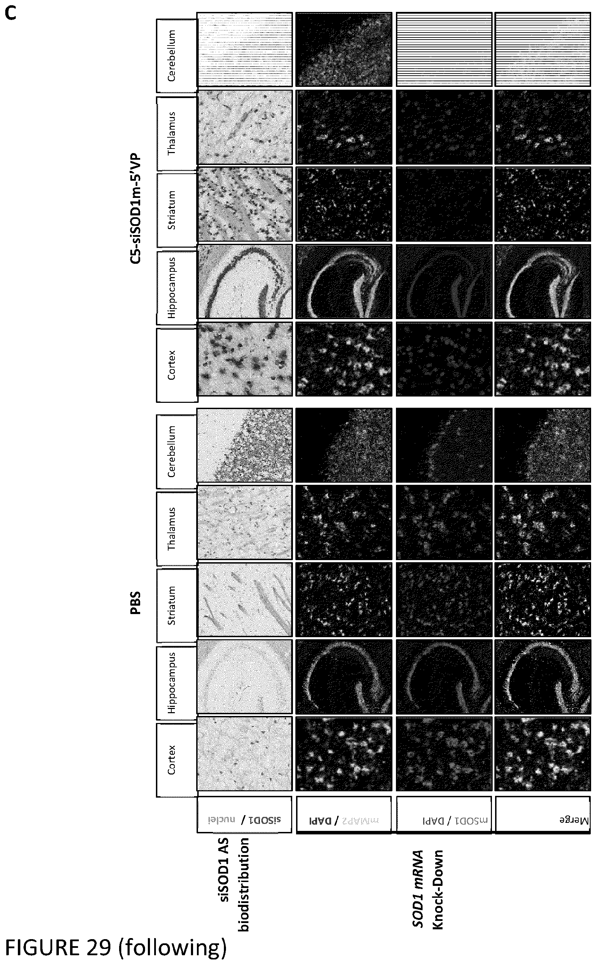

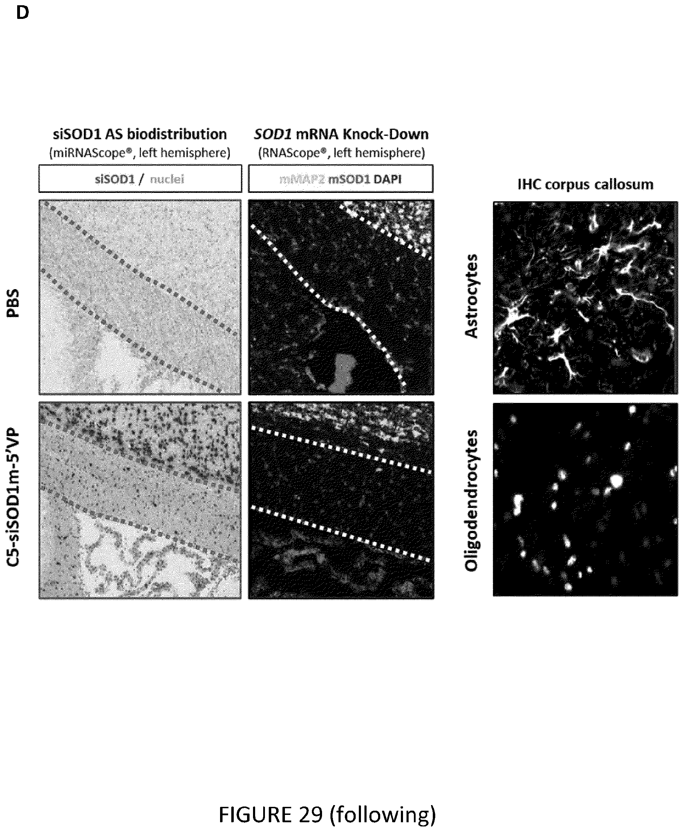

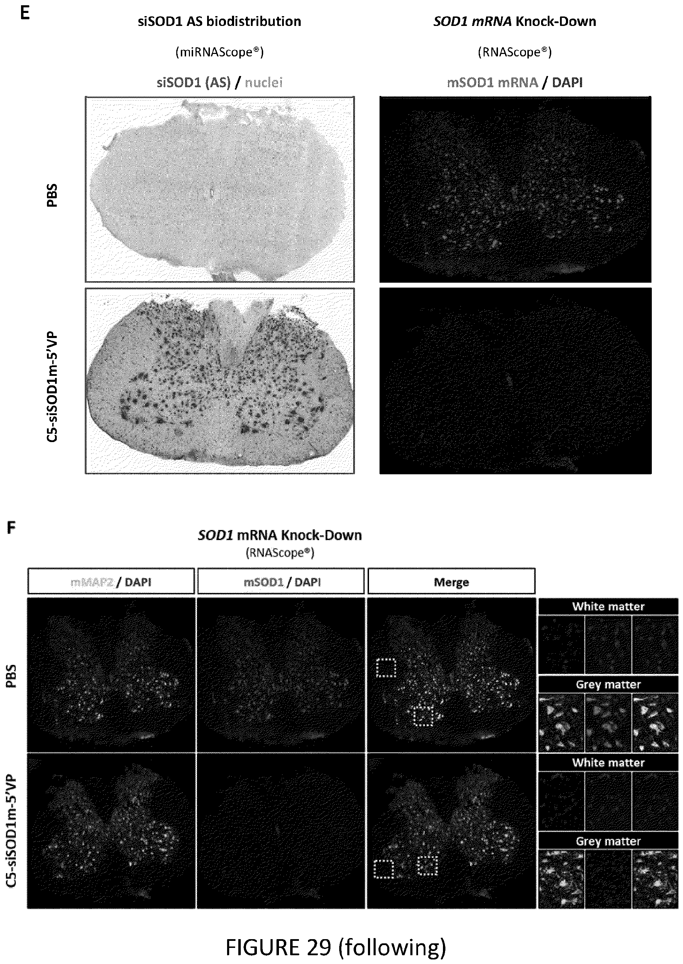

**Figure 29****: CNS knock-down effect and siRNA biodistribution.** The figure illustrates siSOD1m-5'VP biodistribution and murine *SOD1* mRNA levels in CNS brain and spinal cord, as assessed using *in situ* hybridization, after local ICV administration of PBS or VHH-siRNA conjugates (wherein VHH is C5) in wild-type C57B1/6 mice. Figure 29A shows the general sampling and bioanalysis method for the right and left brain hemispheres and spinal cord. Figures 29B and 29E show the general brain (29B, sagittal sections) and spinal cord (29E, coronal sections) distribution of the siSOD1m-5'VP antisense strand (miRNAScope^{®}, left panels), correlating to broad downregulation of murine *SOD1* mRNA levels (RNAScope^{®}, right panels). Figures 29C-D and 29F show zooms and neuron-specific co-staining, using a *Map2* mRNA-specific probe, in different regions of the brain (29B, sagittal sections) and spinal cord (29E, coronal sections).

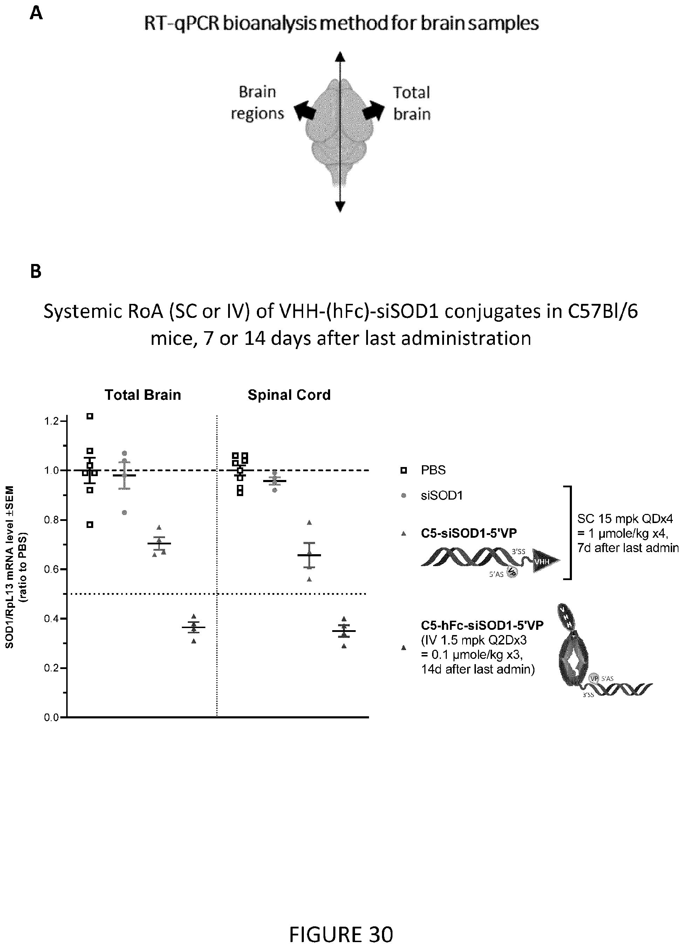

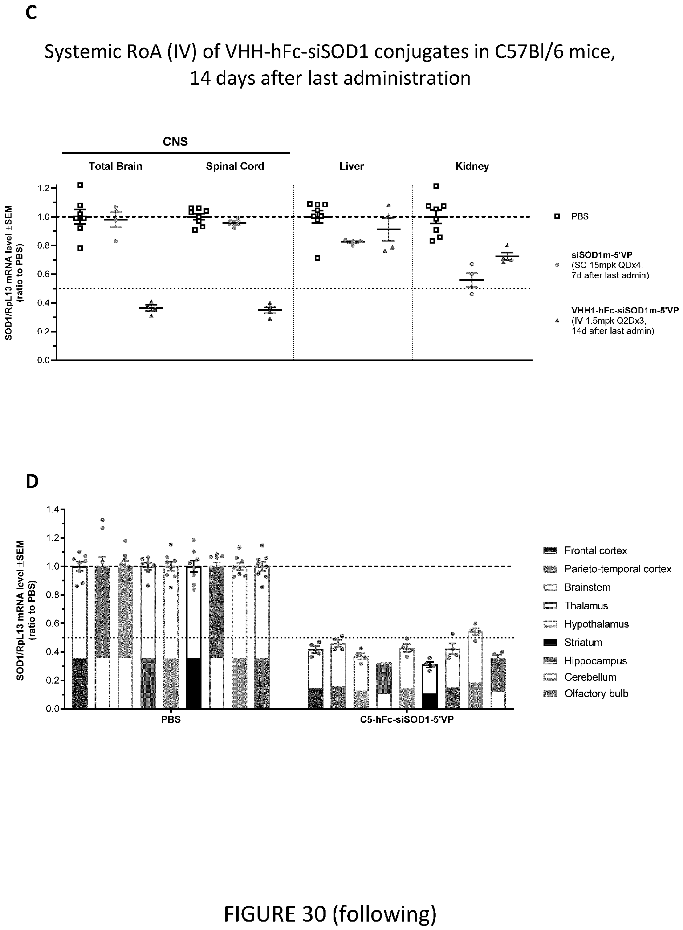

**Figure 30****: Optimization of the CNS functional delivery of VHH-siRNA conjugates.** CNS- specific knock-down of murine *SOD1* mRNA after repeated IV or SC administration of VHH-siRNA or VHH-hFc-siSOD1m-5'VP conjugates in wild-type C57B1/6 mice.

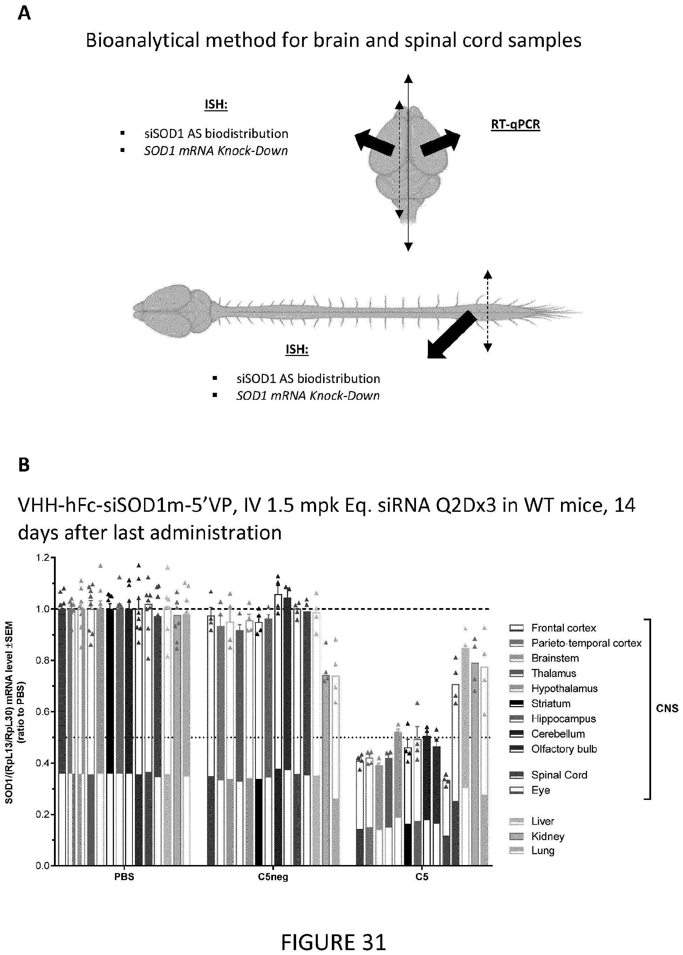

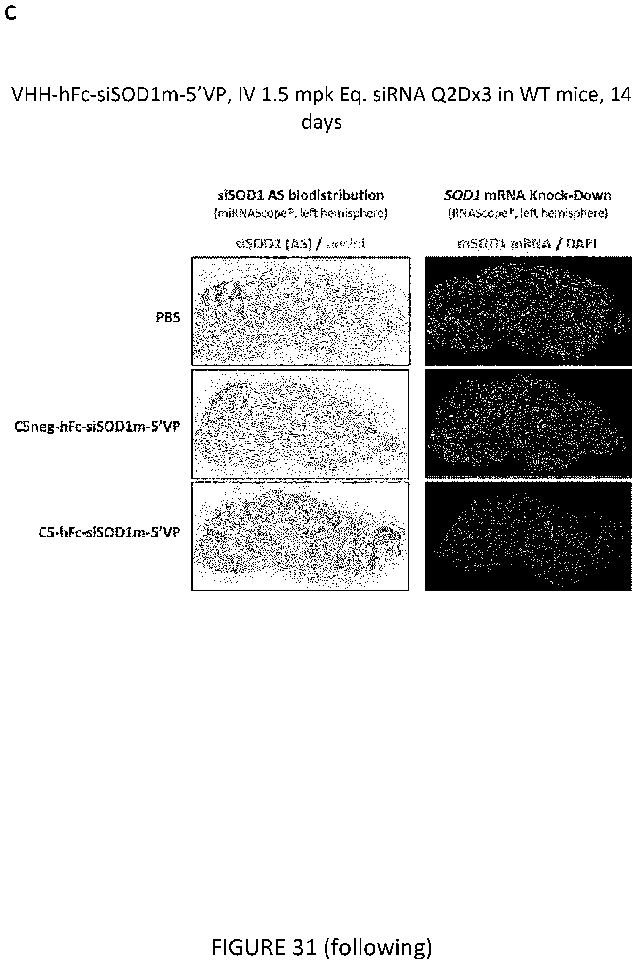

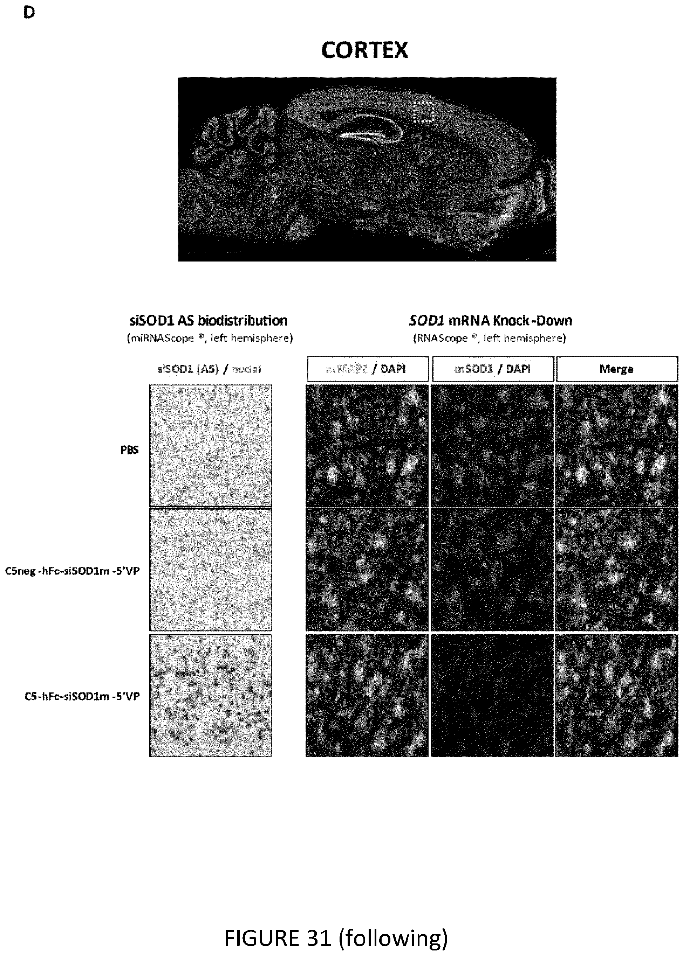

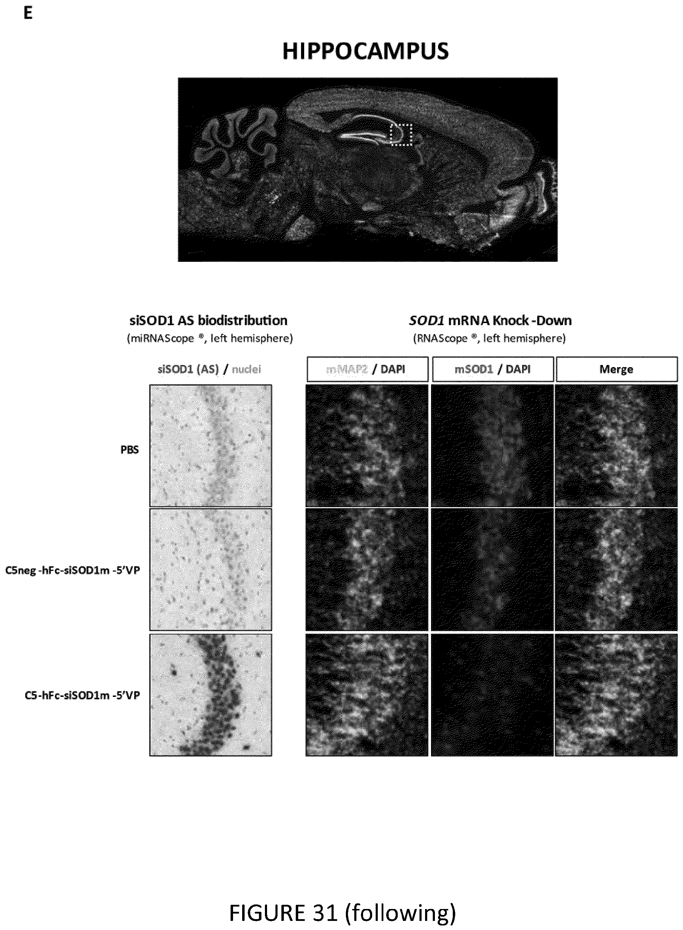

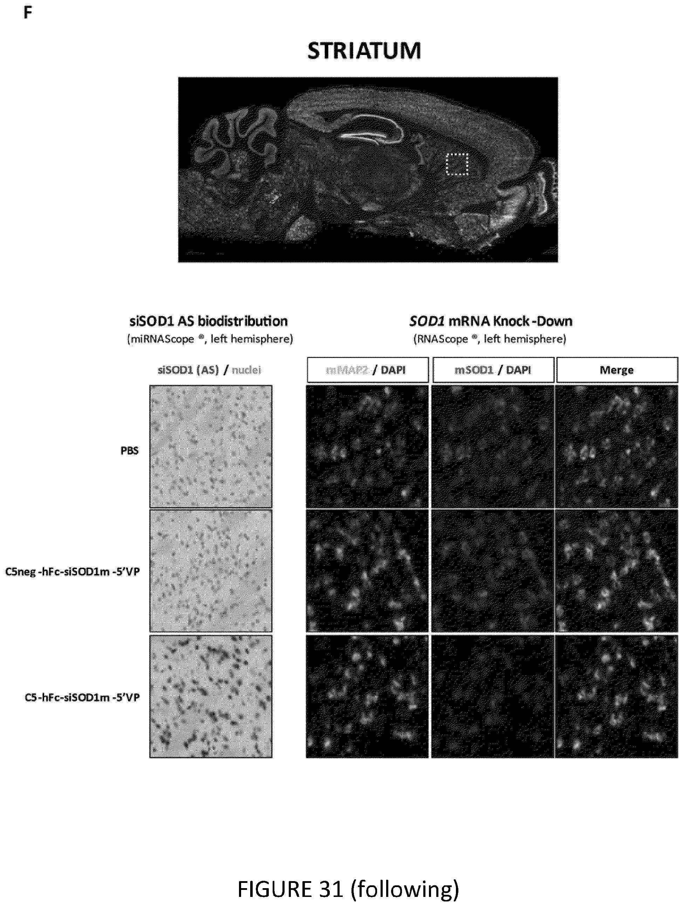

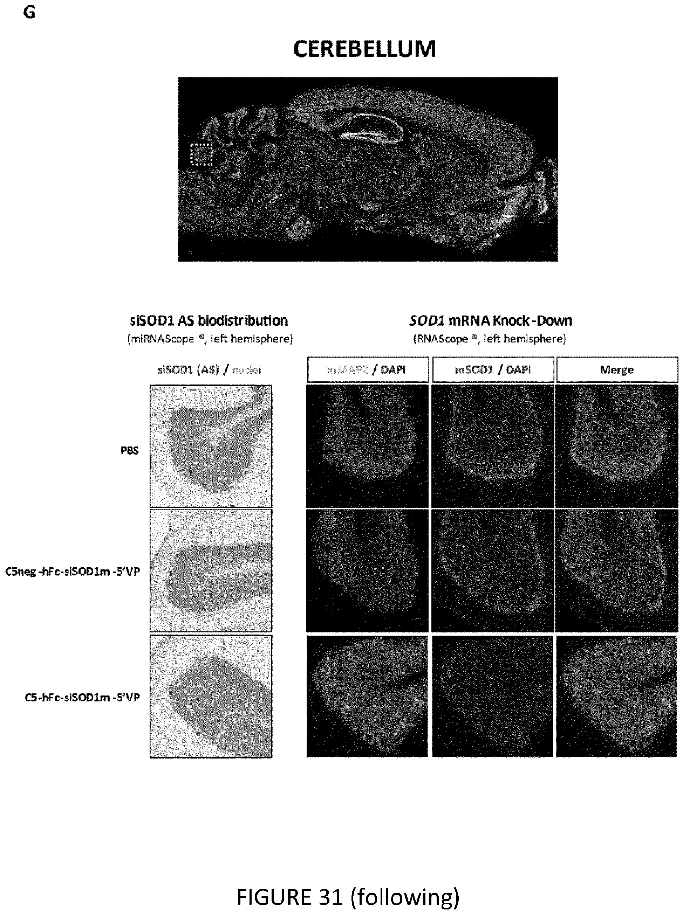

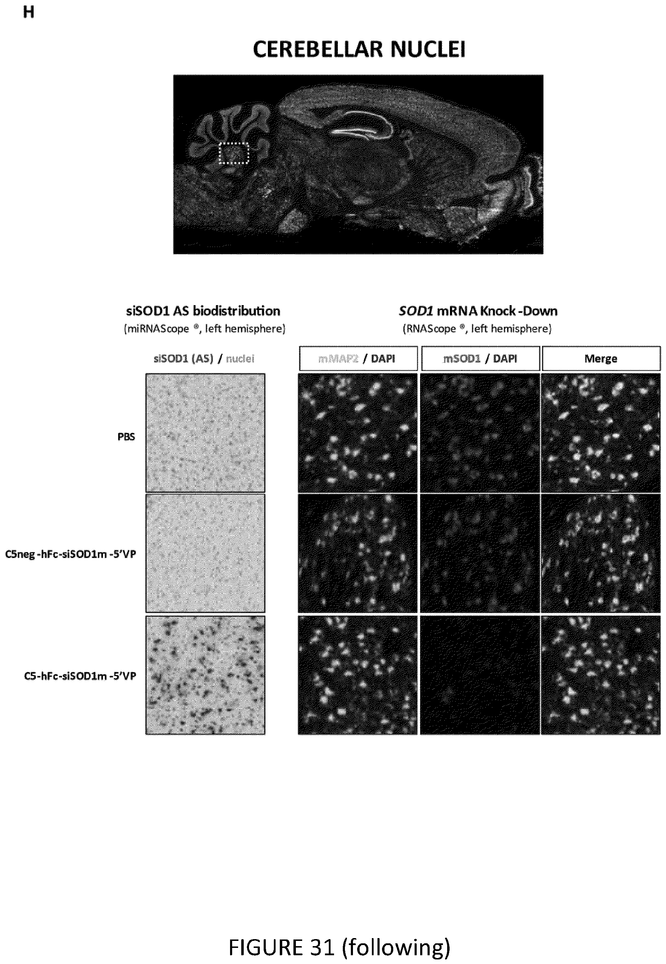

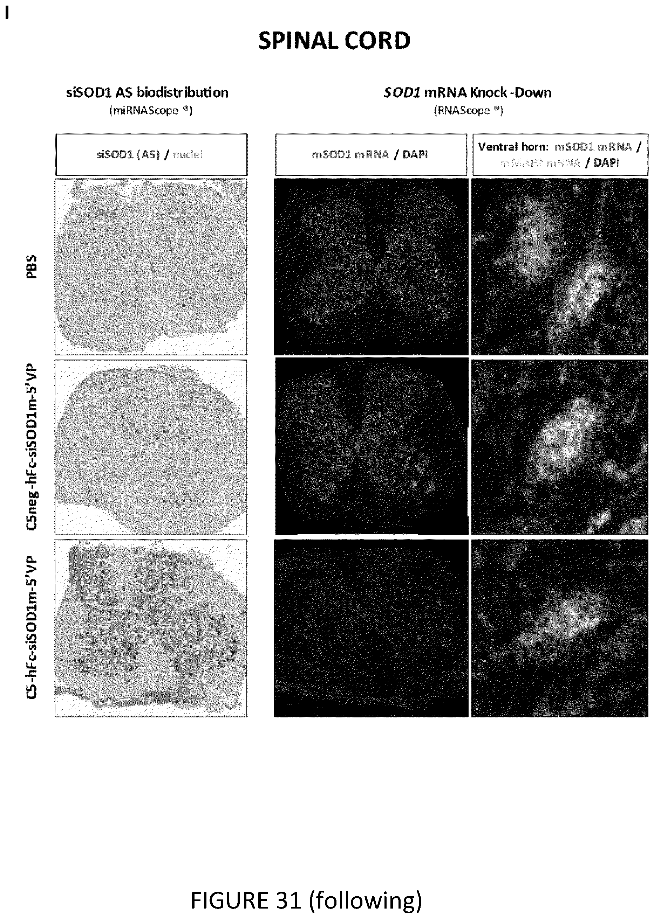

**Figure 31****: CNS knock-down effect and siRNA biodistribution after systemic administration of VHH-hFc-siRNA conjugates.** TfR-dependent CNS knock-down of murine *SOD1* mRNA, as assessed using either RT-qPCR on right hemibrains and spinal cords or using *in situ* hybridization on left hemibrains and part of the lumbar spinal cords, and siSOD1m-5'VP biodistribution on left hemibrains and part of the lumbar spinal cords using *in situ* hybridization, after repeated IV administration of PBS, a TfR-binding VHH-hFc-siSOD1m-5'VP conjugate (wherein VHH is C5) vs. a non-binding VHHneg-hFc-siSOD1m-5'VP conjugate (wherein VHHneg is C5neg) at a total dose of 4.5 mg/kg (siRNA molar equivalent) in wild-type C57B1/6 mice. Figures 31C and 31I show the general brain (31C, sagittal sections) and spinal cord (311, coronal sections) distribution of the siSOD1m-5'VP antisense strand (miRNAScope^{®}, left panels), correlating to broad downregulation of murine *SOD1* mRNA levels (RNAScope^{®}, right panels). Figures 31D-I show zooms and neuron-specific co-staining, using a *Map2* mRNA-specific probe, in different regions of the brain (31D-H, sagittal sections) and spinal cord (311, coronal sections).

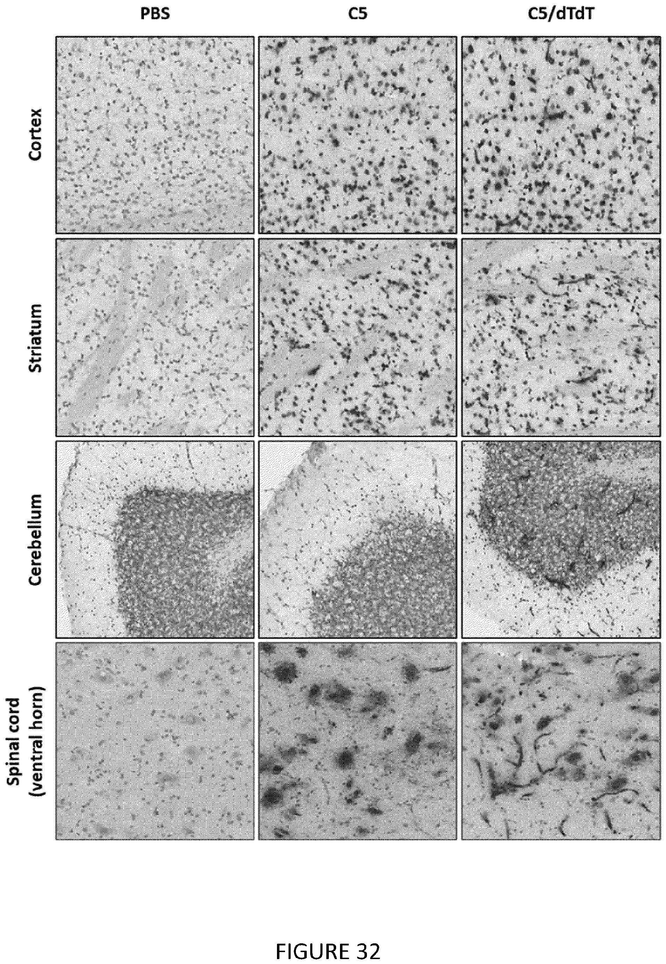

**Figure 32****: Differential biodistribution.** Differential siSOD1m-5'VP biodistribution within microvessels vs. parenchyma in brain and spinal cord following repeated IV administration in wild-type C57Bl/6 mice of PBS or TfR-binding VHH-hFc-siSOD1m-5'VP conjugates (wherein VHH is C5) comprising either a stable linker or a nuclease-sensitive linker (dTdT) between the siRNA 3'SS and the VHH-hFc moiety.

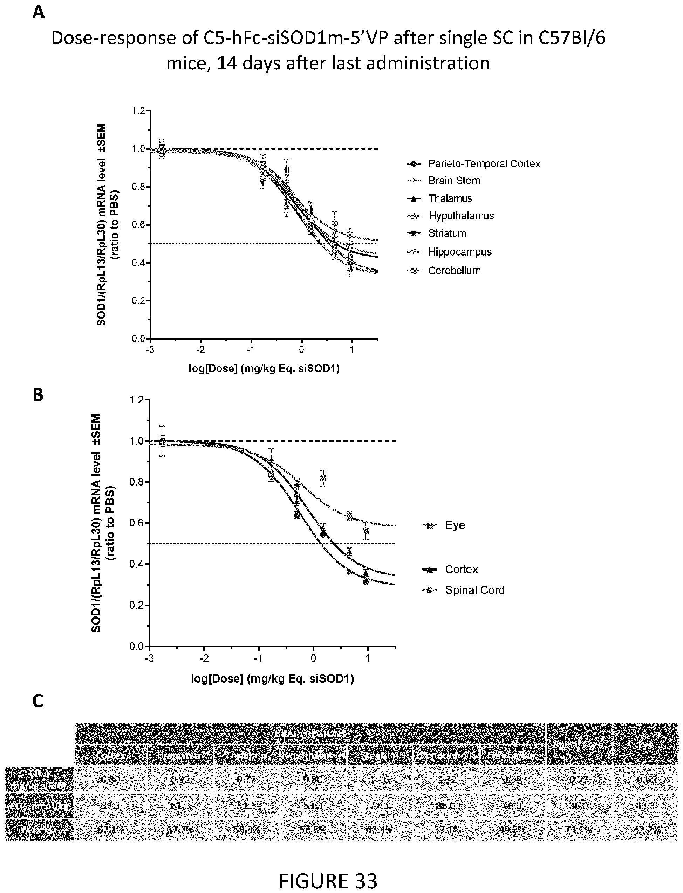

**Figure 33****: Dose-response of the CNS knock-down effect of VHH-hFc-siRNA conjugates.** Dose-dependent downregulation of murine *SOD1* mRNA levels in CNS tissues and brain regions of wild-type C57Bl/6 mice following single subcutaneous administration of a TfR-binding VHH-hFc-siSOD1m-5'VP conjugate (wherein VHH is C5), with estimated ED50 values and maximal downregulation effect (Max KD).

### Detailed description of the invention

The present invention provides novel TfR-binding agents which can be used to transport molecules, such as therapeutic, imaging or diagnostic agents, across the BBB. More particularly, the invention discloses improved VHH molecules which bind TfR, and the uses thereof.

The TfR is involved in the incorporation of iron, transported by its transferrin ligand, and in the regulation of cell growth (Neckers and Trepel 1986). There are two types of transferrin receptors: the TfR1 receptor and a homologous receptor, TfR2, expressed primarily in the liver. In the context of the invention, the term TfR is used to designate the TfR1 homologue. TfR is a type II homodimeric transmembrane glycoprotein consisting of two identical 90 kDa subunits linked by two disulfide bridges (Jing and Trowbridge 1987, McClelland et al., 1984). Each monomer has a short cytoplasmic N-terminal domain of 61 amino acids containing a YTRF (Tyrosine-Threonine-Arginine-Phenylalanine) internalization motif, a single hydrophobic transmembrane segment of 27 amino acids, and a broad C-terminal extracellular domain of 670 amino acids, containing a trypsin cleavage site and a transferrin binding site (Aisen, 2004). Each subunit is capable of binding a transferrin molecule. The extracellular domain has one O-glycosylation site and three N-glycosylation sites, the latter being particularly important for the proper folding and transport of the receptor to the cell surface (Hayes et al., 1997). There are also palmitoylation sites in the intramembranous domain, that presumably anchor the receptor and allow its endocytosis (Alvarez et al., 1990, Omary and Trowbridge, 1981). In addition, an intracellular phosphorylation site is present, whose functions are uncertain, and which plays no role in endocytosis (Rothenberger et al., 1987).

The TfR receptor is expressed at high level by highly proliferating cells, whether healthy or neoplastic (Gatter et al., 1983). Many studies have shown high levels of TfR expression in cancer cells compared to healthy cells. Thus, pathologies such as breast cancer (Yang et al., 2001), gliomas (Prior et al., 1990), pulmonary adenocarcinoma (Kondo et al., 1990), chronic lymphocytic leukemia (Das Gupta and Shah, 1990) or non-Hodgkin's lymphoma (Habeshaw et al., 1983) show increased TfR expression, correlated with tumor grade and stage of disease or prognosis. Targeting drugs to TfR may thus be suitable for cancer treatment, as well as for crossing the BBB.

Using purified membrane preparations from cells expressing high levels of hTfR and mTfR, the inventors generated and selected VHH molecules, in particular the VHH molecules that bind both the human and non-human TfR. The inventors showed that when fused to a human IgG1 Fc region or drug (such as an antibody, siRNA) or imaging agent, these VHH molecules retain TfR binding capacity, transmigrate across an *in vitro* BBB model, and demonstrate brain-targeting properties *in vivo.* They also showed that when fused to an oligonucleotide such as siRNA or ASO or to NODAGA scaffold, these VHH molecules retain TfR binding capacity and efficient delivery *in vivo.* The VHH molecules exhibit appropriate levels of affinity and specificity to undergo proper endocytosis following TfR binding. The invention thus provides novel TfR-binding molecules which represent valuable agents for drug targeting.An object of the invention thus relates to VHH molecules, wherein said VHH molecules bind a human, non-human primate and/or rodent (such as rat or murine) TfR. Preferably, the VHH can cross the human BBB or bind TfR-expressing tissues such as cancers. The invention also relates to chimeric agents comprising such VHH, their manufacture, compositions comprising the same and the use thereof.

### Definitions

Unless otherwise defined herein, all scientific and technical terms used in connection with the present invention have the same meaning as commonly understood by one of ordinary skill in the art. As used herein, the term "treatment", "treat" or "treating" refers to any act intended to ameliorate the health status of patients such as therapy, prevention, prophylaxis and retardation of a disease. The treated disease may be any disease, and in particular, a CNS disease, a neurological disease or a neurodegenerative disease, e.g., Alzheimer's disease, Parkinson's disease, stroke, Creutzfeldt-Jakob disease, bovine spongiform encephalopathy, multiple sclerosis, amyotrophic lateral sclerosis, epilepsy, migraine, encephalitis, CNS pain or glioblastoma; or a cancer. It designates both a curative treatment and/or a prophylactic treatment of a disease. In the context of cancer, this includes, inter alia, the alleviation of symptoms, the reduction of inflammation, the inhibition of cancer cell growth, and/or the reduction of tumor size. For example, in the case of cancer, a response to treatment includes a reduction in cachexia, increase in survival time, elongation in time to tumor progression, reduction in tumor mass, reduction in tumor burden and/or a prolongation in time to tumor metastasis, time to tumor recurrence, tumor response, complete response, partial response, stable disease, progressive disease, progression free survival, overall survival, each as measured for example by standards set by the National Cancer Institute and the U.S. Food and Drug Administration for the approval of new drugs (Johnson et al., J. Clin. Oncol., 2009; 21(7): 1404-1411).

A "therapeutically effective dose" as described herein refers to the dose that gives a therapeutic effect for a given condition and administration schedule. It is typically the average dose of an active substance to administer to appreciably improve some of the symptoms associated with a disease or a pathological state. For example, in treating a cancer of the brain or of other tissue, a pathology, a lesion or a disorder of the CNS, the dose of an active substance that decreases, prevents, delays, eliminates or stops one of the causes or symptoms of the disease or disorder would be therapeutically effective. A "therapeutically effective dose" of an active substance does not necessarily cure a disease or disorder but will provide a treatment for this disease or disorder so that its appearance is delayed, impeded or prevented, or its symptoms are attenuated, or its term is modified or, for example, is less severe, or the recovery of the patient is accelerated.

It is understood that the "therapeutically effective dose" for a person in particular will depend on various factors, including the activity/effectiveness of the active substance, its time of administration, its route of administration, its toxicity, its rate of elimination and its metabolism, drug combinations/interactions and the severity of the disease (or disorder) treated on a preventive or curative basis, as well as the age, weight, overall health, sex and/or diet of the patient. "VHH molecules" as used herein correspond to the variable region of heavy chain only camelid antibodies that are naturally devoid of light chains. VHH have a very small molecular weight of around 12-15 kDa. They contain a single chain molecule that can bind its cognate antigen using a single domain. The antigen-binding surfaces of VHHs are usually more convex (or protruding) than those of conventional antibodies, which are usually flat or concave. More specifically, VHHs are composed of 4 Framework Regions (or FRs) whose sequences and structures are defined as conserved, and three Complementarity Determining Regions (or CDRs) showing high variability both in sequence content and structure conformation, which are involved in antigen binding and provide antigen specificity. Compared to conventional human antibody VH, a few amino acids are substituted in the FR2 region and complementarity-determining regions (CDRs) of VHH. For instance, highly conserved hydrophobic amino acids (such as Val42, Gly49, Leu50, and/or Trp52) in FR2 region are often replaced by hydrophilic amino acids (Phe42, Glu49, Arg50, Gly52), rendering the overall structure more hydrophilic and contributing to high stability, solubility and resistance to aggregation.

VHH molecules according to the present invention are polypeptides comprising (or consisting of, or consisting essentially of) an antigen-binding domain of a heavy chain only antibody (HcAb).

The term "and/or" as used herein is to be taken as specific disclosure of each of the two specified features or components with or without the other. For example, "A and/or B" is to be taken as specific disclosure of each of (i) A, (ii) B and (iii) A and B, just as if each is set out individually.

The term "a" or "an" can refer to one of or a plurality of the elements it modifies (e.g., "a VHH molecule" can mean one or more VHH molecules) unless it is contextually clear either one of the elements or more than one of the elements is described.

### VHH molecules

In order to generate VHH molecules having suitable properties, the inventors tested over 2000 TfR-binding VHH from a library of VHH produced by lama immunization with a TfR immunogen. Following analysis of said clones for binding and specificity, the inventors further selected about 450 clones which were all sequenced and compared. Further VHH with controlled/improved binding properties were produced by mutagenesis or by humanization. The sequences of the relevant domains and preferred VHH are provided in the experimental section and sequence listing. The properties of the VHH and conjugates thereof are also illustrated in the experimental section.

VHH molecules of the invention typically comprise or consist of the formula: FR1-CDR1-FR2-CDR2-FR3-CDR3-FR4, wherein FRn designates framework regions and CDRn designates complementarity determining regions.

In a particular embodiment, VHH molecules of the invention comprise a CDR1 domain comprising or consisting of an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 or 711, or variants thereof having at least 60%, in particular at least 65%, 70% or 75%, for example at least 80% or 85% amino acid identity to any one of said sequences over the entire length thereof, preferably at least 85%, 86%, 87%, 88%, 89%, 90%, 91%, 92%, 93%, 94%, 95%, 96%, 97%, 98% or 99% (the preferred percentages of identity for a particular sequence being preferably percentages corresponding to an integer number of amino acids), said variants retaining a TfR binding capacity. Preferred VHH molecules of the invention contain a CDR1 domain having an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710, 711, or variants thereof having several amino acid modifications, for example at least 3 amino acid modifications, preferably at most 3 or 2 amino acid modifications, in a particular aspect at most 1 amino acid modification.

The "% identity" between amino acid (or nucleic acid) sequences may be determined by techniques known per se in the art. Typically, the % identity between two nucleic acid or amino acid sequences is determined by means of computer programs such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). The % identity between two sequences designates the identity over the entire length of said sequences. As indicated above, the preferred percentages of identity for a particular sequence are preferably percentages corresponding to an integer number of amino acids both in the reference sequence (which is for example SEQ ID NOs: 1, 5, 9, 13, 17, 19, 67, 69, 125, 175, 179, 182, 184, 186, 190, 194, 198, 201, 205, 392, 410, 413, 426, 434 or 437, 607, 610, 671-674, 710, 711, or any other reference sequences herein identified such as SEQ ID NOs: 2, 6, 10, 14, 21, 23, 71, 73, 75, 113, 115, 128, 160, 162, 164, 166, 169, 171, 176, 187, 191, 195, 199, 202, 206, 608, 611, 712, or 3, 7, 11, 15, 25, 27, 29, 31, 33, 77, 79, 81, 83, 85, 117, 119, 121, 123, 177, 180, 188, 192, 196, 200, 203, 207, 609, 612 or 713-715 and in the variant thereof.

Specific examples of VHH molecules of the invention comprise a CDR1 sequence comprising, or consisting essentially of SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 or 711.

In a further particular embodiment, VHH molecules of the invention comprise a CDR2 domain comprising or consisting of an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, said variants retaining a TfR binding capacity. Preferred VHH molecules of the invention contain a CDR2 domain having an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712, or variants thereof having several amino acid modifications, for example at least 3 amino acid modifications, preferably at most 3 or 2 amino acid modifications, in a particular aspect at most 1 amino acid modification.

Specific examples of VHH molecules of the invention comprise a CDR2 sequence comprising, or consisting essentially of SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712.

In a further particular embodiment, VHH molecules of the invention comprise a CDR3 domain comprising or consisting of an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715, or variants thereof having at least 60%, in particular at least 65%, 70% or 75%, for example at least 80% or 85%, amino acid identity to any one of said sequences over the entire length thereof, preferably at least 80%, more preferably at least 85%, 86%, 87%, 88%, 89%, 90%, 91%, 92%, 93%, 94%, 95%, 96%, 97%, 98% or 99%, said variants retaining a TfR binding capacity. Preferred VHH molecules of the invention contain a CDR3 domain having an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612, or 713-715, or variants thereof having several amino acid modifications, for example at least 3 amino acid modifications, preferably at most 3 or 2 amino acid modifications, in a particular aspect at most 1 amino acid modification.

Specific examples of VHH molecules of the invention comprise a CDR3 sequence comprising, or consisting essentially of SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715.

In a further particular embodiment, VHH molecules of the invention comprise:
. a CDR1 domain comprising or consisting of an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 or 711, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
. a CDR2 domain comprising or consisting of an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 416, 419, 431, 206, 608, 611 or 712, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
. a CDR3 domain comprising or consisting of an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715, or variants thereof having at least 60%, in particular at least 65%, 70% or 75%, for example at least 80% or 85%, amino acid identity to any one of said sequences over the entire length thereof, preferably at least 80%, 85%, 86%, 87%, 88%, 89%, 90%, 91%, 92%, 93%, 94%, 95%, 96%, 97%, 98% or 99%, more preferably at least 95%,
said VHH having a TfR-binding capacity.

In a further particular embodiment, VHH molecules of the invention comprise:
- a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710 and 711, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
- a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611 and 712, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
- a CDR3 comprising an amino acid sequence selected from SEQ ID NOs: 3, 7, 11, 117, 119, 121, 123, 177, 180, 188, 192, 196, 200, 203, 207, 452, 455, 609, 612 and 713-715, or variants thereof having at least 60%, in particular at least 65%, 70% or 75%, for example at least 80% or 85%, amino acid identity to any one of said sequences over the entire length thereof, preferably at least 80%, 85%, 86%, 87%, 88%, 89%, 90%, 91%, 92%, 93%, 94%, 95%, 96%, 97%, 98% or 99%, more preferably at least 95%,
said VHH having a TfR-binding capacity,
with the proviso that:
. when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
. when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
. when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.

In a preferred embodiment, the VHH molecules of the invention comprise:
. a CDR1 domain having an amino acid sequence selected from the group consisting of SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710, 711 and variants thereof having at most 3, 2 or 1 amino acid modifications; and
. a CDR2 domain having an amino acid sequence selected from the group consisting of SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611, 712 and variants thereof having at most 3, 2 or 1 amino acid modifications; and
. a CDR3 domain having an amino acid sequence selected from the group consisting of SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612, 713-715 and variants thereof having at most 3, 2 or 1 amino acid modifications.

In another preferred embodiment, the VHH molecules of the invention comprise:
- a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710, 711, and variants thereof having at most 3, 2 or 1 amino acid modifications; and
- a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611, 712, and variants thereof having at most 3, 2 or 1 amino acid modifications; and
- a CDR3 comprising an amino acid sequence selected from SEQ ID NOs: 3, 7, 11, 117, 119, 121, 123, 177, 180, 188, 192, 196, 200, 203, 207, 452, 455, 609, 612, 713-715 and variants thereof having at most 3, 2 or 1 amino acid modifications.
with the proviso that:
. when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
. when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
. when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.

In a more preferred embodiment, the VHH molecules of the invention comprise a CDR1, a CDR2 and a CDR3, wherein said CDR1, CDR2 and CDR3 domains comprise or consist of, respectively:
SEQ ID NOs: 1, 2 and 3; or
SEQ ID NOs: 17, 2 and 3; or
SEQ ID NOs: 19, 2 and 3; or
SEQ ID NOs: 67, 2 and 3; or
SEQ ID NOs: 69, 2 and 3; or
SEQ ID NOs: 1, 21 and 3; or
SEQ ID NOs: 1, 23 and 3; or
SEQ ID NOs: 1, 71 and 3; or
SEQ ID NOs: 1, 73 and 3; or
SEQ ID NOs: 1, 75 and 3; or
SEQ ID NOs: 1, 2 and 25; or
SEQ ID NOs: 1, 2 and 27; or
SEQ ID NOs: 1, 2 and 29; or
SEQ ID NOs: 1, 2 and 31; or
SEQ ID NOs: 1, 2 and 33; or
SEQ ID NOs: 1, 2 and 77; or
SEQ ID NOs: 1, 2 and 79; or
SEQ ID NOs: 1, 2 and 81; or
SEQ ID NOs: 1, 2 and 83; or
SEQ ID NOs: 1, 2 and 85; or
. SEQ ID NOs: 5, 6 and 7; or
. SEQ ID NOs: 9, 10 and 11; or
. SEQ ID NOs: 13, 14 and 15, or
. SEQ ID NOs: 392, 2 and 3; or
. SEQ ID NOs: 1, 113 and 3; or
. SEQ ID NOs: 1, 115 and 3; or
. SEQ ID NOs: 1, 2 and 117; or
. SEQ ID NOs: 1, 2 and 119; or
. SEQ ID NOs: 1, 2 and 121; or
. SEQ ID NOs: 1, 2 and 123; or
. SEQ ID Nos: 125, 2 and 3; or
. SEQ ID NOs: 17, 73 and 3; or
. SEQ ID NOs: 17, 128 and 3; or
. SEQ ID NOs: 5, 160 and 7; or
. SEQ ID NOs: 5, 162 and 7; or
. SEQ ID NOs: 5, 164 and 7; or
. SEQ ID NOs: 5, 166 and 7; or
. SEQ ID NOs: 9, 169 and 11; or
. SEQ ID NOs: 9, 171 and 11; or
. SEQ ID NOs: 175, 176 and 177; or
. SEQ ID NOs: 179, 176 and 180; or
. SEQ ID NOs: 182, 176 and 177; or
. SEQ ID NOs: 184, 176 and 177; or
. SEQ ID NOs: 186, 187 and 188; or
. SEQ ID NOs: 190, 191 and 192; or
. SEQ ID NOs: 194, 195 and 196; or
. SEQ ID NOs: 198, 199 and 200; or
. SEQ ID NOs: 201, 202 and 203; or
. SEQ ID NOs: 205, 206 and 207, or
. SEQ ID NOs: 410, 6 and 7; or
. SEQ ID NOs: 413, 6 and 7; or
. SEQ ID NOs: 5, 416 and 7; or
. SEQ ID NOs: 5, 419 and 7; or
. SEQ ID NOs: 426, 6 and 7; or
. SEQ ID NOs: 5, 431 and 7; or
. SEQ ID NOs: 434, 6 and 7; or
. SEQ ID NOs: 437, 6 and 7; or
. SEQ ID NOs: 5, 6 and 452; or
. SEQ ID NOs: 5, 6 and 455; or
. SEQ ID NOs: 607, 608 and 609; or
. SEQ ID NOs: 610, 611 and 612, or
. SEQ ID NOs: 671, 2 and 3; or
. SEQ ID NOs: 672, 2 and 3; or
. SEQ ID NOs: 673, 6 and 7; or
. SEQ ID NOs: 674, 6 and 7; or
. SEQ ID NOs: 1, 2 and 713; or
. SEQ ID NOs: 5, 6 and 714; or
. SEQ ID NOs: 674, 164 and 7; or
. SEQ ID NOs: 710, 6 and 7; or
. SEQ ID NOs: 5, 6 and 715; or
. SEQ ID NOs: 674, 712 and 7; or
. SEQ ID NOs: 711, 6 and 7; or variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of C5 or of variants thereof (as listed in Table 1), comprising or consisting of, respectively:
. SEQ ID NOs: 1, 2 and 3; or
. SEQ ID NOs: 13, 14 and 15; or
. SEQ ID NOs: 17, 2 and 3; or
. SEQ ID NOs: 19, 2 and 3; or
. SEQ ID NOs: 67, 2 and 3; or
. SEQ ID NOs: 69, 2 and 3; or
. SEQ ID NOs: 1, 21 and 3; or
. SEQ ID NOs: 1, 23 and 3; or
. SEQ ID NOs: 1, 71 and 3; or
. SEQ ID NOs: 1, 73 and 3; or
. SEQ ID NOs: 1, 75 and 3; or
. SEQ ID NOs: 1, 2 and 25; or
. SEQ ID NOs: 1, 2 and 27; or
. SEQ ID NOs: 1, 2 and 29; or
. SEQ ID NOs: 1, 2 and 31; or
. SEQ ID NOs: 1, 2 and 33; or
. SEQ ID NOs: 1, 2 and 77; or
. SEQ ID NOs: 1, 2 and 79; or
. SEQ ID NOs: 1, 2 and 81; or
. SEQ ID NOs: 1, 2 and 83; or
. SEQ ID NOs: 1, 2 and 85; or
. SEQ ID NOs: 392, 2 and 3; or
. SEQ ID NOs: 1, 113 and 3; or
. SEQ ID NOs: 1, 115 and 3; or
. SEQ ID NOs: 1, 2 and 117; or
. SEQ ID NOs: 1, 2 and 119; or
. SEQ ID NOs: 1, 2 and 121; or
. SEQ ID NOs: 1, 2 and 123; or
. SEQ ID NOs: 125, 2 and 3; or
. SEQ ID NOs: 17, 73 and 3; or
. SEQ ID NOs: 17, 128 and 3; or
. SEQ ID NOs: 671, 2 and 3; or
. SEQ ID NOs: 672, 2 and 3; or
. SEQ ID NOs: 1, 2 and 713; or
variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of B6 (as listed in Table 1), comprising or consisting of, respectively:
. SEQ ID NOs: 9, 10 and 11; or
. SEQ ID NOs: 9, 169 and 11; or
. SEQ ID NOs: 9, 171 and 11, or
variants thereof as defined above, preferably variants having at most 3, 2 or 1 amino acid modifications.

In a particular embodiment, the VHH molecules of the invention are B6 or variants thereof, that target the apical domain of the hTfR and comprise a CDR1, a CDR2 and a CDR3, comprising or consisting of, respectively:
. SEQ ID NOs: 9, 10 and 11; or
. SEQ ID NOs: 9, 169 and 11; or
. SEQ ID NOs: 9, 171 and 11; or
. SEQ ID NOs: 175, 176 and 177; or
. SEQ ID NOs: 179, 176 and 180; or
. SEQ ID NOs: 182, 176 and 177; or
. SEQ ID NOs: 184, 176 and 177; or
variants thereof as defined above, preferably variants having at most 3, 2 or 1 amino acid modifications.

In another particular embodiment, the VHH molecules of the invention are VHH molecules targeting the apical domain of the hTfR and comprising a CDR1, a CDR2 and a CDR3 (as listed in Table 1), comprising or consisting of, respectively:
. SEQ ID NOs: 9, 10 and 11; or
. SEQ ID NOs: 9, 169 and 11; or
. SEQ ID NOs: 9, 171 and 11; or
. SEQ ID NOs: 175, 176 and 177; or
. SEQ ID NOs: 179, 176 and 180; or
. SEQ ID NOs: 182, 176 and 177; or
. SEQ ID NOs: 184, 176 and 177; or
. SEQ ID NOs: 186, 187 and 188; or
. SEQ ID NOs: 190, 191 and 192; or
. SEQ ID NOs: 194, 195 and 196; or
. SEQ ID NOs: 198, 199 and 200; or
. SEQ ID NOs: 201, 202 and 203; or
. SEQ ID NOs: 205, 206 and 207, or
variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of B8 or of variants thereof (as listed in Table 1), comprising or consisting of, respectively:
. SEQ ID NOs: 5, 6 and 7; or
. SEQ ID NOs: 5, 160 and 7; or
. SEQ ID NOs: 5, 162 and 7; or
. SEQ ID NOs: 5, 164 and 7; or
. SEQ ID NOs: 5, 166 and 7; or
. SEQ ID NOs: 410, 6 and 7; or
. SEQ ID NOs: 413, 6 and 7; or
. SEQ ID NOs: 5, 416 and 7; or
. SEQ ID NOs: 5, 419 and 7; or
. SEQ ID NOs: 426, 6 and 7; or
. SEQ ID NOs: 5, 431 and 7; or
. SEQ ID NOs: 434, 6 and 7; or
. SEQ ID NOs: 437, 6 and 7; or
. SEQ ID NOs: 5, 6 and 452; or
. SEQ ID NOs: 5, 6 and 455, or
. SEQ ID NOs: 673, 6 and 7; or
. SEQ ID NOs: 674, 6 and 7; or
. SEQ ID NOs: 5, 6 and 714; or
. SEQ ID NOs: 674, 164 and 7; or
. SEQ ID NOs: 710, 6 and 7; or
. SEQ ID NOs: 5, 6 and 715; or
. SEQ ID NOs: 674, 712 and 7; or
. SEQ ID NOs: 711, 6 and 7; or
variants thereof as defined above, preferably variants having at most 3, 2 or 1 amino acid modifications.

In another particular embodiment, the VHH molecules of the invention are cross-reactive with human, mouse and/or non-human primate species, as detailed in Tables 5 and 6 below.

Preferred VHH molecules of the invention comprise FRs domains as defined below.

In a particular embodiment, the FR1 domain comprises or consists of SEQ ID NO: 35 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 80%: EVQLVESGGGLVQPGGSLKLSCAAS (SEQ ID NO: 35). More preferably, the bold amino acid residues are present and the variability occurs only on the other positions.

In a specific embodiment, the E in position 1 may be replaced with Q.

In a specific embodiment, the V in position 5 may be replaced with Q.

In a specific embodiment, the E in position 6 may be replaced with Q.

In a specific embodiment, the G in position 10 may be replaced with K or A.

In a specific embodiment, the L in position 11 may be replaced with V or E.

In a specific embodiment, the P in position 14 may be replaced with A.

In a specific embodiment, the G in position 16 may be replaced with D.

In another specific embodiment, the K in position 19 may be replaced with R.

In a specific embodiment, the A in position 23 may be replaced with V or T.

In another specific embodiment, the S in position 25 may be replaced with D.

More preferably, the FR1 contains at most 4 amino acid modifications by reference to this sequence, even more preferably at most 3, even more preferably at most 2 amino acid modifications in non-bold amino acid residues. In a preferred embodiment, the amino acid modification is R in position 19.

In a further specific embodiment, the FR1 has an amino acid sequence selected from any one of the amino acid sequences listed below:
EVQLVESGGGVVQPGGSLKLSCVAS (SEQ ID NO: 36);
EVQLVESGGGVVQPGGSLRLSCAAS (SEQ ID NO: 37);
EVQLVESGGGLVQPGGSLRLSCTAS (SEQ ID NO: 38); or
EVQLVESGGGEVQPGGSLKLSCVAS (SEQ ID NO: 39); or variants thereof.

Further specific examples of the FR1 of VHH molecules according to the invention are provided below (see also Table 3):
EVQLVESGGGVVQPGGSLKLSCAAS (SEQ ID NO: 331),
EVQLVESGGGLVQPGGSLRLSCAAS (SEQ ID NO: 332),
EVQLVESGGGVVQPGGSLRLSCAAD (SEQ ID NO: 333),
EVQLVESGGGVVQPGGSLRLSCVAS (SEQ ID NO: 400),
QVQLVQSGGGLVQAGGSLTLSCTAS (SEQ ID NO: 334),
EVQLVESGGGLVQAGGSLRLSCTAS (SEQ ID NO: 335),
QVQLVQSGGGLVQPGGSLRLSCAAS (SEQ ID NO: 336),
EVQLVESGGGLVQAGDSLRLSCTAS (SEQ ID NO: 337),
QVQLVQSGGGLVQAGGSLRLSCAAS (SEQ ID NO: 338),
EVQLVQSGGGLVQAGGSLRLSCAAS (SEQ ID NO: 339),
EVQLVESGGGLVQPGESLRLSCTAS (SEQ ID NO: 340),
EVQLVESGGGLVQPGGSLRLSCVSS (SEQ ID NO: 341),
EVQLVESGGGLVQAGDSLRLSCAAS (SEQ ID NO: 619), or
VQLVESGGRLVQAGGSLRLSCTAS (SEQ ID NO: 620).

In a particular embodiment, VHH molecules of the invention comprise a FR2 domain comprising or consisting of SEQ ID NO: 40 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof: MRWYRQAPGKQRELVAT (SEQ ID NO: 40). More preferably, the bold amino acid residues are present and the variability occurs only on the other positions.

In a specific embodiment, the M in position 1 may be replaced with I or V.

In a specific embodiment, the R in position 2 may be replaced with G or H or S.

In a specific embodiment, the Y in position 4 may be replaced with F or V.

In a specific embodiment, the R in position 5 may be replaced with G.

In a specific embodiment, the Q in position 6 may be replaced with R or E.

In a specific embodiment, the A in position 7 may be replaced with R.

In a specific embodiment, the G in position 9 may be replaced with I or E.

In a specific embodiment, the Q in position 11 may be replaced with E or G or I or D.

In a specific embodiment, the R in position 12 may be replaced with L.

In a specific embodiment, the E in position 13 may be replaced with N or H.

In a specific embodiment, the L in position 14 may be replaced with F or W or S or Q.

In a specific embodiment, the V in position 15 may be replaced with Q or I.

In a specific embodiment, the A in position 16 may be replaced with M.

In a specific embodiment, the T in position 17 may be replaced with G or S.

More preferably, the FR2 contains at most 6 amino acid modifications by reference to this sequence, even more preferably at most 5, at most 3, even more preferably at most 2 amino acid modifications in non-bold amino acid residues. In preferred embodiments, the amino acid modifications are V in position 4 and/or G in position 11 and L in position 12 and/or W in position 14.

In a particular embodiment, VHH molecules of the invention comprise at least one of the following amino acids in the FR2 domain: Phe42, Glu49, Arg50 or Gly52 (according to IMGT numbering).

In a further specific embodiment, the FR2 has an amino acid sequence selected from any one of the amino acid sequences listed below:
IRWYRQAPGKQREFVAG (SEQ ID NO: 41);
MRWYRQAPGKQREWVAG (SEQ ID NO: 42);
MGWFRRAPGKERELVAS (SEQ ID NO: 43);
VRWYRQRPGKQREWVAG (SEQ ID NO: 44); or variants thereof.

Further specific examples of the FR2 of VHH molecules according to the invention are provided below (see also Table 3):
IRWVRQAPGKGLEWVAG (SEQ ID NO: 342),
IRWYRQAPGKGLEFVAG (SEQ ID NO: 343),
IRWVRQAPGKGLEFVAG (SEQ ID NO: 344),
IRWYRQAPGKGREFVAG (SEQ ID NO: 345),
IRWVRQAPGKQREFVAG (SEQ ID NO: 346),
IRWYRQAPGKGLEWVAG (SEQ ID NO: 347),
MRWYRQAPGKGLEWVAG (SEQ ID NO: 348),
MRWYGQAPGKQREWVAG (SEQ ID NO: 349),
MRWYREAPGKQREWVAG (SEQ ID NO: 350),
MRWYRQAPIKQREWVAG (SEQ ID NO: 351),
MRWYRQAPGKIREWVAG (SEQ ID NO: 352),
MGWFRRAPGKERNLVAS (SEQ ID NO: 353),
MGWFRRAPGKERESVAS (SEQ ID NO: 354),
MGWFRRAPGKERELQAS (SEQ ID NO: 355),
MGWFRRAPEKERELVAS (SEQ ID NO: 356),
MGWFRRAPGKDRELVAS (SEQ ID NO: 357),
MSWVRQAPGKGRELVAS (SEQ ID NO: 358),
MGWFRRAPGKERELIAS (SEQ ID NO: 359),
LAWHRQIPGKEREWVAG (SEQ ID NO: 360),
MAWHRQAPGKERLWVAG (SEQ ID NO: 361),
VGWYRQAPGEQRVLVAH (SEQ ID NO: 362),
MGWFRQAPGKEREFVAA (SEQ ID NO: 363),
MGWYRQAPGKQRELVAV (SEQ ID NO: 364),
MGWFRQTPGKEREFVAA (SEQ ID NO: 365),
MRWYRQAPGKQREQVAG (SEQ ID NO: 458),
MRWYRQAPGKQREFVAG (SEQ ID NO: 459),
MRWYRQAPGKQRHWVAG (SEQ ID NO: 460),
MIWYRQAPGKQREWVAG (SEQ ID NO: 461),
MEWYRQAPGKQREWVAG (SEQ ID NO: 462),
MRWYRQAPGKQREWVAA (SEQ ID NO: 463),
MRWYRQAPGKQREWVAK (SEQ ID NO: 464),
IGWFRQAPGKEREKVSC (SEQ ID NO: 621), or
MHWFRQAPGKEREFVGA (SEQ ID NO: 622), or
IRWYSQAPGKQREFVAG (SEQ ID NO: 716), or
MRWYRQAPGKQREWVSG (SEQ ID NO: 720).

In a particular embodiment, VHH molecules of the invention comprise a FR3 domain comprising or consisting of SEQ ID NO: 45 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 70%: YYADSVKGRFTISRDNAKNTVYLQMNSLKPEDTAVYYC (SEQ ID NO: 45). More preferably, the bold amino acid residues are present and the variability occurs only on the other positions.

In a specific embodiment, the Y in position 1 may be replaced with N.

In a specific embodiment, the Y in position 2 may be replaced with A.

In a specific embodiment, the A in position 3 may be replaced with P or I.

In a specific embodiment, the D in position 4 may be replaced with S or N.

More preferably, the FR3 contains at most 7 amino acid modifications by reference to this sequence, even more preferably at most 6, at most 3, even more preferably at most 2 amino acid modifications in non-bold amino acid residues.

In a further specific embodiment, the FR3 has an amino acid sequence selected from any one of the amino acid sequences listed below:
NYADSMKGRFTISRDNTKNAVYLQIDSLKPEDTAVYYC (SEQ ID NO: 46);
NYPDSAKGRFTISRDNAKNTVYLQIDSLKPEDTAVYYC (SEQ ID NO: 47);
YAISSVKGRFTISRDNAENTVFLQMNSLKPDDTAVYYC (SEQ ID NO: 48);
NYPDSMKGRFTISRDNAKNTVYLQINSLKSEDTAVYYC (SEQ ID NO: 49); or variants thereof.

Further specific examples of the FR3 of VHH molecules according to the invention are provided below (see also Table 3):
NYADSMKGRFTISRDNTKNALYLQIDSLRPEDTAVYYC (SEQ ID NO: 366)
NYADSVKGRFTISRDNTKNTLYLQIDSLRPEDTAVYYC (SEQ ID NO: 367),
NYADSVKGRFTISRDNAKNTLYLQMNSLRPEDTAVYYC (SEQ ID NO: 368),
NY ADSVKGRFTISRDNTKNTL YLQINSLRPEDTA VYYC (SEQ ID NO: 369),
NYADSMKGRFTISRDNTKNTLYLQMNSLRPEDTAVYYC (SEQ ID NO: 370),
NYADSVKGRFTISRDNAKNTLYLQIDSLRPEDTAVYYC (SEQ ID NO: 371),
NYADSVKGRFTISRDNTKNTLYLQMNSLRPEDTAVYYC (SEQ ID NO: 372),
NYADSVKGRFTISRDNTKNALYLQMNSLRPEDTAVYYC (SEQ ID NO: 373),
NYADSVKGRFTISRDNTKNTLYLQMDSLRPEDTAVYYC (SEQ ID NO: 374),
NYADSVKGRFTISRDNSKNTLYLQMNSLRAEDTAVYYC (SEQ ID NO: 375),
NYADSVKGRFTISRDNAKNAVYLQMNSLRPEDTAVYYC (SEQ ID NO: 376),
NYADSVKGRFTISRDNAKNTLYLQMNSLKPEDTAVYYC (SEQ ID NO: 377),
NYADSMKGRFTISRDNAKNTLYLQMNSLRPEDTAVYYC (SEQ ID NO: 378),
NYPDSVKGRFTISRDNAKNTVYLQMNSLRPEDTAVYYC (SEQ ID NO: 379),
YYADGMRGRFTISRDNSENTVSLQMNNLKPEDTAVYYC (SEQ ID NO: 380),
YYANSMKERFTISRDNAQNTVSLQISSLKPEDTAVYYC (SEQ ID NO: 381),
YYADGMKGRFTISRDNAENTVSLQINSLKPEDTAIYYC (SEQ ID NO: 382),
YYADSSVKGRFTISRDNAENTVSLQMNSLKPEDTAVYYC (SEQ ID NO: 383),
SYRDSVKGRFTISRDNAKNTVFLQMNSLEPEDTGVYYC (SEQ ID NO: 384),
SYADSVKGRFTISRDDAKNTVYLQMDNLTPEDTAVYFC (SEQ ID NO: 385),
EYKDSVKGRFTISRDNARNTIYLEMKNLKPEDTAIYYC (SEQ ID NO: 386),
DYADGVMGRFTISRNSALNTVYLQMDSLKSTDTGVYVC (SEQ ID NO: 387),
KYGDSVKGRFTISRDDAKNTVYLQMNSLKPEDTAVYYC (SEQ ID NO: 388),
TYADSVKGRFTISRDNAKNTVYLQMNSLEPTDTAVYYC (SEQ ID NO: 389),
YYADSVKGRFTISRDTVKDMVYLQMNSLKPEDTAVYYC (SEQ ID NO: 623),
EYADSVKGRFTISRDNAKSTVYLQMNNLKPEDTAVYYC (SEQ ID NO: 624),
VYPDSAKGRFTISRDNAKNTVYLQIDSLKPEDTAVYYC (SEQ ID NO: 625), or
FYPDSAKGRFTISRDNAKNTVYLQIDSLKPEDTAVYYC (SEQ ID NO: 626), or
NYADSMKGRLTISRDNTKNAVYLQIDSLKPEDTAVYYC (SEQ ID NO: 717), or
NYPDIAKGRFTISRDNAKNTVYLQIDSLKPEDTAVYYC (SEQ ID NO: 718), or
NYPDSAKGRFTISEDNAKNTVYLQIDSLKPEDTAVYYC (SEQ ID NO: 719).

In a particular embodiment, VHH molecules of the invention comprise a FR4 domain comprising or consisting of SEQ ID NO: 50 as represented below, or variants thereof having at least 58%, %, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 90%: WGQGTQVTVSS (SEQ ID NO: 50). More preferably, the bold amino acid residues are present and the variability occurs only on the other positions. More preferably, the FR4 contains at most 4 amino acid modifications by reference to this sequence, even more preferably at most 3, even more preferably at most 2 amino acid modifications in non-bold amino acid residues. A specific illustrative example of a FR4 sequence is SEQ ID NO: 50.

Other specific examples of the FR4 according to the invention are the following:
WGQGTLVTVSS (SEQ ID NO: 390),
WGKGTQVTVSS (SEQ ID NO: 391) or
WGRGTQVTVSS (SEQ ID NO: 670).

Specific examples of TfR-binding VHH molecules of the invention are molecules comprising or consisting of an amino acid sequence selected from any one of SEQ ID NOs: 4 (VHH A), 8 (VHH B), 12 (VHH C), 16 (VHH D), 18 (VHH A1), 20 (VHH A2), 22 (VHH A3), 24 (VHH A4), 26 (VHH A5), 28 (VHH A6), 30 (VHH A7), 32 (VHHA8), 34 (VHH A9), 68 (VHH A10), 70 (VHH A11), 72 (VHH A12), 74 (VHHA13), 76 (VHH A14), 78 (VHH A15), 80 (VHH A16), 82 (VHH A17), 84 (VHH A18), 86 (VHH A19), 87 (VHH A20), 88 (VHH A21), 89 (VHH A22), 90 (VHH A23), 91 (VHH A24), and 92 (VHH A25), 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618 and 679-682, 691-700 (as listed in Table 1 below; in these examples, when x is equal to 1, each VHH molecule comprises the following particular tag sequence of SEQ ID NO: 51: AAAEQKLISEEDLNGAAHHHHHHGS).

Other specific examples of TfR-binding VHH molecules of the invention are molecules comprising or consisting of an amino acid sequence selected from any one of SEQ ID NOs: 213-271, 273-299, 412, 415, 418, 421, 423, 425, 428, 430, 433, 436, 439, 441, 443, 445, 447, 449, 451, 454, 457, 613-615 and 675-678 and 701-709 (see Table 1). In the examples corresponding to SEQ ID NOs: 213-271, 273-299, 412, 415, 418, 421, 423, 425, 428, 430, 433, 436, 439, 441, 443, 445, 447, 449, 451, 454, 457, 613-615, 675-678 and 701-709, the VHH molecule does not comprise any tag sequence (when x is 0, as detailed in Table 1 below). In a particular embodiment, the VHHs of the invention are humanized.

For humanization, one or more of the FR and/or CDR domains may be (further) modified by one or more amino acid substitutions. In this respect, in a particular embodiment, the VHH are humanized by selected modification (e.g., amino acid substitution) of the FR1 domain. A FR1 domain consists typically in a sequence of 25 amino acid residues. A typical humanized position in FR1 is 19R or 23A, or both (for example by reference to any one of SEQ ID NOs: 35-39, 331, 332, 400 or to any variants thereof as herein described). Specific examples of such humanized FR1 thus comprise SEQ ID NOs: 37, 331, 400, wherein K19 and/or V23 are respectively modified into 19R and 23A.

Another humanized position in FR1 is 11L, and a specific example of such humanized FR1 thus comprises SEQ ID NO: 332, wherein V11 is modified into 11L and wherein K19 and V23 are respectively modified into 19R and 23A.

In another particular embodiment, the VHH are humanized by selected modification of the FR2 domain. A typical humanized position in FR2 is selected from 1M, 2S or 2H, 4V, 11G, 12L, 14W, or combinations thereof (by reference to e.g., any one of SEQ ID NOs: 40-44, 342-348 or to any variants thereof as described herein).

A specific example of such a humanized FR2 thus comprises SEQ ID NO: 41 wherein one or more or all of I1, R2, Y4, Q11, R12, and F14 are respectively modified into 1M, 2S or 2H, 4V, 11G, 12L, and 14W.

Another specific example of the humanized FR2 comprises SEQ ID NO: 342, wherein Y4, Q 11, R12 and F14 are respectively modified into 4V, 11G, 12L and 14W.

Another specific example of the humanized FR2 comprises SEQ ID NO: 343, wherein Q11 and R12 are respectively modified into 11G and 12L.

Another specific example of the humanized FR2 comprises SEQ ID NO: 344, wherein Y4, Q11 and R12 are respectively modified into 4V, 11G and 12L.

Another specific example of the humanized FR2 comprises SEQ ID NO: 345, wherein Q11 is modified into 11G.

Another specific example of the humanized FR2 comprises SEQ ID NO: 346, wherein Y4 is modified into 4V.

Another specific example of the humanized FR2 comprises SEQ ID NO: 347, wherein Q 11, R12 and F14 are respectively modified into 11G, 12L and 14W.

Another specific example of the humanized FR2 comprises SEQ ID NO: 348, wherein Q11 and R12 are respectively modified into 11G and 12L.

In another particular embodiment, the VHH are humanized by selected modification of the FR3 domain. A typical humanized position in FR3 is selected from 6V, 17A or S, 20T, 21L, 25M, 26N, 29R, 30A, and any combination thereof (by reference to e.g., any one of SEQ ID NOs: 45-49, 366-379 or any variants thereof as herein defined).

A specific example of such a humanized FR3 thus comprises SEQ ID NO: 46 wherein one or more or all of M6, T17, A20, V21, I25, D26, and K29, are respectively modified into 6V, 17A, 20T, 21L, 25M, 26N, and 29R.

Another specific example of the humanized FR3 comprises SEQ ID NO: 366 wherein V21 and K29 are respectively modified into 21L and 29R.

Another specific example of such a humanized FR3 comprises SEQ ID NO: 367 wherein M6, A20, V21 and K29, are respectively modified into 6V, 20T, 21L and 29R.

Another specific example of the humanized FR3 comprises SEQ ID NO: 368 wherein M6, T17, A20, V21, I25, D26, and K29, are respectively modified into 6V, 17A, 20T, 21L, 25M, 26N, and 29R.

Another specific example of the humanized FR3 comprises SEQ ID NO: 369 wherein M6, A20, V21, D26, and K29, are respectively modified into 6V, 20T, 21L, 26N, and 29R.

Another specific example of the humanized FR3 comprises SEQ ID NO: 370 wherein A20, V21, I25, D26, and K29, are respectively modified into 20T, 21L, 25M, 26N, and 29R.

Another specific example of the humanized FR3 comprises SEQ ID NO: 371 wherein M6, T17, A20, V21 and K29, are respectively modified into 6V, 17A, 20T, 21L and 29R.

Another specific example of the humanized FR3 comprises SEQ ID NO: 372 wherein M6, A20, V21, I25, D26, and K29, are respectively modified into 6V, 20T, 21L, 25M, 26N, and 29R.

Another specific example of the humanized FR3 comprises SEQ ID NO: 373 wherein M6, V21, I25, D26, and K29, are respectively modified into 6V, 21L, 25M, 26N, and 29R.

Another specific example of the humanized FR3 comprises SEQ ID NO: 374 wherein M6, A20, V21, I25 and K29, are respectively modified into 6V, 20T, 21L, 25M and 29R.

Another specific example of the humanized FR3 comprises SEQ ID NO: 375 wherein M6, T17, A20, V21, I25, D26, K29 and P30, are respectively modified into 6V, 17S, 20T, 21L, 25M, 26N, 29R and 30A.

Another specific example of the humanized FR3 comprises SEQ ID NO: 376 wherein M6, T17, I25, D26 and K29, are respectively modified into 6V, 17A, 25M, 26N and 29R.

Another specific example of the humanized FR3 comprises SEQ ID NO: 377 wherein M6, T17, A20, V21, I25 and D26, are respectively modified into 6V, 17A, 20T, 21L, 25M and 26N.

Another specific example of the humanized FR3 comprises SEQ ID NO: 378 wherein T17, A20, V21, I25, D26, and K29, are respectively modified into 17A, 20T, 21L, 25M, 26N, and 29R.

Another specific example of the humanized FR3 comprises SEQ ID NO: 379 wherein M6, I25, D26, and K29, are respectively modified into 6V, 25M, 26N, and 29R.

In a further particular embodiment, the FR1 and/or FR2 and/or FR3 are humanized.

Specific examples of humanized TfR-binding VHH molecules of the invention are molecules comprising or consisting of an amino acid sequence selected from any one of SEQ ID NOs: 236-241 or 252-271, 273-275 (see Table 1). In a further particular embodiment, the VHH molecules may further comprise one or several tags, suitable for e.g., purification, coupling, detection, etc. Within the context of this invention, the term "tag" includes any peptide sequence which is attached to a polypeptide VHH molecule of the invention to facilitate easy detection or purification of expressed proteins or to identify their binding to TfR or for site-directed enzymatic chemical/enzymatic conjugation purpose. The tag may be an affinity tag, an epitope tag, a site-specific conjugation tag or a fluorescent tag.

Examples of such tags include a Q-tag which is a tag comprising a glutamine residue inserted in a tag sequence, which is specifically recognized by the TGase, preferably comprising or consisting of the sequence LQR, a myc tag (EQKLISEEDL, SEQ ID NO: 394), a poly-His tag (comprising from 2 to 8 histidine residues, preferably 6-8 His residues, e.g, His6 (SEQ ID NO: 395) or His8 (SEQ ID NO: 396)), a poly-Arg tag (comprising from 2 to 8 arginine residues), a poly-Lys tag (comprising from 2 to 8 lysine residues), an HA tag (e.g., YPYDVPDYA, SEQ ID NO: 397), a FLAG tag (e.g., DYKDDDDK, SEQ ID NO: 398) or a GFP tag, a CBP tag, a Strep II tag, a sortase-tag, a SNAP-tag or a combination thereof (as shown in Table 4 below).

Typically, the one or several tags are located at the C-terminal end of the VHH.

In a further particular embodiment, the VHH molecules may further comprise one or several linkers.

Within the context of this invention, the terms "linker" or "spacer" are used interchangeably. The linkers may be peptide linkers or conjugation linkers. The peptide linker includes one or more amino acid residues, typically from 1 to 10 amino acid residues, used for linking the VHH molecule of the invention and a tag, or between various tags as described herein, provided that the linker does not specifically bind to the target protein which is TfR. The linker may be any amino acid residue for example, glycine (Gly or G), alanine (Ala or A), phenylalanine (Phe or F), serine (Ser or S), cysteine (Cys or C), leucine (Leu or L), asparagine (Asn or N), lysine (Lys or K), glutamate (Glu or E), glutamine (Gln or Q), proline (Pro or P), valine (Val or V), arginine (Arg or R), aspartate (Asp or D), etc., or a combination thereof. The peptide linker may be flexible (e.g., any flexible hydrophilic linker) or rigid (e.g., any alpha-helix rigid linker).

Such a peptide linker differs from conjugation linkers which may be introduced between the VHH and the compounds of interest, such as bi- or multifunctional agents containing alkyl, aryl, thiols, azide, alkynes, nucleotidic or peptide groups by esters, aldehydes or alkyl or aryl acids, anhydride, sulfhydryl or carboxyl groups, groups derived from cyanogen bromide or chloride, carbonyldiimidazole, succinimide esters or sulfonic halides (as described herein in the part "Conjugates").

As a further illustration, the VHH of the invention may comprise a linker, preferably located at the C-terminal end of the VHH. The linker may comprise a Gly residue or a Gly repeat of e.g., 2-7 Gly residues (i.e., Gly2, Gly3, Gly4, Gly5, Gly6 or Gly7, respectively). The VHH of the invention may also comprise a combination of Gly and Ser residues. Specific examples of such Gly and Ser combinatorial linkers include: GlySerGlySer (GSGS; SEQ ID NO: 627); SerGlySerGly5 (SGSGGGGG; SEQ ID NO: 628); (Gly4Ser)n, wherein n is from 1 to 6, for example any of SEQ ID NOs: 629 to 634 (as shown in Table 4 below), preferably SEQ ID NOs: 629 to 631; or any linker comprising such (Gly4Ser)n sequence, for example GlyGly(Gly4Ser)3 (GGGGGGSGGGGSGGGGS; SEQ ID NO: 635).

Other specific examples of peptide linkers according to the invention comprise or consist of EAAAK (SEQ ID NO: 636) or comprise a EAAAK repeat in combination with other amino acid residues, for example any of SEQ ID NOs: 637 to 641, as shown in Table 4.

Other specific examples of peptide linkers according to the invention are the following: GG(AP)17 (SEQ ID NO: 642), ASTKGPSVFPLAP (SEQ ID NO: 643), GSAGSAAGSGEF (SEQ ID NO: 644) or KESGSVSSEQLAQFRSLD (SEQ ID NO: 645), as shown in Table 4.

In a particular embodiment, VHH of the invention may comprise a Gly linker and a Q-tag, preferably located C-terminally. More specific examples of such VHH comprise the following structure: VHH - Gly linker - Q-tag, wherein the Gly Linker comprises or consists of 2-6 Gly residues and the Q-tag contains or consists of LQR. As an illustration, the VHH may comprise, at the C-terminal end, the following tag sequence AAAEQKLISEEDLNGAAHHHHHHGS (SEQ ID NO: 51), wherein simple underlined is a myc tag and double underlined is a His6 tag (the remaining residues being linkers such as an Ala linker AAA or residues resulting from cloning).

Specific examples of such tagged TfR-binding VHH molecules of the invention, comprising the tag sequence of SEQ ID NO: 51 at the C-terminal end, are molecules comprising or consisting of an amino acid sequence selected from any one of 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618, 679-682 and 691-700.

Specific examples of humanized TfR-binding VHH molecules of the invention with tag sequences are molecules comprising or consisting of an amino acid sequence selected from any one of SEQ ID NOs: 87-92 or 130-149, 152-154 (see Table 1).

As another illustration, the VHH may comprise, at the C-terminal end, the following tag sequence GGGGSCHHHHHH (SEQ ID NO: 399), wherein simple underline is a spacer, bold C is a free cysteine available for site-directed chemical conjugation and double underline is a His tag (the remaining residues being linkers or resulting from cloning).

As another illustration, the VHH of the invention may comprise a Q-tag preferably comprising or consisting of the sequence LQR, preferably located at the C-terminal end of the VHH.

As a further illustration, the VHH may comprise, at the C-terminal ("C-ter" or "C-terminus") end, the following tag sequence **GGG**LQR (SEQ ID NO: 111) wherein underlined is a Q-tag and bold is a Gly linker. Other examples are GGGGLQR (SEQ ID NO: 401), GGGGGLQR (SEQ ID NO: 402), GGGGGGLQR (SEQ ID NO: 403) and GGGGGGGLQR (SEQ ID NO: 404). In a preferred aspect of the invention, the VHH comprises the tag sequence of SEQ ID NO: 111.

In a further particular embodiment, VHH of the invention may comprise an Ala linker, a His tag, a Gly linker and a Q-tag. Preferably, the linkers and tags are located C-terminally of the VHH. In other embodiments, the Q-tag at least may be located N-terminal end of the VHH. More specific examples of such VHH comprise the following structure: VHH - Ala linker - His tag - Gly linker - Q-tag, wherein the Ala linker comprises 3 residues, the His tag comprises 2-7 His residues, preferably 6 His residues, the Gly linker comprises 2-6 Gly residues, preferably 3 residues, and the Q tag preferably contains or consists of LQR.

As an illustration, the VHH may comprise, at the C-terminal end, the following tag sequence **AAA**HHHHHH**GGG**LQR (SEQ ID NO: 112) wherein underlined is a Q-tag, bold are an Ala and a Gly linker, double underline is a His tag. Other examples are AAAHHHHHHGGGGLQR (SEQ ID NO: 406), AAAHHHHHHGGGGGLQR (SEQ ID NO: 407), AAAHHHHHHGGGGGGLQR (SEQ ID NO: 408) and AAAHHHHHHGGGGGGGLQR (SEQ ID NO: 409). In a preferred aspect of the invention, the VHH comprises the additional sequence of SEQ ID NO: 112.

As already described herein above, specific examples of such tagged TfR-binding VHH molecules of the invention are molecules comprising or consisting of an amino acid sequence selected from any one of 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618, 679-682, and 691-700.

Further specific examples of TfR-binding VHH molecules of the invention are VHH molecules which competitively inhibit binding of a VHH as defined above to a human and a non-human TfR. The term "competitively inhibits" indicates that the VHH can reduce or inhibit or displace the binding of a said reference VHH to TfR, *in vitro* or *in vivo.* Competition assays can be performed using standard techniques such as, for instance, competitive ELISA or other binding assays. Typically, a competitive binding assay involves a recombinant cell or membrane preparation expressing a TfR, optionally bound to a solid substrate, an unlabeled test VHH (or a phage expressing the same) and a labeled reference VHH (or a phage expressing the same). Competitive inhibition is measured by determining the amount of labeled VHH bound in the presence of the test VHH. Usually, the test VHH is present in excess, such as about 5 to 500 times the amount of reference VHH. Typically, for ELISA, the test VHH is in 100-fold excess. When a test VHH present in excess inhibits or displaces at least 70% of the binding of the reference VHH to TfR, it is considered as competitively inhibiting said reference VHH. Preferred competing VHH bind epitopes that share common amino acid residues.

As shown in the experimental section, VHH molecules bind TfR *in vitro* and *in vivo.* They show adequate affinity, with a Kd (measured for example by SPR or by flow cytometry), from 0.1 nM to 10 µM, preferably from 0.1 nM to 4 µM, and even more preferably from 0.1 nM to 2500 nM, for example from 0.1 nM to 500 nM or from 0.1 nM to 100 nM or from 1 nM to 100 nM or from 10 nM to 100 nM.

In a particular embodiment of the invention, VHH molecules are C5 or variants thereof, humanized or not, comprising or consisting of any of SEQ ID NOs: 213, 216-271, 274, 275, 675, 676 and 701 (as listed in Table 1). In another particular embodiment, VHH molecules are C5 or variants thereof which are cross-species VHH molecules binding to human, non-human primate (NHP) and murine TfR. In a particular embodiment, C5 or variants thereof comprise a tag sequence at the N- and/or C-terminal end. Specific examples of such variants comprise or consist of any of SEQ ID NOs: 4, 16, 18, 20, 22, 24, 26, 28, 30, 32, 34, 68, 70, 72, 74, 76, 78, 80, 82, 84, 86-92, 114, 116, 118, 120, 122, 124, 126, 127, 129 or 130-149, 153-154, 393, 679, 680, 692. In another particular embodiment, VHH molecules are humanized variants of C5 comprising or consisting of any of SEQ ID NOs: 236-241, 252-271, 274-275. In a particular embodiment, the humanized variants of C5 comprise a tag sequence at the N- and/or C-terminal end. Specific examples of such variants comprise or consist of any of SEQ ID NOs: 87-92 or 130-149, 153-154. Examples of humanized variants of C5 are C5h20 variants comprising or consisting of SEQ ID NOs: 265 or 143, or C5V13h20 variants comprising or consisting of SEQ ID NO: 274 or 153.

In another particular embodiment, VHH molecules of the invention are B8 or variants thereof (as listed in Table 1), comprising or consisting of any of SEQ ID NOs: 214, 276-284, 412, 415, 418, 421, 423, 425, 428, 430, 433, 436, 439, 441, 443, 445, 447, 449, 451, 454, 457, 677, 678 and 702-709. In a particular embodiment, B8 or variants thereof comprise a tag sequence at the N- and/or C-terminal end. Specific examples of such variants comprise or consist of any of SEQ ID NOs: 8, 152, 155-159, 161, 163, 165, 167, 411, 414, 417, 420, 422, 424, 427, 429, 432, 435, 438, 440, 442, 444, 446, 448, 450, 453, 456, 681, 682 or 693-700.

In another particular embodiment, VHH molecules of the invention are B8 or variants thereof, which are cross-reactive with human and non-human primate species.

In another particular embodiment, VHH molecule are B8 or variants thereof, humanized or not, comprising an amino acid sequence selected from any one of SEQ ID NOs: 214, 273, 276-284, 412, 415, 418, 421, 423, 425, 428, 430, 433, 436, 439, 441, 443, 445, 447, 449, 451, 454, 457, 677, 678 and 702-709.

In another particular embodiment, VHH molecules of the invention are B8 or variants thereof, comprising or consisting of any of SEQ ID NOs: 677, 678, 681, 682, 702-709 or 693-700.

In another particular embodiment, VHH molecules are humanized variants of B8 comprising or consisting of SEQ ID NOs: 273 or 152 (such as B8h1 variant).

In another particular embodiment, VHH molecules of the invention are B6 or variants thereof (as listed in Table 1), comprising or consisting of any of SEQ ID NOs: 12, 168, 170, 172, 173, 174, 178, 181, 183, 185, 215, 285-293, that bind TfR. B6 and variants thereof according to the invention target the apical domain of the TfR.

In another particular embodiment, VHH molecules of the invention are VHH molecules that bind the apical domain of the TfR, preferably an apical domain of the TfR1, and comprise or consist of SEQ ID NOs: 215, 285-299. In a particular embodiment, such VHH molecules comprise a tag sequence at the N- and/or C-terminal end. Specific examples of such variants comprise or consist of any of SEQ ID NOs: 12, 168, 170, 172-174, 178, 181, 183, 185, 189, 193, 197, 204, 208 or 691.

In another particular embodiment, the VHH molecule is B6 or a variant thereof, comprising an amino acid sequence selected from any one of SEQ ID NOs: 215 and 285-293.

In another particular embodiment, the VHH molecule comprises or consists of an amino acid sequence selected from any one of SEQ ID NOs: 613-618. Interestingly, such VHH molecules comprising or consisting of SEQ ID NOs: 613-618 bind human and non-human primate TfR but they do not bind mouse TfR.

Moreover, binding of said VHH of the invention to a human TfR receptor does not compete with binding of transferrin, the endogenous TfR ligand, and thus does not affect regular functions of said ligand. Conjugates produced with such VHH molecules have further been shown to bind TfR *in vitro* and to be transported across the BBB into the CNS *in vivo,* showing transcytosis. Such VHH thus represent potent agents for drug delivery or targeting.

The VHH of the invention can be synthesized by any technique known to those skilled in the art (biological or genetic synthesis, chemical, etc.). They can be preserved as they are, or be formulated in the presence of a substance of interest or any acceptable excipient. For chemical syntheses, commercial apparatuses that can incorporate natural as well as non-natural amino acids, such as D enantiomers and residues with side chains with hydrophobicities and steric obstructions different from those of their natural homologues (so-called exotic, *i.e.,* non-coded, amino acids), or a VHH sequence containing one or more peptidomimetic bonds that can include notably intercalation of a methylene (-CH₂-) or phosphate (-PO₂-) group, a secondary amine (-NH-) or an oxygen (-O-) or an N-alkylpeptide, are used. During synthesis, it is possible to introduce various chemical modifications, such as for example, inserting, linking or conjugating in the N-terminal and/or C-terminal position or on a side chain a lipid (or phospholipid) derivative or a constituent of a liposome or a nanoparticle, in order to incorporate the VHH of the invention within a lipid membrane such as that of a liposome composed of one or more lipid layers or bilayers, or of a nanoparticle. Liposomes and nanoparticles are examples of a "vehicle" which may be conjugated with one or more VHH molecules of the invention. The VHH of the invention can also be obtained from a nucleic acid sequence coding for the same, as described further below (cf. Table 2 and the Sequence listing).

### Conjugates

A further object of the invention relates to conjugates (also interchangeably called herein "conjugate compounds" or "chimeric agents") comprising one or more VHH molecules as defined above, conjugated to at least one additional compound, in particular to at least one additional molecule, agent, compound or scaffold of interest. This additional compound may be a distinct VHH or a molecule which is not a VHH. The at least one additional molecule, agent or compound of interest may be any molecule, agent or compound such as a half-life extending moiety, a stabilizing group or scaffold, a therapeutic (i.e., active) compound, medicament or drug, a diagnostic agent, an imaging molecule, a tracer, etc., or a vehicle comprising such a therapeutic, diagnostic or imaging compound. In a particular aspect, the chimeric agent (i.e., conjugate) may comprise both kinds of additional compounds, i.e. i) a half-life extending moiety, a stabilizing group or scaffold and ii) a therapeutic, diagnostic or imaging compound, or a vehicle comprising the same.The therapeutic compound is for example selected from a peptide, a polypeptide, a protein, an antibody, a nucleic acid and any fragment thereof. In a particular embodiment, the therapeutic compound is a nucleic acid molecule e.g., a mRNA, a ribozyme or an oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA, bridged nucleic acid (BNA), virus, diagnostic agent, tracer, etc.

Examples of conjugated molecules, agents or compounds of interest include, without limitation, any chemical entity such as a small chemical molecule (for example a chelating agent, an antibiotic, antiviral, immunomodulator, antineoplastic, anti-inflammatory, or adjuvant, etc.); a peptide, polypeptide or protein (for example an enzyme, hormone, neurotrophic factor, neuropeptide, cytokine, apolipoprotein, growth factor, antigen, antibody or part of an antibody, adjuvant, etc.); a nucleic acid (for example a RNA or a DNA of human, viral, animal, eukaryotic, prokaryotic, plant or synthetic origin, etc., including e.g., coding genes, inhibitory nucleic acids such as ribozymes, antisense oligonucleotides (ASOs), interfering nucleic acids (siRNAs), small activating RNAs (saRNAs), mRNAs, full genomes or portions thereof, plasmids, etc); a lipid (nano)particle, a Cell-Derived Vesicle (CDV) such as an exosome, a virus, a marker, or a tracer, for instance. Generally, the "molecule, agent or compound of interest" can be any drug (active) ingredient, whether a chemical, biochemical, natural or synthetic compound. Generally, the expression "small chemical molecule, agent or compound" designates a molecule of pharmaceutical interest with a maximum molecular weight of 1000 Daltons, typically between 300 Daltons and 700 Daltons.

The vehicle may be selected for example from a virus, a virus-like particle (VLP), a Cell-Derived Vesicle (CDV), an exosome, a lipid vehicle and a polymer vehicle, and is preferably a lipid nanoparticle (LNP), a micelle or a liposome.

The conjugated compound is typically a medicament (such as a small drug, nucleic acid or polypeptide, e.g., an antibody or fragment thereof) or an imaging agent suitable for treating or detecting neurological, infectious or cancerous pathologies, preferably pathologies of the CNS, such as the brain, e.g., Alzheimer's disease, Parkinson's disease, stroke, Creutzfeldt-Jakob disease, bovine spongiform encephalopathy, multiple sclerosis, amyotrophic lateral sclerosis, epilepsy, migraine, encephalitis, CNS pain, glioblastoma, etc.

The chimeric agent may also contain, in addition to or instead of said compound of interest, a half-life extending moiety or a stabilizing group to increase the plasma half-life of the VHH or conjugate. Particular chimeric agents of the invention thus comprise (i) at least one VHH, for example several VHH molecules, (ii) a half-life extending moiety or a stabilizing group, (iii) a compound of interest, typically a therapeutic, diagnostic or imaging compound, and optionally (iv) a vehicle, in any order.

In a particular aspect herein described, the compound of interest is at the same time a group allowing the stabilization and/or increasing the plasma half-life of the VHH molecule(s) of the invention. The half-life extending moiety or stabilizing group may be any group known to have substantial plasma half-life (e.g. at least few hours) and essentially no adverse biological activity. Examples of such half-life extending moieties or stabilizing groups include, for instance, an antibody or a fragment thereof such as a Fc fragment of an immunoglobulin, a VHH molecule or variants thereof, preferably VHH molecules binding to albumin, large human serum proteins such as albumin, HSA, or IgGs or PEGs molecules.

In a particular embodiment, the conjugate according to the invention comprises a half-life extending moiety or stabilizing group which is a small organic albumin-moiety that binds to the albumin with a low micromolar affinity thus improving the pharmacokinetic profile of the compound of interest by a progressive release of the conjugate from the albumin. Such small organic albumin-moieties include e.g. a fragment of Evans blue (EB) dye, fatty acids and derivatives thereof such as the C16 group and the 4-(p-iodophenyl)butytryl (PIB) group.

In another particular embodiment, the conjugate according to the invention comprises a half-life extending moiety or stabilizing group which is an Fc fragment of a human IgG1 or IgG4, preferably IgG1. Such a conjugate is of general formula VHH-hFc, for example VHH-hFc-siRNA or VHH-hFc-ASO.

In a further particular embodiment, the conjugate according to the invention comprises a half-life extending moiety or stabilizing group which is an Fc homodimer or heterodimer.

In another embodiment, the conjugate according to the invention comprises a half-life extending moiety or stabilizing group which is a modified Fc fragment homodimer or heterodimer of a IgG1 or IgG4 with attenuated or abolished effector functions and/or extended half-life.

In a further particular embodiment, the conjugate according to the invention comprises a modified Fc fragment of an IgG1 which is an aglycosylated Fc fragment of an IgG1, e.g., with N297 mutation.

In a further particular embodiment, the conjugate according to the invention comprises a modified Fc fragment of an IgG1 which is a fragment of an Fc variant having a symmetric or asymmetric amino acid modification (i.e, on only one or two arms of the Fc dimer) selected from deletion, insertion, inversion or substitution, or a combination thereof e.g., an amino acid substitution at L234A and L235A (i.e., LALA mutation). Such Fc modifications allow to modulate Fc receptor interactions, modulate, reduce or eliminate Fc effector functions such as FcgammaR binding, antibody-dependent cell-mediated cytotoxicity (ADCC), or complement-dependent cytotoxicity (CDC) or to modulate glycosylation.

In a further particular embodiment, the conjugate according to the invention comprises a modified Fc fragment which is a fragment of an IgG1 comprising a mutation at residue position N434, E380, M252, I253, S254, T256 or H433, or a combination thereof. Specific examples of such mutations are E380A M252Y, S254T, T256E, H433K, N434A or N434F.

In another particular embodiment, the conjugate according to the invention comprises a modified Fc fragment which is a fragment of an IgG1 comprising a mutation at residue position E233, L234, L235, G236, G237, S239, D265, D270, P329, A327, A330, or a combination thereof. Specific examples of such mutations are E233P, L234A, L234V, L235A, deltaG236, G237A, S239A, D265A, D265N, D270N, D270A, A327G, P329A, P329G, A330S or P331S.

The conjugate according to the invention may also comprise a modified Fc fragment which comprises any combination of the above mutations, optionally in a further combination with the LALA mutation.

In another particular embodiment, the conjugate according to the invention may comprise a modified Fc fragment which is a fragment of an IgG4 comprising a mutation at residue position L248 or L235, or a combination thereof. Specific examples of such mutations are L248E, L235A or L235E.

In a further particular embodiment, the conjugate according to the invention comprises a half-life extending moiety or stabilizing group which is albumin protein or albumin binding moieties.

The VHH may be conjugated in N-terminal or C-terminal end of the half-life extending moiety or stabilizing group, or both. When the half-life extending moiety or stabilizing group is a Fc fragment, conjugation is typically by genetic fusion. The resulting protein may remain as a monomeric agent, or multimerize, depending on the nature of the half-life extending moiety or stabilizing group. In the case of a Fc fragment, the fusion protein Fc-VHH or VHH-Fc may form homodimers or heterodimers.

In this regard, in a particular embodiment, the VHH molecule of the invention is conjugated to at least one oligonucleotide, i.e., one or more oligonucleotide(s) (e.g., any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), preferably ASO or siRNA, by means of the half-life extending moiety or stabilizing group such as a Fc fragment of a human IgG1 or IgG4, wherein Fc-VHH or VHH-Fc are homodimers or heterodimers.

For example, a heterodimeric VHH-Fc fusion may be generated using the "knob-into-hole" or "KiH" technology and may comprise a VHH at the N-terminus (or N-ter) of one arm ("knob" arm) of a human IgG1-derived Fc dimer (hFc) and a tag sequence specifically recognized by transglutaminase (TGase) enzyme (namely a Q-tag) inserted in C-terminus (or C-ter) of the other arm of the Fc dimer ("hole" arm), and may be site-specifically modified on the Q-tag to introduce an azido-linker. The resulting heterodimeric VHH-hFc-Q-tag-azido intermediate may be conjugated to alkyne-siRNA using copper-free click reaction, yielding VHH-hFc-siRNA conjugates with a stable linker (as described in Example XXIII and shown in Figure 23B and 24C).

In a particular embodiment, the conjugate according to the invention comprises a VHH-Fc heterodimer, wherein Fc comprises the following mutations: T366W on the "knob" arm of the heterodimer, and/or T366S, L368A and Y407V on the "hole" arm of the heterodimer.

In another particular embodiment, the conjugate according to the invention comprises a VHH-Fc heterodimer, wherein Fc comprises T366W, L234A and L235A mutations on the "knob" arm of the heterodimer and/or T366S, L368A, Y407V, L234A and L235A mutations on the "hole" arm of the heterodimer.

In another particular embodiment, the conjugate according to the invention comprises a VHH-Fc heterodimer, wherein Fc comprises T366W, L234A and L235A mutations on the "knob" arm of the heterodimer and T366S, L368A, Y407V, L234A and L235A mutations on the "hole" arm of the heterodimer. In this regard, the VHH-Fc heterodimer may comprise a modified Fc having a sequence of SEQ ID NO: 664 on the "knob" arm, and a modified Fc having a sequence of SEQ ID NO: 665 on the "hole" arm. Specific examples of such VHH-Fc heterodimers comprising SEQ ID NO: 664 and 665 are described, for instance, in Example XXII and in Figures 20 and 22.

All the above mutations are defined according to the standard Kabat system for numbering the amino acid residues in an antibody. In the conjugate compounds of the invention, coupling can be performed by any acceptable means of bonding taking into account the chemical nature, steric hindrance and number of conjugated entities. Coupling can thus be carried out by one or more covalent, ionic, hydrogen, hydrophobic or Van der Waals bonds, cleavable or non-cleavable in physiological medium or within cells, preferably cleavable in particular when the present invention is used in the context of the delivery of at least one active agent on the CNS site. Furthermore, coupling can be made at various reactive groups, and notably at one or more terminal ends and/or at one or more internal or lateral reactive groups. Coupling can also be carried out using genetic engineering.

A strong interaction is necessary between the VHH and different cargos to prevent their dissociation before the conjugate reaches its site of action (i.e., the CNS cell). For this reason, the preferred coupling of the invention is covalent coupling, although non-covalent coupling may also be employed. The compound of interest can be coupled with the VHH either at one of the terminal ends (N-terminal or C-terminal), or at a side chain of one of the constitutive amino acids of the sequence (Majumdar and Siahaan, 2012). The compound of interest can be coupled directly to a VHH, or indirectly by means of a linker or spacer. Means of covalent chemical coupling, calling upon a linker or not, include classical bioconjugation techniques for instance those selected from bi- or multifunctional agents containing alkyl, aryl, thiols, azide, alkynes, nucleotidic or peptide groups by esters, aldehydes or alkyl or aryl acids, anhydride, sulfhydryl or carboxyl groups, groups derived from cyanogen bromide or chloride, carbonyldiimidazole, succinimide esters or sulfonic halides. It also may additionally include specific enzymatic conjugation for example through the bacterial transglutaminase that catalyzes the transamidation on a glutamine, provided that this glutamine is inserted in a specific tag. Illustrative strategies for conjugating a VHH of the invention to a molecule or scaffold are disclosed in Fig 6.

In a particular embodiment, coupling (or conjugation) involves genetic fusion. Such a strategy can be used when the coupled molecule is a peptide or polypeptide. In this case, a nucleic acid molecule encoding the VHH fused to the molecule is prepared and expressed in any suitable expression system, to produce the conjugate.

General structures of VHH-Oligo conjugates of the invention are shown in Figure 24, and illustrative strategies for conjugating a VHH of the invention to a molecule or scaffold are disclosed in Figure 23.

In another particular embodiment, coupling (or conjugation) is performed using the thiol/maleimide chemistry technology. For this reaction to occur, the VHHs are fused to their C-terminus with a peptidic sequence containing an additional cysteine. In particular, the additional peptidic tag is typically GGGGSCHHHHHH (SEQ ID NO: 399), wherein simple underline is a Gly linker that is used as a spacer and double underline is a 6His tag that is used for purification purposes.

Since VHHs only contain cysteines engaged in a disulfide bridge, the additional cysteine introduced in the tag is the only one to be chemically reactive towards maleimides. It allows the specific conjugation of the VHH with maleimide-derivatized molecules of interest. The reaction proceeds in two steps. First it is necessary to smoothly reduce the VHH-GGGGSCHHHHHH since the additional cysteine in the tag can be partially engaged in a disulfide bridge during the production process (formation of a disulfide bond with another VHH-GGGGSCHHHHHH or with a free cysteine). The first step thus consists in the partial reduction using a mild reducing agent such as 2-MEA (2-mercaptoethanol), TCEP (tris(2-carboxyethyl)phosphine) or DTT (dl-1,4-dithiothreitol). In a second step, the VHH-GGGGSCHHHHHH is allowed to react at a pH in the range of 6.5 - 7.5 with maleimide-functionalized molecules of interest to form VHH-molecule conjugates linked in a covalent and stable manner. The two steps can be performed sequentially or all together in situ.

In another particular embodiment, coupling (or conjugation) is performed by enzymatic reaction. In particular, site-specific conjugation onto the VHH can be performed using the transglutaminase enzyme (Tgase). Tgase catalyzes the formation of a stable isopeptidic bond between (i) the side chain of a glutamine residue inserted in a tag sequence specifically recognized by the Tgase (namely a Q-tag) and (ii) an amino-functionalized donor substrate. In this regard, the inventors have developed a particular tag sequence (named "Q-tag") which is recognized by Tgase and may be used to couple VHH of the invention to any molecule of interest, either chemical drugs or agents or a heterobifunctional linker for further conjugation to chemical drugs or agents. For this purpose, VHHs are prepared by genetic fusion to add in tandem (typically to their C-terminus) the following tags: first an optional trialanine linker, then an optional His-tag, then an optional small triglycine linker, and finally a Q-tag. The triglycine linker allows spacing out the Q-tag to allow a better accessibility of the Tgase to the glutamine while the His-tag aims at facilitating the purification of the VHH and its further functionalized versions.

The general conjugation strategy that was developed is a convergent synthesis that is based on a process comprising:
1) introduction onto the glutamine in the Q-tag fused to the VHH of a reactive moiety for further conjugation to a molecule of interest. In this objective, a heterobifunctional conjugation linker having two different reactive ends is allowed to be processed by the Tgase: one suitable primary amine-group toward the Tgase and one orthogonal reactive moiety. Representative examples of such orthogonal and reactive groups include azides, constraints alkynes such as DBCO (dibenzocyclooctyne) or BCN (bicyclo[6.1.0]nonyne), tetrazines, TCO (trans-cyclooctene), free or protected thiols, maleimides, etc.
2) introduction onto the molecule of interest of a reactive moiety complementary to the one incorporated onto the VHH Q-tag. Representative examples of such orthogonal and reactive groups include azides, constrained alkynes such as DBCO or BCN, tetrazines, TCO, free or protected thiols, maleimides, etc.
3) conjugation of both the functionalized VHH and molecule owing to their complementary reactive groups.

Such conjugation strategy represents a further object of the present invention. In particular, an object of the invention resides in a method for coupling two molecules using a Q-tag as defined above through Tgase coupling reaction. A further object of the invention is a VHH comprising a Q-tag. A further object of the invention is a VHH molecule comprising a linker, such as a Gly linker, and a Q-tag.

Preferred VHH of the invention have the following structure: VHH-Linker-Hisₘ-Linker-LQR, wherein : VHH is any VHH molecule; Linker is any molecular linker such as an Ala or Gly linker (preferably the two linkers are different); and m is an integer from 0 to 8, preferably m is 6 or 8.

In a particular embodiment, the invention relates to a conjugate comprising at least one VHH covalently linked to at least one chemical entity. Preferred variants of such conjugates contain one VHH and one chemical entity.

In another particular embodiment, the invention relates to a conjugate comprising at least one VHH covalently linked to at least one nucleic acid. The nucleic acid may be an antisense oligonucleotide ("ASO"), a ribozyme, an aptamer, a siRNA, etc. Preferred variants of such conjugates contain one VHH and one nucleic acid molecule. In a particular embodiment, the nucleic acid is an oligonucleotide (i.e., a short DNA or RNA molecule, also called herein "oligo" or "oligomer") selected from any single- or double-stranded oligonucleotide such as a small interfering RNA (siRNA), small activating RNAs (saRNA), gapmer, antisense oligonucleotide (ASO), shRNA, miRNA, aptamer RNA or bridged nucleic acid (BNA), which can specifically bind to a target mRNA thereby modulating gene expression in the cell. For example, such an oligonucleotide is an ASO or a siRNA capable of modulating (silencing or inhibiting or activating) the expression of a target gene.

The conjugates according to the invention are complementary to a target gene, and, in particular embodiments, they are capable of silencing or inhibiting the expression of a target gene, preferably by at least 40 %, or at least 50 %, or at least 60 %, or at least 70 %, or at least 80 %, or at least 90 %. More specifically, the conjugates according to the invention are capable of silencing or inhibiting the expression of a target gene by about 40 % to about 80 %.

Accordingly, in a particular embodiment, the invention relates to a conjugate compound comprising at least one VHH binding TfR, preferably at the surface of the CNS cell, and covalently linked to at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA thereby modulating gene expression in the cell.

In a particular embodiment, the invention relates to a conjugate compound comprising:
(i) one or more VHH molecule(s) binding TfR at the surface of the CNS cell, comprising:
   . a CDR1 comprising a sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 and 711, and/or,
   . a CDR2 comprising a sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 and 712, and/or
   . a CDR3 comprising a sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 and 713-715; and
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby reducing its level of expression in the cell.

In a particular embodiment, the invention relates to a conjugate compound comprising:
(i) one or more VHH molecule(s) binding TfR at the surface of the CNS cell, comprising:
   - a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710, 711, and variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
   - a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611, 712, and variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
   - a CDR3 comprising an amino acid sequence selected from SEQ ID NOs: 3, 7, 11, 117, 119, 121, 123, 177, 180, 188, 192, 196, 200, 203, 207, 452, 455, 609, 612, 713-715, and variants thereof having at least 60%, in particular at least 65%, 70% or 75%, for example at least 80% or 85%, amino acid identity to any one of said sequences over the entire length thereof, preferably at least 80%, 85%, 86%, 87%, 88%, 89%, 90%, 91%, 92%, 93%, 94%, 95%, 96%, 97%, 98% or 99%, more preferably at least 95%,
   with the proviso that:
   . when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
   . when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
   . when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby reducing its level of expression in the cell.

In another particular embodiment, the invention relates to a conjugate compound comprising:
(i) one or more VHH molecule(s) binding TfR at the surface of the CNS cell, comprising:
   - a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710, 711, and variants thereof having at most 3, 2 or 1 amino acid modifications; and
   - a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611, 712, and variants thereof having at most 3, 2 or 1 amino acid modifications; and
   - a CDR3 comprising an amino acid sequence selected from SEQ ID NOs: 3, 7, 11, 117, 119, 121, 123, 177, 180, 188, 192, 196, 200, 203, 207, 452, 455, 609, 612, 713-715, and variants thereof having at most 3, 2 or 1 amino acid modifications,
   with the proviso that:
   . when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
   . when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
   . when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby reducing its level of expression in the cell.

In another particular embodiment, the invention relates to a conjugate compound comprising:
(i) one or more VHH molecule(s) binding TfR at the surface of the CNS cell, and comprising SEQ ID NOs: 1, 2 and 3; or SEQ ID NOs: 5, 6 and 7; or SEQ ID NOs: 9, 10 and 11; or SEQ ID NOs: 13, 14 and 15; SEQ ID NOs: 17, 2 and 3; or SEQ ID NOs: 19, 2 and 3; or SEQ ID NOs: 1, 21 and 3; or SEQ ID NOs: 1, 23 and 3; or SEQ ID NOs: 1, 2 and 25; or SEQ ID NOs: 1, 2 and 27; or SEQ ID NOs: 1, 2 and 29; or SEQ ID NOs: 1, 2 and 31; or SEQ ID NOs: 1, 2 and 33; or SEQ ID NOs: 67, 2 and 3; or SEQ ID NOs: 69, 2 and 3; or SEQ ID NOs: 1, 71 and 3; or SEQ ID NOs: 1, 73 and 3; or SEQ ID NOs: 1, 75 and 3; or SEQ ID NOs: 1, 2 and 77; or SEQ ID NOs: 1, 2 and 79; or SEQ ID NOs: 1, 2 and 81; or SEQ ID NOs: 1, 2 and 83; or SEQ ID NOs: 1, 2 and 85; or SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7; and
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In a preferred embodiment, the conjugate of the invention comprises: (i) at least one VHH molecule binding TfR at the surface of the CNS cell, and comprising or consisting of any of SEQ ID NOs: 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 213-271, 273-299, 393, 411-412, 414-415, 417-418, 420-425, 427-430, 432-433, 435-436, 438-451, 453-454, 456-457 or 691-709; and (ii) at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) one or more VHH molecule(s) which is C5 or a variant thereof (as listed in Table 1), which is cross-species VHH molecule binding to human, non-human primate (NHP) and murine TfR at the surface of the CNS cell, and which comprises or consists of any of SEQ ID NOs: 4, 16, 18, 20, 22, 24, 26, 28, 30, 32, 34, 68, 70, 72, 74, 76, 78, 80, 82, 84, 86-92, 114, 116, 118, 120, 122, 124, 126, 127, 129 or 130-149, 153-154, 213, 216-271, 274-275, 393, 675, 676, 679, 680, 692 or 701; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) at least one VHH molecule which is a humanized variant of C5 (as listed in Table 1), comprising or consisting of any of SEQ ID NOs: 87-92, 130-149, 153-154, 236-241, 252-271 or 274-275; and (ii) at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) one or more VHH molecule(s) which is B6 or a variant thereof (as listed in Table 1), which binds to the apical domain of the TfR, preferably TfR1 at the surface of the CNS cell, and which comprises or consists of any of SEQ ID NOs: 12, 168, 170, 172-174, 178, 181, 183, 185, 215, 285-293 and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell.

In another preferred embodiment, the conjugate of the invention comprises: (i) one or more VHH molecule(s) binding to the apical domain of the TfR, preferably TfR1 at the surface of the CNS cell, and comprising or consisting of any of SEQ ID NOs: 12, 168, 170, 172-174, 178, 181, 183, 185, 189, 193, 197, 204, 208, 215, 285-299 or 691; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell.

In another preferred embodiment, the conjugate of the invention comprises: (i) one or more VHH molecule(s) which is B8 or a variant thereof (as listed in Table 1), comprising or consists of any of SEQ ID NOs: 8, 152, 155-159, 161, 163, 165, 167, 214, 273, 276-284, 411, 412, 414, 415, 417, 418, 420-425, 427-430, 432, 433, 435, 436, 438-451, 453, 454, 456, 457, 677, 678, 681, 682, 693-700, 702-709; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell
Specific examples of conjugation methods used to conjugate a VHH molecule to an oligonucleotide are the direct thiol-maleimide chemistry thanks to the introduction of an unpaired cysteine to the C-terminus of the VHH or an indirect SPAAC reaction (Strain Promoted Alkyne Assisted Click) thanks to the preliminary site-specific enzyme-assisted reaction onto the VHH to introduce suitable moieties for click chemistry. Different linkers can be used between the two partners VHH and oligos, that can be stable or cleavable. Example of cleavable linkers are disulfide, enzyme-labile peptide linkers such as valine-citrulline or phenylalanine-lysine dipeptide or pH-labile linkers such as hydrazone.

In a particular embodiment, a VHH molecule and oligonucleotide are linked to each other using preferably a copper-free click reaction yielding VHH-Oligo conjugates with a stable or a cleavable linker.

In another particular embodiment, the invention relates to a conjugate comprising a VHH covalently linked to a peptide. The peptide may be an active molecule, a bait, a tag, a ligand, etc. Preferred variants of such conjugates contain one VHH and one peptide.

In another embodiment, the invention relates to a conjugate comprising a VHH covalently linked to a dye.

In another embodiment, the invention relates to a conjugate comprising a VHH covalently linked to a nanoparticle and/or liposome, for example a lipidic particle or nanoparticle ("LNP"). The nanoparticle or liposome may be loaded or functionalized with active agents. Preferred variants of such conjugates contain several VHH molecules coupled to each nanoparticle or liposome.

In a further embodiment, the conjugate comprises, as a half-life extending moiety or a stabilizing group, an antibody or a fragment thereof to which one or several VHH molecules are coupled. Typically, a VHH molecule is coupled to a C- or N-terminal of a heavy or light chain, or both, or to the C- or N-terminal of an Fc fragment. In a particular aspect, the VHH molecule is coupled to the N-terminal end of an Fc. In another aspect, the VHH molecule is coupled to the C-terminal end of an Fc.In another particular aspect, the conjugate comprises, or consists of, a single VHH molecule coupled to an antibody fragment which may be a heavy chain or a light chain. In this aspect, the VHH molecule is indifferently coupled to the C-terminal end or to the N-terminal end of the chain. Preferably, it is coupled to the N-terminal end.

The invention also relates to a method for preparing a conjugate compound such as defined above, characterized in that it comprises a step of coupling between a VHH and a molecule or scaffold, preferably by a chemical, biochemical or enzymatic pathway, or by genetic engineering.

In a chimeric agent of the invention, when several VHH are present, they may have a similar or different binding specificity.

### Nucleic acids, vectors and host cells

A further aspect of the invention relates to a nucleic acid encoding a VHH as defined above, or a conjugate thereof (when the conjugated moiety is an amino acid sequence). The nucleic acid may be single- or double-stranded. The nucleic acid can be DNA (for example a cDNA or a gDNA), a RNA (for example a mRNA or a gRNA), or a mixture thereof. It can be in single stranded form or in duplex form, or it can be a mixture of the two. It can comprise modified nucleotides, comprising for example a modified bond, a modified purine or pyrimidine base, or a modified sugar. It can be prepared by any method known to one of ordinary skill in the art, including chemical synthesis, recombination, and/or mutagenesis. The nucleic acid according to the invention may be deduced from the amino acid sequence of the VHH molecules according to the invention and codon usage may be adapted according to the host cell in which the nucleic acid shall be transcribed. These steps may be carried out according to methods well known to one of ordinary skill in the art and some of which are described in the reference manual Sambrook *et al..*

Specific examples of such nucleic acids sequences include the sequences comprising any one of SEQ ID NOs: 301-329, 52-64 and 95-110, 469-606, 646-663, 683-690 or 721-738, with no tag coding portion or including the optional tag-coding portion of SEQ ID NO: 330, and the complementary sequence thereto, . The corresponding domains encoding CDR1, CDR2 and CDR3 are underlined in Table 2 below. The tag-coding portion of SEQ ID NO: 330 is in bold in Table 2.

The invention also relates to a vector containing such a nucleic acid, optionally under control of regulatory sequences (e.g., promoter, terminator, etc.). The vector may be a plasmid, virus, cosmid, phagemid, artificial chromosome, etc. In particular, the vector may comprise a nucleic acid of the invention operably linked to a regulatory region, i.e. a region comprising one or more control sequences. Optionally, the vector may comprise several nucleic acids of the invention operably linked to several regulatory regions. The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal-peptide sequence and transcription terminator. The term "operably linked" means a configuration in which a control sequence is placed at an appropriate position relative to a coding sequence, in such a way that the control sequence directs expression of the coding region. The present invention further relates to the use of a nucleic acid or vector according to the invention to transform, transfect or transduce a host cell or to produce a composition, including a pharmaceutical composition, to transform, transfect or transduce a host cell.

The present invention also provides a host cell comprising one or several nucleic acids of the invention and/or one or several vectors of the invention. The term "host cell" also encompasses any progeny of a parent host cell that is not identical to the parent host cell due to mutations that occur during replication. Suitable host cells may be prokaryotic (e.g., a bacterium) or eukaryotic (e.g., yeast, plant, insect or mammalian cell). Specific illustrative examples of such cells include E. coli strains, CHO cells, Saccharomyces strains, plant cells, sf9 insect cells etc.

### Uses

VHH molecules of the invention can bind to TfR and thus target/deliver molecules to TfR-expressing cells or organs. Within the context of this invention, binding is preferably specific, so that binding to TfR occurs with higher affinity than binding to any other antigen in the same species. In a particular embodiment, the VHH molecules of the invention specifically bind the human TfR1. In another particular embodiment, the VHH molecules of the invention bind the human and non-human primate TfR1. In another particular embodiment, the VHH molecules of the invention bind the human and rodent TfR1. In another particular embodiment, VHH molecules of the invention bind human TfR1, non-human primate TfR1 and rodent (e.g., a murine or rat) TfR1. In another particular embodiment, the VHH molecules bind the human, non-human primate and murine receptors with a substantially similar affinity.

The invention thus relates to methods of targeting/delivering a compound to/through a TfR-expressing cell or organ, comprising coupling said compound to at least one VHH of the invention.

The invention further relates to the use of a VHH such as defined above, as a vector for the transport of a compound to/through a TfR-expressing cell or organ.

The invention also relates to the use of a VHH such as defined above for preparing a drug capable of crossing the BBB.

The invention also relates to a method for enabling or improving the passage of a molecule across the BBB, comprising the coupling of the molecule to a VHH of the invention.

As explained herein above, the VHH of the invention may be used to transport or deliver any compound, such as for example small drugs, proteins, polypeptides, peptides, amino acids, lipids, nucleic acids, viruses, liposomes, exosomes, etc.

A vehicle may be used to transport or deliver the conjugate (including the VHH) such as for example a virus, a virus-like particle (VLP), a Cell-Derived Vesicle (CDV), an exosome, a lipid vehicle or a polymer vehicle, and is preferably a lipid nanoparticle (LNP), a micelle or a liposome.

The invention also relates to a pharmaceutical composition, in particular a diagnostic or therapeutic composition, characterized in that it comprises at least one VHH or conjugate compound, associated to, or present in, a vehicle or not, for example, in the context of a therapeutic composition, a VHH-drug conjugate such as defined above and one or more pharmaceutically acceptable supports, carriers or excipients.

The invention also in particular relates to a diagnostic composition characterized in that it comprises a VHH or conjugate compound, associated to, or present in, a vehicle or not, for example a VHH-diagnostic or medical imaging agent conjugate compound such as defined above.

The conjugate can be used in the form of any pharmaceutically acceptable salt. The expression "pharmaceutically acceptable salts" refers to, for example and in a non-restrictive way, pharmaceutically acceptable base or acid addition salts, hydrates, esters, solvates, precursors, metabolites or stereoisomers, said vectors or conjugates loaded with at least one substance of interest.

The expression "pharmaceutically acceptable salts" refers to nontoxic salts, which can be generally prepared by reacting a free base with a suitable organic or inorganic acid. These salts preserve the biological effectiveness and the properties of free bases. Representative examples of such salts include water-soluble and water-insoluble salts such as acetates, N-methylglucamine ammonium, amsonates (4,4-diaminostilbene-2,2'-disulphonates), benzenesulphonates, benzonates, bicarbonates, bisulphates, bitartrates, borates, hydrobromides, bromides, buryrates, camsylates, carbonates, hydrochlorates, chlorides, citrates, clavulanates, dichlorhydrates, diphosphates, edetates, calcium edetates, edisylates, estolates, esylates, fumarates, gluceptates, gluconates, glutamates, glycolylarsanylates, hexafluorophosphates, hexylresorcinates, hydrabamines, hydroxynaphthoates, iodides, isothionates, lactates, lactobionates, laurates, malates, maleates, mandelates, mesylates, methylbromides, methylnitrates, methylsulphates, mucates, napsylates, nitrates, 3-hydroxy-2-naphthoates, oleates, oxalates, palmitates, pamoates (1,1-methylene-bis-2-hydroxy-3-naphtoates, or emboates), pantothenates, phosphates, picrates, polygalacturonates, propionates, p-toluenesulphonates, salicylates, stearates, subacetates, succinates, sulphates, sulphosalicylates, suramates, tannates, tartrates, teoclates, tosylates, triethiodides, trifluoroacetates and valerianates.

The compositions of the invention advantageously comprise a pharmaceutically acceptable carrier or excipient. The pharmaceutically acceptable carrier can be selected from the carriers classically used according to each mode of administration. According to the mode of administration envisaged, the compounds can be in solid, semi-solid or liquid form. For solid compositions such as tablets, pills, powders, or granules that are free or are included in gelatin capsules, the active substance can be combined with: a) diluents, for example lactose, dextrose, sucrose, mannitol, sorbitol, cellulose and/or glycine; b) lubricants, for example silica, talc, stearic acid, its magnesium or calcium salt and/or polyethylene glycol; c) binders, for example magnesium and aluminum silicate, starch paste, gelatin, tragacanth, methylcellulose, sodium carboxymethyl cellulose and/or polyvinylpyrrolidone; d) disintegrants, for example starch, agar, alginic acid or its sodium salt, or effervescent mixtures; and/or d) absorbents, dyes, flavoring agents and sweeteners. The excipients can be, for example, mannitol, lactose, starch, magnesium stearate, sodium saccharin, talc, cellulose, glucose, sucrose, magnesium carbonate and analogues of pharmaceutical quality. For semi-solid compositions such as suppositories, the excipient can, for example, be an emulsion or oily suspension, or polyalkylene glycol-based, such as polypropylene glycol. Liquid compositions, in particular injectables or those included in a soft capsule, can be prepared, for example, by dissolution, dispersion, etc., of the active substance in a pharmaceutically pure solvent such as, for example, water, physiological saline solution, aqueous dextrose, glycerol, ethanol, oil and analogues thereof.

The compositions or conjugates of the invention can be administered by any suitable route and, in a non-restrictive way, by parenteral route, such as, for example, in the form of preparations that can be injected by subcutaneous, intravenous or intramuscular route or by intraventricular, intracerebral, intracerebroventicular (ICV) or intrathecal route; by oral route (or *per os*)*,* such as, for example, in the form of coated or uncoated tablets, gelatin capsules, powders, pellets, suspensions or oral solutions (one such form for oral administration can be either with immediate release or with extended or delayed release); by rectal route such as, for example, in the form of suppositories; by topical route, in particular by transdermal route, such as, for example, in the form of patches, pomades or gels; by intranasal route such as, for example, in aerosol and spray form; by perlingual route; or by intraocular route.

The pharmaceutical compositions typically comprise an effective dose of a VHH or conjugate of the invention. The "therapeutically effective dose" of the conjugate of the invention is for example of about 1 nmole to about 500 nmoles per kilo of body weight of the subject who will be administered with said conjugate (nmoles/kg), preferably of about 10 nmoles/kg to about 500 nmoles/kg. It is understood that the "therapeutically effective dose" for a person in particular will depend on various factors, including the activity/effectiveness of the active substance, its time of administration, its route of administration, its toxicity, its rate of elimination and its metabolism, drug combinations/interactions and the severity of the disease (or disorder) treated on a preventive or curative basis, as well as the age, weight, overall health, sex and/or diet of the patient.

Depending on the substance coupled, the conjugates and compositions of the invention can be used for imaging, diagnosing, preventing and/or treating pathologies or disorders, notably pathologies or disorders affecting the CNS, infectious pathologies or cancers. The VHH of the invention have the capacity to target TfR-expressing cells, particularly cells which exhibit marked expression of said receptor, such as notably cancer cells, nervous or non-nervous tissue and/or to cross cell membranes, notably those of the physiological barriers of the CNS and more particularly the blood-tumor barrier (BTB) of cancerous nervous tissue. The TfR is enriched in organs such as bone marrow, placenta and in the gastrointestinal tract. TfR is also highly expressed in brain endothelial cells but not in endothelial cells lining the vessels in other tissues. TfR expression has been confirmed at the plasma membrane of purified brain microvessels and cultured endothelial cells from rat, mouse, pig and non-human primate.

In this respect, the invention relates to the use of pharmaceutical conjugates or compositions as described above for treating or preventing CNS pathologies or disorders, brain tumors or other cancer cells, and bacterial, viral, parasitic or fungal infectious pathologies of the brain or other tissues.

The invention also relates to a VHH, conjugate, or compositions as described above for use for diagnosing, imaging or treating CNS pathologies or disorders, brain tumors or other cancer cells, and bacterial, viral, parasitic or fungal infectious pathologies of the brain or other tissues.

The invention also relates to a VHH, conjugate, or compositions as described above for use for treating, imaging and/or diagnosing a brain tumor or other types of cancer.

The invention to a VHH, conjugate or composition such as defined above for use for preventing, treating, imaging and/or diagnosing neurodegenerative pathologies such as, in a non-restrictive manner, Alzheimer's disease, Parkinson's disease, stroke, Creutzfeldt-Jakob disease, bovine spongiform encephalopathy, multiple sclerosis, amyotrophic lateral sclerosis, etc.

The invention also relates to a VHH, conjugate or composition such as defined above for use for preventing, treating, imaging and/or diagnosing neurological pathologies such as, in a non-restrictive manner, epilepsy, migraine, encephalitis, CNS pain, etc.

The invention also relates to a VHH, conjugate or composition such as defined above for use for preventing, treating, imaging and/or diagnosing rare diseases such as, in non-restrictive manner lysosomal storage diseases, Farber disease, Fabry disease, Gangliosidosis GM1 and GM2, Gaucher disease, different mucopolysaccharidoses etc.

The invention also relates to a VHH, conjugate or composition such as defined above for use for preventing, treating, imaging and/or diagnosing neuropsychiatric pathologies such as, in a non-restrictive manner, depression, autism, anxiety, schizophrenia, etc.

The invention also relates to a VHH, conjugate or composition such as defined above for use for preventing, treating, imaging and/or diagnosing cancers such as, in a non-restrictive manner, glioblastoma, pancreatic cancer, ovarian cancer, hepatocellular cancer, etc.

The invention also relates to a VHH, conjugate or composition such as defined above, wherein the conjugated agent is or comprises a virus or a virus-like particle, such as a recombinant virus. The invention may indeed be used to increase brain or cancer or any TfR enriched tissue delivery of recombinant (e.g., replication-defective or attenuated) viruses used in gene therapy, such as adenoviruses, adeno-associated viruses, lentiviruses, retroviruses, etc, or virus-like particles. Coupling to a virus or VLP may be performed e.g., by coupling to the capsid protein of the virus.

The invention also relates to methods for preventing or treating any of the above conditions or diseases by administering to a subject in need thereof a VHH, conjugate or composition of the invention.

The invention also relates to the use of a VHH, conjugate or composition of the invention for the manufacture of a medicament for treating any of the above conditions or diseases.

Other aspects and advantages of the present invention will become apparent upon consideration of the examples below.

### Examples

### EXAMPLE I: Validation of TfR expression at the BBB.

We analyzed cell membrane expression profile of the TfR in brain endothelium of various species. The kit ProteoExtract Subcellular Proteome Extraction Kit (Calbiochem, La Jolla, CA, USA) was used to prepare membrane extracts of digested or non-digested brain microvessels (BMVs) and of primocultures of brain microvascular endothelial cells (BMEC) from rat, mouse, pig and non-human primate (NHP; rhesus monkey) (Figure 1). Membrane extracts were quantified using the BioRad DC Protein Assay (Bio-Rad, Hercules, CA, USA) following manufacturer's instructions. Membrane proteins were separated by sodium dodecyl sulfate polyacrylamide gel electrophoresis (SDS-PAGE) on 4-12% polyacrylamide gels, and transferred onto nitrocellulose membranes (ThermoFisher Scientific). Proteins were probed with a primary antibody against TfR (Genetex GTX102596; 1/1000), followed by an HRP-conjugated donkey anti-rabbit IgG secondary antibody (Jackson ImmunoResearch) diluted 1/10000. Finally, proteins were detected using chemiluminescence. As shown in Figure 1, TfR is expressed in digested and non-digested brain microvessels from rat, mouse, pig and non-human primate. TfR is also expressed in brain endothelial cells from mouse rat and pig (note that only 1 µg of membrane proteins was loaded on SDS-PAGE for brain microvascular endothelial cells versus 10 µg or 5 µg for brain microvessels). TfR expression is enhanced in digested NHP brain microvessels. These data demonstrate that the TfR represents a valid target for designing molecules for *in vivo* applications.

### EXAMPLE II: Construction of CHO cell lines stably expressing human and mouse TfR.

The prerequisite to the identification and characterization of TfR-binding VHHs was the establishment in eukaryotic cells (Chinese hamster ovary cells, CHO) of stable cell lines expressing hTfR and mTfR, constitutively and at high rates. These cell lines were then used i) for the identification and characterization of agents binding to the receptor expressed at the cell surface, in its native configuration; and ii) to test whether the receptor could internalize such agents by endocytosis. For the construction of these cell lines, the cDNA coding for the hTfR was cloned using sequence information available in databases (accession number: NM_003234.3). The primers necessary for cDNA amplification by RT-PCR were selected (see SEQ ID NOs: 65 and 66, as shown below), comprising at their end (in bold type) the restriction sites (EcoRI and Sall) necessary for cloning in the pEGFP-C1 expression vector (Clontech) (Figure 2-A).

| Receptor | Primer sequences |
|---|---|
| hTfR | (F) ATATATGAATTCGGCTCGGGACGGAGGACGC (SEQ ID NO: 65) |
| | (R) TTAATTGTCGACAGAACTCATTGTCCCAACCGTCAC (SEQ ID NO: 66) |

Total RNA prepared from human brain was used for RT-PCR amplification of the cDNA fragment coding for hTfR. After amplification, the PCR product was digested by EcoRI-SalI restriction enzymes, and ligated in the pEGFP-C 1 expression vector (Clontech), digested by the same restriction enzymes. After transfection in eukaryotic cells, this vector enables the expression, under control of the CMV promoter, of the hTfR fused to EGFP at its N-Terminal end, *i.e.,* at the end of its intracellular domain. After transforming competent *E. coli* DH5α bacteria, obtaining isolated colonies and preparing plasmid DNA, both strands of the construct were fully sequenced for verification. Plasmid coding for the mTfR fused to EGFP was purchased from GeneCopoeia (plasmid reference: EX-Mm05845-M29). Transient transfections in CHO-K1 cells were carried out and used to select stable transfectants by limit dilution and resistance to antibiotic (G418). These cell lines were amplified while maintaining selective pressure. Confocal photomicrographs taken after immunocytochemistry on fixed (PFA) cell lines using Alexa647-conjugated Transferrin (Tf-Alexa647) confirm in Figure 2-B co-localization between EGFP (in green) and Tf-Alexa647 (in red) and therefore, good expression and functional binding of the receptor. Membrane expression of the receptors of the expected size was checked by western blot on cell membranes extracted with ProteoExtract Subcellular Proteome Extraction Kit. Antibodies were directed either against GFP or against the TfR. Proteins corresponding to the combined sizes of EGFP and h/mTfR (170 kDa), were recognized by an anti-GFP antibody and by an anti-TfR antibody (Figure 2-C). A CHO K1 wild type (WT) cell line was used as negative control and antibodies detected no proteins. These data confirm the expression of functional receptor at the cell surface of the CHO cell lines.

### EXAMPLE III:

### (A) Generation of VHHs that bind the TfR.

A llama (*Lama glama*) was immunized subcutaneously 4 times with membrane preparations from CHO stable cell lines expressing the human and murine receptors of interest. VHH library construction was performed as previously described (Alvarez-Rueda et al., 2007, Behar et al., 2009). Briefly, mRNAs coding for VHH were amplified by RT-PCR from the total RNAs of peripheral blood mononuclear cells isolated by ficoll gradient, and cloned into the pHEN1 phagemid. Reiterative selections enabled the isolation of phages presenting VHH exhibiting strong affinity for the TfR expressed at the cell surface. In total, more than 2000 clones were screened for their ability to bind the TfR, and roughly 450 clones were sequenced. VHHs with improved binding (to the murine, the non-human primate and/or the human cell lines), cell penetration and transport properties were obtained.

Illustrative firstly identified VHH are VHH A (C5), VHH B (B8), VHH C (B6), VHH D (H3) (see also the list of sequences). These VHHs do not bind to cells of the control CHO cell line. Furthermore, TfR-binding VHH with appropriate, improved binding properties, were generated by site-directed mutagenesis. More particularly, site directed mutagenesis was performed to introduce single alanine substitutions into the VHH A complementarity-determining regions (CDR) 1, 2 and 3, giving rise to the VHH A1 to A9 (C5^{V1} to CS^{V9}). VHH A1 and A2 were mutated in the CDR1, VHH A3 and A4 were mutated in the CDR2 and VHH A5 to A9 were mutated in the CDR3. Furthermore, single site directed mutagenesis was also performed by substituting some CDR amino acids by structurally-close amino acids. VHH A10-A19 (C5^{V10} to C5^{V19}) were obtained, wherein VHH A10 and A11 were mutated in the CDR1, VHH A12 to A14 were mutated in the CDR2, and VHH A15 to A19 were mutated in the CDR3. Moreover, humanized TfR-binding VHH were generated, to improve *in vivo* efficacy by, e.g., avoiding immunogenicity, and were designated VHH A20-A25 (CS^{h1} to CS^{h6}). In addition, tagged VHH molecules were produced, to facilitate purification and/or coupling. The amino acid sequences of each of these VHH are provided in the Sequence Listing.

### (B) Generation of further VHHs that bind the TfR.

Several strategies were further performed to generate VHHs that bind the TfR with different binding properties. To further improve the binding properties of the C5, site directed mutagenesis strategies in the complementarity-determining regions (CDR) 1, 2 and 3 were pursued, giving rise to the variants CS^{V20} to C5^{V29}. A negative control of the C5 was also generated by introducing 2 alanines in the CDR3, namely C5^{neg}.

Moreover, humanized C5 VHHs were generated to improve *in vivo* efficacy by, e.g., avoiding immunogenicity. Thus, different humanization schemes were applied in the framework regions (FR) 1, 2, 3 and 4, giving rise to the variants C5^{h8} to C5^{h26}, which present humanization rate compared to C5 between 89% and 96%. A humanization pattern was also applied to the VHHs B8, C5^{V13} and C5^{V16}, giving rise to the VHHs B8^{h1}, C5^{V13h20} and C5^{V16h20}.

To generate B8 and B6 variants, as well as new C5 variants, yeast display in combination with deep mutational scanning (DMS) were performed by a CRO (Deeptope, Paris), to give residue level resolution of positions in the VHH interface that are crucial for binding to the TfR. Thanks to the DMS maps highlighting the interesting positions to modify to impact the VHH binding, numerous B8 variants (B8^{V1} to B8^{V40}), C5 variants (C5^{V30} and to C5^{V32}) and B6 variants (B6^{V1} to B6^{V4}) were generated by site directed mutagenesis, in the CDRs or in the FRs.

The TfR receptor encompasses several extracellular domains. A strategy developed here was to use the VHH library described above to perform phage display selections and screenings on the apical domain of the TfR. In total, more than 700 clones were screened for their ability to bind the TfR apical domain using an ELISA assay, and roughly 150 clones were sequenced. Sequence analysis led then to the isolation of 10 VHHs of interest, that showed specific binding to the TfR apical domain. Illustrative VHHs are B7, H7, C12b, E4, E2, C3, F2a, E9, A9a, B4a. Noteworthy, VHHs B7, H7, C12b and E4 showed similar CDR3 sequences compared to B6, suggesting that they should bind to the same epitope.

Finally, a last strategy to enrich the diversity of the VHHs of the invention was to perform phage display selections on a CHO cell line stably expressing the rhesus monkey TfR (rhTfR). Screenings were then performed on CHO cell lines stably expressing the hTfR or the rhTfR, and on a CHO cell line that does not express the TfR (CHO TRVb). In total, more than 180 clones were screened for their ability to bind the rhTfR and the hTfR using flow cytometry experiments, and roughly 30 clones were sequenced. This strategy allowed the identification of particular VHHs that bind both the hTfR and the rhTfR, namely E8, ClOa and H1. The amino acid sequences of all the generated VHHs are provided in the enclosed Sequence Listing and in Table 1 below.

### EXAMPLE IV: Binding and endocytosis of purified VHHs of the invention.

To confirm the ability of selected VHH molecules to bind the TfR, and to be endocytosed, immunocytochemical experiments involving the incubation of VHHs on living CHO cell lines expressing the TfR fused to EGFP, detected using a mouse anti-cMyc primary antibody (ThermoFisher) followed by an Alexa594-conjugated donkey anti-mouse secondary antibody (Jackson ImmunoResearch), were performed and observed with a confocal microscope. The results obtained with VHH A are shown as an example. As shown in Figure 3, the VHH binds to the CHO-hTfR-EGFP (Figure 3-B) and CHO-mTfR-EGFP (Figure 3-A) cell lines and is incorporated by endocytosis to accumulate in the cells as shown using triton permeabilization, which is not the case for the control VHH (VHH Z) (Figure 3-C, D).

### EXAMPLE V: Determination of binding affinity of firstly identified VHHs to the human and mouse TfR.

The binding properties of firstly identified VHHs with affinity for the TfR were tested using flow cytometry, and apparent affinities (K_{d app}) were determined. All experiments were performed in 96 well plates using 2-3 x 10⁵ cells/well, at 4 °C with shaking. CHO cell lines expressing the TfR fused to EGFP or CHO WT cells were saturated with PBS/BSA 2% solution during 30 min to avoid nonspecific binding, followed by incubation with purified VHHs at concentrations ranging from 50 µM to 1 pM for 1 hr. After one wash in PBS/BSA 2%, cells were incubated for 1 hr with an anti-6His tag antibody (mouse), washed twice with PBS/BSA 2%, and incubated for 45 min with an Alexa647-conjugated anti-mouse secondary antibody. After two last washes in PBS/BSA 2%, cells were fixed (or not) by incubation for 15 min with PBS/PFA 2%, washed once with PBS and finally resuspended in PBS. Fluorescence levels were assessed using a MACSQuant flow cytometer (Miltenyi) or an Attune NxT flow cytometer (Thermo Fisher Scientific). There was no nonspecific labelling in the control conditions where cells were incubated with control VHH (VHH Z). All tested VHHs induced a concentration-dependent shift of the signal, confirming binding to the receptor of interest (Figure 4-A). No labeling of the CHO WT control cells was detected with all the tested VHHs (not shown). The VHH K_{d app} were calculated using GraphPad Prism software (Figure 4-B). K_{d app} were in the same range for all VHH, ranging from 7.5 nM (VHH B) to 56 nM (VHH D) on mTfR, and from 1.7 nM (VHH B) to 2.7 nM (VHH A) on hTfR.

### EXAMPLE VI: Competition assays between purified VHHs with affinity for the TfR and the natural ligand.

To evaluate the ability of selected VHHs to compete with Transferrin (Tf), the TfR natural ligand, for the binding to the receptor, competition assays using flow cytometry experiments were performed. In a first step, competitors in dilution series were incubated on CHO cells expressing the receptor of interest fused to EGFP, for 1 hr at 4°C. Secondly, tracers at EC90 were added and incubated 1 hr more, and were then detected with the appropriate revelation system (Figure 5). TfR-binding VHHs were used as tracers (Figure 5-B) and competitors (Figure 5-C). In all conditions, there was no competition between VHHs and the ligand Tf, suggesting than VHHs bind to TfR on an epitope different than that of Tf.

### EXAMPLE VII:

### (A) Determination of binding affinity of VHH A1-A19 to the human and mouse TfR.

The binding properties of VHH A1-A19 (C5^{V1} to C5^{V19}) for the TfR were tested using flow cytometry, and apparent affinities (K_{d app}) were determined. All experiments were performed in 96 well plates using 2 × 10⁵ cells/well, at 4 °C with shaking. CHO cell lines stably expressing the hTfR or the mTfR fused to EGFP or CHO WT cells were saturated with PBS/BSA 2% solution during 30 min to avoid non-specific binding, followed by incubation with purified VHHs at concentrations ranging from 50 µM to 5 pM for 1 hr. After one wash in PBS/BSA 2%, cells were incubated for 1 hr with an anti-6His tag antibody (mouse), washed twice with PBS/BSA 2%, and incubated for 45 min with an Alexa647-conjugated anti-mouse secondary antibody. After two last washes in PBS/BSA 2%, cells were fixed by incubation for 15 min with PBS/PFA 2%, washed once with PBS and finally resuspended in PBS and stored at 4 °C. Fluorescence levels were assessed using an Attune NxT Flow Cytometer (Thermo Fisher Scientific). VHH A1-A19 all induced a concentration-dependent shift of the signal on both cell lines (with the exception of VHH A12) confirming their efficient binding to the receptor of interest (Figures 11; 12). While VHH A, VHH A1 to A4, and VHH A10 to A15, showed similar Bₘₐₓ (plateau of the curve) on both hTfR and mTfR expressing cell lines, VHH A6 to A9 and VHH A16 to A19 showed slight to drastic lower Bₘₐₓ on both cell lines, as well as slight to strong curve shift. Only VHH A12 showed a lower Bₘₐₓ and a strong curve shift on the hTfR expressing cell line compared to the other VHH Ax. No labeling of the CHO WT control cells was detected with all the tested VHHs (not shown). The VHH K_{d app} were calculated using GraphPad Prism software (Figure 11-B; 12-B). Regarding the binding to the human TfR, VHH A, A1 to A4, A6, A9 to A11, and A13 to A17, all showed similar K_{d app} of about 3-4 nM. Conversely, VHH A5, A8, A18 and 19 showed slightly lower affinities of 9.2 to 25 nM, while VHH A7 and A12 showed drastically lower affinities of 255 nM and 363 nM, respectively. Regarding the binding to the mouse TfR, VHH A and A9 showed similar K_{d app} of about 50 nM. All other VHH Ax showed slightly lower affinities of 131 to 259 nM, with the exception of VHH A5, A8 and A18 that showed significantly lower affinities of 604 nM, 427 nM and 416 nM, respectively.

### (B) Determination of VHH binding properties to hTfR, mTfR and rhTfR.

The binding properties of VHHs with affinity for the TfR from different species (i.e., human (h), mouse (m) and rhesus monkey (rh)) were tested using flow cytometry, and apparent affinities (Kd app) were determined. All experiments were performed in 96 well plates using 2 × 10⁵ cells/well, at 4 °C with shaking. CHO cell lines expressing the hTfR, mTfR or rhTfR fused to EGFP were saturated with PBS/BSA 2% solution during 30 min to avoid nonspecific binding, followed by incubation with purified VHHs at increasing concentrations for 1 hr. After one wash in PBS/BSA 2%, cells were incubated for 1 hr with an anti-6His tag antibody (mouse), washed twice with PBS/BSA 2%, and incubated for 1 hr with an Alexa647-conjugated anti-mouse secondary antibody. After two last washes in PBS/BSA 2%, cells were fixed by incubation for 15 min with PBS/PFA 2%, washed once with PBS and finally resuspended in PBS. Fluorescence levels were assessed using an Attune NxT flow cytometer (Thermo Fisher Scientific). Experimental data were fitted with GraphPad Prism^{®} software using a nonlinear fit to determine the apparent Kd constants. There was no nonspecific labelling in the control conditions where cells were incubated with control VHH D12 or C5^{neg}, validating the C5 negative control. All tested VHHs induced a concentration-dependent shift of the signal on the hTfR, confirming binding to the receptor of interest. Table 5 hereafter summarizes apparent Kd obtained for the tested VHHs. Most VHH showed cross-species reactivity and bound all three human, mouse and rhesus monkey TfR with apparent Kd ranging from 0, 1 nM to 4 µM, highlighting the great interest and diversity of the VHH library generated.

**Table 5:**

| **VHH name** | **Apparent K_{d} on human TfR (nM)** | **Apparent K_{d} on mouse TfR (nM)** | **Apparent K_{d} on rhesus monkey TfR (nM)** |
|---|---|---|---|
| C5 | 2,7 (± 0,4) | 50 (± 13) | 4,9 (± 1,3) |
| B8 | 1,7 (± 0,3) | 7,5 (± 1,1) | 2,2 (± 1,2) |
| B6 | 2,1 (± 0,4) | NB | NB |
| H3 | 1,9 (± 0,3) | 56 (± 4,7) | 1,5 (± 0,3) |
| C5^{V1} | 3,2 (± 0,5) | 259 (± 62) | 13 (± 2,4) |
| C5^{V2} | 3,2 (± 0,6) | 136 (± 41) | 5,7 (± 1,8) |
| C5^{V3} | 3,3 (± 0,6) | 138 (± 42) | 7,0 (± 3,5) |
| C5^{V4} | 3,1 (± 0,5) | 179 (± 51) | 3,4 (± 0,7) |
| C5^{V8} | 23 (± 6,9) | 427 (± 146) | 494 (± 112) |
| C5^{V9} | 3,4 (± 0,5) | 47 (± 17) | 4,6 (± 1,4) |
| C5^{V10} | 3,4 (± 0,9) | 159 (± 70) | 5,1 (± 1,5) |
| C5^{V11} | 3,4 (± 0,8) | 197 (± 78) | 20 (± 12) |
| C5^{V13} | 3,5 (± 1,0) | 187 (± 76) | 28 (± 20) |
| C5^{V14} | 3,3 (± 0,8) | 158 (± 69) | 3,9 (± 0,9) |
| C5^{V15} | 3,7 (± 1,0) | 207 (± 93) | 10 (± 1,6) |
| C5^{V16} | 4,7 (± 1,2) | 131 (± 55) | 425 (± 39) |
| C5^{V18} | 12 (± 3,1) | 416 (± 151) | 481 (± 55) |
| C5^{V20} | 2,5 (± 0,5) | 125 (± 101) | 6,1 (± 2,3) |
| C5^{V21} | 2,4 (± 0,3) | 120 (± 60) | 8,2 (± 3,8) |
| C5^{V22} | 2,7 (± 0,7) | 131 (± 69) | 11 (± 6,2) |
| C5^{V23} | 4,5 (± 1,5) | 254 (± 169) | 353 (± 19) |
| C5^{V24} | 4,3 (± 0,9) | 218 (± 80) | 564 (± 114) |
| C5^{V25} | 4,5 (± 0,5) | 136 (± 31) | 429 (± 78) |
| C5^{V26} | 7,9 (± 1,2) | 211 (± 131) | 335 (± 53) |
| C5^{V27} | 3,1 (± 0,8) | 197 (± 74) | 178 (± 81) |
| C5^{V28} | 3,3 (± 0,2) | 160 (± 23) | 371 (± 93) |
| C5^{V29} | 2,9 (± 0,8) | 207 (± 95) | 66 (± 19) |
| C5^{V30} | 31 (± 12) | 696 (± 142) | 362 (± 68) |
| C5^{V31} | 11 (± 2,3) | 445 (± 45) | 256 (± 74) |
| C5^{V32} | 66 (± 31) | 1744 | 653 (± 82) |
| C5^{h2} | 267 (± 53) | 225 (± 100) | ND |
| C5^{h5} | 193 (± 113) | ND | ND |
| C5^{h8} | 987 (± 114) | 656 | ND |
| C5^{h9} | 6,1 (± 0,5) | 431 (± 59) | 493 (± 128) |
| C5^{h10} | 1069 (± 315) | 845 | NB |
| C5^{h11} | 1133 (± 302) | 1369 | 1244 (± 551) |
| C5^{h12} | 4,3 (± 0,7) | 331 (± 100) | 361 (± 42) |
| C5^{h13} | 8,3 (± 2,8) | 554 (± 241) | 730 (± 188) |
| C5^{h14} | 1105 (± 438) | 164 (± 29) | ND |
| C5^{h15} | 1091 (± 359) | 452 | ND |
| C5^{h16} | 8,1 (± 2,1) | 582 (± 59) | 760 (± 202) |
| C5^{h17} | 630 (± 375) | 898 (± 311) | NB |
| C5^{h18} | 11 (± 5,7) | 834 (± 309) | 957 (± 253) |
| C5^{h19} | 5,4 (± 1,7) | 1030 (± 250) | 521 (± 214) |
| C5^{h20} | 4,1 (± 1,7) | 22 (± 14) | 40 (± 7,9) |
| C5^{h21} | 8,8 (± 5,2) | 600 (± 67) | 1009 (± 91) |
| C5^{h22} | 4,9 (± 1,4) | 602 (± 113) | 755 (± 120) |
| C5^{h23} | 594 (± 179) | 1545 (± 877) | 1995 (± 173) |
| C5^{h24} | 4,1 (± 1,3) | 624 (± 211) | 641 (± 131) |
| C5^{h25} | 777 (± 173) | 755 (± 364) | NB |
| C5^{h26} | 4,0 (± 0,9) | 675 (± 177) | 656 (± 76) |
| B8^{V1} | 192 (± 63) | 313 (± 129) | 138 (± 31) |
| B8^{V2} | 0,1 (± 0,01) | 0,2 (± 0,02) | 0,1(± 0,02) |
| B8^{V3} | 36 (± 10) | 52 (± 14) | 19 (± 3,9) |
| B8^{V4} | 0,4 (± 0,08) | 0,6 (± 0,02) | 0,3 (± 0,08) |
| B8^{V5} | 2,3 (± 0,5) | 3,8 (± 0,3) | 2,1 (± 0,5) |
| B8^{V6} | 3,8 (± 0,9) | 16 (± 9,4) | 2,7 (± 0,4) |
| B8^{V7} | 5,7 (± 4,9) | 41 (± 19) | 2,6 (± 0,6) |
| B8^{V8} | 2,5 (± 0,8) | 8,1 (± 1,8) | 2,1 (± 0,6) |
| B8^{V9} | 2,8 (± 0,9) | 31 (± 10) | 24 (± 0,5) |
| B8^{V11} | 4,6 (± 0,5) | 22 (± 11) | 2,0 (± 0,3) |
| B8^{V12} | 4,7 (± 0,8) | 743 (± 210) | 6,9 (± 4,3) |
| B8^{V14} | 4,8 (± 0,2) | 41 (± 20) | 1,9 (± 0,3) |
| B8^{V15} | 4,7 (± 1,0) | 684 (± 114) | 4,3 (± 1,3) |
| B8^{V16} | 6,2 (± 1,6) | 3,6 (± 0,5) | 307 (± 234) ! |
| B8^{V17} | 4,8 (± 0,6) | 5,2 (± 0,6) | 2,5 (± 0,7) |
| B8^{V18} | 3,8 (± 0,8) | 43 (± 29) | 1,6 (± 0,2) |
| B8^{V19} | 6,2 (± 1,6) | 7,6 (± 0,9) | 2,3 (± 0,07) |
| B8^{V20} | 4,5 (± 0,4) | 6,4 (± 2,4) | 1,8 (± 0,08) |
| B8^{V21} | 29 (± 6,6) | 3352 (± 1293) | 469 (± 218) |
| B8^{V22} | 321 (± 131) | NB | 971 (± 135) |
| B8^{V23} | 29 (± 6,6) | 1551 (± 766) | 939 (± 332) |
| B8^{V24} | 1011 (± 114) | NB | LB |
| B8^{V25} | 14 (± 2,4) | 2353 (± 1099) | 425 (± 226) |
| B8^{V27} | 6,8 (± 0,4) | 760 (± 205) | 16 (± 3,6) |
| B8^{V28} | 31 (± 3,9) | 2499 (± 1220) | 755 (± 427) \| |
| B8^{V29} | 9,0 (± 1,6) | 1667 (± 696) | 72 (± 22) |
| B8^{V30} | 24 (± 3,7) | 3319 (± 1284) | 505 (± 320) |
| B8^{V31} | 14 (± 0,7) | 1689 (± 462) | 20 (± 3,5) |
| B8^{V32} | 7,1 (± 2,7) | 1208 (± 259) | 14 (± 0,08) |
| B8^{V33} | 50 (± 42) | 230 | 170 (± 35) \| |
| B8^{V34} | 13 (± 4,2) | 1069 | 68 (± 27) |
| B8^{V35} | 7,1 (± 1,6) | 513 | 23 (± 10) |
| B8^{V36} | 27 (± 16) | 891 | 130 (± 35) |
| B8^{V37} | 97 (± 31) | 582 | 334 (± 150) |
| B8^{V38} | 23 (± 13) | 1776 | 123 (± 71) |
| B8^{V39} | 16 (± 8,9) | 1290 | 200 (± 149) |
| B8^{V40} | 42 (± 29) | 1582 | 69 (± 24) |
| C5^{V13h20} | 3,8 (± 1,5) | 246 (± 163) | 549 (± 258) |
| C5^{V16h20} | 23 (± 14) | 622 (± 361) | 793 (± 147) |
| B8^{h1} | 4,1 (± 2,3) | 2,8 (± 1,1) | 1,9 (± 0,3) |
| C5^{neg} | NB | NB | NB |
| B6^{V1} | 2,3 (± 0,9) | NB | NB |
| B6^{V2} | 643 (± 127) | NB | NB |
| B6^{V3} | 2,6 (± 1,1) | NB | NB |
| B6^{V4} | 5,9 (± 0,8) | NB | NB |
| B7 | 1,9 (± 0,8) | NB | NB |
| H7 | 2,9 (± 0,9) | NB | NB |
| C12b | 630 (± 465) | NB | NB |
| E4 | 3,1 (± 2,0) | NB | NB |
| E2 | 2,5 (± 1,8) | NB | NB |
| C3 | 1,8 (± 1,1) | NB | 4227 (± 752) |
| F2a | 2,9 (± 1,8) | NB | NB |
| E9 | 1287 (± 400) | 1307 (± 1035) | 1537 (± 924) |
| A9a | 1864 (± 428) | 841(± 841) | 965 (± 394) |
| B4a | 692 (± 335) | 409 (± 203) | 479 (± 137) |
| E8 | 3,4 (± 0,4) | NB | 2,1 (± 0,7) |
| ClOa | 4,1 (± 0,9) | NB | 3,2 (± 1,0) |
| H1 | 11 (± 2,7) | NB | 3,7 (± 1,2) |
| D12 (neg ctrl) | NB | NB | NB |

| | | | |
|---|---|---|---|
| NB: no binding | | | |

Binding properties of some VHHs were also evaluated using Surface Plasmon Resonance (SPR) experiments. The human, mouse and rhesus monkey TfR (having respectively GeneBank number NM_003234.2, NM_011638, NC_041755.1) ectodomains were fused to the N-terminal end of a mouse IgG1 Fc fragment and the recombinant proteins were produced and purified in-house. Interaction of VHHs with the receptors was tested using a Biacore T200 (GE Healthcare). Receptors were immobilized on an HC 1500M or a CMS sensor chip (Xantec) either directly or on an anti-mouse IgG previously immobilized. VHHs were injected over flowcell by using the single-cycle kinetic or the multiple-cycle kinetic methods. Table 6 hereafter summarizes the binding properties of the tested VHHs. These results illustrate once again the cross-species reactivity of the VHHs, and the diversity of binding parameters.

**Table 6:**

| **VHH name** | **On-rate Kₒₙ (M⁻¹ s⁻¹)** | **Off-rate K_{off} (s⁻¹)** | **K_{D} (M)** | **K_{D} at equilibrium (M)** |
|---|---|---|---|---|
| **Human TfR** | | | | |
| C5 | 6,65E+05 | 1,79E-04 | 2,06E-10 | ND |
| B8 | 6,77E+05 | 3,06E-05 | 5,59E-11 | ND |
| B6 | 1,73E+06 | 1,21E-03 | 6,59E-10 | ND |
| H3 | 1.01E+06 | 3,30E-05 | 3,33E-11 | ND |
| C5^{V1} | 5,55E+05 | 4,87E-04 | 8,76E-10 | ND |
| C5^{V5} | 3,32E+05 | 5,09E-02 | 1,54E-07 | 1.80E-07 |
| C5^{V8} | 5,04E+05 | 5,58E-02 | 9,48E-08 | 8,43E-08 |
| C5^{V11} | 7,90E+05 | 3,11E-04 | 3,95E-10 | ND |
| C5^{V13} | 1.14E+06 | 3,92E-04 | 3,57E-10 | ND |
| C5^{V15} | 7,00E+05 | 1,76E-04 | 2,52E-10 | ND |
| C5^{V16} | 4,59E+05 | 5,77E-03 | 1,45E-08 | ND |
| C5^{V18} | 4,87E+05 | 2,10E-02 | 4,33E-08 | 3,78E-08 |
| C5^{V19} | 4,26E+05 | 9,70E-03 | 2,23E-08 | 7,13E-09 |
| C5^{V29} | 1.17E+06 | 9,82E-04 | 8,46E-10 | ND |
| C5^{V30} | 3,19E+05 | 3,00E-02 | 9,40E-08 | 1,12E-07 |
| C5^{V31} | 3,83E+05 | 1,82E-02 | 4,75E-08 | 6,48E-08 |
| C5^{V32} | 6,92E+05 | 2,47E-02 | 3,58E-08 | 3,86E-08 |
| C5^{h9} | 5,49E+05 | 5,82E-03 | 1,06E-08 | ND |
| C5^{h18} | 4,62E+05 | 1,30E-02 | 3,09E-08 | 2,73E-08 |
| C5^{h19} | 6,24E+05 | 9,04E-03 | 1.63E-08 | 1,32E-08 |
| C5^{h20} | 8,66E+05 | 1,05E-03 | 9,85E-10 | ND |
| C5^{h22} | 1.50E+06 | 9,06E-03 | 8,96E-09 | ND |
| C5^{h25} | ND | ND | ND | 1,55E-06 |
| B8^{V1} | 1.83E+05 | 1.10E-03 | 6,04E-09 | ND |
| B8^{V3} | 6,69E+04 | 3,38E-05 | 5,05E-10 | ND |
| B8^{V12} | 1.04E+06 | 1.69E-03 | 9,84E-10 | ND |
| B8^{V16} | 1.03E+06 | 1,33E-02 | 1,28E-08 | 7,63E-08 |
| B8^{V22} | 6,98E+05 | ND | 4,75E-07 | 4,80E-07 |
| B8^{V27} | 1.01E+06 | 7,22E-03 | 7,29E-09 | 3,22E-09 |
| B8^{V29} | 6,82E+05 | 1.69E-02 | 4,40E-08 | 2,18E-08 |
| B8^{V31} | 3,64E+05 | 2,21E-02 | 6,08E-08 | 6,38E-08 |
| B8^{V32} | 4,20E+05 | 1,26E-02 | 3,01E-08 | 3,65E-08 |
| B8^{V33} | 7,10E+05 | 3,28E-02 | 4,62E-08 | 4,65E-08 |
| B8^{V34} | 7,33E+05 | 2,44E-02 | 3,33E-08 | 3,24E-08 |
| B8^{V35} | 6,98E+05 | 1,43E-02 | 2,06E-08 | 2,39E-08 |
| B8^{V36} | 5,71E+05 | 1.90E-02 | 3,33E-08 | 3,92E-08 |
| B8^{V37} | 6,99E+05 | 4,93E-02 | 7,05E-08 | 7,55E-08 |
| B8^{V38} | 5,96E+05 | 1.92E-02 | 3,22E-08 | 3,58E-08 |
| B8^{V39} | 4,48E+05 | 1,75E-02 | 3,91E-08 | 4,03E-08 |
| B8^{V40} | 6,72E+05 | 2,83E-02 | 4,21E-08 | 4,25E-08 |
| C5^{V13h20} | 7,86E+05 | 3,57E-03 | 4,55E-09 | ND |
| C5^{V16h20} | 3,60E+05 | 3,99E-02 | 1.12E-07 | 1.15E-07 |
| B8^{h1} | 4,44E+05 | 9,49E-05 | 2,12E-10 | ND |
| B7 | 1,16E+06 | 7,58E-04 | 6,52E-10 | ND |
| H7 | 1,13E+06 | 1,47E-03 | 1,30E-09 | ND |
| C12b | ND | ND | ND | 2,44E-06 |
| E4 | 1,51E+06 | 1.89E-03 | 1,25E-09 | ND |
| C3 | 5,90E+06 | 2,49E-03 | 5,59E-10 | ND |
| F2a | 1,39E+06 | 7,52E-04 | 5,45E-10 | ND |
| C10a | 1,88E+05 | 7,26E-03 | 4,46E-08 | 8,68E-08 |
| H1 | 1,08E+05 | 5,54E-03 | 5,77E-08 | 2,24E-07 |

| **Mouse TfR** | | | | |
|---|---|---|---|---|
| C5 | 9,15E+05 | 4,47E-02 | 8,85E-08 | 8,82E-08 |
| B8 | 1,23E+06 | 2,43E-02 | 2,70E-08 | 3,75E-08 |
| H3 | 5,81E+06 | 9,09E-03 | 1,56E-08 | ND |
| C5^{V1} | 5,46E+05 | 1,20E-01 | 2,44E-07 | 2,28E-07 |
| C5^{V2} | 5,83E+05 | 5,79E-02 | 1,09E-07 | 1,08E-07 |
| C5^{V3} | 6,47E+05 | 4,67E-02 | 8,35E-08 | 9,88E-08 |
| C5^{V4} | 6,52E+05 | 5,32E-02 | 8,97E-08 | 9,18E-08 |
| C5^{V9} | 5,24E+05 | 1,97E-02 | 4,08E-08 | 4,05E-08 |
| C5^{V11} | 3,55E+05 | 2,18E-02 | 6,13E-08 | 7,47E-08 |
| C5^{V13} | 2,31E+05 | 1,82E-02 | 7,89E-08 | 1,13E-07 |
| C5^{V15} | 7,97E+05 | 5,63E-02 | 7,06E-08 | 6,11E-08 |
| C5^{V27} | 4,93E+05 | 1,02E-01 | 2,07E-07 | 3,79E-07 |
| C5^{V29} | 4,72E+05 | 6,75E-02 | 1,43E-07 | 2,40E-07 |
| C5^{h18} | 8,16E+05 | 2,35E-01 | 3,36E-07 | 3,00E-07 |
| C5^{h19} | 1,28E+04 | 8,25E-03 | 6,43E-07 | 2,75E-06 |
| B8^{V1} | 6,51E+03 | 1,25E-02 | 1,96E-06 | ND |
| B8^{V3} | 1,78E+05 | 3,43E-02 | 1,93E-07 | 2,06E-07 |
| C5^{V16h20} | 2,51E+05 | 7,50E-02 | 3,01E-07 | ND |

| **Rhesus monkey TfR** | | | | |
|---|---|---|---|---|
| C5 | 1,55E+06 | 7,74E-03 | 5,35E-09 | 1,31E-08 |
| B8 | 1,00E+06 | 9,54E-05 | 9,S 1E-11 | ND |
| C5^{V27} | 3,32E+05 | 2,78E-02 | 8,39E-08 | ND |
| C5^{V30} | 5,15E+05 | 1,92E-01 | 3,73E-07 | 3,39E-07 |
| C5^{V31} | 5,24E+05 | 1,23E-01 | 2,34E-07 | 2,42E-07 |
| C5^{V32} | 7,45E+05 | 2,66E-01 | 3,58E-07 | 2,65E-07 |
| B8^{V12} | 9,97E+05 | 1,19E-02 | 1,21E-08 | 2,S 1E-08 |
| B8^{V16} | 3,25E+05 | 4,38E-02 | 1,35E-07 | 2,22E-07 |
| B8^{V27} | 4,55E+05 | 2,06E-02 | 5,38E-08 | 3,68E-08 |
| B8^{V29} | 1,16E+06 | 9,42E-02 | 2,02E-07 | 1,00E-07 |
| B8^{V31} | 1,59E+06 | 5,09E-02 | 3,21E-08 | 3,54E-08 |
| B8^{V32} | 1,28E+06 | 4,18E-02 | 3,27E-08 | 3,S 1E-08 |
| B8^{V33} | 6,93E+05 | 5,09E-02 | 7,34E-08 | 8,60E-08 |
| B8^{V34} | 1,97E+06 | 1,02E-01 | 5,15E-08 | 4,94E-08 |
| B8^{V35} | 8,98E+05 | 3,03E-02 | 3,37E-08 | 3,44E-08 |
| B8^{V36} | ND | ND | ND | 2,06E-07 |
| B8^{V37} | 7,00E+05 | 1,03E-01 | 1,48E-07 | 1,75E-07 |
| B8^{V38} | 5,49E+05 | 2,55E-02 | 4,63E-08 | 5,54E-08 |
| B8^{V39} | 3,62E+05 | 3,27E-02 | 9,05E-08 | 9,33E-08 |
| B8^{V40} | 5,75E+05 | 2,00E-02 | 3,48E-08 | 3,98E-08 |
| C10a | 9,03E+05 | 1,78E-02 | 2,46E-08 | 3,81E-08 |
| H1 | 6,62E+05 | 1,09E-02 | 2,22E-08 | 2,78E-08 |

| | | | | |
|---|---|---|---|---|
| ND: not determined | | | | |

### EXAMPLE VIII: Binding and endocytosis of purified VHH-Fc fusion molecules with affinity for TfR and affinity determination.

Anti-TfR VHH molecules of the invention were fused to an IgG Fc fragment. To produce the fusion protein, DNA fragments encoding the VHHs (with no tag) were amplified by PCR and cloned into the pINFUSE-IgG1-Fc2 vector (InvivoGen) in order to encode a human IgG1-Fc fragment encompassing in its N-ter or in its C-ter the VHHs. Fusion proteins were prepared using the Expi293 Expression System according to the manufacturer's instructions (Life Technologies). Seventy-two hrs post-transfection, supernatants were recovered and purified using Protein A GraviTrap columns (GE Healthcare). The purified fusion proteins were quantified using an in-house anti-Fc ELISA. Immunocytochemistry experiments on CHO cell lines expressing the TfR fused to EGFP, involving the incubation of VHH-Fc fusion proteins on living cells, detected using an Alexa594-conjugated anti-hFc antibody (Jackson ImmunoResearch), photographed with a confocal microscope, were performed to confirm the ability of fusion proteins to bind the targeted receptor of interest. The results demonstrate that conjugates of the invention can bind and be endocytosed by cells (Figure 7). No binding of a control VHH-Fc conjugate (VHH Z-Fc) on cells was observed, showing the specificity of the interaction.

The binding properties of VHH-Fc and Fc-VHH fusion proteins with an affinity for the TfR were tested in flow cytometry experiments, and apparent affinity (K_{d app}) were determined. All experiments were performed in 96 well plates using 2-3 × 10⁵ cells/well, at 4 °C with shaking. CHO cell lines expressing the receptors of interest fused to EGFP or CHO WT cells were saturated with PBS/BSA 2%, followed by an incubation with purified VHH-Fcs or Fc-VHHs at concentrations ranging from 350 nM to 0,03 pM for 1 hr. After washes, cells were incubated for 1 hr with an Alexa647-conjugated anti-hFc antibody (Jackson ImmunoResearch). After 3 last washes and cells resuspension in PBS, fluorescence was immediately measured using a MACSQuant flow cytometer (Miltenyi), and results were analyzed with the MACSQuant software. All VHH-Fc and Fc-VHH fusion proteins induced a concentration-dependent shift of the signal, confirming binding to the receptor of interest. The VHH-Fc and Fc-VHH K_{d app} were calculated using GraphPad Prism software (Figure 8-B). The K_{d app} of almost all VHHs were greatly improved by the conjugation with an Fc fragment, with K_{d app} ranging from 0.44 nM to 51 nM for TfR-binding VHH-Fcs and Fc-VHHs.

### EXAMPLE IX: Endocytosis and transport of VHHs of the invention in an in vitro BBB model.

We used rat or mouse brain microvascular endothelial cells (BMEC) and rat or mouse astrocytes to set up the co-culture model. This type of *in vitro* BBB model is used to evaluate the passive passage or active transport of numerous molecules, notably pharmacological agents, across BMEC and thus, by extrapolation, their capacity to reach CNS tissue *in vivo.* The different models developed to date (bovine, porcine, murine, human) have ultrastructural properties characteristic of the brain endothelium, notably tight junctions, absence of fenestrations, low permeability to hydrophilic molecules and high electrical resistance. Moreover, these models have shown solid correlations between the results of measurements taken on various molecules evaluated *in vitro* and *in vivo* for their property of passing across the BBB. To date, all the data obtained show that these *in vitro* BBB models mimic the situation *in vivo* by reproducing some of the complexities of the cell environment that exist *in vivo,* while preserving the advantages associated with cell culture experimentation. For example, the *in vitro* rat BBB model brings into play a co-culture of BMEC and astrocytes (Molino et al., 2014). Prior to cell culture, membrane inserts (Corning, Transwell 1.0 µm porosity, for 96-well or 12-well plates) were treated on the upper part with collagen type IV and fibronectin in order to enable optimal adhesion of BMEC and to create the conditions of a basal lamina. Primary cultures of mixed astrocytes were established from neonatal rat cerebral cortex. Briefly, meninges were removed and the cortical pieces were mechanically, then enzymatically dissociated in a trypsin solution. Dissociated cells were seeded into cell culture flasks in glial cell media (GCM) containing DMEM supplemented with 10% fetal bovine serum then frozen in liquid nitrogen for later use. Primary cultures of BMEC were prepared from 5-6 weeks old Wistar rats. Briefly, the cortical pieces were mechanically then enzymatically dissociated in a collagenase/dispase solution. The digested tissues were separated by a density-dependent centrifugation in 25% bovine serum albumin. The microvessels pellet were seeded on culture flask, pre-coated with collagen type IV and fibronectin, in endothelial cell media (ECM) containing DMEM/F12 supplemented with 20% bovine platelet poor plasma derived serum and basic fibroblast growth factor (bFGF) 2 ng/ml. Five days before the establishment of the co-culture, astrocytes were thawed and plated in 12-well or 96-well plates (abluminal compartment). The BMEC were then distributed on the upper surface of the filters (luminal compartment) in co-culture. Under these conditions, *in vitro* models differentiate, express junction-related proteins within 3 days and remain optimally differentiated during 3 more days. The binding/uptake at the BBB of inventive VHHs conjugated to the human Fc fragment of an IgG1 antibody (VHH-Fc) was verified on the *in vitro* rat model described above (Figure 9). VHH A-Fc or VHH B-Fc were co-incubated with Tf-Alexa647 for 2 hrs on live rBMEC monolayers at 37°C (Figure 9A). Following this co-incubation, the cell monolayer was washed extensively and fixed with PFA 4%. The cell monolayer was permeabilized with a solution of 0.1% triton X-100. VHH-Fcs were detected using immunostaining with an antibody against the human Fc fragment. Then confocal microscopy was used to assess the co-localization between fluorescence signal of VHH A-Fc or VHH B-Fc with Tf-A647 (Figure 9A). The results show that, following this 2 hr co-incubation, VHH A-Fc and VHH B-Fc were readily endocytosed and co-localized almost perfectly with Tf-Alexa647. This analysis of co-localization of different TfR ligands (VHH A-Fc, VHH B-Fc and Tf-A647) confirmed the specificity of the inventive VHHs to their target receptor. For transport across the rBMEC monolayers to the abluminal compartment, the VHH-Fcs were incubated at 10 nM in the luminal compartment of the culture system for 24 hrs to 72 hrs (Figure 9-C, D). Prior to experiment, filter inserts, containing rBMEC monolayers were placed in 96-well plates containing fresh transport buffer (75 µl in the luminal and 250 µl in the abluminal compartments). To evaluate the integrity of the BBB *in vitro* and the absence of toxicity for the endothelial cells, VHH-Fcs were co-incubated with lucifer yellow (LY), a small fluorescent molecule that does not cross the BBB. 24 hrs after incubation, the inserts were transferred to another 96-well plate containing fresh transport buffer for another interval of 48 hrs. At the end of transport, LY accumulated in the abluminal compartment was quantified by fluorescence spectrophotometry and results were expressed as endothelial surface permeability (or Pe) in 10⁻³ cm/min. The *in vitro* barrier was considered "permeable" or "open" if the Pe value of LY was greater than 0.6×10⁻³ cm/min. Transendothelial electrical resistance (TEER), measured with an ohmmeter and expressed in ohm.cm², also makes it possible to measure BBB integrity *in vitro* during tests of passage across the BBB. The quality threshold value is set at >400 ohm.cm². The experiments carried out show an absence of toxicity of the VHH-Fcs, and an absence of deleterious effects on the permeability properties of the BBB (not shown). The content of Fc-fragment of inventive VHH-Fcs in the inputs (T0), the luminal compartments at the end of transport experiment (T72 hrs, product recovery) and the abluminal compartments (transport intervals of 24 hrs and +48hrs) were quantified using an in-house anti-Fc ELISA assay with sensitivity between 0.5-50 femtomoles. Absorbance units were transformed in femtomoles per insert (surface area of 0, 143 cm² for inserts of a 96-well plate). Our results show that VHH B-Fc and VHH A-Fc conjugates show higher transport than VHH Z-Fc (negative control), around 10-fold at 24 hrs and 5-fold at 72 hrs. This transport reached an apparent saturation between 24 hrs and 72 hrs, further suggesting the involvement of a specific and saturable receptor mediated process (Figure 9-D).

### EXAMPLE X: Pharmacokinetic and organ uptake of VHH-Fc conjugates in vivo.

To assess the potential of VHH-Fc conjugates of the invention to target organs enriched with receptors of interest *in vivo,* conjugates VHH A-Fc, VHH A-Fc-Agly and VHH Z-Fc were injected into tail vein at 5 mg/kg and the mice were perfused with saline at different times. Plasmas and brains were collected. Brains were processed by the capillary depletion method to isolate brain parenchyma from capillary. The amount of VHH-Fc in plasma, brain parenchyma and microvessels was measured using an in-house anti-Fc ELISA. Results are presented as concentrations (nM), or by organ-to-plasma ratio (Figure 10). TfR-binding conjugates VHH A-Fc and VHH A-Fc-Agly, exhibit a significant brain targeting at 2 hrs pi, with concentrations of 0.25 and 0.32 nM in brain parenchyma for VHH A-Fc and VHH A-Fc-Agly respectively, compared to 0.07 nM for the control VHH Z-Fc (Figure 10-B). When looking at parenchyma-to-plasma ratios, a clear advantage is confirmed, especially at 24 hrs pi where VHH A-Fc-Agly is still measurable in brain parenchyma whereas there is only 8 nM present in plasma (Figure 10-D). In microvessels, VHH A-Fc and VHH A-Fc-Agly accumulate significantly more than VHH Z-Fc at 2 hrs pi, with concentrations 9 and 5 times higher, respectively. Moreover, VHH A-Fc concentration in microvessels is still 3 times higher than VHH Z-Fc at 24 hrs pi (Figure 10-C). These results were confirmed when looking at microvessel-to-plasma ratios (Figure 10-E). These results demonstrate that TfR-targeting VHH of the invention can be used to effectively deliver or to improve pharmacokinetic properties of agents, notably protein cargos.

### EXAMPLE XI: Design and production of a therapeutic antibody fused to a VHH.

Anti-TfR VHH A, A1 A5, A6, A7 and A8 of the invention (with no tag) were fused to the mouse IgG1 13C3 monoclonal antibody, with high specific affinity for the protofibril form of β-amyloid peptide (WO2009/065054). To produce the 13C3-HC-VHH fusion proteins, a DNA fragment encoding the selected VHH was synthetized and cloned into the 13C3 heavy chain (HC) vector in order to encode the 13C3-HC-VHH conjugate containing, in its C-ter, the selected VHH sequence fused to the antibody heavy chain C-ter amino acid residue. In another set of experiments, the DNA fragment encoding the selected VHH was cloned into the 13C3 light chain (LC) vector in order to encode the 13C3 LC conjugate containing in its C-ter the selected VHH sequence fused to the antibody light chain C-ter amino acid residue. Fusion proteins were produced using the Expi293^{™} Expression System according to the manufacturer's instructions (Life Technologies). Seventy-two hrs post-transfection, supernatants were recovered and purified using HiTrap^{®} Protein G High Performance columns (GE Healthcare). The purified fusion proteins were quantified using 280 nm absorbance measurement.

The amino acid sequence of a 13C3-HC-VHHA conjugate is provided as SEQ ID NO: 93:

QVQLQQSGPELVRPGVSVKISCKGSGYTFTDYAMHWVKQSHAKSLEWIGVISTKYGKTNY
NQKFKGKATMTVDKSSSTAYMELARLTSEDSAIYYCARGDDGYSWGQGTSVTVSSAKTTPP
SVYPLAPGSAAQTNSMVTLGCLVKGYFPEPVTVTWNSGSLSSGVHTFPAVLQSDLYTLSSSVTVP
SSTWPSETVTCNVAHPASSTKVDKKIVPRDCGCKPCICTVPEVSSVFIFPPKPKDVLTITLTPKVTC
VVVDISKDDPEVQFSWFVDDVEVHTAQTQPREEQFNSTFRSVSELPIMHQDWLNGKEFKCRVNS
AAFPAPIEKTISKTKGRPKAPQVYTIPPPKEQMAKDKVSLTCMITDFFPEDITVEWQWNGQPAEN
YKNTQPIMDTDGSYFVYSKLNVQKSNWEAGNTFTCSVLHEGLHNHHTEKSLSHSPGGGGGMA
EVQLVESGGGVVQPGGSLKLSCVASGTDFSINFIRWYRQAPGKQREFVAGFTATGNTNYADSMK
GRFTISRDNTKNAVYLQIDSLKPEDTAVYYCYMLDKWGQGTQVTVSSAAA

In bold is the 13C3 Variable Heavy Chain sequence; underlined is the 13C3 Constant Heavy Chain sequence; bold and underlined is a Gly linker; double underline MA and C-ter AAA residues result from cloning and may be optionally removed. The remaining is the VHH.

The amino acid sequence of a 13C3-LC-VHHA conjugate is provided as SEQ ID NO: 94:

DVVMTQTPLSLPVSLGDQASISCRSGQSLVHSNGNTYLHWYLQKPGQSPKLLIYTVSNRFSG
VPDRFSGSGSGSDFTLKISRVEAEDLGVYFCSQNTFVPWTFGGGTKLEIKRADAAPTVSIFPPS
SEQLTSGGASVVCFLNNFYPKDINVKWKIDGSERQNGVLNSWTDQDSKDSTYSMSSTLTLTKDE
YERHNSYTCEATHKTSTSPIVKSFNRNECSGSGGGGGMAEVQLVESGGGVVQPGGSLKLSCVA
SGTDFSINFIRWYRQAPGKQREFVAGFTATGNTNYADSMKGRFTISRDNTKNAVYLQIDSLKPED
TAVYYCYMLDKWGQGTQVTVSSAAA

In bold is the 13C3 Variable kappa Light Chain sequence; FGGGTK is the J region; LEIKR is a multiple cloning site; underlined is the 13C3 Constant kappa Light Chain sequence; bold and underlined is a Gly linker; double underline MA and C-ter AAA residues result from cloning and may be optionally removed. The remaining is the VHH.

### Determination of binding affinity

The binding properties of 13C3 conjugates of the invention for the TfR were tested using flow cytometry, and apparent affinities (K_{d app}) were determined. All experiments were performed in the same conditions than described in example VII, with 13C3 constructs incubated at concentrations ranging from 15 µM to 7 pM and detected with an Alexa647-conjugated anti-mouse antibody. All 13C3-HC-VHH fusion proteins induced a concentration-dependent shift of the signal on both hTfR and mTfR expressing cell lines, confirming binding to the receptor (Figure 13). All 13C3 fusion proteins showed the same hTfR binding profile, with the exception of the VHH A7 fusion that showed a slightly lower Bₘₐₓ. All fusion proteins showed different binding profiles on the mTfR, with the 13C3-HC-VHH A1 fusion showing a 2-fold lower Bₘₐₓ than the 13C3-HC-VHH A, while A5 to A8 13C3 fusions showed very low Bₘₐₓ. The 13C3 fusions K_{d app} were calculated using GraphPad Prism software (Figure 13-B). Affinities for the hTfR were similar for all fusions, with K_{d app} of about 10-20 nM. Despite different Bₘₐₓ, VHH A, A1 and A6 13C3 HC fusions showed similar affinities of 10 to 20 nM, while 13C3-HC-VHH A8 and 13C3-LC-VHH A fusions showed lower affinities of 315 nM and 106 nM, respectively.

### EXAMPLE XII: Brain uptake of 13C3-HC-VHH and 13C3-LC-VHH fusions in vivo.

To assess the potential of VHH of the invention to promote the brain uptake of antibodies, 13C3-HC-VHH A and 13C3-HC-VHH A1 conjugates, or unvectorized 13C3 were injected into C57Bl6 mice tail vein at the dose of 35 nmoles/kg. The mice were perfused with saline solution at different times. Brains were collected at 2 hrs and 6 hrs time points post-injection (p.i.). Half of mice brains were processed to isolate the capillary network from the brain parenchyma by a capillary depletion method that consists in centrifugation on 20% Dextran solution (Sigma Aldrich) of the resuspended half brain homogenate and recovery of the parenchyma fraction. The second halves of mice brains were directly processed (homogenized and lysed) for total brain quantification. The amount of 13C3-HC-VHH conjugate in total brain and brain parenchyma was measured using an in-house qualified Meso Scale Discovery (MSD) direct coating (Abeta) immunoassay. (CV<20% and recovery ± 30%). Results are presented as concentrations (nM) (Figure 14). Results show that TfR-binding conjugates 13C3-HC-VHH A and 13C3-HC-VHH A1 exhibited a significant brain uptake advantage at 2 and 6 hrs p.i. by comparison to the control unvectorized 13C3 antibody (Figure 14-A). The total brain concentrations of 13C3-HC-VHH A and 13C3-HC-VHH A1 are 8 and 5-fold more important than that of the unvectorized 13C3 antibody at 6 hrs pi, respectively. Crossing of the BBB by 13C3-HC-VHH A and 13C3-HC-VHH A1 was confirmed by the fact that, at 6 hrs pi, the concentrations measured in brain parenchyma, depleted of the microcapillary network, were 10- and 9-fold more important than that of the unvectorized 13C3, respectively (Figure 14-B). Additional brain uptake investigations further confirmed that 13C3-HC-VHH A and 13C3-LC-VHH A (the light chain vectorized version) demonstrated BBB crossing at the dose of 70 nmoles/kg with parenchyma accumulation respectively 6-fold and 5-fold higher than unvectorized 13C3 antibody at 4 hrs p.i..

### EXAMPLE XIII: Synthesis of VHH-siRNA conjugates.

An anti-GFP siRNA comprising chemical modifications for high resistance to nucleases, namely siGFPst1, was conjugated to a tagged VHH A to generate a VHH A-siGFPst1 bioconjugate. The same conjugation strategy was used to conjugate siGFPst1 to the irrelevant VHH Z as a negative control with the same structure and size as the VHH A-siGFPst1 conjugate but with no TfR-targeting capacity. The conjugation strategy involved a convergent synthesis with the parallel modification of i) the VHH to site-specifically introduce an azido-linker; and ii) the siGFPst1 to introduce a constrained azido moiety complementary to the azido functional group. In a final step, both functionalized VHH-azide and alkyne-siGFPst1 precursors are linked to each other using a copper-free click reaction.

### Synthesis of the VHH-azide

Site-specific conjugation to the VHH was performed using a Bacterial Transglutaminase (BTG)-based ligation strategy. The BTG enzyme catalyzes the formation of an isopeptidic bond between a glutamine residue inserted in a tag sequence specifically recognized by the BTG enzyme (namely a Q-tag) and an amino-functionalized substrate. The amino-functionalized substrate introduced was a heterobifunctional linker containing at one end an amino moiety that we proved to be a substrate of the BTG enzyme and at the other end an azido moiety for the conjugation to the siGFPst1 through copper-free click chemistry.

### BTG-conjugation protocol:

3-azido-1-propanamine (20.eq/Gln) was dissolved in PBS (1X) and added to the Q-tagged VHH produced in-house. BTG (Zedira, Darmstadt, Germany) was then introduced in the mixture (0. 1U/nmol of Gln) which was allowed to react at 37°C overnight. Purification of the crude mixture was performed through chromatography on a Protino Ni-ida 1000 packed column according to the manufacturer's instructions to isolate the VHH-azide from excess of starting material as well as potential by-products. Absorbance was read at 280 nm to calculate the amount of purified VHH-azide construct and thus the conjugation yield (in the 70-80% range). Final VHH-azide were characterized by LCMS analysis to check their identity and purity.

### Synthesis of the alkyne-siGFPst1

siGFPst1 was purchased from Dharmacon with a 3'amine modification on the sense strand (N6-siGFPst1) to allow its further functionalization by the alkyne moiety required for the click chemistry conjugation with the VHH-azide.

### siGFPst1 functionalization protocol

N6-siGFPst1 (1eq) was dissolved in a NaB (0.09M; pH 8.5) conjugation buffer to obtain a final concentration between 0.3 and 0.8 mM. DBCO-NHS (20eq, DMSO) was then added to this solution. Reaction mixture was stirred for 2 hours at room temperature. Alkyne-siGFPst1 was purified by precipitation in cold absolute ethanol. Absorbance was read at 260 nm to calculate the amount of purified alkyne-siGFPst1 construct and thus the conjugation yield (in the 40-50% range). Final alkyne-siGFPst1 was characterized by analytical HPLC to check its identity and purity.

### Synthesis of the VHH-siGFPst1

Both VHH-azide and alkyne-siGFPst1 precursors were finally conjugated by a copper-free click chemistry reaction to obtain the final conjugate VHH-siGFPst1.

### VHH-siGFPst1 conjugation protocol:

Alkyne-siGFPst1 (2 eq.) was dissolved in PBS (1X) and added to the VHH-azide (1 eq., final concentration in the 100µM range in PBS (1X)). Reaction mixture was allowed to stir overnight at room temperature. Final conjugate was first purified by gel filtration chromatography onto a Superdex75 column and second, concentrated using an Amicon Ultra-centrifugation filter (10K). Absorbance was read at 260 nm to calculate the amount of purified VHH-siGFPst1 construct and thus the conjugation yield (overall yield in the 30% range). Final VHH-siGFPst1 (VHH A-siGFPst1 and VHH Z-siGFPst1) were characterized by analytical SEC-HPLC and agarose-gel electrophoresis to check their identity and purity.

### EXAMPLE XIV: In vitro gene silencing activity of a VHH-siRNA bioconjugate.

Specific cellular targeting and productive intracellular delivery of therapeutic nucleic acids, especially siRNAs, oligonucleotides remain a major challenge. The structural and physico-chemical features of these molecules, being multiply charged hydrophilic oligomers, prevent them from entering any subcellular compartment if unassisted. VHH of the invention were used to transport a small interfering RNA (siRNA) across cellular membranes to access the cytosol.

First, the apparent hTfR-binding affinity (K_{d app}) of the VHH A-siGFPst1 and VHH B-siGFPst1 bioconjugates was evaluated as described in Example VII (Determination of binding affinity of VHH A1-A19) by adding concentrations ranging from 2 µM to 30 pM during 1 hr at 4°C on the same CHO-hTfR-GFP cells. Quantification of the cell-surface bound molecules was performed by anti-6His immunocytochemistry and experimental data were fit with a nonlinear regression using GraphPad Prism^{®} software. As previously shown with the free VHH A and VHH B, the VHH A-siGFPst1 and VHH B-siGFPst1 bioconjugates demonstrated concentration-dependent and saturable binding to the cell-surface target hTfR, with K_{d app} values in the same low nanomolar range as unconjugated VHH A and VHH B (Figure 15A). No significant binding was observed with the control VHH Z. This, in turn, confirmed that coupling of the VHH A and VHH B to an siRNA does not alter their ability to bind specifically and efficiently to hTfR.

Second, the intrinsic silencing activity of the VHH-siGFPst1 bioconjugate was assessed in living CHO cell lines stably expressing the TfR fused to EGFP (CHO-hTfR-EGFP cells) by transfection of the conjugate at 25 nM using Dharmafect 1 (Dharmacon) for direct delivery into the cytosol. The total cellular amount of GFP was quantified 72 hours post-transfection using flow cytometry. The results demonstrate that the VHH A-siGFPst1 conjugate induced a *ca.* 85% reduction of GFP protein levels, in the same range than the unconjugated siGFPst1 or the control VHH Z-siGFPst1 conjugate (Figure 15B). This confirms that coupling of either the VHH A or Z does not hamper the siRNA to undergo RISC loading and exert its silencing activity. In another series of experiments, the VHH A-siGFPst1 conjugate was transfected on CHO-hTfR-EGFP cells at concentrations ranging from 10 nM to 1 pM and the total cellular amount of GFP was quantified 120 hours post-transfection using flow cytometry. This resulted in a concentration-dependent reduction of GFP protein levels, with an IC50 of 50.4 pM and a maximum silencing efficiency in this condition of -90.2 % (Figure 15C).

Third, the ability of the VHH A, once conjugated to siGFPst1, to trigger hTfR-mediated endocytosis and subsequent delivery into the cytosol of target cells in pharmacological amounts was assessed. The VHH A-siGFPst1 or the control VHH Z-siGFPst1 bioconjugates were incubated on CHO-hTfR-GFP cells at 1 µM during 120 hrs at 37°C to allow free uptake, delivery to the cytosol and gene silencing to take place at the mRNA transcript and protein levels. This led to a significant *ca.* -70% reduction of GFP protein levels with the TfR-binding VHH A-siGFPst1 bioconjugate, while no silencing was observed with the control VHH Z-siGFPst1 bioconjugate (Figure 15D). Next, the VHH A-siGFPst1 bioconjugate was incubated on CHO-hTfR-GFP cells at concentrations ranging from 3 µM to 10 pM during 120 hrs. This resulted in a concentration-dependent reduction of GFP protein levels, with an IC50 of 2.73 ± 0.23 nM and a maximum silencing efficiency in this condition of -61.6 ± 2.9 % (Figure 15E). This demonstrates that cell-surface binding to hTfR and subsequent endocytosis of VHH A-siGFPst1 bioconjugate allows its delivery into the cytosol in pharmacological amounts, with an IC50 in the same nanomolar range than hTfR-binding affinity of the bioconjugate.

Fourth, the involvement of the hTfR in the observed silencing effect of the VHH A-siGFPst1 bioconjugate upon free uptake on CHO-hTfR-GFP cells was confirmed in a competition assay. In this experiment, VHH A-siGFPst1 was incubated during 120 hrs at 37°C at the saturating concentration of 30 nM, as defined from the previous experiment, either alone or in the presence of a 100X excess of the free VHHs A, B or Z. The results demonstrated that the *ca.* 60% reduction of GFP protein levels was almost completely abrogated in the presence of the free VHH A or VHH B (GFP protein levels were maintained at 85% and 96% of the control levels, respectively). Importantly, no competition was observed when using an excess of the irrelevant VHH Z (Figure 15F). This unequivocally confirmed that the silencing effect of the VHH A-siGFPst1 bioconjugate was indeed due to hTfR-mediated cellular uptake and subsequent delivery into the cell cytoplasm.

Fifth, the TfR-mediated GFP-silencing effect of the VHH A-siGFPst1 bioconjugate was evaluated using a pulse-chase procedure. CHO-hTfR-GFP cells were exposed to VHH A-siGFPst1 at concentrations ranging from 300 nM to 1 pM during a short duration (6 hours), followed by chase in ligand-free medium up to a total duration of 120 hrs. This experiment allowed to evaluate the contribution of early cellular uptake to the silencing effect previously observed by continuous incubation during 120 hrs. As observed using continuous incubation, the VHH A-siGFPst1 bioconjugate again induced a concentration-dependent reduction of GFP protein levels, with a similar IC50 of 1.24 nM and a maximum silencing efficiency of - 54.2 % (Figure 15G). This result suggests that most of the effect previously observed upon 120 hrs continuous incubation was due to productive TfR-mediated uptake within the first 6 hrs. This finding is of particular interest since *in vivo* the plasma pharmacokinetic profile of such bioconjugates generally allows tissue exposure at therapeutic levels during only a few hours when administered by intravenous or subcutaneous bolus injection. The TfR-targeting VHH described here hence represents a viable tool for targeted and efficient gene silencing *in vivo.*

Finally, the ability of the VHH B to trigger hTfR-mediated endocytosis and subsequent gene silencing was evaluated by incubating the VHH B-siGFPst1 bioconjugate on CHO-hTfR-GFP cells at 30 nM during 120 hrs. The result showed a *ca.* -60% reduction in GFP levels, similar to that obtained with the VHH A-siGFPst1 bioconjugate, confirming that these VHHs display a similar TfR-targeting and intracellular delivery potential (Figure 15H). To the best of our knowledge, receptor-mediated hepatocyte uptake through the asialoglycoprotein receptor (ASGPR) using triantennary GalNAc as a targeting ligand is the only ligand/receptor system able to trigger specific and efficient gene silencing at nanomolar concentrations. However, the use of this system for *in vivo* therapeutic applications with therapeutic nucleic acids is restricted to hepatic targets, since ASGPR is expressed *in vivo* exclusively in hepatocytes. Therefore, the present invention provides a new ligand/receptor system for the targeting and intracytoplasmic delivery at nanomolar concentrations of therapeutic nucleic acids, such as siRNAs, into extra-hepatic organs and tissues expressing the TfR.

### EXAMPLE XV: Synthesis of VHH-NODAGA conjugates.

### Design of the Q-tagged VHH A

In the present example, a DNA fragment encoding VHH A with an AlaLinker, a HisTag, a GlyLinker and a Q-tag (AAA-HisTag-GGG-LQR sequence) introduced at its C-terminal end) was synthetized and cloned into the pHEN1 vector.

### BTG-based preparation of the VHH A-azide:

3-azido-1-propanamine (20.eq/Gln) was dissolved in PBS (1X) and added to the LQR-tagged VHH A produced in-house. BTG (Zedira, Darmstadt, Germany) was introduced in the mixture (0.1U/nmol of Gln). The reaction mixture was then allowed to react at 37°C overnight. Purification of the crude mixture was performed through chromatography on a Protino Ni-ida 1000 packed column according to the manufacturer's instructions to isolate the VHH A-azide from excess of starting material as well as potential by-products. Absorbance was read at 280 nm to calculate the amount of purified VHH A-azide construct and thus the conjugation yield (in the 70-80% range). Final VHH A-azide was characterized by LCMS analysis to check its identity and the purity.

### Click chemistry reaction to conjugate VHH A-azide to commercial alkyne-NODAGA

VHH A-azide (1 eq.) was allowed to react with the heterobifunctional NODAGA-BCN (5 eq.) (Chematech, Dijon, France) in PBS at room temperature. Reaction was monitored by LCMS. After completion of the reaction, the final conjugate was purified through chromatography on a Protino Ni-ida 1000 packed column according to the manufacturer's instructions to isolate the VHH A-azide from excess of starting material as well as potential by-products. Absorbance was read at 280 nm to calculate the amount of purified VHH A-NODAGA construct and thus the conjugation yield (in the 50-60% range). Final VHH A-NODAGA was characterized by LCMS analysis to check its identity and purity.

### EXAMPLE XVI: PET imaging of a VHH-68Ga bioconjugate in a subcutaneous mouse model of glioblastoma tumor.

Glioblastoma is the most common primary malignant brain tumor and the U87 cell line, a human primary glioblastoma cell line, is known to express a high TfR levels. In order to assess the glioblastoma targeting of VHH of the invention, the radiolabeled VHH A-NODAGA bioconjugate was intravenously administrated to mice previously implanted with glioblastoma cells (xenograft model) and PET-Scan imaging was performed.

### Radiolabeling of VHH A-NODAGA and binding affinity validation

First, VHH A-NODAGA was radiolabeled using 68Ga chloride. Gallium was obtained in 68Ga3+ form using a commercial TiO2-based 68Ge/68Ga generator (Obninsk). A radiolabeling reaction was conducted by reacting 60µg of VHH A-NODAGA with 74-148 MBq (2-4 mCi) of 68Ga in 400 µL of ammonium acetate buffer (1M, pH 6) at 25°C for 10 minutes. The VHH A-68Ga radiochemical purity (RPC) obtained was >95%. Following radiolabeling, the apparent hTfR-binding affinities (K_{d app}) of the VHHA-NODAGA and VHH A -68Ga bioconjugates were evaluated as described in Example VII (Determination of binding affinity of VHH A1-19) by adding concentrations ranging from 2 µM to 30 pM during 1 hr at 4°C on the same CHO-hTfR-GFP cells. Quantification of the cell-surface bound VHH A bioconjugates was performed by anti-6His immunocytochemistry and experimental data were fit with a nonlinear regression using GraphPad Prism^{®} software. VHH A-NODAGA and VHH A-68Ga bioconjugates demonstrated concentration-dependent and saturable binding to the cell-surface target receptor hTfR, with K_{d app} values in the same low nanomolar range as the unconjugated VHH A (Figure 16A). No significant binding was observed with the control VHH Z. This confirmed that coupling of the VHH A to a NODAGA ligand and radiolabeling protocol does not alter its ability to bind specifically to hTfR.

### PET-Scan imaging

Animal studies were performed according to the protocols approved by the Aix-Marseille Ethic comity (Comity 14). Four weeks old BALB/c Nude Mouse female were obtained from Charles River Inc. Mice (n=6) were implanted subcutaneously between the shoulders with U87-MG cells (2×10⁶) in 100 µL of complete medium containing 50% Matrigel (Corning). On day 28 following implantation (when the tumors reached a volume comprised between 300-700 mm3), the animals were administered with an intravenous single bolus dose of 5± 1MBq of VHH A-68Ga. Following administration, the biodistribution in the glioblastoma cancer xenograft and other tissues was assessed using PET-imaging. PET/CT scans were acquired during 2 hrs for 3 mice and at 2 hrs post injection (p.i.) for the 3 other mice. PET and PET/CT studies were performed on a microPET/microCT rodent model scanner (nanoPET/CT^{®}, Mediso). Anesthesia was induced with 5% isoflurane and maintained at 1.5%. To improve image quality, 20 million coincidence events per mouse were acquired for every static PET emission scan (energy window, 400-600 keV; time: 20 minutes for one FOV). For dual modality PET/CT, CT images (35 kVp, exposure time of 350ns and medium zoom) were obtained, and anatomical registration, as well as attenuation of correction, was applied to the corresponding PET scans. Imaging pictures of animals injected with VHH A-68Ga showed a significant accumulation at the tumor site (Figure 16B, 1.46% of ID/g) and a good tumor/muscle ratio (4.0). Thus, experiments showed a clear and selective imaging and labeling of glioblastoma cancer with VHH A-68Ga at day 28, consistent with the known high expression levels of the TfR.

### EXAMPLE XVII: Competition assays between purified VHHs and phage-VHHs.

To evaluate whether selected VHHs compete one with the other for binding to the TfR receptor, competition assays using flow cytometry experiments were performed. All experiments were performed in 96 well plates using 2 × 10⁵ cells/well, at 4 °C with shaking. CHO cell line expressing the hTfR fused to EGFP was saturated with PBS/BSA 2% solution during 30 min to avoid nonspecific binding, followed by incubation with VHH (competitors) at increasing concentrations for 1 hr. Then, phage-VHHs (tracers) at EC80 were added and incubated 1 hr more. To detect the phage-VHHs, cells were washed twice with PBS/BSA 2% and incubated for 1 hr with an Alexa647-conjugated anti-M13 antibody. After two last washes with PBS/BSA 2%, cells were fixed by incubation for 15 min with PBS/PFA 2%, washed once with PBS and finally resuspended in PBS. Fluorescence levels were assessed using an Attune NxT flow cytometer (Thermo Fisher Scientific). Experimental data were fitted with GraphPad Prism^{®} software using a nonlinear fit.

VHHs E8 and C10a induced a concentration-dependent loss of signal of the phage-H1, indicating that their binding to the TfR hinders the binding of the phage-H1. These three VHHs (i.e., E8, C10a and H1) were selected by phage display on the rhTfR and these results indicate that they should bind a close epitope. Moreover, VHHs B6 and C5 did not interfere with the phage-H1 binding. Thus, H1, E8 and C10a should bind a distinct epitope than B6 and C5 (Figure 17-A). This is confirmed by Figure 17-B which shows that none of E8 and C10a VHHs interfered with phage-C5 binding. As shown in Figure 17-C, VHHs C3 and F2a induced a concentration-dependent inhibition of phage-B6 binding. Those three VHHs (C3, F2a and B6) bind to the apical domain of the TfR, on a close epitope as suggested by these results. VHH C10a and C5 did not interfere with phage-B6 binding, confirming the results described above. Thus, C3, F2a and B6 should bind to a close epitope, distinct from that of C10a and C5. Noteworthy, VHHs B7, H7, C12b and E4 were not evaluated here as they have nearly the same CDR3 as B6 and bind the same epitope, but they can be considered as part of the same family. Figure 17-D also illustrates that no VHHs interfered with phage-CS binding.

Overall, those results indicate that various groups of VHHs of the invention (such as B6 and variants thereof; C5 and variants thereof; or E8, C10a, H1 and variants thereof) have distinct epitopes. These results highlight once again the diversity of the VHHs of the invention, allowing to target several TfR epitopes with potentially different effects.

### EXAMPLE XVIII: Competition assays between purified VHHs with affinity for the TfR and the natural ligand (Tf).

To evaluate the ability of selected VHHs to compete with Transferrin (Tf), the TfR natural ligand, for the binding to the receptor, competition assays using flow cytometry experiments were performed. All experiments were performed in 96 well plates using 2 × 105 cells/well, at 4 °C with shaking. CHO cell line expressing the hTfR fused to EGFP was saturated with PBS/BSA 2% solution during 30 min to avoid nonspecific binding, followed by incubation with competitors (VHHs or Tf) at increasing concentrations for 1 hr. Then, tracers (VHHs or Tf-A647) at EC80 were added and incubated 1 hr more. In the condition where Tf-A647 was the tracer, cells were washed 3 times with PBS/BSA 2%, fixed by incubation for 15 min with PBS/PFA 2%, washed once with PBS and finally resuspended in PBS. In the condition where VHHs were tracers, cells were washed once with PBS/BSA 2% and incubated for 1 hr with an anti-6His tag antibody (mouse). After two washes with PBS/BSA 2%, cells were incubated for 1 hr with an Alexa647-conjugated anti-mouse secondary antibody. Cells were finally fixed as in the condition where Tf-A647 was the tracer. Fluorescence levels were assessed using an Attune NxT flow cytometer (Thermo Fisher Scientific). Experimental data were fitted with GraphPad Prism^{®} software using a nonlinear fit.

TfR-binding VHHs were used as tracers (Figure 18-A) and competitors (Figure 18-B). In both conditions, there was no competition between VHHs and the ligand Tf, suggesting that VHHs bind to TfR on an epitope different from that of Tf. Keeping the Tf binding to its receptor was a prerequisite to limit side effects and allow the TfR to play its natural role.

### EXAMPLE XIX: Brain uptake of homodimeric 13C3-HC-VHH fusions having a bivalent TfR binding mode vs unvectorized 13C3 in vivo in C57Bl/6 mice.

To assess the potential of some VHH variants of the invention having different mTfR binding properties to promote the brain uptake of the murine 13C3 monoclonal antibody, homodimeric fusions 13C3-HC-C5h20, 13C3-HC-C5h9, 13C3-HC-C5h16, 13C3-HC-C5h24 or non-vectorized 13C3 were first produced and evaluated for their apparent binding affinity (K_{d app}) and maximum level of binding (Bmax) to mTfR as described in Example XI (Design and production of a therapeutic antibody fused to a VHH). Unlike the fusions 13C3-HC-C5h20 / C5h9 / C5h16 / C5h24 that specifically and efficiently bind to mTfR, no significant binding was observed with the control 13C3. Thanks to the determination of the Bmax and Kd app parameters, the binding potential of the different fusions to mTfR was calculated as the ratio of normalized Bmax/K_{d app} × 100. As shown in the Figure 19-A, 13C3-HC-C5h20 has the strongest mTfR binding potential (436.8) while 13C3-HC-C5h16 has a ≈ 3-time lower mTfR binding potential (128.2).

Second, homodimeric fusions 13C3-HC-C5h20 / C5h9 / C5h16 / C5h24 or non-vectorized 13C3 were injected into C57Bl/6 mice tail vein at the dose of 35 nmoles/kg. Blood was collected and the mice were perfused with saline solution at 6 hrs and 18 hrs post-injection (p.i.). Brains were collected and one half was processed to isolate the capillary network from the brain parenchyma and the brain extra-cellular fluid (ECF) by using a capillary depletion method already described in Example XII (Brain uptake of 13C3-HC-VHH and 13C3-LC-VHH fusions *in vivo*)*.* The second halves of mice brains were directly processed (homogenized and lysed) for total brain quantification. The amount of 13C3-HC-VHH in total brain, brain parenchyma and ECF was measured using an in-house qualified Meso Scale Discovery (MSD) direct coating (Abeta) immunoassay. (CV<20% and recovery ± 30%). Results are presented as concentrations (nM) (Figure 19-B, C, E, F) or as brain tissue-to-plasma ratio percentage (Figure 19-D).

Results show that TfR-binding conjugates 13C3-HC-C5h20 / C5h9 / C5h16 / C5h24 concentrations in plasma were lower at 18 hrs than the levels of the 13C3 control antibody, in line with target mediated clearance of the conjugates targeting TfR (Figure 19-B). However, 13C3-HC-C5h20 / C5h9 / C5h16 / C5h24 exhibited a significant brain uptake advantage at 6 hrs p.i. by comparison to the non-vectorized 13C3 control antibody (Figure 19-C) and this advantage remained significant at 18 hrs p.i for 13C3-HC-CSh9. When looking at brain-to-plasma ratios, a clear advantage is confirmed for all the TfR-binding conjugates 13C3-HC-C5h20 / C5h9 / C5h16 / C5h24, especially at 18 hrs p.i.

The BBB crossing ability of 13C3-HC-C5h20 / C5h9 / C5h16 / C5h24 in the post-vascular brain compartments was further confirmed by the fact that concentrations of 13C3-HC-C5h20 / C5h9 / C5h16 / C5h24 are 7.5 to 9-fold and 2 to 7.2-fold more important than that of the non-vectorized 13C3 antibody at 6 hrs p.i in the brain parenchyma and ECF respectively, and remain significatively 2.2 to 2.5-fold more concentrated than 13C3 in the brain parenchyma at 18 hrs p.i for 13C3-HC-C5h9 / CSh24. Interestingly, these two last constructs have an intermediate mTfR binding potential.

Overall, these results demonstrate that TfR-targeting VHH of the invention can be used to effectively deliver a therapeutic antibody to the brain. Moreover, the intensity and the duration of the antibody delivery can be modulated thanks to the TfR binding potential of the VHH of the invention.

### EXAMPLE XX: Brain uptake of heterodimeric VHH-Fc fusions having a monovalent TfR binding mode in vivo in C57Bl/6 mice.

To assess the potential of some VHH variants of the invention having a monovalent TfR binding mode to promote the brain uptake of a prototypic protein like an effector function-less human IgG1 Fc fragment, heterodimeric (VHH)₁ₖ-hFc_{LALA} fusions were first generated by using the "knob-into-hole" technology.

To produce the fusion proteins, DNA fragments encoding the (VHHs)₁ₖ-hFc_{LALA} knob (with Fc knob carrying the T366W mutation and the L234A, L235A mutations to diminish effector functions-wherein the amino acid numbering is according to the EU index as in Kabat of a full length hIgG1) and HFc_{LALA} hole (carrying T366S, L368A, Y407V and L234A, L235A mutations) were synthetized and cloned into the pCDNA3.4 vector (ThermoFisher Scientific) in order to encode an effector function-less human IgG 1- Fc fragment encompassing in its N-ter one single VHH. Fusion proteins were prepared using the Expi293 expression system according to the manufacturer's instructions (Life Technologies) by co-transfection of the plasmids coding for VHH-hFc knob and hole at a 1 to 4 ratio. Seventy-two hrs post-transfection, supernatants were recovered and purified using first an affinity chromatography HiTrap Prot A (Cytiva) followed by a size exclusion chromatography (Hiload 16/600 Superdex 200-(Cytiva) on an AKTA pure system (GE Healthcare). The purified fusion proteins were validated by SDS-PAGE, SEC-HPLC and LC-MS to ensure their purity (over 92 %) and correct identity.

Second, TfR targeting heterodimeric (C5h18)ₗₖ-hFc_{LALA}, (C5h19)ₗₖ-hFc_{LALA} fusions and (C5neg)ₗₖ-hFc_{LALA} negative control fusion were evaluated for their binding potential to mTfR as described in Example XIX. As shown in the Figure 20-A both (C5h18)ₗₖ-hFc_{LALA}, (C5h19)ₗₖ-hFc_{LALA} demonstrated a low mTfR binding potential of around 1, in line with the low apparent affinity of the unconjugated VHH C5h18 / C5h19 for mTfR (see Table 5). No significant TfR binding was observed with the (C5neg)ₗₖ-hFc_{LALA} control.

Third, TfR targeting heterodimeric (C5h18)ₗₖ-hFc_{LALA}, (C5h19)ₗₖ-hFc_{LALA} fusions and (C5neg)ₗₖ-hFc_{LALA} negative control fusion were injected subcutaneously into C57Bl/6 mice tail vein at the dose of 70 nmoles/kg. Plasma, brain, post-vascular brain compartments (i.e parenchyma and ECF depleted from brain microvessels) and liver were recovered at 6, 18 and 42 hrs p.i. The amount of (VHH)ₗₖ-hFc_{LALA} fusion in each tissue was measured using an in-house anti-Fc ELISA assay. Results are presented as concentrations (nM).

The plasma pharmacokinetic of (C5h18)ₗₖ-hFc_{LALA}, (C5h19)ₗₖ-hFc_{LALA} fusions was close to the one of the negative (C5neg)ₗₖ-hFc_{LALA} control indicating a low TfR-mediated clearance, in accordance with the low TfR binding potential of (C5h18)ₗₖ-hFc_{LALA} and (C5h19)ₗₖ-hFc_{LALA} fusions (Figure 20-B). Interestingly, in brain and brain post-vascular compartments, (C5h18)ₗₖ-hFc_{LALA} and (C5h19)ₗₖ-hFc_{LALA} fusions demonstrated a significative and sustained uptake up to 42 hrs post-injection by comparison with the (C5neg)ₗₖ-hFc_{LALA} negative control fusion (Figure 20-C, D, E). (C5h19)ₗₖ-hFc_{LALA} reaches its maximal brain concentration at 42 hrs p.i (4 nM) while (C5h18)ₗₖ-hFc_{LALA} peaked at 18 hrs p.i. (4.8 nM) and the (C5neg)ₗₖ-hFc_{LALA} control was stable between 18 and 42 hrs at 1.7 nM. No accumulation differences were seen in the liver between (C5h18/C5h19)ₗₖ-hFc_{LALA}, and the control (C5neg)ₗₖ-hFc_{LALA} (Figure 20-F).

Overall, these results demonstrate that TfR-targeting VHH of the invention having a low TfR binding potential can be used to effectively deliver a protein of interest to the brain across the BBB without drastically modifying its plasma pharmacokinetic.

### EXAMPLE XXI: Targeted delivery in the CNS of the neurotensin peptide (NT(8-13)) in C57Bl/6 and B-hTfR mice.

Neurotensin is a 13 amino-acid neuropeptide that can induce a variety of central effects, including hypothermia. However, upon systemic administration, neurotensin peptide does not elicit any central effect due to inefficient BBB crossing. The potential of some VHH variants of the invention to promote the brain uptake of the shorter active fragment of neurotensin peptide (NT (8-13)) and induce a central hypothermia was assessed in mice expressing the wild type and the human ectodomain of the TfR (C57Bl/6 and B-hTfR (Biocytogen) mice respectively).

To produce the fusion proteins, DNA fragments encoding the VHHs-NT(8-13) were synthetized and cloned into the pHEN1 vector to encode a protein encompassing in its N-ter end one single VHH copy and in its C-Ter the NT(8-13) peptide. A sequence coding for a hexahistidine affinity tag followed by a (G4S)2 linker was introduced between the sequence coding for the VHHs and NT(8-13). VHHs-NT(8-13) were then expressed in the bacteria E.coli (strain BL21-DE3) and purified by Immobilized Metal Affinity Chromatography based on His-tag. Endotoxins, were removed by performing a polishing step consisting in a size exclusion chromatography and filtering on Mustang Q or E membranes (Pall). The purified fusion proteins were validated by SDS-PAGE, SEC-HPLC and LC-MS to ensure their purity (over 95 %) and correct identity. A LAL assay (Pierce) was also performed to determine endotoxin levels.

The apparent affinities of the VHHs B8h1-NT(8-13) (Kd app 2.8 nM) and B8V1-NT(8-13) (Kd app 8.3 nM) for mTfR (Figure 21-A) and of C5V30-NT(8-13) (Kd app 16.7 nM), C5h18-NT(8-13) (Kd app 16.8 nM), B8V31-NT(8-13) (Kd app 25.6 nM) and C12b-NT(8-13) (Kd app 1865 nM) to hTfR (Figure 21-B) were evaluated in a cell-based assay. No significant m/hTfR binding was observed with the control C5neg-NT(8-13).

Hypothermia induction of some VHH-NT(8-13) fusions of the invention was next monitored using rectal temperature measurement after single intravenous injection at 500 nmoles/Kg in C57Bl/6 or B-hTfR mice (Figure 21-C, D). The results are presented as mice body temperature in °C as a function of time (minutes post-injection) on curve graphs or in area under the curve (AUC) relative to a mouse mean basal body temperature (38°C) on the bar graphs.

The negative control C5neg-NT(8-13) had no significant effect on body temperature of C57Bl/6 or B-hTfR mice. On the contrary, B8h1-NT(8-13) and B8V1-NT(8-13) induced a significative drop in C57Bl/6 mice body temperature reaching a maximum of minus 3-4°C at 60-75 min p.i respectively. C5h18-NT(8-13), B8V31-NT(8-13), CSV30-NT(8-13) and C12b-NT(8-13) induced a significative drop in B-hTfR mice body temperature reaching a maximum of minus 5-7 °C at 45-60 min p.i respectively. Interestingly, C5h18 that was demonstrated in Example XX to be able to address to C57Bl/6 (wild type) mice brain an IgG1 Fc fragment was also demonstrated in this example as potent for NT(8-13) delivery to the brain of mice expressing the hTfR ectodomain (B-hTfR), validating here its cross-species TfR targeting potential.

Overall, these results demonstrate that TfR-targeting VHH of the invention allow NT(8-13) to cross the BBB and reach its brain target in pharmacologically active amount in mice expressing the human or the mouse TfR versions.

### Example XXII: Brain uptake of heterodimeric VHH-Fc-NT fusions having a monovalent TfR binding mode in vivo in C57Bl/6 mice.

To assess the potential of some VHH variants of the invention having a monovalent TfR binding mode to promote the brain uptake of an effector function-less human IgG1 Fc fragment fused to NT(8-13) peptide, heterodimeric (VHH)ₗₖ-hFc_{LALA}-NT(8-13) fusions were generated by using the "knob-into-hole" technology. To produce the fusion proteins, DNA fragments encoding the (VHHs)ₗₖ-hFc_{LALA} knob (with Fc knob carrying the T366W mutation and the L234A, L235A mutations to diminish effector functions-wherein the amino acid numbering is according to the EU index as in Kabat of a full length hIgG1) and hFc_{LALA} hole-NT(8-13) (carrying T366S, L368A, Y407V and L234A, L235A mutations plus a sequence coding for a (G₄S)₃ linker followed by NT(8-13) added at its C-terminal end) were synthetized and cloned into the pCDNA3.4 vector (ThermoFisher Scientific) in order to encode an effector function-less human IgG1-Fc fragment encompassing in its N-ter one single VHH copy and in its C-ter one single NT(8-13) copy. Fusion proteins were prepared as described in example XIX.

Second, TfR targeting heterodimeric (C5h18)ₗₖ-hFc_{LALA}-NT(8-13), (C10a)ₗₖ-hFc_{LALA}-NT(8-13), (H1)ₗₖ-hFc_{LALA}-NT(8-13) fusions (C5neg)ₗₖ-hFc_{LALA}-NT(8-13) negative control fusion were evaluated for their apparent affinity for hTfR as described in Example XIX. As shown in the table of Figure 22-A both (C10a)ₗₖ-hFc_{LALA}-NT(8-13) and (H1)ₗₖ-hFc_{LALA}-NT(8-13) demonstrated a similar apparent affinity of around 16 nM for hTfR, and (C5h18)ₗₖ-hFc_{LALA}-NT(8-13) demonstrated a slightly lower apparent affinity of 22 nM. No significant TfR binding was observed with the (C5neg)ₗₖ-hFc_{LALA} control-NT(8-13).

Third, TfR targeting heterodimeric (C5h18)ₗₖ-hFc_{LALA}-NT(8-13), (C10a)ₗₖ-hFc_{LALA}-NT(8-13), (H1)ₗₖ-hFc_{LALA}-NT(8-13) fusions and (C5neg)ₗₖ-hFc_{LALA}-NT(8-13) negative control fusion were injected intravenously into B-hTfR mice tail vein (n=4) at the dose of 20 nmoles/kg. B-hTfR mice were also injected with 150 µl of PBS buffer used as (VHH)ₗₖ-hFc_{LALA}-NT(8-13) formulation buffer. At 24 hrs post-injection, blood of the previously injected mice was collected, and mice were perfused with a saline solution. Brains were collected and one half was processed to isolate the capillary network from the brain parenchyma and the brain extra-cellular fluid (ECF) by using a capillary depletion method described in Example XII (Brain uptake of 13C3-HC-VHH and 13C3-LC-VHH fusions *in vivo*)*.* The second halves of mice brains were directly processed (homogenized and lysed) for total brain quantification. The amount of amount of (VHH)ₗₖ-hFc_{LALA}-NT(8-13) fusions in each tissue was measured using an in-house anti-Fc ELISA assay. Results are presented as concentrations (nM) (Figure 22-B, C, D, E).

The plasma concentrations of (C5h18)ₗₖ-hFc_{LALA}-NT(8-13), (C10a)ₗₖ-hFc_{LALA}-NT(8-13), (H1)ₗₖ-hFc_{LALA}-NT(8-13) fusions were lower to the one of the negative (C5neg)ₗₖ-hFc_{LALA} control in line with a TfR-mediated clearance and in accordance with their hTfR apparent affinity. Of note, while (C10a)ₗₖ-hFc_{LALA}-NT(8-13) and (H1)ₗₖ-hFc_{LALA}-NT(8-13) have a similar or even more stronger affinity than (C5h18)ₗₖ-hFc_{LALA}-NT(8-13) for hTfR, they were 3-times less distributed (Figure 22-C). One hypothesis to explain this distributional difference could be that VHH C10a, and H1 (which are from the same family), target a different epitope on hTfR from the one targeted by C5h18 (derived from C5 VHH and thus targeting a similar epitope on hTfR) as illustrated in Figure 17-A & D.

Interestingly, in brain and brain post-vascular compartments, (C5h18)ₗₖ-hFc_{LALA}-NT(8-13), (C10a)ₗₖ-hFc_{LALA}-NT(8-13) and (H1)ₗₖ-hFc_{LALA}-NT(8-13) fusions demonstrated significantly higher concentration by comparison with the (C5neg)ₗₖ-hFc_{LALA} NT(8-13) negative control fusion (Figure 22-C, D, E). In particular (C10a)ₗₖ-hFc_{LALA}-NT(8-13) accumulates 6.4-times more than (C5neg)ₗₖ-hFc_{LALA} NT(8-13) in total brain (Figure 22-C) and (H1)ₗₖ-hFc_{LALA}-NT(8-13), (C10a)ₗₖ-hFc_{LALA}-NT(8-13) and , (C5h18)ₗₖ-hFc_{LALA}-NT(8-13) 17- to 7-times more in parenchyma (Figure 22-E).

Finally, to investigate the potential of (C5h18)ₗₖ-hFc_{LALA}-NT(8-13) to induce a CNS-dependant hypothermia, the molecule was injected at 20 nmoles/Kg to B-hTfR mice. (C5neg)ₗₖ-hFc_{LALA}-NT(8-13) negative control fusion and PBS buffer were used as controls. Rectal temperature was monitored until 4 hrs post-injection (Figure 22-F). The control molecule and the molecule formulation buffer (PBS buffer) did not elicit any hypothermia while (C5h18)ₗₖ-hFc_{LALA}-NT(8-13) induced a maximum drop in mouse body temperature of-5.8°C at 90 min post-injection, lasting until ≈ 200 min post-injection, confirming the ability of C5h18 VHH of the invention to cross the BBB and to shuttle rapidly Fc_{LALA}-NT(8-13) into the brain in pharmacology active amounts.

Overall, these results demonstrate that TfR-targeting VHH of the invention having a medium TfR affinity can be used to effectively deliver a protein of interest to the brain across the BBB. Moreover, different VHH of the invention targeting different epitope can be used to vectorize a payload, depending on the need for a rapid or much slower brain delivery.

### EXAMPLE XXIII: Oligonucleotide sequences and their conjugation to TfR-binding VHH.

The potential of some VHH variants of the invention to promote functional delivery of oligonucleotides in the CNS was evaluated using VHHs conjugated to RNAi-competent, chemically modified siRNA sequences targeting the ubiquitous mouse and rat superoxide dismutase 1 (SOD1) mRNA, described in WO2019217459, as listed in **Table 7** below. The single-stranded gapmer, rNase H-competent antisense oligonucleotide (ASO) used in the following examples targets the ubiquitously expressed long non-coding RNA (lncRNA) MALAT-1, which is preferentially enriched in nuclear speckles where it regulates post-transcriptional RNA processing, adapted from Tripathi et al. by introduction, in a sequence of SEQ ID NO: 405 of Table 7 below, of a modification 'mc'( i.e., 2'-O-methoxyethyl-5-methyl-cytidine ; 2'MOE meC)' instead of 2'-MOE-C (i.e., 2'-O-methoxyethyl-cytidine).

**Table 7:**

| **SEQ ID NO** | **Oligo ID** | **Strand (5'-3')** | **Sequence** |
|---|---|---|---|
| SEQ465 | siSOD1m (mouse/rat) | S | c•a•uuuu*A*a*UCC*ucacucuaaaL |
| SEQ466 | | AS | u•*U*•uagAg*UG*agga*U*u*A*aaaug•a•g |
| SEQ465 | siSOD1m-5'VP (mouse/rat) | S | c•a•uuuuAaUCCucacucuaaaL |
| (VP)-SEQ466 | | AS | (VP)-u•*U*•uagAg*UG*agga*U*u*A*aaaug•a•g |
| SEQ 467 | dTdT-siSOD1m-5'VP (mouse/rat) | S | c•a•uuuu*A*a*UCC*ucacucuaaadTdTL |
| (VP)-SEQ 468 | | AS | (VP)-u•*U*•uagAg*UG*agga*U*u*A*aaaug•a•g |
| SEQ405 | *hMALATI*-ASO | AS | Lg•g•g•a•g•dT•dT•dA•dC•dT•dT•dG•dC •dC•dA•a•mc•t•t•g |

| | | | |
|---|---|---|---|
| siSOD1m: siRNA duplex targeting the mouse and rat superoxide dismutase 1 (SOD1) mRNA; *hMALAT1-*ASO: gapmer antisense oligonucleotide (ASO) targeting the human MALAT-1 long non-coding RNA; lower case indicates 2'-*O*-methyl (2'-OMe) sugar modifications, respectively, to adenosine, cytidine, guanosine or uridine nucleotides; italicized upper case indicates 2'-deoxy-2'-fluoro (2'F) sugar modifications, respectively, to adenosine, cytidine, guanosine or uridine nucleotides; dN indicates deoxynucleotides (e.g., dT indicates deoxythymidine); • indicates phosphorothioate (PS) internucleosidic linkage; L indicates linker (conjugation handle); VP indicates vinyl-phosphonate; italicized lower case indicates 2'-O-methoxyethyl (2'-MOE) sugar modifications to adenosine, guanosine or thymidine nucleotides; mc indicates 2'-O-methoxyethyl-5-methyl-cytidine (2'MOE meC). | | | |

Oligonucleotides were synthesized or purchased from Horizon Discovery Biosciences Ltd. or GeneLink Inc. with conjugation handles, such as hexylamino-linker, introduced on the 3'-end of the sense strand of siRNA duplexes.

The general conjugation strategy involved a convergent synthesis such as the strategy described in WO 2020/144233, consisting of the parallel modification of: i) the VHH or the VHH-hFc heterodimer to site-specifically introduce for example an azido-linker; and ii) the oligonucleotide to introduce for example a constrained alkyne group complementary to the azido functional group. In a final step, both functionalized azido-VHH, or VHH-hFc-azido, and alkyne-Oligo precursors are linked to each other using preferably a copper-free click reaction, yielding VHH-Oligo or heterodimeric VHH-hFc-Oligo conjugates with a stable linker (cf. **Figure 23**). Alternative methods were employed by inventors to generate VHH-Oligo conjugates with either other types of linkers such as cleavable disulfide linkers or longer PEG linkers or another conjugation chemistry such as thiol-maleimide chemistry (cf. **Figure 24**).

### EXAMPLE XXIV: Evaluation of the binding of VHH-Oligo and VHH-hFc-Oligo conjugates to mTfR in living cells.

The binding of VHH-Oligo and VHH-hFc-Oligo conjugates to mTfR was evaluated using a competition assay on living cells expressing the receptor of interest, namely murine neuroblastoma Neuro-2a cells (N2a). N2a cells were grown in Dulbecco's Modified Eagle Medium (DMEM) GlutaMAX supplemented with 10% v/v FBS in 5% CO₂ at 37°C. Cells were seeded two days before experiments in 96-well plates at a density of 50.000 cells/well. On the day of the experiment, free VHHs, VHH-Oligo or VHH-hFc-Oligo conjugates diluted at various concentrations in DMEM supplemented with 1% bovine serum albumin (BSA) were co-incubated at various concentrations on each cell line during 3 hrs at 37°C with a sub-saturating concentration of the fluorescent reference compound CS-Alexa680 (100 nM). After treatment, cells were washed with D-PBS and dissociated by incubating with Trypsin/EDTA for 5 mins at 37°C, before the addition of cold culture media (4°C) to inhibit Trypsin activity. Resuspended cells were transferred in a 96-deepwell plate (V-bottom) containing 1% fetal bovine serum (FBS) / 0.02% sodium azide in phosphate-buffered saline (PBS) and then centrifuged for 5 min at 2000 rpm at 4°C. After removing the supernatant, 5 mM EDTA in D-PBS and 4% paraformaldehyde (PFA) (v/v 1:1) were added into wells to fix cells for 15 min at room temperature. PFA was removed by centrifugation (5 min, 3000 rpm, 4°C) and cells were resuspended with 5 mM EDTA in D-PBS. Then the cellular A680-associated fluorescence signal was quantified using an Attune^{™} NxT flow cytometer (Thermo Fisher Scientific) equipped with Attune^{™} NxT v3.1.2. Experimental data (experimental triplicates) were fitted with GraphPad Prism^{®} software using a nonlinear fit to determine the apparent *K*ᵢ inhibition constants. Graphs of **Figure 25** show representative inhibition curves and tables show means ± SD of at least 2 independent experiments.

All tested conjugates (e.g., C5-siSOD1m-5'VP, C5-hFc-siSOD1m-5'VP or B8-MALAT1-ASO) induced a concentration-dependent inhibition of the binding and uptake of the reference compound in cells expressing the murine TfR, with similar apparent *K*ᵢ values to that of free VHHs (e.g., C5 or B8), demonstrating efficient binding to the TfR.

### EXAMPLE XXV: In vitro functional delivery and gene silencing potential of TfR-binding VHH-Oligo and VHH-hFc-Oligo conjugates after free uptake in murine cells.

Murine neuroblastoma (Neuro-2a) cells were grown in DMEM GlutaMAX supplemented with 10% v/v FBS in 5% CO₂ at 37°C. For free uptake experiments, cells were seeded in 96-well plates at a density of 2.000 cells/well. Twenty-four hours later, the medium was removed, and cells were further incubated during 3 days in DMEM supplemented with 1% v/v FBS containing various concentrations of free or conjugated oligos. Cells were then washed with D-PBS and harvested in lysis buffer from the Nucleospin RNA XS Kit (Macherey-Nagel), according to the manufacturer's recommendations. Cell lysates were stored at -20°C before RNA extraction, and reverse-transcription was performed using the High-Capacity RNA-to-cDNA^{™} Kit (Applied Biosystems). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 or MALAT-1 genes (Applied Biosystems). Expression data were analyzed using the DDCq method normalized to the expression of reference RpL13 or GAPDH genes, based on raw quantification cycle (Cq) values (Bustin et al.) and are presented as mRNA levels relative to untreated cells. Experimental data (experimental triplicates) are presented as means ± standard deviation (SD) and were analysed with GraphPad Prism^{®} software using a three-parameter log(inhibitor) vs. response nonlinear regression to determine the IC50. **Figure 26A** **and** **26C** show representative inhibition curves.

Whereas the unconjugated siSOD1m-5'VP showed no effect on *mSOD1* mRNA levels, TfR-binding VHH-siSOD1m-5'VP and VHH-hFc-siSOD1m-5'VP conjugates (such as C5-siSOD1m-5'VP or C5-hFc-siSOD1m-5'VP) showed potent and concentration-dependent *mSOD1* mRNA downregulation, with mean IC50 of 7.4 nM and 8.7 nM, respectively (**Figure 26A**). A similar specific and potent knock-down effect was observed with various VHH-siSOD1m conjugates (i.e., conjugates comprising the following VHH molecules which are B8 or C5 or their variants such as B8h1, C5, C5V1, CSV7, CSV8, C5V13, C5h18, C5h19, as listed in Table 1) as well as the VHH-*MALAT1*-ASO conjugate (comprising a VHH molecule such as B8 as described in Table 1) (**Figures 26B** **and** **26C****,** respectively). These results illustrate that the VHH-Oligo or VHH-hFc-Oligo conjugates of the invention efficiently bind to cell surface TfR followed by TfR-mediated endocytosis, endosomal escape and cytosol or nuclear delivery of active oligonucleotides, thereby allowing effective modulation of target gene expression using different oligonucleotide modalities.

### EXAMPLE XXVI: TfR distribution in brain microvessels and parenchyma cells.

TfR distribution in wild-type C57Bl/6 mouse brain was evaluated by immunohistochemistry (IHC) on brain cryosections prepared from snap-frozen (dry ice) mouse hemibrains. Mouse TfR was detected using the 8D3 antibody (Novus Biologicals, NB100-64979) and its colocalization with either microvessels, stained using an anti-collagen IV antibody (United States Biological, C7510-50H) or neurons, stained using an anti-NeuN antibody (Merck Millipore, MAB377), was assessed using confocal imaging. **Figure 27** shows broad expression of TfR in all regions of mouse brains. Examples are shown in the cortex or the hippocampus where TfR is found highly expressed at the BBB, as evidenced by extensive colocalization with collagen IV-positive microvessels, but also in parenchyma cells, such as neurons as evidenced by a strong colocalization with the neuron marker NeuN. Therefore, TfR distribution in the brain demonstrates that the VHH ligands of the invention targeting TfR have the potential not only to cross the BBB but also to mediate endocytosis and intracellular delivery of therapeutic payloads in parenchymal cells.

### EXAMPLE XXVII: CNS knock-down effect of VHH-siRNA conjugates after local ICV administration in wild-type C57Bl/6 mice.

VHH-siSOD 1m conjugates were assessed for their potential to mediate murine *SOD1* mRNA knock-down in the brain and spinal cord following intracerebroventicular (ICV) administration in wild-type C57Bl/6 mice. Wild-type C57Bl/6 mice (4 per group) were injected a single ICV dose (right lateral ventricle, 5-10 µL at 0.75 µL/min) of 100 µg (siRNA molar equivalent) of PBS vehicle (control), unconjugated siSOD1m-5'VP or the indicated VHH-siSOD1m-5'VP conjugates. Seven days after injection, tissue samples including total brain (right hemisphere), brain regions (from the left hemisphere), spinal cord and the liver were withdrawn, snap-frozen in 10 vol. ofNucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 and RpL13 genes (Applied Biosystems). Expression data were analyzed using the ΔΔCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS. Both the unconjugated siSOD1m-5'VP and the non-binding C5neg-siSOD1m-5'VP induced only moderate (brain) and minor (spinal cord) knock-down (KD) effect in CNS tissues and no effect the liver, indicating no retention and uptake in parenchymal cells, nor size-related improvement with the non-binding C5neg-siSODAm-5'VP conjugate. On the contrary, the TfR-binding C5-siSOD1m-5'VP conjugate induced a strong KD effect in both brain and spinal cord, with *ca.* 80-90% KD (**Figure 28B**), demonstrating efficient and TfR-dependent retention and functional uptake of TfR-binding VHH-siRNA conjugates in brain parenchymal cells and spinal cord.

In addition, brain regional analysis of the left hemisphere showed very homogenous KD effect of the TfR-binding C5-siSOD1m-5'VP conjugate among all regions tested (60-90% KD), including deep structures such as striatum or hippocampus, as opposed to the moderate and highly heterogenous effect of the unconjugated siSOD1m-5'VP (10-60% KD) (**Figure 28C**). These results demonstrate early TfR-mediated uptake of TfR-binding VHH-siRNA conjugates in CNS tissues after single ICV injection. This efficient TfR-targeting and functional uptake potential translates into strong and stable intracellular depot in all regions of the brain and spinal cord, with sustained release of RNAi-competent siRNA from endo-lysosomal compartments to the cytosol, RNA-induced silencing complex (RISC)-loading and continuous degradation of target mRNA molecules. This CNS-specific functional delivery pattern validates these TfR-binding VHH-siRNA conjugates for use in neurological and neuromuscular disorders.

### EXAMPLE XXVIII: CNS knock-down effect and siRNA biodistribution after local ICV administration of VHH-siRNA conjugates in wild-type C57Bl/6 mice.

Biodistribution of VHH-siRNA conjugates and CNS knock-down of the target *SOD1* mRNAVHH-siSODlm conjugates were evaluated using *in situ* hybridization methods (ISH) on brain and spinal cord samples following single ICV administration in wild-type mice. Wild-type C57Bl/6 mice were injected a single ICV (right lateral ventricle) dose of 100 µg (siRNA molar equivalent) of PBS vehicle (control) or a TfR-binding VHH-siSOD1m-5'VP conjugate. Seven days after injection, the right (ipsilateral) brain hemispheres were withdrawn, snap-frozen in 10 vol. of NucleoProtect RNA stabilization reagent (Macherey-Nagel) and stored at -20°C until processing for qPCR quantification of *SOD1* mRNAs, while the left (contralateral) brain hemispheres and part of the cervical spinal cords were isolated, snap-frozen on dry ice and stored at -80°C until preparation for further ISH and imaging as described below (**Figure 29A**).

For RT-qPCR bioanalysis, frozen tissue samples in NucleoProtect were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} LTNG kit (Applied Biosystems) and commercially available TaqMan^{™} probes for murine SOD1 and RpL13 genes (Applied Biosystems). Expression data were analyzed using the ΔΔCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS. Mice injected with the TfR-binding C5-siSOD1m-5'VP conjugate induced a strong, 70-90% downregulation of *SOD1* mRNA in all regions tested of the brain ipsilateral (right) hemisphere (e.g. frontal cortex, parieto-temporal cortex, thalamus, hypothalamus, hippocampus, striatum, cerebellum, brain stem, olfactory bulb) compared to PBS-injected mice.

For ISH bioanalysis, 20 µm sagittal cryosections of left brain hemispheres or coronal sections of spinal cords were prepared using a cryostat at -20°C (Leica), collected onto superfrost plus slides and stored at - 80°C until processing. For RNAScope^{®} or miRNAScope^{®} assays, slides containing cyosections were immediately transferred and fixed in 10% Neutral buffered formalin (NBF) for 1h30 at RT. Fluorescent RNAscope^{®} assay for mRNAs detection or chromogenic miRNAscope^{®} assay for siRNA detection were performed according to the manufacter's protocols (Advanced Cell Diagnostics, ACD). RNAscope^{®} assay was performed using RNAscope Multiplex Fluorescent Reagent Kit v.2 (ACD) and miRNAscope^{®} assay was performed using the miRNAscope HD Reagent Kit RED Assay (ACD). After tissue pretreatment, sections were hybridized with probes specific to mouse *SOD1* and the neuronal-specific *MAP2* mRNAs (ACD) or probe specific to siSODlm antisense strand (AS) (ACD). After signal amplification, sections were counterstained with DAPI (RNAscope^{®}) or hematoxylin solution (miRNAscope^{®}) and then images were acquired using a Zeiss Axio Scan.Z1 Slide Scanner. Mice injected with the TfR-binding CS-siSOD1m-5'VP conjugate showed a broad biodistribution of the siSOD1m AS strand in all brain regions on midsagittal mouse brain tissue sections and also in the white matter and grey matter of the spinal cord, as assessed using miRNAScope^{®} (**Figure 29B** **and** **29E****, left panels**). This distribution correlates with a broad and potent *SOD1* mRNA knock-down, as assessed using RNAScope^{®} (**Figure 29B** **and** **29E****, right panels**). Co-detection of *SOD1* and *MAP2* mRNAs shows a potent knock-down of *SOD1* mRNA in MAP2-expressing neurons in all regions tested, such as cortical and hippocampal pyramidal neurons, cerebellar Purkinje cells or neurons of the spinal cord (**Figure 29C** **and** **29F**). A particular aspect of these results is that a strong KD is observed not only in regions with intense distribution, such as the cortex, hippocampus, striatum or the grey matter of spinal cord, but also in some regions showing lower distribution, such as the thalamus, cerebellum. A significant *SOD1* mRNA KD was also observed in regions containing only few neurons but large number of non-neuronal cells such as GFAP-positive astrocytes or Olig2-positive oligodendrocytes, as observed in the corpus callosum (**Figure 29D**), in the fornix or in the white matter of spinal cord (**Figure 29F**).

These results demonstrate that the TfR-binding VHH-siRNA conjugates of the invention are endowed with potent functional delivery potential in neuronal and non-neuronal cells of the brain parenchyma and spinal cord, with efficient egress from endo-lysosomal compartments to cytosol even with low intracellular depot, ultimately leading to RISC-loading and potent RNAi activity.

### EXAMPLE XXIX: Optimization of the CNS functional delivery of VHH-siRNA conjugates by systemic administration in wild-type C57Bl/6 mice.

VHH-siSOD1m-5'VP conjugates or heterodimeric VHH-hFc-siSOD1m-5'VP conjugates were generated and compared for their potential to trigger BBB-crossing and functional delivery in CNS tissues following systemic administration. Wild-type C57Bl/6 mice (4-8 per group) were administered with either four daily SC doses (QDx4) of unconjugated siSOD1m-5'VP or the C5-siSOD1m-5'VP conjugate at 15 mg/kg (siRNA molar equivalent), or three IV doses of the C5-hFc-siSOD1m-5'VP conjugate every two days (Q2Dx3) at 1.5 mg/kg (siRNA molar equivalent), compared to PBS injected mice. One week (siSOD1m-5'VP and VHH-siSOD1m-5'VP groups) or two weeks (VHH-hFc-siSOD1m-5'VP) following the last administration, tissue samples including total brain (right hemisphere), brain regions (from the left hemisphere), spinal cord, the liver and kidneys were withdrawn, snap-frozen in 10 vol. of NucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} LTNG kit (Applied Biosystems) and commercially available TaqMan^{™} probes for the target murine SOD1 and reference RpL13 and PUM1 genes (Applied Biosystems). Expression data were analyzed using the □□Cq method normalized to the expression of the reference RpL13 gene, for brain regions, or RpL13 and PUM1 genes for total brain, spinal cord, liver, kidney (multiplex qPCR), based on raw quantification cycle (Cq) values (Bustin et al.). The results are expressed as means ± standard error of the mean (SEM) and are presented as mRNA levels relative to the control animals injected with PBS.

Despite extensive chemical stabilization, the unconjugated siSOD1m-5'VP showed no KD effect in brain and spinal cord seven days following SC administration of a 60 mg/kg total dose (4x 15 mg/kg siRNA molar equivalent, i.e. 4 µmole/kg). On the contrary, TfR-binding conjugates such as the C5-siSOD1m-5'VP conjugate or the heterodimeric C5-hFc-siSOD1m-5'VP conjugate showed significant downregulation of the target *SOD1* mRNAs in both total brain and spinal cord, reaching 30-40% KD with the same dosing regimen of the C5-siSOD1m-5'VP conjugate, and up to 65% with the C5-hFc-siSOD1m-5'VP conjugate at a 13-fold lower (3x 1.5 mg/kg siRNA molar equivalent, i.e. 0.3 µmole/kg) (**Figure 30B**). This demonstrates efficient BBB-crossing and functional uptake in CNS tissues of TfR-binding conjugates of the invention, with further improvement of the effect and at a much lower dose with the C5-hFc-siSOD1m-5'VP conjugate engineered to display extended plasma half-life, compared to the C5-siSOD1m-5'VP conjugate. In addition, the C5-hFc-siSOD1m-5'VP conjugate induced no or minor effects in excretory organs (**Figure 30C**). Brain regional analysis on the left hemibrains of mice treated with this conjugate shows very homogenous KD effect, with 50-70% KD including in deep structures such as the striatum or the thalamus (**Figure 30D**). This demonstrates that efficient TfR-mediated crossing of the BBB of the VHHs of the invention, together with the dense vasculature found in the brain, allows for broad exposure of brain parenchymal cells in pharmacological amount, leading to potent functional uptake and RNAi activity in the brain following systemic administration at low dose. Additionally, the TfR-binding VHH of the invention demonstrate potent functional uptake potential after systemic administration at low dose in CNS tissues, allowing their use for therapeutic intervention in neurological and neuromuscular disorders, while sparing other tissues such as excretory organs.

### EXAMPLE XXX: CNS knock-down effect and siRNA biodistribution after systemic administration of VHH-hFc-siRNA conjugates in wild-type C57Bl/6 mice.

TfR-binding or non-binding heterodimeric VHH-hFc-siSOD1m-5'VP conjugates were generated and compared for their potential to trigger BBB-crossing and functional delivery in CNS tissues following systemic administration. Wild-type C57Bl/6 mice (4-8 per group) were administered with three IV doses of the TfR-binding C5-hFc-siSOD1m-5'VP conjugate or the non-binding C5neg-hFc-siSOD1m-5'VP conjugate every two days (Q2Dx3) at 1.5 mg/kg (siRNA molar equivalent), compared to PBS injected mice. Two weeks following the last administration, tissue samples including brain regions (from the right hemisphere), spinal cord, right eye (total), the liver, kidneys and lungs were withdrawn, snap-frozen in 10 vol. of NucleoProtect RNA stabilization reagent (Macherey-Nagel) and stored at -20°C until processing for qPCR quantification of *SOD1* mRNAs, while the left brain hemispheres, part of the lumbar spinal cords and the left eye were isolated, snap-frozen on dry ice and stored at -80°C until preparation for further ISH and imaging as described below (**Figure 31A**). For RT-qPCR bioanalysis, frozen tissue samples in NucleoProtect were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} LTNG kit (Applied Biosystems) and commercially available TaqMan^{™} probes for murine SOD1, RpL13 and RpL30 genes (Applied Biosystems). Expression data were analyzed using the ΔΔCq method normalized to the expression of the reference RpL13 and RpL30 genes (multiplex qPCR), based on raw quantification cycle (Cq) values (Bustin et al.). The results are expressed as means ± standard error of the mean (SEM) and are presented as mRNA levels relative to the control animals injected with PBS. Quantification of *SOD1* mRNA levels in tissues is shown in **Figure 31B****.** As observed in previous examples, the C5-hFc-siSOD1m-5'VP conjugate showed a potent 50-70% KD effect in all brain regions and spinal cord, with maximum effect observed in spinal cord, while no effect was observed with the non-binding C5neg-hFc-siSOD1m-5'VP conjugate. This confirms that the BBB-crossing and CNS functional uptake potential of the VHHs of the invention is specifically mediated through binding to TfR. A significant and TfR-specific KD effect of *ca.* 40% was also observed in the eye with the C5-hFc-siSOD1m-5'VP conjugate. On the contrary, only minor or no KD effect was observed with the TfR-binding conjugate in other tissues, such as the liver, kidney or lung, confirming tissue-specificity.

For ISH bioanalysis, 20 µm sagittal cryosections of left brain hemispheres or coronal sections of spinal cords were prepared using a cryostat at -20°C (Leica), collected onto superfrost plus slides and stored at - 80°C until processing. For RNAScope^{®} or miRNAScope^{®} assays, slides containing cyosections were immediately transferred and fixed in 10% Neutral buffered formalin (NBF) for 1h30 at RT. Fluorescent RNAscope^{®} assay for mRNAs detection or chromogenic miRNAscope^{®} assay for siRNA detection were performed according to the manufacter's protocols (Advanced Cell Diagnostics, ACD). RNAscope^{®} assay was performed using RNAscope Multiplex Fluorescent Reagent Kit v.2 (ACD) and miRNAscope^{®} assay was performed using the miRNAscope HD Reagent Kit RED Assay (ACD). After tissue pretreatment, sections were hybridized with probes specific to mouse *SOD1* and the neuronal-specific *MAP2* mRNAs (ACD) or probe specific to siSODlm antisense strand (AS) (ACD). After signal amplification, sections were counterstained with DAPI (RNAscope^{®}) or hematoxylin solution (miRNAscope^{®}) and then images were acquired using a Zeiss Axio Scan.Z 1 Slide Scanner. Fourteen days following IV administration, mice injected with the TfR-binding C5-hFc-siSOD1m-5'VP conjugate showed a broad biodistribution of the siSODlm AS strand in all brain regions on midsagittal mouse brain tissue sections and also in the white matter and grey matter of the spinal cord, as assessed using miRNAScope^{®} (Figure 31C and 31I, left **panels**). On the contrary, the non-binding C5neg-hFc-siSOD1m-5'VP conjugate did not show evidence of tissue retention, consistent with TfR-mediated BBB-crossing of the TfR-binding conjugate followed by uptake and retention in brain parenchyma and spinal cord. This distribution correlates with a broad TfR-dependent and potent *SOD1* mRNA knock-down, as assessed using RNAScope^{®} (**Figure 31C** and 31I, **right panels**). Co-detection of *SOD1* and *MAP2* mRNAs shows a potent knock-down of *SOD1* mRNA in MAP2-expressing neurons in all regions tested, such as cortical and hippocampal pyramidal neurons, neurons of the striatum, cerebellar Purkinje cells, neurons of cerebellar nuclei or neurons of the spinal cord (Figure 31D-I). A particular aspect of these results is that a strong KD is observed in regions showing both high or low tissue retention, indicating that low level of TfR-mediated intracellular delivery is sufficient to mediate efficient functional delivery in neural cells with RISC-loading and potent RNAi activity. These results demonstrate efficient and TfR-specific BBB-crossing and broad functional delivery of therapeutic oligonucleotides conjugated to the VHHs of the invention in brain and spinal cord cells, including neurons, after systemic administration at low dose.

An example of cleavable linker was evaluated and compared to the C5-hFc-siSOD1m-5'VP for its biodistribution in CNS tissues following systemic administration. A C5-hFc-dTdT-siSOD1m-5'VP conjugate was generated, with an additional dTdT overhang at the 3' end of the sense strand of the siRNA to increase its metabolic susceptibility to intracellular nucleases. Both C5-hFc-siSOD1m-5'VP and C5-hFc-dTdT-siSOD1m-5'VP conjugates were administered with three IV doses every two days (Q2Dx3) at 1.5 mg/kg (siRNA molar equivalent), compared to PBS injected mice. Two weeks following the last administration, the left brain hemisphere and part of the lumbar spinal cords were isolated, snap-frozen on dry ice and stored at -80°C until preparation for further ISH and imaging as previously described. Tissue distribution of the siRNA AS in CNS tissues following systemic administration is shown in **Figure 32****.** Interestingly, the TfR-binding conjugate comprising an additional dTdT linker displays a much higher retention in microvessels in all brain regions, such as the cortex, striatum or cerebellum, and also in spinal cord, compared to the C5-hFc-siSOD1m-5'VP conjugate. This indicates a potential to modulate the CNS distribution and retention in microvessels of therapeutic oligonucleotides conjugated to TfR-binding VHHs of the invention, by introducing specific linkers or spacers endowed with metabolic susceptibility to some components present during trans-endothelial trafficking. This potential enables the use of the VHH of the invention for functional delivery of therapeutic oligonucleotides in endothelial cells of CNS tissues, in conditions where modulation of gene expression can provide a therapeutic opportunity (e.g. vascular dementia).

### EXAMPLE XXXI: Dose-response of the CNS knock-down effect of heterodimeric VHH-hFc-siRNA conjugates after single subcutaneous administration in wild-type C57Bl/6 mice.

Wild-type C57Bl/6 mice (4-8 per group) were administered with a single subcutaneous (SC) dose of the TfR-binding C5-hFc-siSOD1m-5'VP conjugate at 0.17, 0.5, 1.5, 4.5 and 9 mg/kg (siRNA molar equivalent), corresponding to 0.011, 0.033, 0.1, 0.3 and 0.6 µmole/kg, compared to PBS injected mice. Two weeks following administration, tissue samples including brain regions (from the right hemisphere), spinal cord, right eye (total), the liver, kidneys and lungs were withdrawn, snap-frozen in 10 vol. of NucleoProtect RNA stabilization reagent (Macherey-Nagel) and stored at -20°C until processing for qPCR quantification of *SOD1* mRNAs. Frozen tissue samples in NucleoProtect were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1, RpL13 and RpL30 genes (Applied Biosystems). Expression data were analyzed using the ΔΔCq method normalized to the expression of the reference RpL13 and RpL30 genes (multiplex qPCR), based on raw quantification cycle (Cq) values (Bustin et al.). The results are expressed as means ± standard error of the mean (SEM) and are presented as mRNA levels relative to the control animals injected with PBS.

Quantification of *SOD1* mRNA levels in tissues, dose-response curves and estimated ED₅₀ values and maximum KDs are shown in **Figure 33** As observed above following multi-IV dosing (Q2Dx3) of the C5-hFc-siSOD1m-5'VP conjugate with a total dose of 4.5 mg/kg (siRNA molar equivalent), a single 4.5 mg/kg SC dose induces a potent and similar 50-70% KD effect in all brain regions and spinal cord, with maximum effect observed in spinal cord. Consistent with previous experiments, a *ca.* 45% KD was also observed in the eye at the highest dose tested. The estimated ED₅₀ values were very homogenous across all CNS tissues tested, with a mean 55 nmoles/kg (corresponding to 825 µg/kg siRNA).

The results presented here demonstrate that a single subcutaneous administration of the TfR-binding VHH-hFc-siRNA conjugates of the invention is sufficient to mediate efficient BBB-crossing and homogenous exposure of CNS tissues in pharmacologically active amounts, allowing functional uptake and target mRNA knock-down at low dose.

### LIST OF SEQUENCES

**Table 1 : List of amino acid sequences of the VHH molecules of the invention as well as of negative controls C5neg and D 12, the corresponding CDR1-3, and their identifiers, i.e. SEQ ID NO or SEQ.**

| **sdAb** | **Amino acid sequence** | **CDR1** | **CDR2** | **CDR3** |
|---|---|---|---|---|
| A (C5) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ 4 (x=1) or SEQ213 (x=0) | GTDFSI NF SEQ 1 | FTATG NT SEQ 2 | YMLDKSEQ3 |
| B (B8) | EVQLVESGGGVVQPGGSLRLSCAASGEIFSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ8 (x=1) or SEQ214 (x=0) | GEIFSIN F SEQ5 | FTRDGS T SEQ6 | YMLDTSEQ7 |
| C (B6) | EVQLVESGGGLVQPGGSLRLSCTASGGPIEQYPMGWFRRAPGKERE LVASISRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 SEQ12 (x=1) or SEQ215 (x=0) | GGPIEQ YP SEQ9 | ISRSGD GTY SEQ10 | GAGINP TKI SEQ11 |
| D (H3) | EVQLVESGGGEVQPGGSLKLSCVASGTDFSINFVRWYRQRPGKQRE WVAGFTANGDTNYPDSMKGRFTISRDNAKNTVYLQINSLKSEDTAV YYCYMLDNWGQGTQVTVSS(AAAEQKLISEEDLNGAAHHHHHHGS )x SEQ16 (x=1) or SEQ216 (x=0) | GTDFSI NF SEQ13 | FTANG DT SEQ14 | YLKDNSEQ15 |
| A1 (C5^{v1}) | EVQLVESGGGVVQPGGSLKLSCVASGADFSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ18 (x=1) or SEQ217 (x=0) | GADFSI NF SEQ17 | FTATG NT SEQ2 | YMLDKSEQ3 |
| A2 (C5^{v2}) | EVQLVESGGGVVQPGGSLKLSCVASGTAFSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ20 (x=1) or SEQ218 (x=0) | GTAFSI NF SEQ19 | FTATG NT SEQ2 | YMLDKSEQ3 |
| A3 (C5^{v3}) | EVQLVESGGGVVQPGGSLKLSCVASGTDFSINFIRWYRQAPGKQRE FVAGFTAAGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ22 (x=1) or SEQ219 (x=0) | GTDFSI NF SEQ1 | FTAAG NT SEQ21 | YMLDKSEQ3 |
| A4 (C5^{v4}) | EVQLVESGGGVVQPGGSLKLSCVASGTDFSINFIRWYRQAPGKQRE FVAGFTATGATNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ24 (x=1) or SEQ220 (x=0) | GTDFSI NF SEQ1 | FTATG AT SEQ23 | YMLDKSEQ3 |
| A5 (C5^{v5}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCAMLDKWGQGTQVTVSS(AAAEQKLISEEDLNGAAHHHHHHGS )x, with x=1 or 0 SEQ26 (x=1) or SEQ221 (x=0) | GTDFSI NF SEQ1 | FTATG NT SEQ2 | AMLDKSEQ25 |
| A6 (C5^{v6}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYALDKWGQGTQVTVSS(AAAEQKLISEEDLNGAAHHHHHHGS) x, with x=1 or 0 | GTDFSI NF SEQ1 | FTATG NT SEQ2 | YALDKSEQ27 |
| | SEQ28 (x=1) or SEQ222 (x=0) | | | |
| A7 (C5^{v7}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMADKWGQGTQVTVSS(AAAEQKLISEEDLNGAAHHHHHHGS )x, with x=1 or 0 SEQ30 (x=1) or SEQ223 (x=0) | GTDFSI NF SEQ1 | FTATG NT SEQ2 | YMADK SEQ29 |
| A8 (C5^{v8}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMLAKWGQGTQVTVSS(AAAEQKLISEEDLNGAAHHHHHHGS )x, with x=1 or 0 SEQ32 (x=1) or SEQ224 (x=0) | GTDFSI NF SEQ1 | FTATG NT SEQ2 | YMLAK SEQ31 |
| A9 (C5^{v9}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMLDAWGQGTQVTVSS(AAAEQKLISEEDLNGAAHHHHHHGS )x, with x=1 or 0 SEQ34 (x=1) or SEQ225 (x=0) | GTDFSI NF SEQ1 | FTATG NT SEQ2 | YMLDA SEQ33 |
| A10 (C5^{v10}) | EVQLVESGGGVVQPGGSLKLSCVASGTDFSLNFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ68 (x=1) or SEQ226 (x=0) | GTDFSL NF SEQ67 | FTATG NT SEQ2 | YMLDK SEQ3 |
| A11 (C5^{v11}) | EVQLVESGGGVVQPGGSLKLSCVASGTDFSINYIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x SEQ70 (x=1) or SEQ227 (x=0) | GTDFSI NY SEQ69 | FTATG NT SEQ2 | YMLDK SEQ3 |
| A12 (C5^{v12}) | EVQLVESGGGVVQPGGSLKLSCVASGTDFSINFIRWYRQAPGKQRE FVAGITATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ72 (x=1) or SEQ228 (x=0) | GTDFSI NF SEQ1 | ITATGN T SEQ71 | YMLDK SEQ3 |
| A13 (C5^{v13}) | EVQLVESGGGVVQPGGSLKLSCVASGTDFSINFIRWYRQAPGKQRE FVAGFSATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ74 (x=1) or SEQ229 (x=0) | GTDFSI NF SEQ1 | FSATGN T SEQ73 | YMLDK SEQ3 |
| A14 (C5^{v14}) | EVQLVESGGGVVQPGGSLKLSCVASGTDFSINFIRWYRQAPGKQRE FVAGFTATGNSNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ76 (x=1) or SEQ230 (x=0) | GTDFSI NF SEQ1 | FTATG NS SEQ75 | YMLDK SEQ3 |
| A15 (C5^{v15}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCFMLDKWGQGTQVTVSS(AAAEQKLISEEDLNGAAHHHHHHGS) x, with x=1 or 0 SEQ78 (x=1) or SEQ231 (x=0) | GTDFSI NF SEQ1 | FTATG NT SEQ2 | FMLDK SEQ77 |
| A16 (C5^{v16}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYILDKWGQGTQVTVSS(AAAEQKLISEEDLNGAAHHHHHHGS) x, with x=1 or 0 SEQ80 (x=1) or SEQ232 (x=0) | GTDFSI NF SEQ1 | FTATG NT SEQ2 | YILDK SEQ79 |
| A17 (C5^{v17}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMIDKWGQGTQVTVSS(AAAEQKLISEEDLNGAAHHHHHHGS) x, with x=1 or 0 SEQ82 (x=1) or SEQ233 (x=0) | GTDFSI NF SEQ1 | FTATG NT SEQ2 | YMIDK SEQ81 |
| A18 (C5^{v18}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMVDKWGQGTQVTVSS(AAAEQKLISEEDLNGAAHHHHHHGS )x, with x=1 or 0 SEQ84 (x=1) or SEQ234 (x=0) | GTDFSI NF SEQ1 | FTATG NT SEQ2 | YMVDK SEQ83 |
| A19 (C5^{v19}) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYMLEKWGQGTQVTVSS(AAAEQKLISEEDLNGAAHHHHHHGS )x, with x=1 or 0 SEQ86 (x=1) or SEQ235 (x=0) | GTDFSI NF SEQ1 | FTATG NT SEQ2 | YMLEK SEQ85 |
| A20 (C5^{h1}) | EVQLVESGGGVVQPGGSLRLSCAASGTDFSINFMSWVRQAPGKGLE WVAGFTATGNTNYADSVKGRFTISRDNAKNTLYLQMNSLRPEDTA VYYCYMLDKWGQGTQVTVSS(AAAEQKLISEEDLNGAAHHHHHHG S)x, with x=1 or 0 SEQ87 (x=1) or SEQ236 (x=0) | GTDFSI NF SEQ1 | FTATG NT SEQ2 | YMLDK SEQ3 |
| A21 (C5^{h2}) | EVQLVESGGGVVQPGGSLRLSCAASGTDFSINFIRWVRQAPGKQREF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ88 (x=1) or SEQ237 (x=0) | GTDFSI NF SEQ1 | FTATG NT SEQ2 | YMLDK SEQ3 |
| A22 (C5^{h3}) | EVQLVESGGGVVQPGGSLRLSCAASGTDFSINFMHWVRQAPGKGLE WVAGFTATGNTNYADSVKGRFTISRDNAKNTLYLQMNSLRPEDTA VYYCYMLDKWGQGTQVTVSS(AAAEQKLISEEDLNGAAHHHHHHG S)x, with x=1 or 0 SEQ89 (x=1) or SEQ238 (x=0) | GTDFSI NF SEQ1 | FTATG NT SEQ2 | YMLDK SEQ3 |
| A23 (C5^{h4}) | EVQLVESGGGVVQPGGSLRLSCAASGTDFSINFMSWVRQAPGKQRE FVAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ90 (x=1) or SEQ239 (x=0) | GTDFSI NF SEQ1 | FTATG NT SEQ2 | YMLDK SEQ3 |
| A24 (C5^{h5}) | EVQLVESGGGVVQPGGSLRLSCAASGTDFSINFIRWVRQAPGKGLE WVAGFTATGNTNYADSVKGRFTISRDNAKNTLYLQMNSLRPEDTA VYYCYMLDKWGQGTQVTVSS(AAAEQKLISEEDLNGAAHHHHHHG S)x, with x=1 or 0 SEQ91 (x=1) or SEQ240 (x=0) | GTDFSI NF SEQ1 | FTATG NT SEQ2 | YMLDK SEQ3 |
| A25 (C5^{h6}) | EVQLVESGGGVVQPGGSLRLSCAASGTDFSINFIHWVRQAPGKGLE WVAGFTATGNTNYADSVKGRFTISRDNAKNTLYLQMNSLRPEDTA VYYCYMLDKWGQGTQVTVSS(AAAEQKLISEEDLNGAAHHHHHHG S)x, with x=1 or 0 SEQ92 (x=1) or SEQ241 (x=0) | GTDFSI NF SEQ1 | FTATG NT SEQ2 | YMLDK SEQ3 |
| | | | | |
| C5^{v20} | EVQLVESGGGVVQPGGSLKLSCVASGHDFSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ393 (x=1) or SEQ242 (x=0) | GHDFSI NF SEQ392 | FTATG NT SEQ2 | YMLDK SEQ3 |
| C5^{v21} | EVQLVESGGGVVQPGGSLKLSCVASGTDFSINFIRWYRQAPGKQRE FVAGWTATGNTNYADSMKGRFTISRDNTKNAVYLQIDSLKPEDTA VYYCYMLDKWGQGTQVTVSS(AAAEQKLISEEDLNGAAHHHHHHG S)x, with x=1 or 0 SEQ114 (x=1) or SEQ243 (x=0) | GTDFSI NF SEQ1 | WTATG NT SEQ113 | YMLDK SEQ3 |
| C5^{v22} | EVQLVESGGGVVQPGGSLKLSCVASGTDFSINFIRWYRQAPGKQRE FVAGYTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ116 (x=1) or SEQ244 (x=0) | GTDFSI NF SEQ1 | YTATG NT SEQ115 | YMLDK SEQ3 |
| C5^{v23} | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCHMLDKWGQGTQVTVSS(AAAEQKLISEEDLNGAAHHHHHHGS )x, with x=1 or 0 SEQ118 (x=1) or SEQ245 (x=0) | GTDFSI NF SEQ1 | FTATG NT SEQ2 | HMLDK SEQ 117 |
| C5^{v24} | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCWMLDKWGQGTQVTVSS(AAAEQKLISEEDLNGAAHHHHHHG S)x, with x=1 or 0 SEQ120 (x=1) or SEQ246 (x=0) | GTDFSI NF SEQ1 | FTATG NT SEQ2 | WMLDK SEQ119 |
| C5^{v25} | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYLLDKWGQGTQVTVSS(AAAEQKLISEEDLNGAAHHHHHHGS) x, with x=1 or 0 SEQ122 (x=1) or SEQ247 (x=0) | GTDFSI NF SEQ1 | FTATG NT SEQ2 | YLLDK SEQ121 |
| C5^{v26} | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCYVLDKWGQGTQVTVSS(AAAEQKLISEEDLNGAAHHHHHHGS) x, with x=1 or 0 SEQ124 (x=1) or SEQ248 (x=0) | GTDFSI NF SEQ1 | FTATG NT SEQ2 | YVLDK SEQ123 |
| C5^{v27} | EVQLVESGGGVVQPGGSLKLSCVASGADFSINYIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ126 (x=1) or SEQ249 (x=0) | GADFSI NY SEQ125 | FTATG NT SEQ2 | YMLDK SEQ3 |
| C5^{v28} | EVQLVESGGGVVQPGGSLKLSCVASGADFSINFIRWYRQAPGKQRE FVAGFSATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ127 (x=1) or SEQ250 (x=0) | GADFSI NF SEQ17 | FSATGN T SEQ73 | YMLDK SEQ3 |
| C5^{v29} | EVQLVESGGGVVQPGGSLKLSCVASGADFSINFIRWYRQAPGKQRE FVAGFTATGHTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ129 (x=1) or SEQ251 (x=0) | GADFSI NF SEQ17 | FTATG HT SEQ128 | YMLDK SEQ3 |
| C5^{v30} | EVQLVESGGGVVQPGGSLKLSCVASGTDKSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ679 (x=1) or SEQ675 (x=0) | GTDKSI NF SEQ671 | FTATG NT SEQ2 | YMLDK SEQ3 |
| C5^{v31} | EVQLVESGGGVVQPGGSLKLSCVASGTDRSINFIRWYRQAPGKQRE FVAGFTATGNTNYADSMKGRFTISRDNTKNAVYLQIDSLKPEDTAV YYCYMLDKWGQGTQVTVSS(AAAEQKLISEEDLNGAAHHHHHHGS )x, with x=1 or 0 SEQ680 (x=1) or SEQ676 (x=0) | GTDRSI NF SEQ672 | FTATG NT SEQ2 | YMLDK SEQ3 |
| C5^{v32} | EVQLVESGGGVVQPGGSLKLSCVASGTDFSINFIRWYSQAPGKQREF VAGFTATGNTNYADSMKGRLTISRDNTKNAVYLQIDSLKPEDTAVY YCYMLGKWGQGTQVTVSS (AAAEQKLISEEDLNGAAHHHHHHGS)x, with x=1 or 0 SEQ692 (x=1) or SEQ701 (x=0) | GTDFSI NF SEQ1 | FTATG NT SEQ2 | YMLGK SEQ713 |
| C5^{h7} | EVQLVESGGGVVQPGGSLKLSCVASGTDFSINFIRWVRQAPGKGLE WVAGFTATGNTNYADSMKGRFTISRDNTKNALYLQIDSLRPEDTAV YYCYMLDKWGQGTQVTVSS(AAAEQKLISEEDLNGAAHHHHHHGS )x, with x=1 or 0 SEQ130 (x=1) or SEQ252 (x=0) | GTDFSI NF SEQ1 | FTATG NT SEQ2 | YMLDK SEQ3 |
| C5^{h8} | EVQLVESGGGVVQPGGSLRLSCAASGTDFSINFIRWVRQAPGKGLE WVAGFTATGNTNYADSVKGRFTISRDNTKNTLYLQIDSLRPEDTAV | GTDFSI NF | FTATG NT | YMLDK SEQ3 |
| | YYCYMLDKWGQGTQVTVSS(AAAEQKLISEEDLNGAAHHHHHHGS )x, with x=1 or 0 SEQ131 (x=1) or SEQ253 (x=0) | SEQ1 | SEQ2 | |
| C5^{h9} | EVQLVESGGGVVQPGGSLRLSCAASGTDFSINFIRWYRQAPGKGLEF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ132 (x=1) or SEQ254 (x=0) | GTDFSI NF SEQ1 | FTATG NT SEQ2 | YMLDK SEQ3 |
| C5^{h10} | EVQLVESGGGVVQPGGSLKLSCAASGTDFSINFIRWVRQAPGKGLE WVAGFTATGNTNYADSVKGRFTISRDNTKNTLYLQINSLRPEDTAV YYCYMLDKWGQGTQVTVSS(AAAEQKLISEEDLNGAAHHHHHHGS )x, with x=1 or 0 SEQ133 (x=1) or SEQ255 (x=0) | GTDFSI NF SEQ1 | FTATG NT SEQ2 | YMLDK SEQ3 |
| C5^{h11} | EVQLVESGGGVVQPGGSLRLSCAASGTDFSINFIRWVRQAPGKGLE WVAGFTATGNTNYADSMKGRFTISRDNTKNTLYLQMNSLRPEDTA VYYCYMLDKWGQGTQVTVSS(AAAEQKLISEEDLNGAAHHHHHHG S)x, with x=1 or 0 SEQ134 (x=1) or SEQ256 (x=0) | GTDFSI NF SEQ1 | FTATG NT SEQ2 | YMLDK SEQ3 |
| C5^{h12} | EVQLVESGGGVVQPGGSLRLSCAASGTDFSINFIRWYRQAPGKGLEF VAGFTATGNTNYADSVKGRFTISRDNAKNTLYLQIDSLRPEDTAVY YCYMLDKWGQGTQVTVSS(AAAEQKLISEEDLNGAAHHHHHHGS)x, with x=1 or 0 SEQ135 (x=1) or SEQ257 (x=0) | GTDFSI NF SEQ1 | FTATG NT SEQ2 | YMLDK SEQ3 |
| C5^{h13} | EVQLVESGGGVVQPGGSLRLSCAASGTDFSINFIRWYRQAPGKGLEF VAGFTATGNTNYADSVKGRFTISRDNTKNTLYLQMNSLRPEDTAVY YCYMLDKWGQGTQVTVSS(AAAEQKLISEEDLNGAAHHHHHHGS)x, with x=1 or 0 SEQ136 (x=1) or SEQ258 (x=0) | GTDFSI NF SEQ1 | FTATG NT SEQ2 | YMLDK SEQ 3 |
| C5^{h14} | EVQLVESGGGVVQPGGSLRLSCAASGTDFSINFIRWVRQAPGKGLE WVAGFTATGNTNYADSVKGRFTISRDNTKNALYLQMNSLRPEDTA VYYCYMLDKWGQGTQVTVSS(AAAEQKLISEEDLNGAAHHHHHHG S)x, with x=1 or 0 SEQ137 (x=1) or SEQ259 (x=0) | GTDFSI NF SEQ1 | FTATG NT SEQ2 | YMLDK SEQ3 |
| C5^{h15} | EVQLVESGGGVVQPGGSLRLSCVASGTDFSINFIRWVRQAPGKGLE WVAGFTATGNTNYADSVKGRFTISRDNTKNTLYLQMDSLRPEDTA VYYCYMLDKWGQGTQVTVSS(AAAEQKLISEEDLNGAAHHHHHHG S)x, with x=1 or 0 SEQ138 (x=1) or SEQ260 (x=0) | GTDFSI NF SEQ1 | FTATG NT SEQ2 | YMLDK SEQ3 |
| C5^{h16} | EVQLVESGGGLVQPGGSLRLSCAASGTDFSINFIRWYRQAPGKGLEF VAGFTATGNTNYADSVKGRFTISRDNSKNTLYLQMNSLRAEDTAV YYCYMLDKWGQGTLVTVSS(AAAEQKLISEEDLNGAAHHHHHHGS )x, with x=1 or 0 SEQ139 (x=1) or SEQ261 (x=0) | GTDFSI NF SEQ1 | FTATG NT SEQ2 | YMLDK SEQ3 |
| C5^{h17} | EVQLVESGGGVVQPGGSLKLSCVASGTDFSINFIRWVRQAPGKGLEF VAGFTATGNTNYADSMKGRFTISRDNTKNALYLQIDSLRPEDTAVY YCYMLDKWGQGTQVTVSS(AAAEQKLISEEDLNGAAHHHHHHGS)x, with x=1 or 0 SEQ140 (x=1) or SEQ262 (x=0) | GTDFSI NF SEQ1 | FTATG NT SEQ2 | YMLDK SEQ3 |
| C5^{h18} | EVQLVESGGGVVQPGGSLKLSCVASGTDFSINFIRWYRQAPGKGLEF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ141 (x=1) or SEQ263 (x=0) | GTDFSI NF SEQ1 | FTATG NT SEQ2 | YMLDK SEQ3 |
| C5^{h19} | EVQLVESGGGVVQPGGSLRLSCAASGTDFSINFIRWYRQAPGKQREF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 | GTDFSI NF SEQ1 | FTATG NT SEQ2 | YMLDK SEQ3 |
| | SEQ142 (x=1) or SEQ264 (x=0) | | | |
| C5^{h20} | EVQLVESGGGVVQPGGSLRLSCAASGTDFSINFIRWYRQAPGKGLEF VAGFTATGNTNYADSVKGRFTISRDNAKNAVYLQMNSLRPEDTAV YYCYMLDKWGQGTQVTVSS(AAAEQKLISEEDLNGAAHHHHHHGS )x, with x=1 or 0 SEQ143 (x=1) or SEQ265 (x=0) | GTDFSI NF SEQ1 | FTATG NT SEQ2 | YMLDK SEQ3 |
| C5^{h21} | EVQLVESGGGVVQPGGSLRLSCAASGTDFSINFIRWYRQAPGKGLEF VAGFTATGNTNYADSVKGRFTISRDNAKNTLYLQMNSLKPEDTAV YYCYMLDKWGQGTQVTVSS(AAAEQKLISEEDLNGAAHHHHHHGS )x, with x=1 or 0 SEQ144 (x=1) or SEQ266 (x=0) | GTDFSI NF SEQ1 | FTATG NT SEQ2 | YMLDK SEQ3 |
| C5^{h22} | EVQLVESGGGVVQPGGSLRLSCAASGTDFSINFIRWYRQAPGKGREF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ145 (x=1) or SEQ267 (x=0) | GTDFSI NF SEQ1 | FTATG NT SEQ2 | YMLDK SEQ3 |
| C5^{h23} | EVQLVESGGGVVQPGGSLRLSCAASGTDFSINFIRWVRQAPGKQREF VAGFTATGNTNYADSVKGRFTISRDNAKNTLYLQMNSLRPEDTAV YYCYMLDKWGQGTQVTVSS(AAAEQKLISEEDLNGAAHHHHHHGS )x, with x=1 or 0 SEQ146 (x=1) or SEQ268 (x=0) | GTDFSI NF SEQ1 | FTATG NT SEQ2 | YMLDK SEQ3 |
| C5^{h24} | EVQLVESGGGVVQPGGSLRLSCAASGTDFSINFIRWYRQAPGKGLEF VAGFTATGNTNYADSMKGRFTISRDNAKNTLYLQMNSLRPEDTAV YYCYMLDKWGQGTQVTVSS(AAAEQKLISEEDLNGAAHHHHHHGS )x, with x=1 or 0 SEQ147 (x=1) or SEQ269 (x=0) | GTDFSI NF SEQ1 | FTATG NT SEQ2 | YMLDK SEQ3 |
| C5^{h25} | EVQLVESGGGVVQPGGSLRLSCAASGTDFSINFIRWVRQAPGKGLEF VAGFTATGNTNYADSVKGRFTISRDNAKNTLYLQIDSLRPEDTAVY YCYMLDKWGQGTQVTVSS(AAAEQKLISEEDLNGAAHHHHHHGS)x, with x=1 or 0 SEQ148 (x=1) or SEQ270 (x=0) | GTDFSI NF SEQ1 | FTATG NT SEQ2 | YMLDK SEQ3 |
| C5^{h26} | EVQLVESGGGVVQPGGSLRLSCAASGTDFSINFIRWYRQAPGKGLE WVAGFTATGNTNYADSVKGRFTISRDNAKNTLYLQIDSLRPEDTAV YYCYMLDKWGQGTQVTVSS(AAAEQKLISEEDLNGAAHHHHHHGS )x, with x=1 or 0 SEQ149 (x=1) or SEQ271 (x=0) | GTDFSI NF SEQ1 | FTATG NT SEQ2 | YMLDK SEQ3 |
| C5^{neg} (control) | EVQLVESGGGVVQPGGSLKLSCVASGTDFSINFIRWYRQAPGKQRE FVAGFTATGNTNYADSMKGRFTISRDNTKNAVYLQIDSLKPEDTAV YYCAMADKWGQGTQVTVSS(AAAEQKLISEEDLNGAAHHHHHHGS )x, with x=1 or 0 SEQ151 (x=1) or SEQ272 (x=0) | GTDFSI NF SEQ1 | FTATG NT SEQ2 | AMADK SEQ150 |
| B8^{h1} | EVQLVESGGGVVQPGGSLRLSCAASGEIFSINFMRWYRQAPGKGLE WVAGFTRDGSTNYPDSVKGRFTISRDNAKNTVYLQMNSLRPEDTA VYYCYMLDTWGQGTQVTVSS(AAAEQKLISEEDLNGAAHHHHHHG S)x, with x=1 or 0 SEQ152 (x=1) or SEQ273 (x=0) | GEIFSIN F SEQ5 | FTRDGS T SEQ6 | YMLDT SEQ7 |
| C5^{V13h20} | EVQLVESGGGVVQPGGSLRLSCAASGTDFSINFIRWYRQAPGKGLEF VAGFSATGNTNYADSVKGRFTISRDNAKNAVYLQMNSLRPEDTAV YYCYMLDKWGQGTQVTVSS(AAAEQKLISEEDLNGAAHHHHHHGS )x, with x=1 or 0 SEQ153 (x=1) or SEQ274 (x=0) | GTDFSI NF SEQ1 | FSATGN T SEQ73 | YMLDK SEQ3 |
| C5^{V16h20} | EVQLVESGGGVVQPGGSLRLSCAASGTDFSINFIRWYRQAPGKGLEF VAGFTATGNTNYADSVKGRFTISRDNAKNAVYLQMNSLRPEDTAV | GTDFSI NF SEQ1 | FTATG NT SEQ2 | YILDK SEQ79 |
| | YYCYILDKWGQGTQVTVSS(AAAEQKLISEEDLNGAAHHHHHHGS)x , with x=1 or 0 SEQ154 (x=1) or SEQ275 (x=0) | | | |
| B8^{V1} | EVQLVESGGGVVQPGGSLRLSCAADGEIFSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ155 (x=1) or SEQ276 (x=0) | GEIFSIN F SEQ5 | FTRDGS T SEQ6 | YMLDT SEQ7 |
| B8^{V2} | EVQLVESGGGVVQPGGSLRLSCAASGEIFSINFMRWYGQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ156 (x=1) or SEQ277 (x=0) | GEIFSIN F SEQ5 | FTRDGS T SEQ6 | YMLDT SEQ7 |
| B8^{V3} | EVQLVESGGGVVQPGGSLRLSCAASGEIFSINFMRWYREAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ157 (x=1) or SEQ278 (x=0) | GEIFSIN F SEQ5 | FTRDGS T SEQ6 | YMLDT SEQ7 |
| B8^{V4} | EVQLVESGGGVVQPGGSLRLSCAASGEIFSINFMRWYRQAPIKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ158 (x=1) or SEQ279 (x=0) | GEIFSIN F SEQ5 | FTRDGS T SEQ6 | YMLDT SEQ7 |
| B8^{V5} | EVQLVESGGGVVQPGGSLRLSCAASGEIFSINFMRWYRQAPGKIRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ159 (x=1) or SEQ280 (x=0) | GEIFSIN F SEQ5 | FTRDGS T SEQ6 | YMLDT SEQ7 |
| B8^{V6} | EVQLVESGGGVVQPGGSLRLSCAASGEIFSINFMRWYRQAPGKQRE WVAGFTRDGFTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ161 (x=1) or SEQ281 (x=0) | GEIFSIN F SEQ5 | FTRDGF T SEQ160 | YMLDT SEQ7 |
| B8^{V7} | EVQLVESGGGVVQPGGSLRLSCAASGEIFSINFMRWYRQAPGKQRE WVAGFTRDGYTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ163 (x=1) or SEQ282 (x=0) | GEIFSIN F SEQ5 | FTRDG YT SEQ162 | YMLDT SEQ7 |
| B8^{V8} | EVQLVESGGGVVQPGGSLRLSCAASGEIFSINFMRWYRQAPGKQRE WVAGFTRDGSLNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ165 (x=1) or SEQ283 (x=0) | GEIFSIN F SEQ5 | FTRDGS L SEQ164 | YMLDT SEQ7 |
| B8^{V9} | EVQLVESGGGVVQPGGSLRLSCAASGEIFSINFMRWYRQAPGKQRE WVAGFTRDGSKNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ167 (x=1) or SEQ284 (x=0) | GEIFSIN F SEQ5 | FTRDGS K SEQ166 | YMLDT SEQ7 |
| B6^{V1} | EVQLVESGGGLVQPGGSLRLSCTASGGPIEQYPMGWFRRAPGKERN LVASISRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 SEQ168 (x=1) or SEQ285 (x=0) | GGPIEQ YP SEQ9 | ISRSGD GTY SEQ10 | GAGINP TKI SEQ11 |
| B6^{V2} | EVQLVESGGGLVQPGGSLRLSCTASGGPIEQYPMGWFRRAPGKERE LVASFSRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 | GGPIEQ YP SEQ9 | FSRSGD GTY SEQ169 | GAGINP TKI SEQ11 |
| | SEQ170 (x=1) or SEQ286 (x=0) | | | |
| B6^{V3} | EVQLVESGGGLVQPGGSLRLSCTASGGPIEQYPMGWFRRAPGKERE LVASSSRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 SEQ172 (x=1) or SEQ287 (x=0) | GGPIEQ YP SEQ9 | SSRSGD GTY SEQ171 | GAGINP TKI SEQ11 |
| B6^{V4} | EVQLVESGGGLVQPGGSLRLSCTASGGPIEQYPMGWFRRAPGKERE SVASISRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 SEQ173 (x=1) or SEQ288 (x=0) | GGPIEQ YP SEQ9 | ISRSGD GTY SEQ10 | GAGINP TKI SEQ11 |
| B6^{V5} | EVQLVESGGGLVQPGGSLRLSCTASGGPIEQYPMGWFRRAPGKERE LQASISRSGDGTYYAISSVKGRFTISRDNAENTVFLQMNSLKPDDTA VYYCGAGINPTKIWGQGTQVTVSS(AAAEQKLISEEDLNGAAHHHH HHGS)x, with x=1 or 0 SEQ174 (x=1) or SEQ289 (x=0) | GGPIEQ YP SEQ9 | ISRSGD GTY SEQ10 | GAGINP TKI SEQ11 |
| B7 | QVQLVQSGGGLVQAGGSLTLSCTASGRPLSDYPMGWFRRAPEKER ELVASISRTGDGTYYADGMRGRFTISRDNSENTVSLQMNNLKPEDT AVYYCGAGINPTKYWGQGTQVTVSS(AAAEQKLISEEDLNGAAHHH HHHGS)x, with x=1 or 0 SEQ178 (x=1) or SEQ290 (x=0) | GRPLSD YP SEQ175 | ISRTGD GT SEQ176 | GAGINP TKY SEQ177 |
| H7 | EVQLVESGGGLVQAGGSLRLSCTASGRPISDYPMGWFRRAPGKDRE LVASISRTGDGTYYANSMKERFTISRDNAQNTVSLQISSLKPEDTAV YYCGAGINPTRYWGQGTQVTVSS(AAAEQKLISEEDLNGAAHHHHH HGS)x, with x=1 or 0 SEQ181 (x=1) or SEQ291 (x=0) | GRPISD YP SEQ179 | ISRTGD GT SEQ176 | GAGINP TRY SEQ180 |
| C12b | QVQLVQSGGGLVQPGGSLRLSCAASGFTFSAYAMSWVRQAPGKGR ELVASISRTGDGTYYADGMKGRFTISRDNAENTVSLQINSLKPEDTA IYYCGAGINPTKYWGQGTQVTVSS(AAAEQKLISEEDLNGAAHHHH HHGS)x, with x=1 or 0 SEQ183 (x=1) or SEQ292 (x=0) | GFTFSA YA SEQ182 | ISRTGD GT SEQ176 | GAGINP TKY SEQ177 |
| E4 | EVQLVESGGGLVQAGDSLRLSCTASGLPLSNYPMGWFRRAPGKERE LIASISRTGDGTYYADSSVKGRFTISRDNAENTVSLQMNSLKPEDTA VYYCGAGINPTKYWGQGTQVTVSS(AAAEQKLISEEDLNGAAHHHH HHGS)x, with x=1 or 0 SEQ185 (x=1) or SEQ293 (x=0) | GLPLSN YP SEQ184 | ISRTGD GT SEQ176 | GAGINP TKY SEQ177 |
| E2 | QVQLVQSGGGLVQAGGSLRLSCAASGSIFSDITLAWHRQIPGKERE WVAGISRSGPSYRDSVKGRFTISRDNAKNTVFLQMNSLEPEDTGV YYCYSRGYWGQGTQVTVSS(AAAEQKLISEEDLNGAAHHHHHHGS) x, with x=1 or 0 SEQ189 (x=1) or SEQ294 (x=0) | GSIFSDI T SEQ186 | ISRSGS P SEQ187 | YSRGY SEQ188 |
| C3 | EVQLVQSGGGLVQAGGSLRLSCAASGRDFSDTSMAWHRQAPGKER LWVAGIRSGSTSTSYADSVKGRFTISRDDAKNTVYLQMDNLTPEDT AVYFCYSKGYWGQGTQVTVSS(AAAEQKLISEEDLNGAAHHHHHH GS)x, with x=1 or 0 SEQ193 (x=1) or SEQ295 (x=0) | GRDFS DTS SEQ190 | IRSGST ST SEQ191 | YSKGY SEQ192 |
| F2a | EVQLVESGGGLVQPGESLRLSCTASESISIYGVGWYRQAPGEQRVLV AHLKRNGLTEYKDSVKGRFTISRDNARNTIYLEMKNLKPEDTAIYY CNTALGSVLPWGRGTQVTVSS(AAAEQKLISEEDLNGAAHHHHHHG S)x, with x=1 or 0 SEQ197 (x=1) or SEQ296 (x=0) | ESISIYG SEQ194 | LKRNG LT SEQ195 | NTALG SVLP SEQ196 |
| E9 | EVQLVESGGGLVQPGGSLRLSCVSSATFGGYSMGWFRQAPGKEREF VAAITWNGGIIKDYADGVMGRFTISRNSALNTVYLQMDSLKSTDTG VYVCAGDRTYYAGGYVPTSEREYAFWGQGTQVTVSS(AAAEQKLIS EEDLNGAAHHHHHHGS)x, with x=1 or 0 SEQ691 (x=1) or SEQ297 (x=0) | ATFGG YS SEQ198 | ITWNG GIIK SEQ199 | AGDRT YYAGG YVPTSE REYAF SEQ200 |
| A9a | QVQLVQSGGGLVQAGGSLRLSCAASGSIFLINDMGWYRQAPGKQR ELVAVRGGSTKYGDSVKGRFTISRDDAKNTVYLQMNSLKPEDTAV YYCHRESGLGSSEYWGQGTQVTVSS(AAAEQKLISEEDLNGAAHHH HHHGS)x, with x=1 or 0 SEQ204 (x=1) or SEQ298 (x=0) | GSIFLIN D SEQ201 | RGGST SEQ202 | HRESGL GSSEY SEQ203 |
| B4a | EVQLVESGGGLVQAGGSLRLSCTASGRTVSNYRMGWFRQTPGKER EFVAAIAWAGGSLTYADSVKGRFTISRDNAKNTVYLQMNSLEPTDT AVYYCKGSRIFPTRDYWGKGTQVTVSS(AAAEQKLISEEDLNGAAH HHHHHGS)x, with x=1 or 0 SEQ208 (x=1) or SEQ299 (x=0) | GRTVS NYR SEQ205 | IAWAG GSL SEQ206 | KGSRIF PTRDY SEQ207 |
| D12 (control) | EVQLVESGGGLVQPGGSLRLSCAASGFTFGSYVMSWVRQAPGKGP EWVSTVSSGGGTTYYADSVKGRFTISRDNAQNTLYLQMNSLKPEDT AVYYCATGRTGLGEGDHRGQGTQVTSS(AAAEQKLISEEDLNGAAH HHHHHGS)x, with x=1 or 0 SEQ212 (x=1) or SEQ300 (x=0) | GFTFGS YV SEQ209 | VSSGG GTT SEQ210 | ATGRT GLGEG DH SEQ211 |
| B8^{V11} | EVQLVESGGGVVQPGGSLRLSCAASDEIFSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ411 (x=1) or SEQ412 (x=0) | DEIFSIN F SEQ410 | FTRDGS T SEQ6 | YMLDT SEQ7 |
| B8^{V12} | EVQLVESGGGVVQPGGSLRLSCAASGEIFYINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ414 (x=1) or SEQ415 (x=0) | GEIFYI NF SEQ413 | FTRDGS T SEQ6 | YMLDT SEQ7 |
| B8^{V14} | EVQLVESGGGVVQPGGSLRLSCAASGEIFSINFMRWYRQAPGKQRE WVAGFTRLGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ417 (x=1) or SEQ418 (x=0) | GEIFSIN F SEQ5 | FTRLGS T SEQ416 | YMLDT SEQ7 |
| B8^{V15} | EVQLVESGGGVVQPGGSLRLSCAASGEIFSINFMRWYRQAPGKQRE WVAGFTRDDSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ420 (x=1) or SEQ421 (x=0) | GEIFSIN F SEQ5 | FTRDDS T SEQ419 | YMLDT SEQ7 |
| B8^{V16} | EVQLVESGGGVVQPGGSLRLSCAASGEIFSINFMRWYRQAPGKQRE QVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ422 (x=1) or SEQ423 (x=0) | GEIFSIN F SEQ5 | FTRDGS T SEQ6 | YMLDT SEQ7 |
| B8^{V17} | EVQLVESGGGVVQPGGSLRLSCAASGEIFSINFMRWYRQAPGKQRE FVAGFTRDGSTNYPDSAKGRFTISRDNAKNTVYLQIDSLKPEDTAVY YCYMLDTWGQGTQVTVSS(AAAEQKLISEEDLNGAAHHHHHHGS)x, with x=1 or 0 SEQ424 (x=1) or SEQ425 (x=0) | GEIFSIN F SEQ5 | FTRDGS T SEQ6 | YMLDT SEQ7 |
| B8^{V18} | EVQLVESGGGVVQPGGSLRLSCAASGEIHSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ427 (x=1) or SEQ428 (x=0) | GEIHSI NF SEQ426 | FTRDGS T SEQ6 | YMLDT SEQ7 |
| B8^{V19} | EVQLVESGGGVVQPGGSLRLSCAASGEIFSINFMRWYRQAPGKQRH WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ429 (x=1) or SEQ430 (x=0) | GEIFSIN F SEQ5 | FTRDGS T SEQ6 | YMLDT SEQ7 |
| B8^{V20} | EVQLVESGGGVVQPGGSLRLSCAASGEIFSINFMRWYRQAPGKQRE WVAGFTRDGSHNYPDSAKGRFTISRDNAKNTVYLQIDSLKPEDTAV | GEIFSIN F | FTRDGS H | YMLDT SEQ7 |
| | YYCYMLDTWGQGTQVTVSS(AAAEQKLISEEDLNGAAHHHHHHGS )x, with x=1 or 0 SEQ432 (x=1) or SEQ433 (x=0) | SEQ5 | SEQ431 | |
| B8^{V21} | EVQLVESGGGVVQPGGSLRLSCAASGEIFSINNMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ435 (x=1) or SEQ436 (x=0) | GEIFSIN N SEQ434 | FTRDGS T SEQ6 | YMLDT SEQ7 |
| B8^{V22} | EVQLVESGGGVVQPGGSLRLSCAASGEIFSINQMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ438 (x=1) or SEQ439 (x=0) | GEIFSIN Q SEQ437 | FTRDGS T SEQ6 | YMLDT SEQ7 |
| B8^{V23} | EVQLVESGGGVVQPGGSLRLSCAASGEIFSINFMIWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ440 (x=1) or SEQ441 (x=0) | GEIFSIN F SEQ5 | FTRDGS T SEQ6 | YMLDT SEQ7 |
| B8^{V24} | EVQLVESGGGVVQPGGSLRLSCAASGEIFSINFMEWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ442 (x=1) or SEQ443(x=0) | GEIFSIN F SEQ5 | FTRDGS T SEQ6 | YMLDT SEQ7 |
| B8^{V25} | EVQLVESGGGVVQPGGSLRLSCAASGEIFSINFMRWYRQAPGKQRE WVAAFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ444 (x=1) or SEQ445 (x=0) | GEIFSIN F SEQ5 | FTRDGS T SEQ6 | YMLDT SEQ7 |
| B8^{V26} | EVQLVESGGGVVQPGGSLRLSCAASGEIFSINFMRWYRQAPGKQRE WVAKFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ446 (x=1) or SEQ447 (x=0) | GEIFSIN F SEQ5 | FTRDGS T SEQ6 | YMLDT SEQ7 |
| B8^{V27} | EVQLVESGGGVVQPGGSLRLSCAASGEIFSINFMRWYRQAPGKQRE WVAGFTRDGSTVYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ448 (x=1) or SEQ449 (x=0) | GEIFSIN F SEQ5 | FTRDGS T SEQ6 | YMLDT SEQ7 |
| B8^{V28} | EVQLVESGGGVVQPGGSLRLSCAASGEIFSINFMRWYRQAPGKQRE WVAGFTRDGSTFYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ450 (x=1) or SEQ451(x=0) | GEIFSIN F SEQ5 | FTRDGS T SEQ6 | YMLDT SEQ7 |
| B8^{V29} | EVQLVESGGGVVQPGGSLRLSCAASGEIFSINFMRWYRQAPGKQRE WVAGFTRDGSTNYPDSAKGRFTISRDNAKNTVYLQIDSLKPEDTAV YYCYMADTWGQGTQVTVSS(AAAEQKLISEEDLNGAAHHHHHHGS )x, with x=1 or 0 SEQ453 (x=1) or SEQ454(x=0) | GEIFSIN F SEQ5 | FTRDGS T SEQ6 | YMADT SEQ452 |
| B8^{V30} | EVQLVESGGGVVQPGGSLRLSCAASGEIFSINFMRWYRQAPGKQRE WVAGFTRDGSTNYPDSAKGRFTISRDNAKNTVYLQIDSLKPEDTAV YYCYMQDTWGQGTQVTVSS(AAAEQKLISEEDLNGAAHHHHHHGS )x, with x=1 or 0 SEQ456 (x=1) or SEQ457(x=0) | GEIFSIN F SEQ5 | FTRDGS T SEQ6 | YMQDT SEQ455 |
| B8^{V31} | EVQLVESGGGVVQPGGSLRLSCAASGEIKSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 | GEIKSI NF SEQ673 | FTRDGS T SEQ6 | YMLDT SEQ7 |
| | SEQ681 (x=1) or SEQ677 (x=0) | | | |
| B8^{V32} | EVQLVESGGGVVQPGGSLRLSCAASGEIRSINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ682 (x=1) or SEQ678 (x=0) | GEIRSI NF SEQ674 | FTRDGS T SEQ6 | YMLDT SEQ7 |
| B8^{V33} | EVQLVESGGGVVQPGGSLRLSCAASGEIFSINFMRWYRQAPGKQRE WVAGFTRDGSTNYPDSAKGRFTISRDNAKNTVYLQIDSLKPEDTAV YYCYMLKTWGQGTQVTVSS(AAAEQKLISEEDLNGAAHHHHHHGS )x, with x=1 or 0 SEQ693 (x=1) or SEQ702 (x=0) | GEIFSIN F SEQ5 | FTRDGS T SEQ6 | YMLKT SEQ714 |
| B8^{V34} | EVQLVESGGGVVQPGGSLRLSCAASGEIRSINFMRWYRQAPGKQRE WVAGFTRDGSTNYPDIAKGRFTISRDNAKNTVYLQIDSLKPEDTAV YYCYMLDTWGQGTQVTVSS(AAAEQKLISEEDLNGAAHHHHHHGS )x, with x=1 or 0 SEQ694 (x=1) or SEQ703 (x=0) | GEIRSI NF SEQ674 | FTRDGS T SEQ6 | YMLDT SEQ7 |
| B8^{V35} | EVQLVESGGGVVQPGGSLRLSCAASGEIRSINFMRWYRQAPGKQRE WVAGFTRDGSLNYPDSAKGRFTISRDNAKNTVYLQIDSLKPEDTAV YYCYMLDTWGKGTQVTVSS(AAAEQKLISEEDLNGAAHHHHHHGS )x, with x=1 or 0 SEQ695 (x=1) or SEQ704 (x=0) | GEIRSI NF SEQ674 | FTRDGS L SEQ164 | YMLDT SEQ7 |
| B8^{V36} | EVQLVESGGGVVQPGGSLRLSCAASGEIFSINVMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ696 (x=1) or SEQ705 (x=0) | GEIFSIN V SEQ710 | FTRDGS T SEQ6 | YMLDT SEQ7 |
| B8^{V37} | EVQLVESGGGVVQPGGSLRLSCAASGEIFSINFMRWYRQAPGKQRE WVAGFTRDGSTNYPDSAKGRFTISEDNAKNTVYLQIDSLKPEDTAV YYCYMLRTWGQGTQVTVSS(AAAEQKLISEEDLNGAAHHHHHHGS )x, with x=1 or 0 SEQ697 (x=1) or SEQ706 (x=0) | GEIFSIN F SEQ5 | FTRDGS T SEQ6 | YMLRT SEQ715 |
| B8^{V38} | EVQLVESGGGVVQPGGSLRLSCAASGEIRSINFMRWYRQAPGKQRE WVAGFTRTGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ698 (x=1) or SEQ707 (x=0) | GEIRSI NF SEQ674 | FTRTGS T SEQ712 | YMLDT SEQ7 |
| B8^{V39} | EVQLVESGGGVVQPGGSLRLSCAASGEIFKINFMRWYRQAPGKQRE WVAGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ699 (x=1) or SEQ708 (x=0) | GEIFKI NF SEQ711 | FTRDGS T SEQ6 | YMLDT SEQ7 |
| B8^{V40} | EVQLVESGGGVVQPGGSLRLSCAASGEIKSINFMRWYRQAPGKQRE WVSGFTRDGSTNYPDSAKGRFTISRDNAKNTVYLQIDSLKPEDTAV YYCYMLDTWGQGTQVTVSS(AAAEQKLISEEDLNGAAHHHHHHGS )x, with x=1 or 0 SEQ700 (x=1) or SEQ709 (x=0) | GEIKSI NF SEQ673 | FTRDGS T SEQ6 | YMLDT SEQ7 |
| C10a | EVQLVESGGGLVQAGDSLRLSCAASGFASHLYAIGWFRQAPGKERE KVSCISSNDGSTYYADSVKGRFTISRDTVKDMVYLQMNSLKPEDTA VYYCATDLTVHCFMAEYDWWGQGTQVTVSS(AAAEQKLISEEDLN GAAHHHHHHGS)x, with x=1 or 0 SEQ616 (x=1) or SEQ613 (x=0) | GFASHL YA SEQ607 | ISSNDG ST SEQ608 | ATDLT VHCFM AEYDW SEQ609 |
| H1 | EVQLVESGGGLVQPGGSLRLSCAASGFASHLYAIGWFRQAPGKERE KVSCISSNDGSTYYADSVKGRFTISRDTVKDMVYLQMNSLKPEDTA VYYCATDLTVHCFMAEYDWWGQGTQVTVSS(AAAEQKLISEEDLN GAAHHHHHHGS)x, with x=1 or 0 SEQ617 (x=1) or SEQ614 (x=0) | GFASHL YA SEQ607 | ISSNDG ST SEQ608 | ATDLT VHCFM AEYDW SEQ609 |
| E8 | VQLVESGGRLVQAGGSLRLSCTASGLAFSTHTMHWFRQAPGKEREF VGAIRWSDEDTEYADSVKGRFTISRDNAKSTVYLQMNNLKPEDTAV YYCAAARGVGTSGHMKYWGQGTQVTVSS(AAAEQKLISEEDLNGA AHHHHHHGS)x, with x=1 or 0 SEQ618 (x=1) or SEQ615 (x=0) | GLAFST HT SEQ610 | IRWSDE DT SEQ611 | AAARG VGTSG HMKY SEQ612 |

**Table 2: List of nucleotide sequences coding for the VHH molecules**

| **sdAb** | **SEQ ID** | **nucleotide sequences including or not an optional tag sequence of SEQ ID NO: 330 (in bold)** |
|---|---|---|
| A | 52 (x=1)or 301 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A1 | 53 (x=1)or 302 (x=0) | GAGGTGcAGCTGGTGGAGTCTGGGGGAGGCGTGGTGCAGCCTGGGGGGTCTCTAAAACTCTCCTG CGTAGCCTCGGGAGCGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A2 | 54 (x=1)or 303 (x=0) | GAGGTGcAGCTGGTGGAGTCTGGGGGAGGCGTGGTGCAGCCTGGGGGGTCTCTAAAACTCTCCTG CGTAGCCTCGGGAACGGCGTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A3 | 55 (x=1)or 304 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGGCGGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A4 | 56 (x=1)or 305 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTGCGACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x,with x=1 or 0 |
| A5 | 57 (x=1)or 306 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCGCGATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x,with x=1 or 0 |
| A6 | 58 (x=1)or 307 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC |
| | | AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATGCGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A7 | 59 (x=1)or 308 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGGCGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A8 | 60 (x=1)or 309 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGCGAAGTGGGGCCAGGGGACCCAGGTCACCG TCTCCTCAGCGGCCGCAGAACAAAAACTCATCTCAGAAGAGGATCTGAATGGGGCCGCACAT CACCACCATCACCATGGGAGCTAG)x, with x=1 or 0 |
| A9 | 61 (x=1)or 310 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACGCGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| B | 62 (x=1)or 311 (x=0) | GAgGTGCAGCTGGTGGAGTCTGGGGGagGCGTGGTGCAGCCTGGGGGGTCTCTGAGACTCTCCTGT GCAGCCTCTGGAGAGATCTTCAGTATCAATTTTATGCGCTGGTACCGCCAGGCTCCAGGGAAGCA GCGCGAGTGGGTCGCAGGTTTTACTAGGGATGGAAGCACAAACTATCCAGACTCCGCGAAGGGCC GATTCACCATCTCTAGAGACAACGCCAAGAACACGGTGTATCTGCAAATAGACAGCCTGAAACCT GAGGACACGGCCGTCTATTATTGTTATATGTTGGACACCTGGGGCCAGGGGACCCAGGTCACTGT CTCCTCA(GCGGCCGCAGAACAAAAACTCATCTCAGAAGAGGATCTGAATGGGGCCGCACAT CACCACCATCACCATGGGAGCTAG)x, with x=1 or 0 |
| C | 63 (x=1)or 312 (x=0) | GAGGTGCAGCTGGTGGAGTCTGGGGGAGGCTTGGTGCAGCCTGGGGGTTCTCTGAGACTCTCCTG TACAGCCTCTGGAGGCCCCATCGAGCAGTATCCCATGGGCTGGTTCCGCCGGGCCCCAGGAAAGG AGCGTGAATTGGTAGCAAGTATTAGCCGAAGTGGAGATGGCACATACTATGCAATCTCTTCCGTG AAGGGCCGATTCACCATCTCTAGAGACAACGCCGAGAACACGGTATTTCTGCAAATGAACAGCCT GAAACCTGACGACACGGCCGTTTATTACTGTGGGGCTGGTATAAACCCAACCAAGATCTGGGGCC AGGGGACCCAGGTCACCGTCTCCTCA(GCGGCCGCAGAACAAAAACTCATCTCAGAAGAGGAT CTGAATGGGGCCGCACATCACCACCATCACCATGGGAGCTAG)x, with x=1 or 0 |
| D | 64 (x=1)or 313 (x=0) | GAGGTGCAGCTGGTGGaGTCTGGGGGAggCGAGGTGCAGCCtgGgGGGTCTCTGAAACTCTCCTGTG TAGCCTCTGGAACCGACTTCAGTATCAATTTTGTGCGCTGGTACCGTCAGCGTCCAGGGAAGCAG CGCGAGTGGGTCGCAGGATTTACTGCGAATGGTGATACAAACTATCCAGACTCCATGAAGGGGCG ATTCACCATTTCCAGAGACAACGCCaAGAATACGGTGTATCTACAGATAAACAGCCTGAAATCTG AGGACACGGCCGTCTATTATTGCTATATGTTAgATAATTGGGGCCAGGGGACCCAGGTCACCGTCT CCTCA(GCGGCCGCAGAACAAAAACTCATCTCAGAAGAGGATCTGAATGGGGCCGCACATCA CCACCATCACCATGGGAGCTAG)x, with x=1 or 0 |
| A10 | 95 (x=1)or 314 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtctgAATTTTATACGCTGGTACCGCCAGGCTCCAGGGAAGCAGCGCGA GTTCGTCGCAGGATTTACTGCGACTGgTAACACAAACTATGCAGACTCCAtgAAGGGGCGATTCAC CATCTCCAGAGACAACACCAAGAACGCGGTGTATCTGCAAATAGACAGCCTGAAACCTGAGGAC ACGGCCGTGTATTACTGCTATATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| A11 | 96 (x=1)or 315 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATtacATACGCTGGTACCGCCAGGCTCCAGGGAAGCAGCGCGA GTTCGTCGCAGGATTTACTGCGACTGgTAACACAAACTATGCAGACTCCAtgAAGGGGCGATTCAC CATCTCCAGAGACAACACCAAGAACGCGGTGTATCTGCAAATAGACAGCCTGAAACCTGAGGAC ACGGCCGTGTATTACTGCTATATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| A12 | 97 (x=1)or 316 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGAATTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A13 | 98 (x=1)or 317 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTTCAGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A14 | 99 (x=1)or 318 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACTCAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A15 | 100 (x=1)or 319 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTTTATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| A16 | 101 (x=1)or 320 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATTTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| A17 | 102 (x=1)or 321 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGATTGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A18 | 103 (x=1)or 322 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGGTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A19 | 104 (x=1)or 323 (x=0) | GagGTGCAGCTGGTGGagtCTGGGGgaGgCGTGGTGCAGCctGGGGGGTCTCtAAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGAAAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| A20 | 105 (x=1)or 324 (x=0) | GAGGTcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGAGCTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A21 | 106 (x=1)or 325 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACGCTGGGTTCGCCAGGCTCCAGGGAAGC AGCGCGAGTTCGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A22 | 107 (x=1)or 326 (x=0) | GAGGTcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGCATTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A23 | 108 (x=1)or 327 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGAGCTGGGTTCGCCAGGCTCCAGGGAAGC AGCGCGAGTTCGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A24 | 109(x=1)or 328 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACGCTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| A25 | 110 (x=1)or 329 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACATTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| DNA sequences of C5neg (negative control), C5V20-V32, C5h8-h26, B8hl, C5V13h20, C5V16h20, B8V1-V9, V11, V12, V14-V40, B6V1-V4, B7, H7, C12b, E4, E2, C3, F2a, E9, A9a, B4a, C10a, Hl, E8 are listed in the enclosed Sequence listing ST26 as SEQ ID NO: 469 to 606, 646-663, 683-690 and 721-738. | | |

**Table 3: List of FR (Framework Regions) sequences of the VHH molecules**

| VHH ref. | Amino acid FR sequences | | | |
|---|---|---|---|---|
| | **FR1** | **FR2** | **FR3** | **FR4** |
| C5^{v20} to C5²⁹ | EVQLVESGGGVV QPGGSLKLSCVAS SEQ 36 | IRWYRQAPGKQREFVAGSEQ41 | NYADSMKGRFTISRDNTKNAVYLQI DSLKPEDTAVYYC SEQ46 | WGQGTQVT VSS SEQ50 |
| C5^{h7} | | IRWVRQAPGKLEWVAGSEQ342 | NYADSMKGRFTISRDNTKNALYLQI DSLRPEDTAVYYC SEQ366 | |
| C5^{h8} | EVQLVESGGGVV QPGGSLRLSCAAS SEQ37 | | NYADSVKGRFTISRDNTKNTLYLQI DSLRPEDTAVYYC SEQ367 | |
| C5^{h9} | | IRWYRQAPGKGLEFVAG SEQ343 | NYADSVKGRFTISRDNAKNTLYLQ MNSLRPEDTAVYYC SEQ368 | |
| C5^{h10} | EVQLVESGGGVV QPGGSLKLSCAAS SEQ331 | IRWVRQAPGKGLEWVAG SEQ342 | NYADSVKGRFTISRDNTKNTLYLQI NSLRPEDTAVYYC SEQ369 | |
| C5^{h11} | EVQLVESGGGVV QPGGSLRLSCAAS SEQ37 | | NYADSMKGRFTISRDNTKNTLYLQ MNSLRPEDTAVYYC SEQ370 | |
| C5^{h12} | | IRWYRQAPGKGLEFVAG SEQ343 | NYADSVKGRFTISRDNAKNTLYLQI DSLRPEDTAVYYC SEQ371 | |
| C5^{h13} | | | NYADSVKGRFTISRDNTKNTLYLQ MNSLRPEDTAVYYC SEQ372 | |
| C5^{h14} | EVQLVESGGGVV QPGGSLRLSCVAS SEQ400 | IRWVRQAPGKGLEWVAG SEQ342 | NYADSVKGRFTISRDNTKNALYLQ MNSLRPEDTAVYYC SEQ373 | |
| C5^{h15} | | | NYADSVKGRFTISRDNTKNTLYLQ MDSLRPEDTAVYYC SEQ374 | |
| C5^{h16} |  SEQ332 | IRWYRQAPGKGLEFVAG SEQ343 | NYADSVKGRFTISRDNSKNTLYLQ MNSLRAEDTAVYYC SEQ375 | WGQGTLVT VSS SEQ39 |
| C5^{h17} | EVQLVESGGGVV QPGGSLKLSCVAS SEQ36 | FRWVRQAPGKGLEFVAG SEQ344 | NYADSMKGRFTISRDNTKNALYLQI DSLRPEDTAVYYC SEQ366 | WGQGTQVT VSS SEQ50 |
| C5^{h18} | EVQLVESGGGVV QPGGSLKLSCVAS SEQ36 | IRWYRQAPGKGLEFVAG SEQ343 | NYADSVKGRFTISRDNAKNTLYLQ MNSLRPEDTAVYYC SEQ368 | |
| C5^{h19} | EVQLVESGGGVV QPGGSLRLSCAAS SEQ37 | IRWYRQAPGKQREFVAG SEQ41 | | |
| C5^{h20} | | IRWYRQAPGKGLEFVAG SEQ343 | NYADSVKGRFTISRDNAKNAVYLQ MNSLRPEDTAVYYC SEQ376 | |
| C5^{h21} | | | NYADSVKGRFTISRDNAKNTLYLQ MNSLKPEDTAVYYC SEQ377 | |
| C5^{h22} | | IRWYRQAPGKGREFVAG SEQ345 | NYADSVKGRFTISRDNAKNTLYLQ MNSLRPEDTAVYYC SEQ368 | |
| C5^{h23} | | FRWVRQAPGKQREFVAG SEQ346 | | |
| C5^{h24} | | IRWYRQAPGKGLEFVAG SEQ343 | NYADSMKGRFTISRDNAKNTLYLQ MNSLRPEDTAVYYC SEQ378 | |
| C5^{h25} | | FRW**V**RQAPGK**GL**EFVAG SEQ344 | NYADSVKGRFTISRDNAKNTLYLQI DSLRPEDTAVYYC SEQ371 | |
| C5^{h26} | | IRWYRQAPGK**GL**E**W**VAG SEQ347 | | |
| C5^{v32} | SEQ36 | IRWY**S**QAPGKQREFVAG SEQ716 | NYADSMKGRLTISRDNTKNAVYLQ IDSLKPEDTAVYYC SEQ717 | |
| C5^{neg} | EVQLVESGGGVV QPGGSLKLSCVAS SEQ36 | IRWYRQAPGKQREFVAG SEQ41 | NYADSMKGRFTISRDNTKNAVYLQI DSLKPEDTAVYYC SEQ46 | |
| B8^{h1} | EVQL VESGGGVV QPGGSLRLSCAAS SEQ37 | MRWYRQAPGK**GL**EWVAG SEQ348 | NYPDSVKGRFTISRDNAKNTVYLQ MNSLRPEDTAVYYC SEQ379 | |
| C5^{V13} h20 | | IRWYRQAPGK**GL**EFVAG SEQ343 | NYADSVKGRFTISRDNAKNAVYLQ MNSLRPEDTAVYYC SEQ376 | |
| C5^{V16} h20 | | | | |
| B8^{V1} | EVQLVESGGGVV QPGGSLRLSCAAD SEQ333 | MRWYRQAPGKQREWVAG SEQ42 | NYPDSAKGRFTISRDNAKNTVYLQI DSLKPEDTAVYYC SEQ47 | |
| B8^{V2} | EVQLVESGGGVV QPGGSLRLSCAAS SEQ37 | MRWY**G**QAPGKQREWVAG SEQ349 | | |
| B8^{V3} | | MRWYR**E**APGKQREWVAG SEQ350 | | |
| B8^{V4} | | MRWYRQAP**I**KQREW VAG SEQ351 | | |
| B8^{V5} | | MRWYRQAPGK**I**REWVAG SEQ352 | | |
| B8^{V6-V9} | | MRWYRQAPGKQREWVAG SEQ42 | | |
| B6^{V1} | EVQLVESGGGLV QPGGSLRLSCTAS SEQ38 | MGWFRRAPGKER**N**LVAS SEQ353 | YAISSVKGRFTISRDNAENTVFLQM NSLKPDDTAVYYC SEQ48 | |
| B6^{V2-V3} | | MGWFRRAPGKEREL VAS SEQ42 | | |
| B6^{V4} | | MGWFRRAPGKERE**S**VAS SEQ354 | | |
| B6^{V5} | | MGWFRRAPGKEREL**Q**AS SEQ355 | | |
| B7 | QVQLVQSGGGLV QAGGSLTLSCTAS SEQ334 | MGWFRRAPEKEREL VAS SEQ356 | YYADGMRGRFTISRDNSENTVSLQ MNNLKPEDTAVYYC SEQ380 | |
| H7 | EVQLVESGGGLV QAGGSLRLSCTAS SEQ335 | MGWFRRAPGKDREL VAS SEQ357 | YYANSMKERFTISRDNAQNTVSLQI SSLKPEDTAVYYC SEQ381 | |
| C12b | QVQLVQSGGGLV QPGGSLRLSCAAS SEQ336 | MSWVRQAPGKGRELVAS SEQ358 | YYADGMKGRFTISRDNAENTVSLQI NSLKPEDTAIYYC SEQ382 | |
| E4 | EVQLVESGGGLV QAGDSLRLSCTAS SEQ337 | MGWFRRAPGKERELIAS SEQ359 | YYADSSVKGRFTISRDNAENTVSLQ MNSLKPEDTAVYYC SEQ383 | |
| E2 | QVQLVQSGGGLV QAGGSLRLSCAAS SEQ338 | LAWHRQIPGKEREWVAG SEQ360 | SYRDSVKGRFTISRDNAKNTVFLQM NSLEPEDTGVYYC SEQ384 | |
| C3 | EVQLVQSGGGLV QAGGSLRLSCAAS SEQ339 | MAWHRQAPGKERLWVAG SEQ361 | SYADSVKGRFTISRDDAKNTVYLQ MDNLTPEDTAVYFC SEQ385 | |
| F2a | EVQLVESGGGLV QPGESLRLSCTAS SEQ340 | VGWYRQAPGEQRVLVAH SEQ362 | EYKDSVKGRFTISRDNARNTIYLEM KNLKPEDTAIYYC SEQ386 | WGRGTQVT VSS SEQ670 |
| E9 | EVQLVESGGGLV QPGGSLRLSCVSS SEQ341 | MGWFRQAPGKEREFVAA SEQ363 | DYADGVMGRFTISRNSALNTVYLQ MDSLKSTDTGVYYC SEQ387 | |
| A9a | QVQLVQSGGGLV QAGGSLRLSCAAS SEQ338 | MGWYRQAPGKQRELVAV SEQ364 | KYGDSVKGRFTISRDDAKNTVYLQ MNSLKPEDTAVYYC SEQ388 | |
| B4a | EVQLVESGGGLV QAGGSLRLSCTAS SEQ335 | MGWFRQTPGKEREFVAA SEQ365 | TYADSVKGRFTISRDNAKNTVYLQ MNSLEPTDTAVYYC SEQ389 | WGKGTQVT VSS SEQ391 |
| B8^{V16} | SEQ37 | MRWYRQAPGKQREQVAG SEQ458 | SEQ47 | SEQ50 |
| B8^{V17} | | MRWYRQAPGKQREFVAG SEQ459 | | |
| B8^{V19} | | MRWYRQAPGKQRHWVAG SEQ460 | | |
| B8^{V23} | | M**I**WYRQAPGKQREWVAG SEQ461 | | |
| B8^{V24} | | MEWYRQAPGKQREWVAG SEQ462 | | |
| B8^{V25} | | MRWYRQAPGKQREWVAA SEQ463 | | |
| B8^{V26} | | MRWYRQAPGKQREWVAK SEQ464 | | |
| B8^{V27} | | SEQ42 | VYPDSAKGRFTISRDNAKNTVYLQI DSLKPEDTAVYYC SEQ625 | |
| B8^{V28} | | | FYPDSAKGRFTISRDNAKNTVYLQI DSLKPEDTAVYYC SEQ626 | |
| B8^{V34} | SEQ37 | SEQ42 | NYPDIAKGRFTISRDNAKNTVYLQI DSLKPEDTAVYYC SEQ718 | |
| B8^{V37} | | | NYPDSAKGRFTISEDNAKNTVYLQI DSLKPEDTAVYYC SEQ719 | |
| B8^{V40} | | MRWYRQAPGKQREWVSG SEQ720 | SEQ47 | |
| C10a | EVQLVESGGGLV QAGDSLRLSCAAS SEQ619 | IGWFRQAPGKEREKVSC SEQ621 | YYADSVKGRFTISRDTVKDMVYLQ MNSLKPEDTAVYYC SEQ623 | |
| H1 |  SEQ332 | IGWFRQAPGKEREKVSC SEQ621 | YYADSVKGRFTISRDTVKDMVYLQ MNSLKPEDTAVYYC SEQ623 | |
| E8 | VQLVESGGRLVQ AGGSLRLSCTAS SEQ620 | MHWFRQAPGKEREFVGA SEQ622 | EYADSVKGRFTISRDNAKSTVYLQ MNNLKPEDTAVYYC SEQ624 | |

**Table 4: Examples of tag sequences and linkers**

| SEQ ID NO | nucleotide or amino acid tag sequence |
|---|---|
| 51 | AAAEQKLISEEDLNGAAHHHHHHGS |
| 330 | GCGGCCGCAGAACAAAAACTCATCTCAGAAGAGGATCTGAATGGGGCCGCACATCACCACCATCACC ATGGGAGCTAG |
| 394 | EQKLISEEDL |
| 395 | HHHHHH |
| 396 | HHHHHHHH |
| 397 | YPYDVPDYA |
| 398 | DYKDDDDK |
| 399 | GGGGSCHHHHHH |
| 111 | GGGLQR |
| 401 | GGGGLQR |
| 402 | GGGGGLQR |
| 403 | GGGGGGLQR |
| 404 | GGGGGGGLQR |
| 112 | AAAHHHHHHGGGLQR |
| 406 | AAAHHHHHHGGGGLQR |
| 407 | AAAHHHHHHGGGGGLQR |
| 408 | AAAHHHHHHGGGGGGLQR |
| 409 | AAAHHHHHHGGGGGGGLQR |
| 627 | GlySerGlySer or GSGS |
| 628 | SerGlySerGly5 or SGSGGGGG |
| 629 | Gly4Ser or GGGGS |
| 630 | (Gly4Ser)2 or GGGGSGGGGS |
| 631 | (Gly4Ser)3 or GGGGSGGGGSGGGGS |
| 632 | (Gly4Ser)4 or GGGGSGGGGSGGGGSGGGGS |
| 633 | (Gly4Ser)5 or GGGGSGGGGSGGGGSGGGGSGGGGS |
| 634 | (Gly4Ser)6 or GGGGSGGGGSGGGGSGGGGSGGGGSGGGGS |
| 635 | GlyGly(Gly4Ser)3 or GGGGGGSGGGGSGGGGS |
| 636 | EAAAK |
| 637 | A(EAAAK)2A or AEAAAKEAAAKA |
| 638 | A(EAAAK)3A or AEAAAKEAAAKEAAAKA |
| 639 | GG(EAAAK)2EA or GGEAAAKEAAAKEA |
| 640 | GG(EAAAK)4EA or GGEAAAKEAAAKEAAAKEAAAKEA |
| 641 | GGA(EAAAK)4ALEA(EAAAK)4A or GGAEAAAKEAAAKEAAAKEAAAKALEAEAAAKEAAAKEAAAKEAAAKA |
| 642 | GG(AP)17 or GGAPAPAPAPAPAPAPAPAPAPAPAPAPAPAPAPAP |
| 643 | ASTKGPSVFPLAP |
| 644 | GSAGSAAGSGEF |
| 645 | KESGSVSSEQLAQFRSLD |

### REFERENCES

Alvarez E, et al. Biochem J. 1990 Apr 1;267(1):31-5.
Alvarez-Rueda Net al, Mol Immunol. 2007 Mar;44(7):1680-90.
Barrientos T. et al. (2015). EBioMedicine 2(11): 1705-1717.
Behar G et al. FEBS J. 2009 Jul;276(14):3881-93.
Bustin et al., Clin Chem. 2009 Apr;55(4):611-22.
Candelaria PV, et al . Front Immunol. 2021 Mar 17;12:607692.
Chan and Gerhardt, J Biol Chem 1992 Apr 25;267(12):8254-9.
Chang J, et al. Int J Pharm. 2009 Sep 11;379(2):285-92.
Das Gupta A, Shah VI. Hematol Pathol. 1990;4(1):37-41.
De Boer et al., 2007, Clin. Pharmacokinet., 46(7), 553-576.
Debacker, A. J., et al. (2020). Mol Ther 28(8): 1759-1771.
Fang F, et al Mater Sci Eng C Mater Biol Appl. 2017 Jul 1;76: 1316-1327.
Fishman JB, et al. J Neurosci Res. 1987;18(2):299-304.
Gatter KC, et al. J Clin Pathol. 1983 May;36(5):539-45.
Habeshaw JA, et al. Lancet. 1983 Mar 5;1(8323):498-501.
Hayes GR, et al. Biochemistry. 1997 Apr 29;36(17):5276-84.
Jain A et al. J Microencapsul. 2011;28(1):21-8.
Jain, 2008, Jain PharmaBiotech Report, Drug Delivery in CNS disorders
Jefferies et al., Nature 1984 Nov 8-14;312(5990):162-3.
Jing SQ, Trowbridge IS. J Biol Chem. 1990 Jul 15;265(20):11555-9.
Jing SQ, Trowbridge IS. EMBO J. 1987 Feb;6(2):327-31.
Johnsen K.B. et al, Prog Neurobiol. 2019 Oct;181: 101665.
Johnson et al., J. Clin. Oncol., 2009; 21(7): 1404-1411
Jones AR, Shusta EV. Pharm Res. 2007 Sep;24(9):1759-71.
Levin, 1980, J. Med. Chem., 23, 682-684
Majumdar S. and Siahaan TJ., Med Res Rev. 2012 May; 32(3):637-58.
McClelland A et al ; Cell. 1984 Dec;39(2 Pt 1):267-74.
Molino et al., 2014, J. Vis. Exp. 88, e51278
Moos T, Morgan EH. Cell Mol Neurobiol. 2000 Feb;20(1):77-95.
Nair AB, et al. Molecules. 2019 Dec 13;24(24):4566.
Nair JK et al., , J. Am. Chem. Soc. 136 (2014) 16958-16961.
Neckers LM, Trepel JB. Cancer Invest. 1986;4(5):461-70.
Needleman, S.B. and Wunsch, C.D., (1970), Journal of Molecular Biology, 48, 443-453.
Neuwelt et al., 2008, Lancet Neurol., 7, 84-96
Omary MB, Trowbridge IS. J Biol Chem. 1981 May 25;256(10):4715-8.
Pardridge WM et al. Metabolism1987 Sep;36(9):892-5.
Pardridge WM, et al. West J Med. 1992 Mar;156(3):281-6.
Pardridge WM. Expert Opin Ther Targets. 2015;19(8):1059-72.
Pardridge WM. Mol Interv 2003 Mar;3(2):90-105, 51.
Pardridge WM et al. , J Pharmacol Exp Ther. 1991 Oct;259(1):66-70.
Prior R et al. Virchows Arch A Pathol Anat Histopathol. 1990;416(6):491-6.
Rothenberger S et al. Cell. 1987 May 8;49(3):423-31.
Sambrook J, Russell D (2001) Molecular cloning: a laboratory manual, Third Edition Cold Spring Harbor.
Schackert et al., 1989, Selective Cancer Ther., 5, 73-79
Tai, W. Molecules. 2019 Jun 13;24(12).
Tripathi et al. Mol Cell. 2010 Sep 24;39(6):925-38.
Tuma P, Hubbard AL et al. Physiol Rev. 2003 Jul;83(3):871-932.
Ulbrich et al . Eur J Pharm Biopharm. 2009 Feb;71(2):251-6.
Xiao G, Gan LS. Int J Cell Biol. 2013;2013:703545.
Xu W., et al. (2015). "Lethal Cardiomyopathy in Mice Lacking Transferrin Receptor in the Heart." Cell Rep 13(3): 533-545.
Yan F, et al. J Mater Sci Mater Med. 2013 Oct;24(10):2371-9
Yang DC et al. Anticancer Res. 2001 Jan-Feb;21(1B):541-9.
Zhang Y, Pardridge WM. J Pharmacol Exp Ther. 2005 Jun;313(3): 1075-81.
Zhou et al., 1992, J. Control. Release, 19, 459-486.
Zhou QH et al. Mol Pharm. 2010 Dec 6;7(6):2148-55.

## Claims

1. A conjugate compound comprising:
(i) one or more VHH molecule(s) of formula FR1-CDR1-FR2-CDR2-FR3-CDR3-FR4, and
(ii) at least one therapeutic compound or a vehicle comprising such a therapeutic compound,
wherein said VHH molecule binds TfR at the surface of a cell of the central nervous system (CNS), and wherein said VHH molecule comprises:
- a CDR1 comprising an amino acid sequence selected from SEQ ID NOs: 1, 5, 9, 17, 125, 175, 179, 182, 184, 186, 190, 194, 198, 201, 205, 392, 410, 413, 426, 434, 437, 607, 610, 671-674, 710 and 711, and
- a CDR2 comprising an amino acid sequence selected from SEQ ID Nos: 2, 6, 73, 113, 115, 128, 160, 162, 164, 166, 169, 171, 176, 187, 191, 195, 199, 202, 206, 416, 419, 431, 608, 611 and 712, and
- a CDR3 comprising an amino acid sequence selected from SEQ ID NOs: 3, 7, 11, 117, 119, 121, 123, 177, 180, 188, 192, 196, 200, 203, 207, 452, 455, 609, 612 and 713-715,
with the proviso that:
. when the CDR1 comprises SEQ ID NOs: 1, 5, 9 or 17, then the CDR2 is not selected from SEQ ID NOs: 2, 6 and 73, and the CDR3 is not selected from SEQ ID NOs: 3, 7 and 11, simultaneously;
. when the CDR2 comprises SEQ ID NOs: 2, 6, or 73, then the CDR1 is not selected from SEQ ID NOs: 1, 5, 9 and 17, and the CDR3 is not selected from SEQ ID NOs: 3, 7 and 11, simultaneously;
. when the CDR3 comprises SEQ ID NOs: 3, 7 or 11, then the CDR1 is not selected from SEQ ID NOs: 1, 5, 9 and 17, and the CDR2 is not selected from SEQ ID NOs: 2, 6 and 73, simultaneously.

2. The conjugate compound of claim 1, wherein said VHH molecule comprises: SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7.

3. The conjugate compound of claim 1 or 2, wherein said VHH molecule comprises an amino acid sequence selected from any one of SEQ ID NOs: 273, 276-284, 412, 415, 418, 421, 423, 425, 428, 430, 433, 436, 439, 441, 443, 445, 447, 449, 451, 454, 457, 677, 678 and 702-709.

4. The conjugate compound of claim 1 or 2, wherein said VHH molecule comprises an amino acid sequence selected from any one of SEQ ID NOs: 242-271, 274, 275, 675, 676 and 701.

5. The conjugate compound of claim 1 or 2, wherein said VHH molecule comprises an amino acid sequence selected from any one of SEQ ID NOs: 285-299.

6. The conjugate compound of claim 1 or 2, wherein said VHH molecule comprises an amino acid sequence selected from any one of SEQ ID NOs: 613-615.

7. The conjugate compound of any one of the preceding claims, wherein said VHH molecule further comprises a tag and/or a linker.

8. The conjugate compound of any one of the preceding claims, wherein said VHH molecule is humanized, preferably selected from SEQ ID NO: 130-149, 152-154, 252-271 and 273-275.

9. The conjugate compound of any one of the preceding claims, wherein said VHH molecule binds the human, non-human primate and/or rodent TfR1.

10. The conjugate compound of any one of the preceding claims, wherein said VHH molecule binds to TfR with an affinity (Kd) of 0.1 nM to 2500 nM.

11. The conjugate compound of any one of the preceding claims, wherein the CNS cell is a brain or spinal cord cell.

12. The conjugate compound of any one of the preceding claims, wherein the therapeutic compound is selected from a peptide, a polypeptide, a protein, an antibody and a nucleic acid, the nucleic acid being preferably selected from a mRNA, a ribozyme or an oligonucleotide selected from any single- or doublestranded oligonucleotide such as small interfering RNA (siRNA), small activating RNAs (saRNA), gapmer, antisense oligonucleotide (ASO), shRNA, miRNA, aptamer RNA and bridged nucleic acid (BNA).

13. The conjugate compound of any one of the preceding claims, further comprising at least one additional compound, preferably a half-life extending moiety or stabilizing group or scaffold such as an antibody or a fragment thereof (such as a Fc fragment), a VHH molecule, PEG, a serum albumin protein, or a serum albumin moiety, more preferably a Fc fragment, wherein the Fc fragment is even more preferably a Fc heterodimer comprising a modified Fc having a sequence of SEQ ID NO: 664 on the knob arm, and a modified Fc having a sequence of SEQ ID NO: 665 on the hole arm.

14. A pharmaceutical composition comprising the conjugate compound of claim 1, and a pharmaceutically acceptable support, carrier or excipient.

15. The conjugate compound of any one of claims 1 to 13, or the composition according to claim 14, for use in the treatment of a CNS disease preferably selected from the group consisting of Alzheimer's disease, Parkinson's disease, stroke, Creutzfeldt-Jakob disease, bovine spongiform encephalopathy, multiple sclerosis, amyotrophic lateral sclerosis, epilepsy, migraine, encephalitis, CNS pain and glioblastoma.

16. The conjugate compound of any one of claims 1 to 13, or the composition according to claim 14, or the conjugate compound or composition for use according to claim 15, wherein the conjugate compound or composition is administered systemically, intravenously, intramuscularly, subcutaneously, intracerebrally, intracerebroventricularly or intrathecally.
